(19) Europäisches Patentamt / European Patent Office / Office européen des brevets

(11) EP 4 268 856 A1

(12) EUROPEAN PATENT APPLICATION
published in accordance with Art. 153(4) EPC

(43) Date of publication:
01.11.2023 Bulletin 2023/44

(21) Application number: 21915278.2

(22) Date of filing: 27.12.2021

(51) International Patent Classification (IPC):
A61L 27/44 (2006.01) A61K 35/12 (2015.01)
A61L 27/20 (2006.01) A61L 27/34 (2006.01)
A61L 27/38 (2006.01) A61L 27/50 (2006.01)
A61L 27/58 (2006.01)

(52) Cooperative Patent Classification (CPC):
A61K 35/12; A61L 27/20; A61L 27/34; A61L 27/38; A61L 27/44; A61L 27/50; A61L 27/58

(86) International application number:
PCT/JP2021/048566

(87) International publication number:
WO 2022/145419 (07.07.2022 Gazette 2022/27)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 28.12.2020 JP 2020219024

(71) Applicant: Mochida Pharmaceutical Co., Ltd.
Tokyo 160-8515 (JP)

(72) Inventors:
- FURUSAKO Shoji
Tokyo 160-8515 (JP)
- TSUDA Naoto
Tokyo 160-8515 (JP)

(74) Representative: Hoffmann Eitle
Patent- und Rechtsanwälte PartmbB
Arabellastraße 30
81925 München (DE)

# (54) MULTILAYER STRUCTURE USING CHEMICALLY CROSSLINKED ALGINIC ACID

(57) There has been required a novel and practicable multilayer structure that has enhanced structure stability and, therefore, is improved in the enlargement of device size, easiness in processing, etc. A structure according to the present invention that comprises a core layer comprising a pharmacological ingredient embedded in a chemically crosslinked alginic acid, a cationic polymer layer coating the core layer, and an anionic polymer layer coating the cationic polymer layer.

Fig. 1

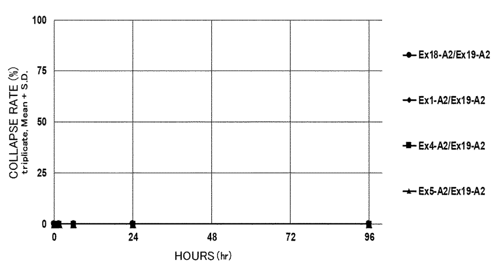


EP 4 268 856 A1

## Description

[Technical Field]

The present invention relates to a multilayer structure using chemically crosslinked alginic acid. More specifically, the present invention relates to a structure comprising a core layer comprising a chemically crosslinked alginic acid in which a pharmacological ingredient such as a cell is embedded, a cationic polymer layer coating the core layer, and an anionic polymer layer, such as alginic acid or a chemically crosslinked alginic acid, coating the cationic polymer layer, a method for manufacturing the same, and the like.

[Background Art]

In recent years, techniques for transplanting cells or the like encapsulated in polymer beads or polymer capsules into bodies have been studied. For example, as cell delivery devices that are used for transplantation, microcapsules in which alginic acid is used are known. Alginic acid instantly gelates in the presence of divalent ions such as calcium ions or barium ions and is thus used to encapsulate cells in gel. In addition, three-layer (alginic acid/PLL or PLO/alginic acid) alginate capsules in which alginic acid is coated with poly-L-lysine (PLL) or poly-L-ornithine (PLO), which is a cationic polymer, and this is further coated with alginic acid have been reported (for example, Non Patent Literature 1 and Patent Literature 1).

Non Patent Literature 2 describes that, in a three-layer alginate capsule, when alginic acid in the outermost layer is degraded in vivo, and PLL is exposed, an immune reaction can be induced. In addition, Non Patent Literature 2 discloses experimental results of comparing the physical properties of an alginate capsule coated with PLO instead of PLL with those in the case of using PLL. According to the document, the alginate capsule coated with PLO is considered to have a higher physical strength than and superior permselectivity to the alginate capsule coated with PLL. Non Patent Literature 3 describes that a PLO-coated alginate capsule has a lower degree of fibrosis than a PLL-coated alginate capsule.

Under such circumstances, a variety of alginate capsules having a three-layer structure for which a material on which structural modification has been performed more chemically is used have been developed (for example, Patent Literatures 2 to 6).

As chemically crosslinked alginic acid, for example, alginic acids for which a Huisgen reaction is used (Patent Literature 7 and Patent Literature 9, which is a basis of the priority of the present application and published after filing) and alginic acid for which a photocrosslinking reaction is used have been reported (Patent Literature 8).

[Citation List]

[Patent Literature]

[Patent Literature 1] WO 2001/052871
[Patent Literature 2] WO 2014/171842
[Patent Literature 3] WO 2018/151186
[Patent Literature 4] WO 98/049202
[Patent Literature 5] CN 105078923 A
[Patent Literature 6] WO 2010/139061
[Patent Literature 7] WO 2019/240219
[Patent Literature 8] WO 2019/168058
[Patent Literature 9] WO 2021/125255


[Non Patent Literature]

[Non Patent Literature 1] Yakugaku Zasshi, Vol. 125, No. 8, pp. 601 to 615, 2005
[Non Patent Literature 2] Biomaterials, Vol. 26, No. 34, 6846 to 52, 2005
[Non Patent Literature 3] American Diabetes Association 60th Scientific Sessions, 2000, Abstract, No. 448-P

[Summary of Invention]

[Technical Problem]

For conventional multilayer structures in which alginic acid is used for a core layer, applications thereof have been limited to application to microcapsules and the like due to their structural stability. There has been a demand for a novel practical multilayer structure in which device enlargement, easy processing or the like has been improved by improvement in the stability of the structure.

[Solution to Problem]

As a result of repeating intensive research, the present inventors found the following (1) to (7) and completed the present invention based on these findings.

(1) A structure in which a core layer produced using a chemically crosslinked alginic acid is coated with a cationic polymer layer and further coated with an anionic polymer layer such as alginic acid or a chemically crosslinked alginic acid (hereinafter, abbreviated as the structure of the present invention) exhibits a high physical strength even under conditions where there is no intermolecular crosslinking of alginic acid by a divalent metal ion compared with a similar structure in which alginic acid is used as a core layer (hereinafter, abbreviated as a conventional structure).
(2) In the case of being produced in, for example, a bead shape, the structure of the present invention can be produced to have a larger size than the conventional structure.
(3) Since the structure of the present invention has high stability, it is also possible to produce large structures with a free shape, such as a flat plate or a fiber, and these structures are capable of maintaining structural stability for a long period of time.
(4) The structure of the present invention comprising cells encapsulated in the core layer can be actually produced, and the cells are capable of surviving for a long period of time in the structure of the present invention.
(5) Therefore, the structure of the present invention can be used in a wide range of applications, such as storing or culturing of cells or the like in the stable core layer.
(6) In a case where pancreatic β-cell-derived cells are encapsulated in the core layer, the structure can also be used for the transplantation of pancreatic islets or device cells for cell transplantation since insulin has been secreted for a long period of time while the structure is maintained.
(7) The structure of the present invention is suitable for in vivo use since a chemically modified alginic acid or alginic acid is used as the layer coating the cationic polymer and can be applied not only to cell transplantation but also to a variety of in vivo uses since the structure has the structural properties described in the (1) to (6).

The chemically crosslinked alginic acid that is obtained by forming a crosslink between a chemically modified alginic acid derivative represented by Formula (HB-I) and a chemically modified alginic acid derivative represented by Formula (HB-II), which will be described below, using both alginic acid derivatives has not thus far been known.

Exemplary embodiments of the present invention are as described in the following [1] to [13-9].

[1] A structure comprising a core layer comprising a pharmacological ingredient embedded in a chemically crosslinked alginic acid, a cationic polymer layer coating the core layer, and an anionic polymer layer coating the cationic polymer layer.

[2] The structure described in [1], in which the chemically crosslinked alginic acid is obtained by forming a crosslink between a chemically modified alginic acid derivative represented by Formula (H-I) and a chemically modified alginic acid derivative represented by Formula (H-II) using both alginic acid derivatives:

[chemically modified alginic acid derivative represented by Formula (H-I)]
a chemically modified alginic acid represented by Formula (H-I) below:

[C1]

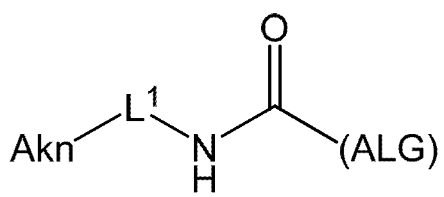

(H-I)

[in Formula (H-I), (ALG) represents alginic acid; -NHCO- represents an amide bond through an arbitrary carboxyl group of the alginic acid; $-L^1-$ is a divalent linker that bonds to a cyclic alkyne group (Akn)];
[chemically modified alginic acid derivative represented by Formula (H-II)]
a chemically modified alginic acid represented by Formula (H-II) below:

[C2]

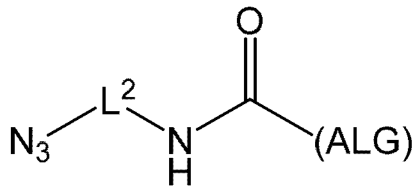

(H-II)

[in Formula (H-II), (ALG) represents alginic acid; -NHCO- represents an amide bond through an arbitrary carboxyl group of the alginic acid; $-L^2-$ is a divalent linker that bonds to an azide group].

[2-1] The structure described in [1] or [2], in which the chemically crosslinked alginic acid is obtained by forming a crosslink between a chemically modified alginic acid derivative represented by Formula (H-I) and a chemically modified alginic acid derivative represented by Formula (H-II) using both alginic acid derivatives:
[chemically modified alginic acid derivative represented by Formula (H-I)]
a chemically modified alginic acid derivative represented by Formula (H-I) below:

[C3]

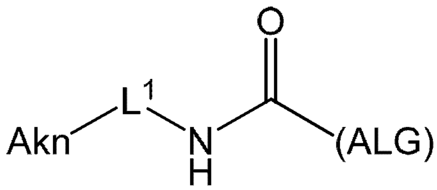

(H-I)

[in Formula (H-I), (ALG) represents alginic acid; -NHCO- represents an amide bond through an arbitrary carboxyl group of the alginic acid; $-L^1-$ represents a linker selected from the group consisting of partial structural formulae [in each formula, the outsides of the broken lines at both ends are not included] shown in the following table:

[Table 1-1]

| No. | $-L^1-$ | |
|---|---|---|
| (LN-1) | 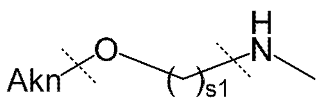 | s1=2-6 |
| (LN-2) | 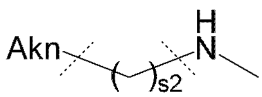 | s2=2-6 |
| (LN-3) | 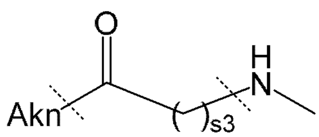 | s3=2-6 |

(continued)

| No. | -L¹- | |
|---|---|---|
| (LN-4) | 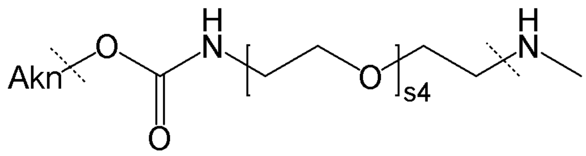 | s4=1-6 |
| (LN-5) | 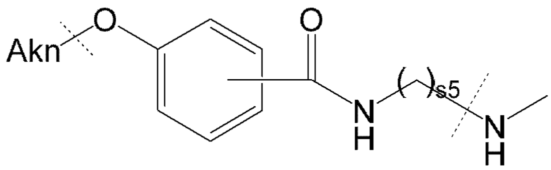 | s5=2-6 |
| (LN-6) | 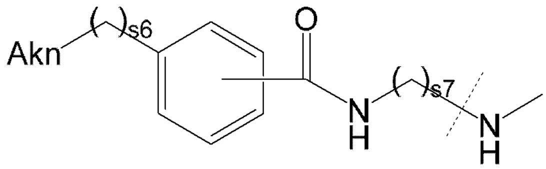 | s6=1-6<br>s7=2-6 |
| (LN-7) | 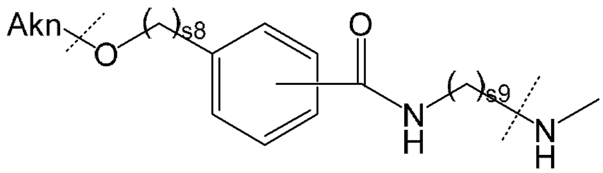 | s8=1-6<br>s9=2-6 |
| (LN-8) | 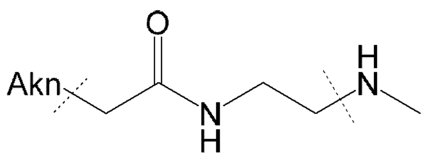 | |
| (LN-9) | 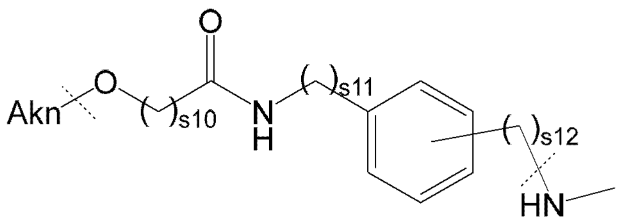 | s10=1-6<br>s11=0-6<br>s12=1-6 |

[Table 1-2]

| No. | -L¹- | |
|---|---|---|
| (L1-1) | 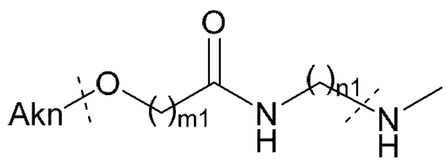 | m1=1-6<br>n1=2-6 |
| (L1-2a) | 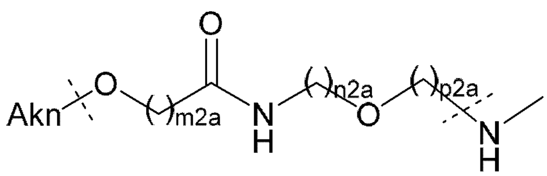 | m2a= 1 -6<br>n2a=2-6<br>p2a=2-6 |

(continued)

| No. | $-L^1-$ | |
|---|---|---|
| (L1-2b) | 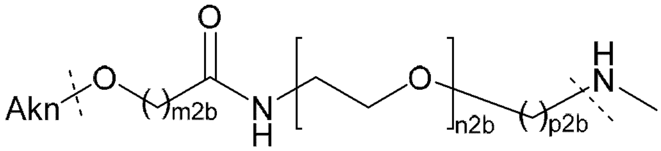 | m2b=1-6<br>n2b=1-6<br>p2b=2-6 |
| (L1-3) | 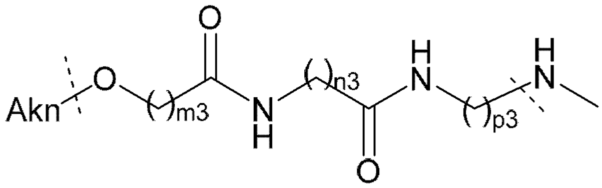 | m3=1-6<br>n3=1-6<br>p3=2-6 |
| (L 1-4a) | 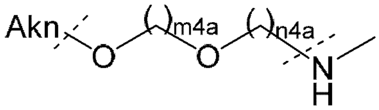 | m4a=2-6<br>n4a=2-6 |
| (L 1-4b) | 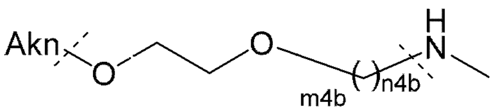 | m4b=0-6<br>n4b=2-6 |
| (L1-5a) | 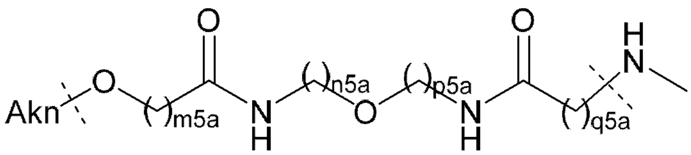 | m5a=1-6<br>n5a=2-6<br>p5a=2-6<br>q5a=1-6 |
| (L1-5b) | 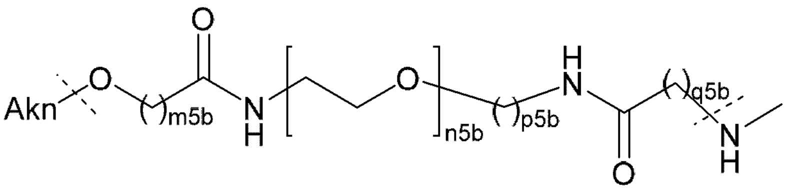 | m5b=1-6<br>n5b=1-6<br>p5b=2-6<br>q5b=1-6 |
| (L1-6a) | 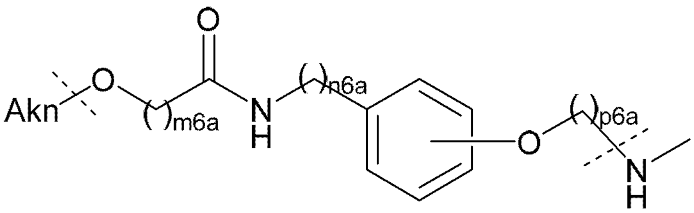 | m6a=1-6<br>n6a=0-6<br>p6a=2-6 |

[Table 1-3]

| | | |
|---|---|---|
| (L1-6b) | 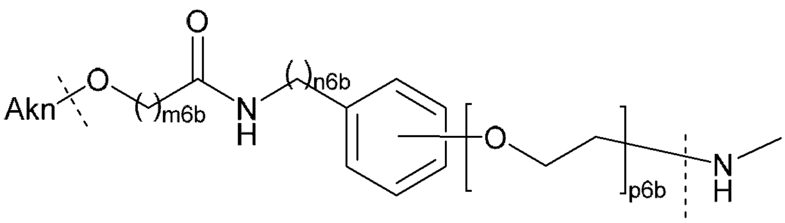 | m6b=1-6<br>n6b=1-6<br>p6b=2-6 |

(continued)

| | | |
|---|---|---|
| (L1-7) | 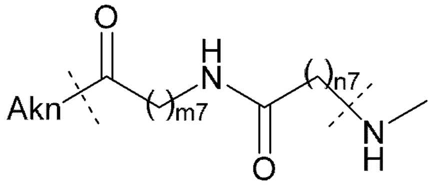 | m7=1-6<br>n7=1-6 |
| (L1-8a) | 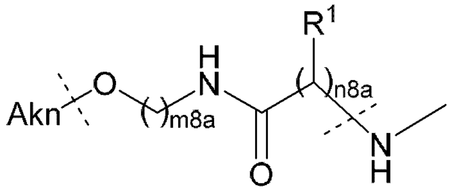 | m8a=2-6<br>n8a=1-6<br>$R^1$=H,Me,Et,Bn |
| (L1-8b) | 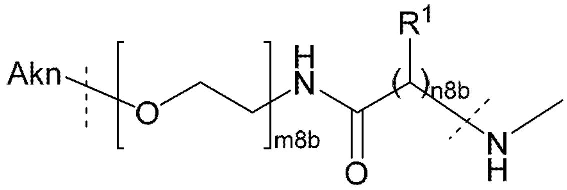 | m8b=1-6<br>n8b=1-6<br>$R^1$=H,Me,Et,Bn |
| (L1-9a) | 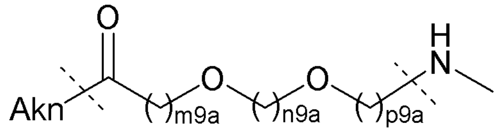 | m9a= 1 -6<br>n9a=2-6<br>p9a=2-6 |
| (L1-9b) | 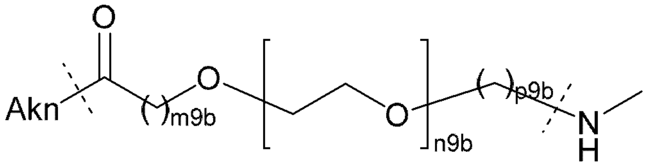 | m9b=1-6<br>n9b=1-6<br>p9b=2-6 |
| (L1-10) | 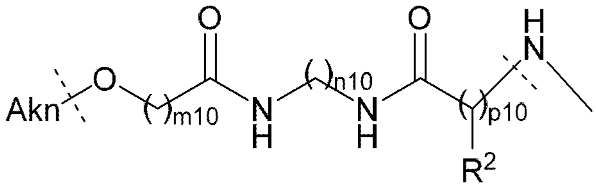 | m10=1-6<br>n10=2-6<br>p10=1-6<br>$R^2$=H,Me,Et,Bn |

and

Akn represents a cyclic alkyne group selected from the group consisting of partial structural formulae [in each formula, the right side of the broken line is not included] shown in the following table:

[Table 2]

| No. | Akn | No. | Akn | No. | Akn |
|---|---|---|---|---|---|
| (AK-1) | 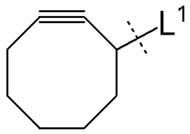 | (AK-5) | 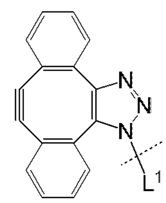 | (AK-9) | 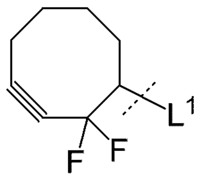 |

(continued)

| No. | Akn | No. | Akn | No. | Akn |
|---|---|---|---|---|---|
| (AK-2) | 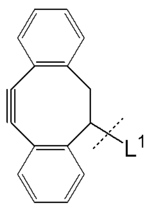 | (AK-6) | 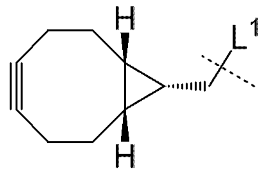 | (AK-10) | 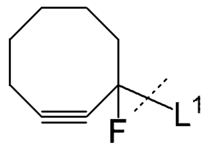 |
| (AK-3) | 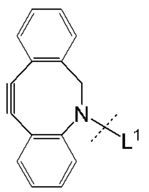 | (AK-7) | 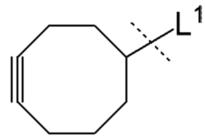 | (AK-11) | 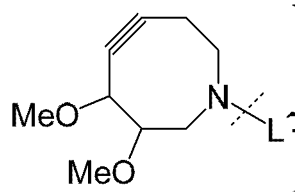 |
| (AK-4) | 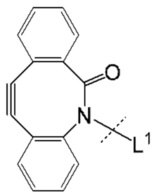 | (AK-8) | 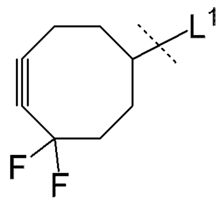 | (AK-12) | 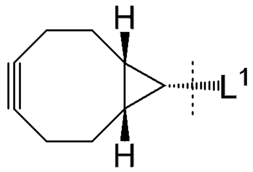 |

and

[chemically modified alginic acid derivative represented by Formula (H-II)]

a chemically modified alginic acid derivative represented by Formula (H-II) below:

[C4]

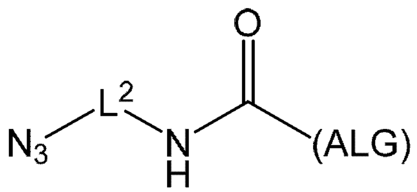

(H-II)

(in Formula (H-II), (ALG) represents alginic acid; -NHCO- represents an amide bond through an arbitrary carboxyl group of the alginic acid; $-L^2-$ represents a linker selected from the group consisting of partial structural formulae [in each formula, the outsides of the broken lines at both ends are not included] shown in the following table:

[Table 3-1]

| No. | $-L^2-$ | |
|---|---|---|
| (LK-1) | 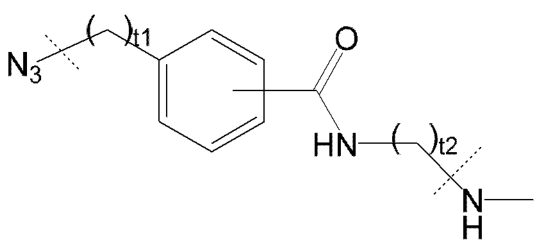 | 11 = 1-6<br>t2=2-6 |

(continued)

| No. | $-L^2-$ | |
|---|---|---|
| (LK-2) | 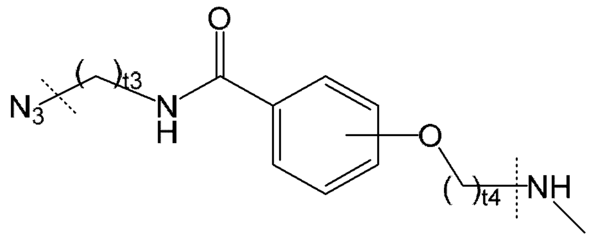 | t3=2-6 t4=2-6 |
| (LK-3) | 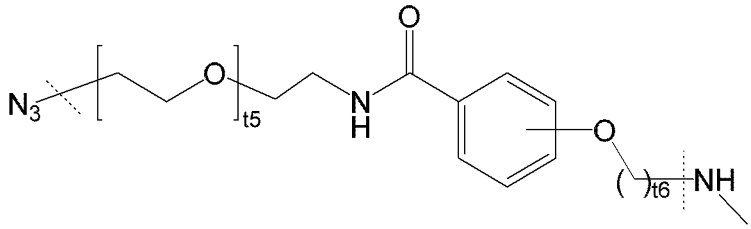 | t5=1-6<br>t6=2-6 |
| (LK-4) | 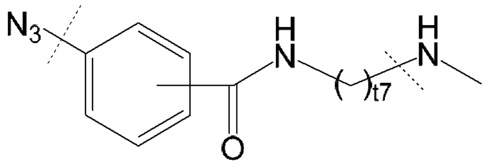 | t7=2-6 |
| (LK-5) | 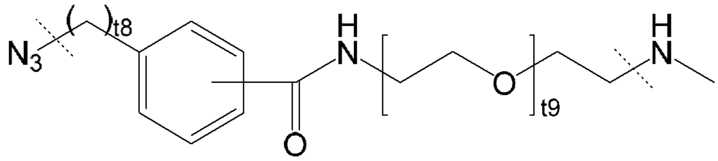 | t8=1-6<br>t9=1-6 |
| (LK-6) | 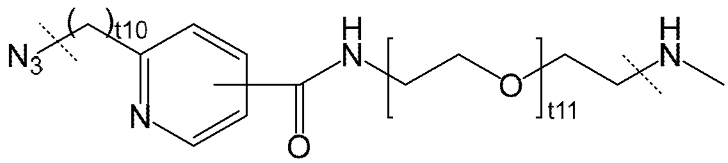 | 110=1-6<br>t11=1-6 |
| (LK-7) | 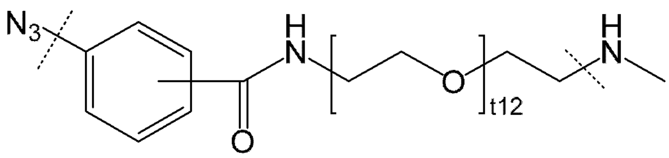 | t12=1-6 |

[Table 3-2]

| No. | $-L^2-$ | |
|---|---|---|
| (L2-1) | 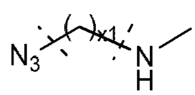 | x1=2-6 |
| (L2-2a) | 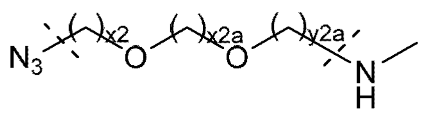 | x2=2-6<br>x2a=2-6<br>y2a=2-6 |

(continued)

| No. | -L²- | |
|---|---|---|
| (L2-2b) | 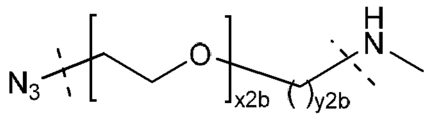 | x2b=1-6<br>y2b=2-6 |
| (L2-3) | 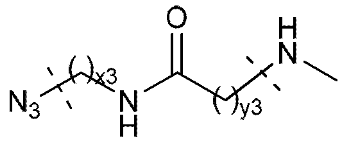 | x3=2-6<br>y3=1-6 |
| (L2-4) | 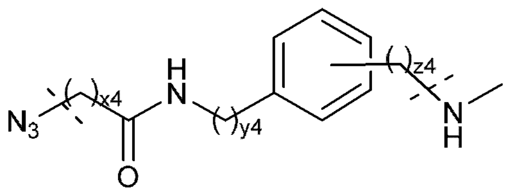 | x4=1-6<br>y4=1-6<br>z4=1-6 |
| (L2-5a) | 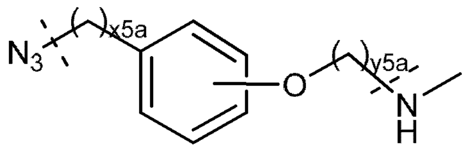 | x5a=0-4<br>y5a=2-6 |
| (L2-5b) | 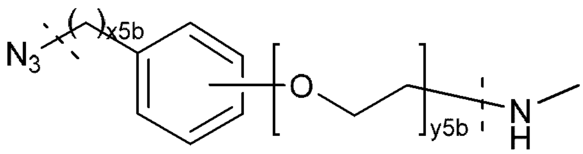 | x5b=0-4<br>y5b=1-6 |
| (L2-6a) | 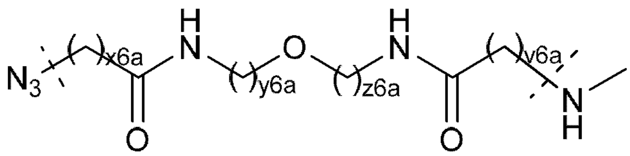 | x6a=1-6<br>y6a=2-6<br>z6a=2-6<br>v6a=1-6 |
| (L2-6b) | 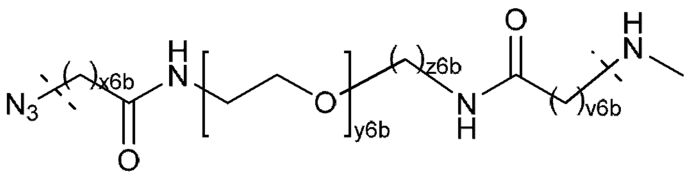 | x6b=1-6<br>y6b=1-6<br>z6b=2-6<br>v6b=1-6 |
| (L2-7a) | 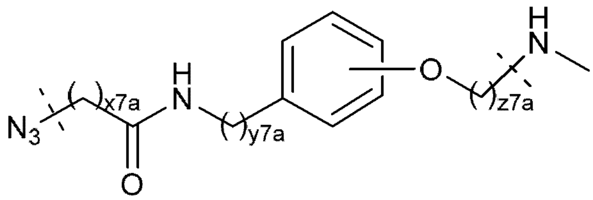 | x7a=1-6<br>y7a=1-6<br>z7a=2-6 |
| (L2-7b) | 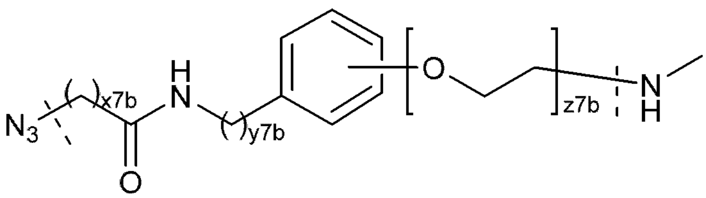 | x7a=1-6<br>x7a=1-6<br>z7b=1-6 |

[Table 3-3]

| No. | -L²- | |
|---|---|---|
| (L2-8a) | 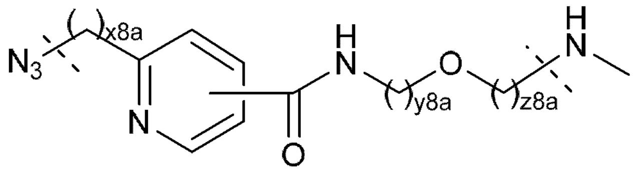 | x8a=0-4<br>y8a=2-6<br>z8a=3-6 |
| (L2-8b) | 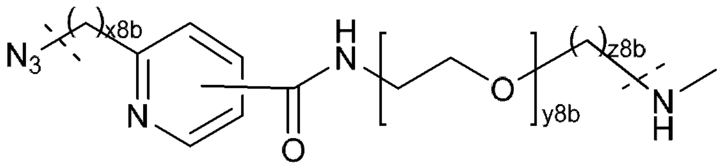 | x8b=0-4<br>y8b=1-6<br>z8b=3-6 |
| (L2-9a) | 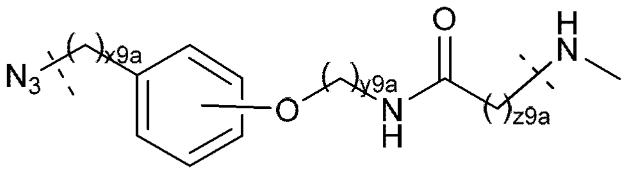 | x9a=0-4<br>y9a=2-6<br>z9a=1-6 |
| (L2-9b) | 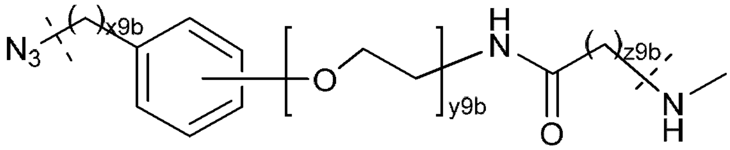 | x9b=0-4<br>y9b=1-6<br>z9b=1-6 |

[2-2] The structure described in [2-1] that is manufactured using a chemically crosslinked alginic acid that is obtained by forming a crosslink between a chemically modified alginic acid derivative in which the combination of Akn-$L^1$- in Formula (H-I) is selected from formulae in the following table:

[Table 4]

| Akn | -$L^1$- |
|---|---|
| (AK-1) | Any one linker selected from the group of (LN-4-X), (LN-9-X), (L1-1-X), (L1-2-X), (L1-3-X1), (L1-3-X2), (L1-4-X1), (L1-4-X2), (L1-5-X), (L1-6-X), (L1-8-X1), (L1-8-X2), (LN-3-X1), (LN-3-X2) or (L1-7-X) |
| (AK-3) | Any one linker selected from the group of (LN-3-X1), (LN-3-X2) or (L1-7-X) |
| (AK-6) | Any one linker selected from the group of (LN-4-X), (LN-9-X), (L1-1-X), (L1-2-X), (L1-3-X1), (L1-3-X2), (L1-4-X1), (L1-4-X2), (L1-5-X), (L1-6-X), (L1-8-X1), (L1-8-X2), (LN-3-X1), (LN-3-X2) or (L1-7-X) |

(here, Akn in the table is the same as the definition in [2-1], each reference symbol represented by -$L^1$- represents partial structural formulae below:

[C5]

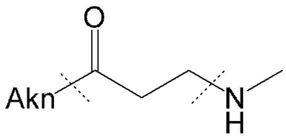

(LN-3-X1)

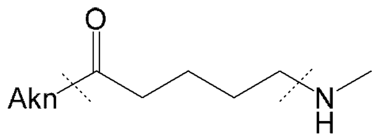

(LN-3-X2)

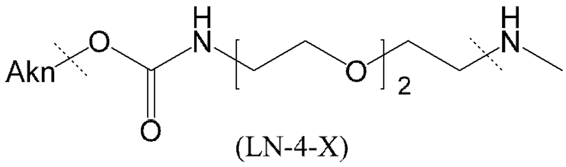

(LN-4-X)

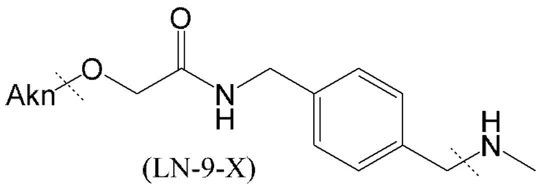

(LN-9-X)

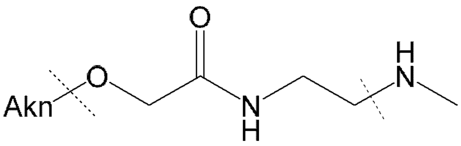

(L1-1-X)

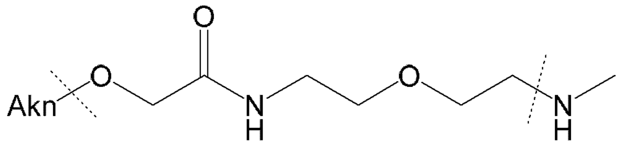

(L1-2-X)

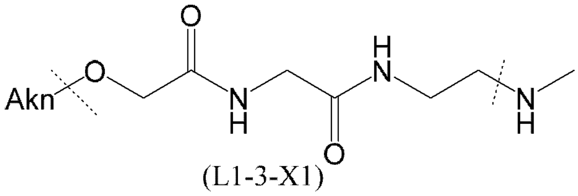

(L1-3-X1)

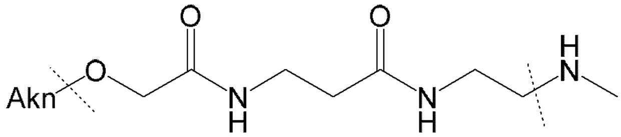

(L1-3-X2)

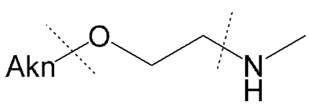

(L1-4-X1)

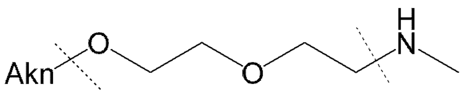

(L1-4-X2)

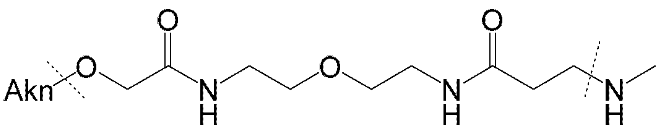

(L1-5-X)

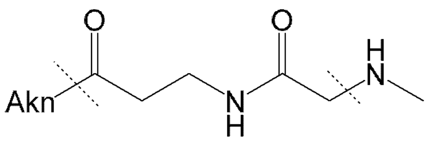

(L1-7-X)

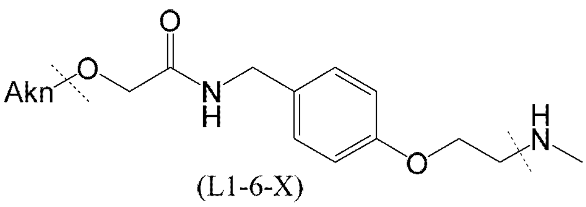

(L1-6-X)

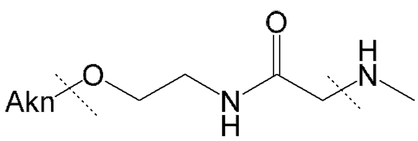

(L1-8-X1)

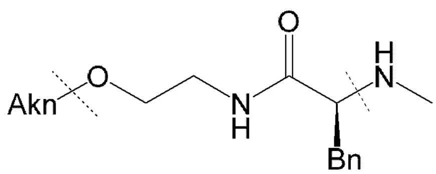

(L1-8-X2)

[in each formula, the outsides of the broken lines at both ends are not included]) and a chemically modified alginic acid derivative in which -$L^2$- in Formula (H-II) is selected from partial structural formulae shown in the following formulae:

[C6]

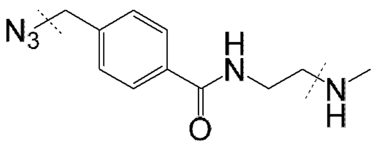

(LK-1-X)

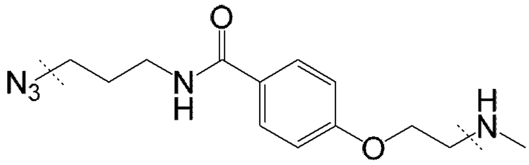

(LK-2-X)

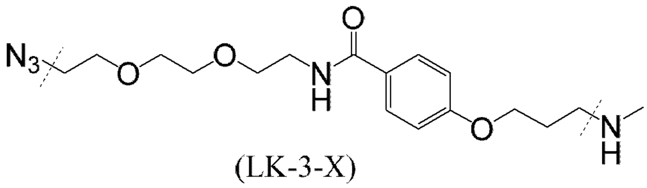

(LK-3-X)

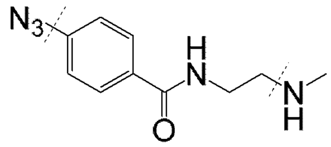

(LK-4-X)

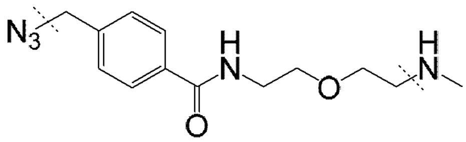

(LK-5-X1)

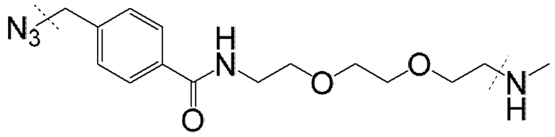

(LK-5-X2)

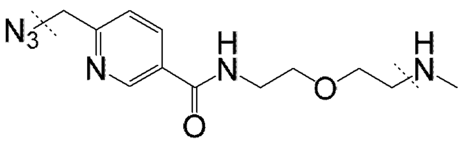

(LK-6-X)

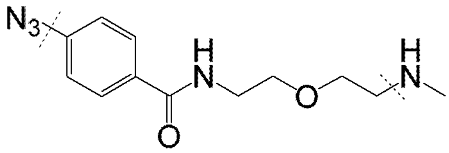

(LK-7-X1)

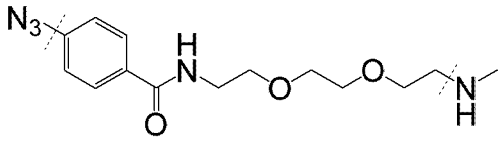

(LK-7-X2)

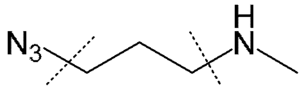
(L2-1-X)

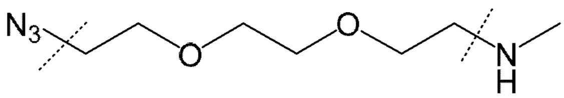
(L2-2-X)

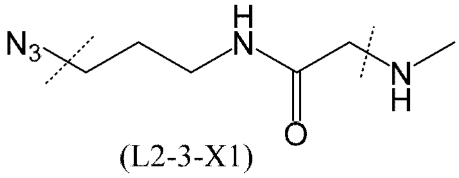
(L2-3-X1)

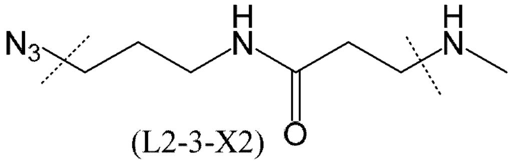
(L2-3-X2)

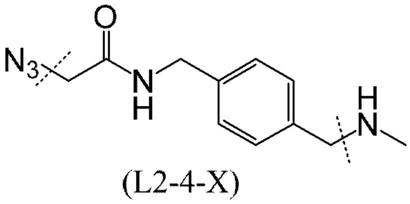
(L2-4-X)

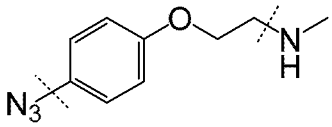
(L2-5-X)

[in each formula, the outsides of the broken lines at both ends are not included] using both alginic acid derivatives.

[2-2b] The structure described in [2-2] that is manufactured using a chemically modified alginic acid derivative for which Akn in Formula (H-I) is (AK-1) and $-L^1-$ is (LN-4-X), (LN-9-X), (L1-1-X), (L1-2-X), (L1-3-X1), (L1-3-X2), (L1 -4-X1), (L1-4-X2), (L1-5-X), (L1-6-X), (L1-8-X1) or (L1-8-X2).

[2-2c] The structure described in [2-2] that is manufactured using a chemically modified alginic acid derivative for which Akn in Formula (H-I) is (AK-3) and $-L^1-$ is (LN-3-X1), (LN-3-X2) or (L1-7-X).

[2-2d] The structure described in [2-2] that is manufactured using a chemically modified alginic acid derivative for which Akn in Formula (H-I) is (AK-9) and $-L^1-$ is (LN-4-X), (LN-9-X), (L1-1-X), (L1-2-X), (L1-3-X1), (L1-3-X2), (L1 -4-X1), (L1-4-X2), (L1-5-X), (L1-6-X), (L1-8-X1) or (L1-8-X2).

[2-3] The structure described in [2-2] that is manufactured using a chemically modified alginic acid derivative for which Akn in Formula (H-I) is (AK-1) or (AK-3).

[2-4] The structure described in [2-1] that is manufactured using a chemically modified alginic acid derivative for which Akn in Formula (H-I) is (AK-1) and $-L^1-$ is (LN-3) or (LN-9), or Akn is (AK-3) and $-L^1-$ is (LN-3), and a chemically modified alginic acid derivative for which $-L^2-$ in Formula (H-II) is (LK-2), (LK-4), (LK-5) or (LN-7). Particularly preferably, the structure for which $-L^2-$ is (LK-2).

[2-4b] The structure described in [2-4] that is manufactured using a chemically modified alginic acid derivative for which Akn in Formula (H-I) is (AK-1) and $-L^1-$ is (LN-9), or Akn is (AK-3) and $-L^1-$ is (LN-3), and a chemically modified alginic acid derivative for which $-L^2-$ in Formula (H-II) is (LK-2), (LK-4), (LK-5) or (LN-7). Particularly preferably, the structure for which $-L^2-$ is (LK-2).

[2-5] The structure described in [2-2] that is manufactured using a chemically modified alginic acid derivative for which Akn in Formula (H-I) is (AK-1) and $-L^1-$ is (LN-3-X1) or (LN-9-X) in the table in [2-2], or Akn is (AK-3) and $-L^1-$ is (LN-3-X1), and a chemically modified alginic acid derivative for which $-L^2-$ in Formula (H-II) is (LK-2-X), (LK-4-X), (LK-5-X1) or (LN-7-X1). Particularly preferably, the structure for which $-L^2-$ is (LK-2-X).

[2-6] The structure described in [2-1], in which the chemically modified alginic acid derivative of Formula (H-I) is Formula (A01) below:

[C7]

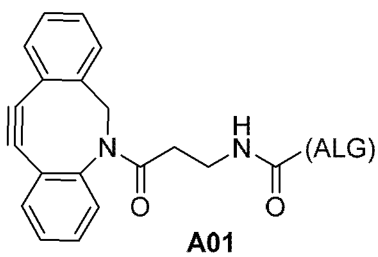
**A01**

the chemically modified alginic acid derivative of Formula (H-II) is Formula (N01) below:

[C8]

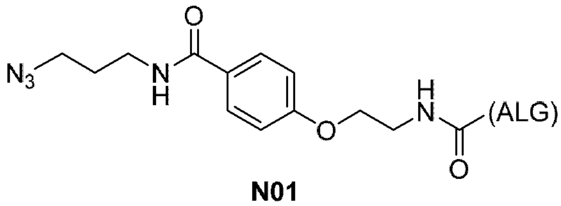

N01

[2-7] The structure described in [2-1], in which the chemically modified alginic acid derivative of Formula (H-I) is Formula (A01) in [2-6], and the chemically modified alginic acid derivative of Formula (H-II) is Formula (N02) below:

[C9]

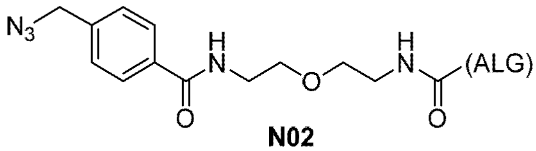

N02

[2-8] The structure described in [2-1], in which the chemically modified alginic acid derivative of Formula (H-I) is Formula (A01) in [2-6], and the chemically modified alginic acid derivative of Formula (H-II) is Formula (N03) below:

[C10]

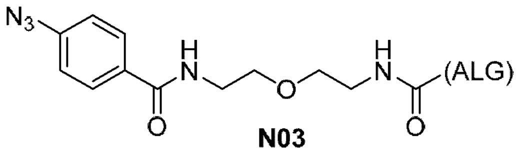

N03

[2-9] The structure described in [2-1], in which the chemically modified alginic acid derivative of Formula (H-I) is Formula (A01) in [2-6], and the chemically modified alginic acid derivative of Formula (H-II) is Formula (N04) below:

[C11]

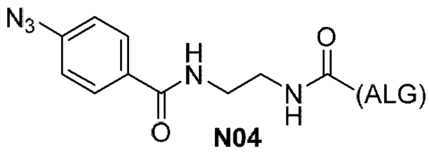

N04

[2-10] The structure described in [2-1], in which the chemically modified alginic acid derivative of Formula (H-I) is Formula (A02) below:

[C12]

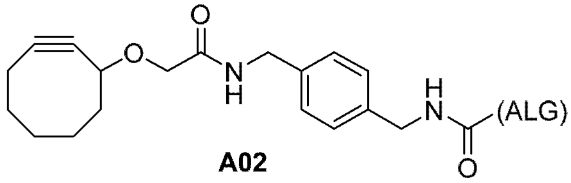

A02

the chemically modified alginic acid derivative of Formula (H-II) is Formula (N01) below:

[C13]

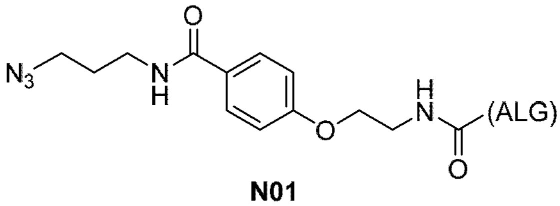

N01

.

[2-11] The structure described in [2-1], in which the chemically modified alginic acid derivative of Formula (H-I) is Formula (A02) in [2-10], and the chemically modified alginic acid derivative of Formula (H-II) is Formula (N02) below:

[C14]

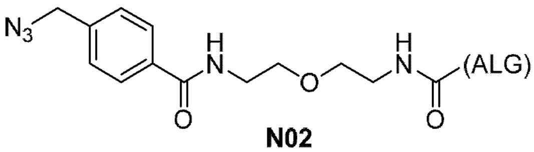

N02

.

[2-12] The structure described in [2-1], in which the chemically modified alginic acid derivative of Formula (H-I) is Formula (A02) in [2-10], and the chemically modified alginic acid derivative of Formula (H-II) is Formula (N03) below:

[C15]

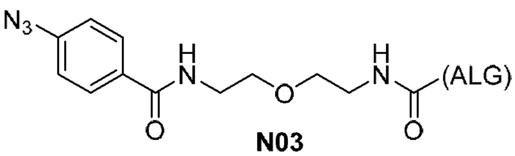

N03

.

[2-13] The structure described in [2-1], in which the chemically modified alginic acid derivative of Formula (H-I) is Formula (A02) in [2-10], and the chemically modified alginic acid derivative of Formula (H-II) is Formula (N04) below:

[C16]

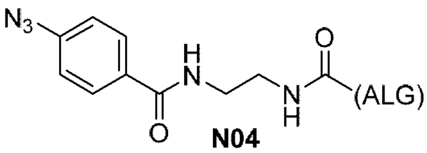

N04

.

[2-14] The structure described in [2], in which, in Formula (H-I), $-L^1-$ is $-(CH_2)_{n1}-$(here, n1 is 1 to 50, $-CH_2-$ in the group is optionally substituted with one to 10 groups selected from -CO-, -CONH-, -NHCO-, -O-CONH-, -NHCO-O-, -O- and -NH-, or a benzene ring, and a hydrogen atom in the $-CH_2-$ is optionally substituted with a $C_{1-3}$ alkyl group or a $C_{1-3}$ alkyl group substituted with a phenyl group),

Akn is a compound that is an eight-membered cyclic alkyne group (here, the cyclic alkyne group is optionally an eight-membered cyclic group in which, furthermore, one or two benzene rings, cyclopropane rings or 1,2,3-triazole rings are optionally condensed and optionally bond to $-L^1-$ in a condensed ring, and $-CH_2-$ in the eight-membered cyclic alkyne group is optionally substituted with one or two groups selected from -C(=O)-, -CONH- and - NH-, and a hydrogen atom in $-CH_2-$ in the alkyne group is optionally substituted with one or two groups selected from a $C_{1-3}$ alkyl group, a fluorine atom, a hydroxyl group or a $C_{1-3}$ alkyloxy group), and
in Formula (H-II), $-L^2-$ is $-(CH_2)_{n2}-$ (here, n2 is 1 to 50, $-CH_2-$ in the group is optionally substituted with one to 10 groups selected from -CONH-, -NHCO-, -O- and -NH-, a benzene ring or a pyridine ring, and a hydrogen atom in the $-CH_2-$ is optionally substituted with a $C_{1-3}$ alkyl group).

[2-15] The structure described in [2], in which, in Formula (H-I), $-L^1-$ is $-(CH_2)_{n1}-$(here, n1 is 1 to 15 (for example, 2 to 15), $-CH_2-$ in the group is optionally substituted with one to five groups selected from -CO-, -CONH-, -NHCO-, -O-CONH-, -NHCO-O-, -O- and - NH- or a benzene ring, and a hydrogen atom in the $-CH_2-$ is optionally substituted with a $C_{1-3}$ alkyl group or a $C_{1-3}$ alkyl group substituted with a phenyl group),

Akn is a compound that is an eight-membered cyclic alkyne group (here, the cyclic alkyne group is optionally an eight-membered cyclic group in which, furthermore, one or two benzene rings are optionally condensed or $-CH_2-$ in the eight-membered cyclic alkyne group is substituted with -NH-), and
in Formula (H-II), $-L^2-$ is $-(CH_2)_{n2}-$ (here, n2 is 1 to 15 (for example, 2 to 15), $-CH_2-$in the group is optionally substituted with one to five groups selected from -CONH-, - NHCO-, -O- and -NH-, or a benzene ring).

[3-1] The structure described in [1], [2] or [2-1], in which the chemically crosslinked alginic acid is obtained by forming a crosslink between a chemically modified alginic acid derivative represented by Formula (HB-I) and a chemically modified alginic acid derivative represented by Formula (HB-II) using both alginic acid derivatives:

[chemically modified alginic acid derivative represented by Formula (HB-I)]
a chemically modified alginic acid represented by Formula (HB-I):

[C17]

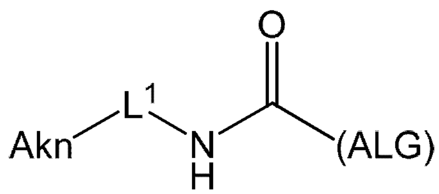

(HB-I)

[in Formula (HB-I), (ALG) represents alginic acid; -NHCO- represents an amide bond through an arbitrary carboxyl group of the alginic acid; $-L^1-$ represents a linker selected from the group consisting of partial structural formulae (LN-3), (LN-9) and (L1-3) to (L1-10) in the partial structural formulae of $-L^1-$ in [2-1] [in each formula, the outsides of the broken lines at both ends are not included];
Akn represents a cyclic alkyne group selected from the group consisting of partial structural formulae [in each formula, the right side of the broken line is not included] shown in the partial structural formulae of Akn in [2-1];
[chemically modified alginic acid derivative represented by Formula (HB-II)]
a chemically modified alginic acid derivative represented by Formula (HB-II) below:

[C18]

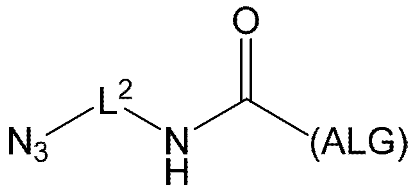

(HB-II)

[in Formula (HB-II), (ALG) represents alginic acid; -NHCO- represents an amide bond through an arbitrary carboxyl group of the alginic acid; $-L^2-$ represents a linker selected from the group consisting of partial structural formulae (LK-2) and (L2-1) to (L2-9b) in the partial structural formulae of $-L^2-$ in [2-1] [in each formula, the outsides of the broken lines at both ends are not included].

(With the proviso that a chemically crosslinked alginic acid that is obtained by performing a crosslinking reaction using a derivative for which, in the chemically modified alginic acid derivative represented by Formula (HB-I), $-L^1-$ is any one linker selected from the group of (LN-3) or (LN-9) and a derivative for which, in the chemically modified alginic acid derivative represented by Formula (HB-II), $-L^2-$ is the linker of (LK-2) is excluded).

[3-2] The structure described in [3-1], in which $-L^1-$ in Formula (HB-I) is selected from partial structural formulae [in each formula, the outsides of the broken lines at both ends are not included] shown in formulae in the following table:

[Table 5-1]

| No. | $-L^1-$ | |
|---|---|---|
| (L1-1-A) | 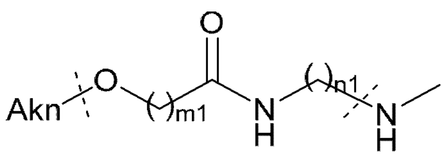 | m 1=5, 6<br><br>n1=2-6 |
| (L1-2a-A) | 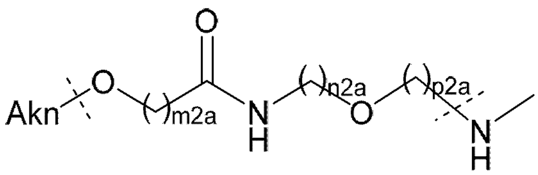 | m2a= 1 -6<br><br>n2a=2-6<br>p2a=2-6<br>(With the proviso that a combination of m2a=1, n2a=2 and p2a=2 is excluded) |
| (L1-2b-A) | 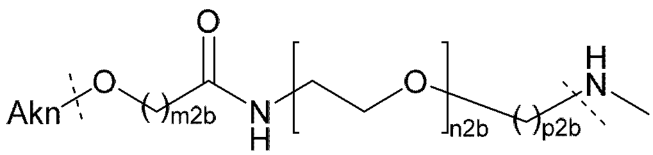 | m2b=1-6<br><br>n2b=1-6<br>p2b=3-6 |
| (L1-3) | 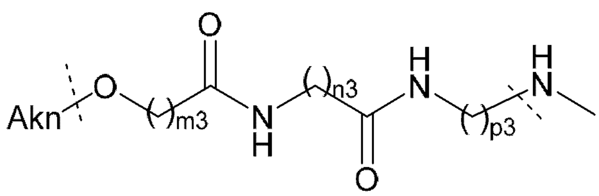 | m3=1-6<br>n3=1-6<br>p3=2-6 |
| (L 1-4a) | 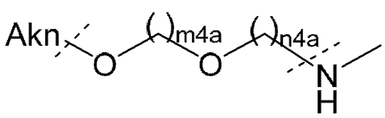 | m4a=2-6<br>n4a=2-6 |

(continued)

| No. | -L[1]- | |
|---|---|---|
| (L1-4b) | 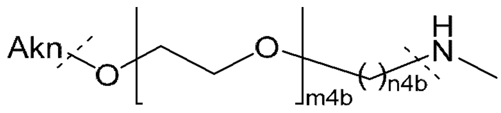 | m4b=1-6<br>n4b=2-6 |
| (L1-5a) | 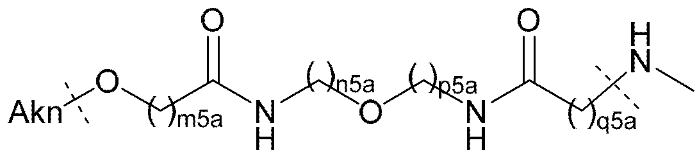 | m5a=1-6<br>n5a=2-6<br>p5a=2-6<br>q5a=1-6 |
| (L1-5b) | 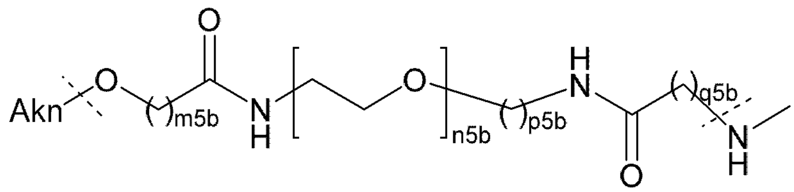 | m5b=1-6<br>n5b=1-6<br>p5b=2-6<br>q5b=1-6 |
| (L1-6a) | 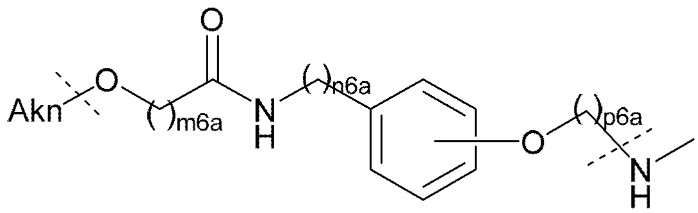 | m6a=1-6<br>n6a=0-6<br>p6a=2-6 |

[Table 5-2]

| No. | -L[1]- | |
|---|---|---|
| (L1-6b) | 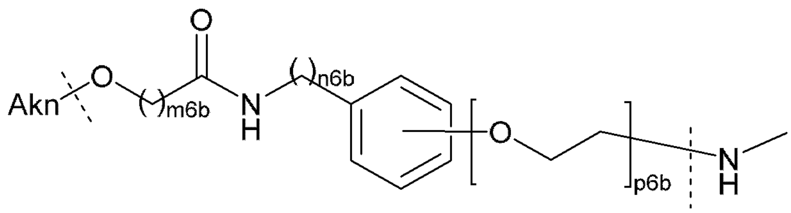 | m6b=1-6<br>n6b=1-6<br>p6b=2-6 |
| (L1-7) | 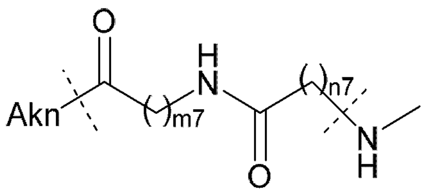 | m7=1-6<br>n7=1-6 |
| (L1-8a) | 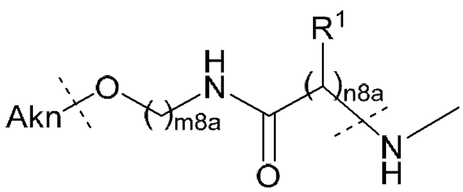 | m8a=2-6<br>n8a=1-6<br>$R^1$=H,Me,Et,Bn |

(continued)

| No. | -$L^1$- | |
|---|---|---|
| (L1-8b) | 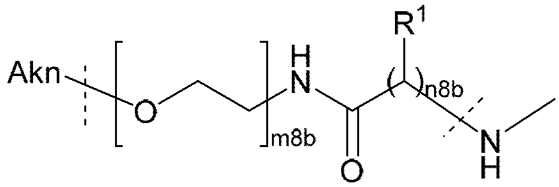 | m8b=1-6<br>n8b=1-6<br>$R^1$=H,Me,Et,Bn |
| (L1-9a) | 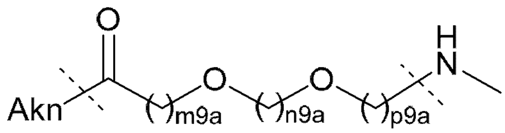 | m9a= 1 -6<br>n9a=2-6<br>p9a=2-6 |
| (L1-9b) | 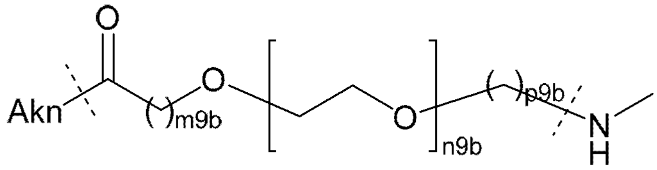 | m9b=1-6<br>n9b=1-6<br>p9b=2-6 |
| (L1-10) | 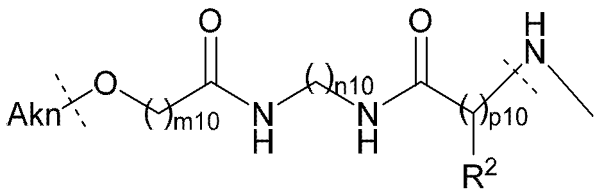 | m10=1-6<br>n10=2-6<br>p10=1-6<br>R2=H,Me,Et,Bn |

Akn is selected from partial structural formulae [in each formula, the right side of the broken line is not included] shown in the table of Akn in [2-1];

-$L^2$- in Formula (HB-II) is selected from the group consisting of partial structural formulae [in each formula, the outsides of the broken lines at both ends are not included] shown in the following table:

[Table 6-1]

| No. | -$L^2$- | |
|---|---|---|
| (L2-1-A) | 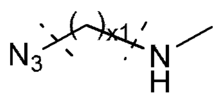 | x1=2,4,5,6 |
| (L2-2a) | 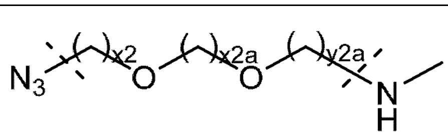 | x2=2-6<br>x2a=2-6<br>y2a=2-6 |
| (L2-2b-A) | 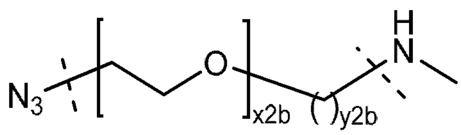 | x2b=1-6<br><br>y2b=2-6 (With the proviso that a combination of x2b=3, y2b=2, x2b=6 and y2b=2 is excluded) |

(continued)

| No. | -L²- | |
|---|---|---|
| (L2-3) | 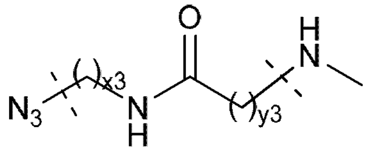 | x3=2-6<br>y3=1-6 |
| (L2-4) | 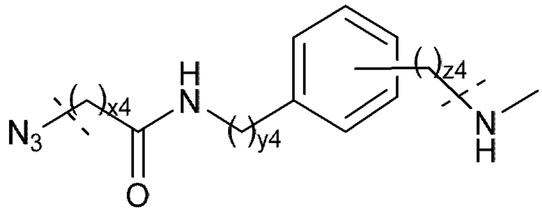 | x4=1-6<br>y4=1-6<br>z4=1-6 |
| (L2-5a) | 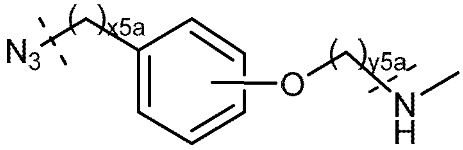 | x5a=0-4<br>y5a=2-6 |
| (L2-5b) | 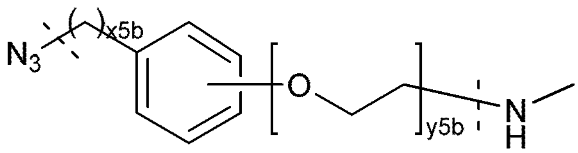 | x5b=0-4<br>y5b=1-6 |
| (L2-6a) | 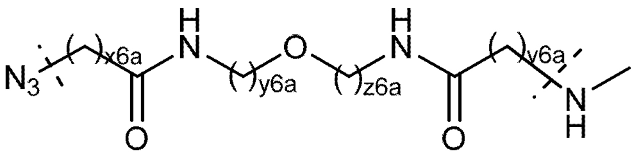 | x6a=1-6<br>y6a=2-6<br>z6a=2-6<br>v6a=1-6 |
| (L2-6b) | 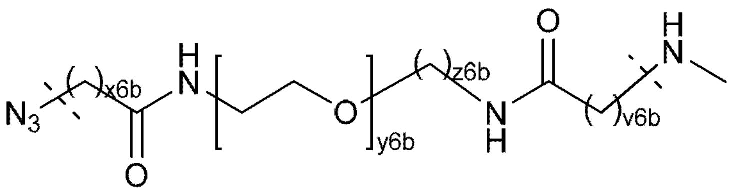 | x6b=1-6<br>y6b=1=6<br>z6b=2-6<br>v6b=1-6 |

[Table 6-2]

| No. | -L²- | |
|---|---|---|
| (L2-7a) | 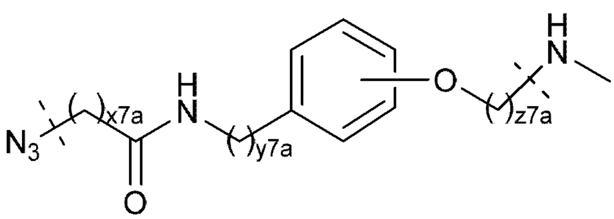 | x7a=1-6<br>y7a=1-6<br>z7a=2-6 |

(continued)

| No. | $-L^2-$ | |
|---|---|---|
| (L2-7b) | 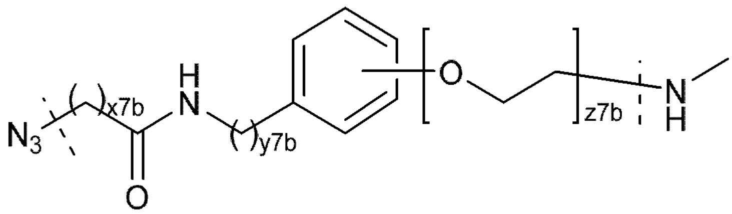 | x7a=1-6<br>x7a=1-6<br>z7b=1-6 |
| (L2-8a) | 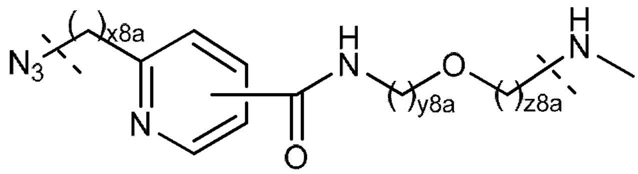 | x8a=0-4<br>y8a=2-6<br>z8a=3-6 |
| (L2-8b) | 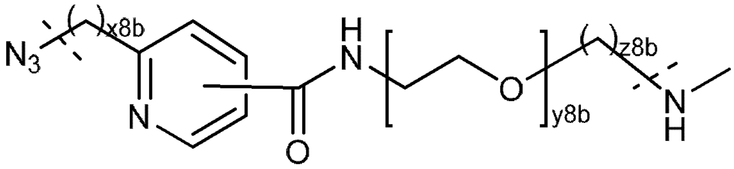 | x8b=0-4<br>y8b=1-6<br>z8b=3-6 |
| (L2-9a) | 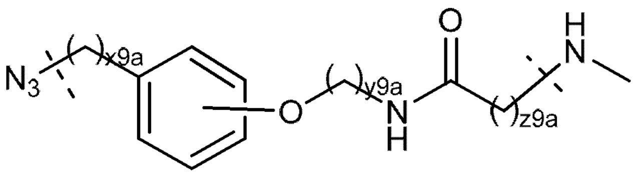 | x9a=0-4<br>y9a=2-6<br>z9a=1-6 |
| (L2-9b) | 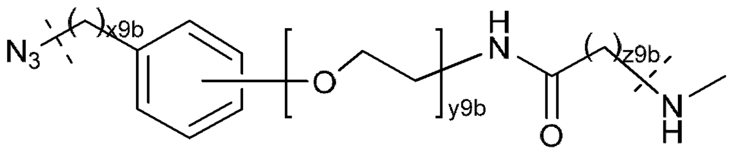 | x9b=0-4<br>y9b=1-6<br>z9b=1-6 |

[3-3] The structure described in [3-2], in which $-L^2-$ in Formula (HB-II) is selected from the group consisting of partial structural formulae [in each formula, the outsides of the broken lines at both ends are not included] shown in the following table:

[Table 7-1]

| No. | $-L^2-$ | |
|---|---|---|
| (L2-3) | 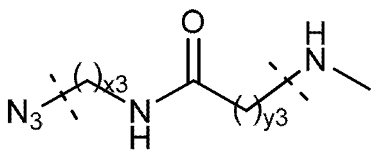 | x3=2-6<br>y3=1-6 |
| (L2-4) | 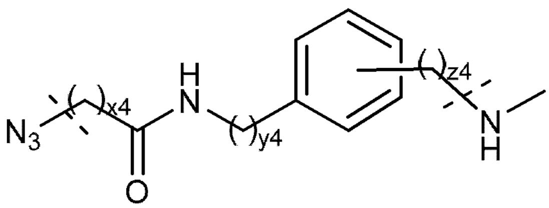 | x4=1-6<br>y4=1-6<br>z4=1-6 |

(continued)

| No. | -L²- | |
|---|---|---|
| (L2-5a) | 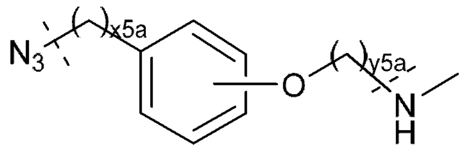 | x5a=0-4<br>y5a=2-6 |
| (L2-5b) | 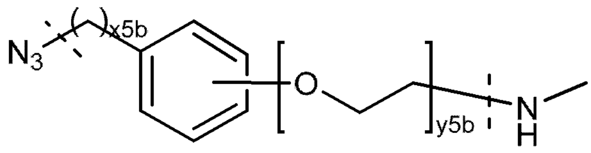 | x5b=0-4<br>y5b=1-6 |
| (L2-6a) | 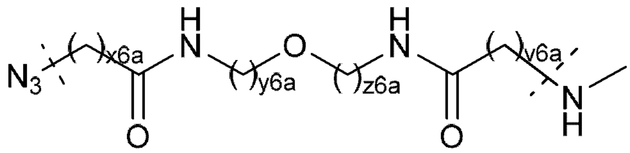 | x6a=1-6<br>y6a=2-6<br>z6a=2-6<br>v6a=1-6 |
| (L2-6b) | 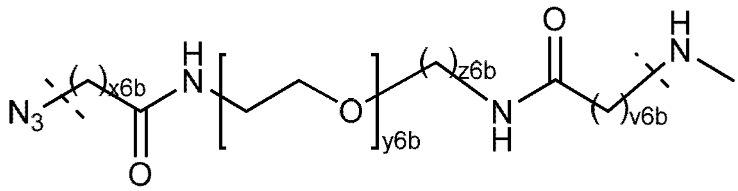 | x6b=1-6<br>y6b=1=6<br>z6b=2-6<br>v6b=1-6 |
| (L2-7a) | 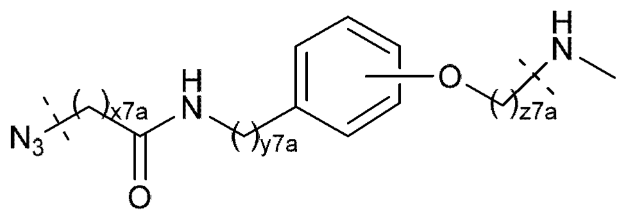 | x7a=1-6<br>y7a=1-6<br>z7a=2-6 |

[Table 7-2]

| No. | -L²- | |
|---|---|---|
| (L2-7b) | 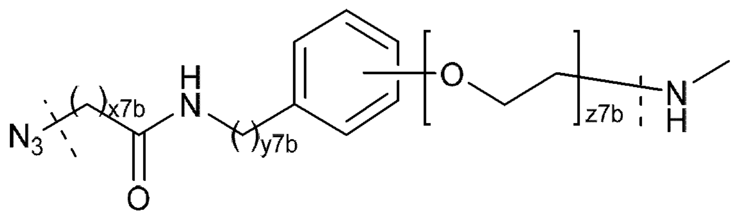 | x7a=1-6<br>x7a=1-6<br>z7b=1-6 |
| (L2-8a) | 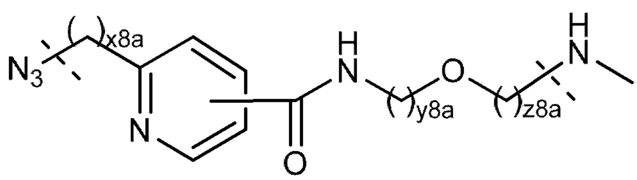 | x8a=0-4<br>y8a=2-6<br>z8a=3-6 |
| (L2-8b) | 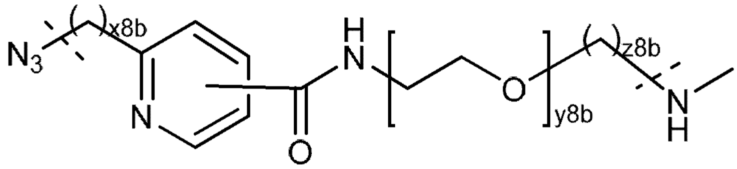 | x8b=0-4<br>y8b=1-6<br>z8b=3-6 |

(continued)

| No. | $-L^2-$ | |
|---|---|---|
| (L2-9a) | 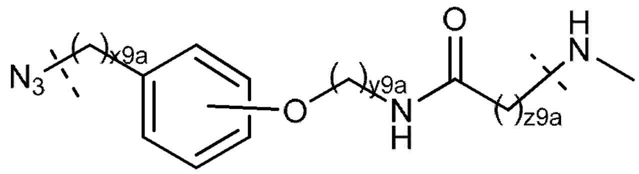 | x9a=0-4<br>y9a=2-6<br>z9a=1-6 |
| (L2-9b) | 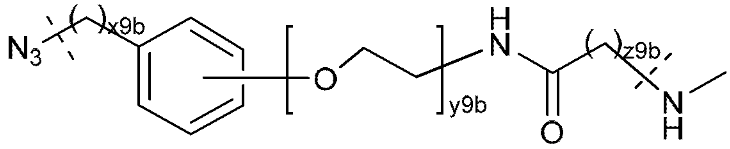 | x9b=0-4<br>y9b=1-6<br>z9b=1-6 |

[3-4] The structure described in [3-3], in which $-L^2-$ in Formula (HB-II) is selected from the group consisting of partial structural formulae [in each formula, the outsides of the broken lines at both ends are not included] shown in the following table:

[Table 8]

| No. | $-L^2-$ | |
|---|---|---|
| (L2-3-1) | 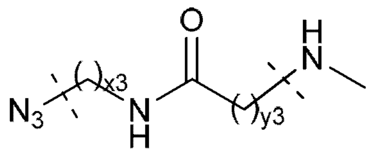 | x3=2-4<br>y3=1-3 |
| (L2-4-p2) | 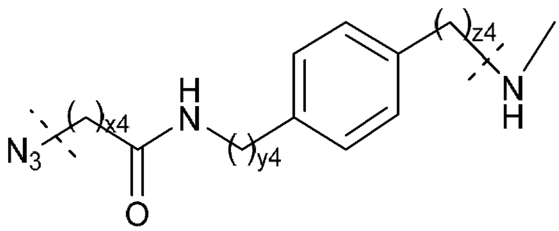 | x4=1-3<br>y4=1-3<br>z4=1-3 |
| (L2-5a-p2) | 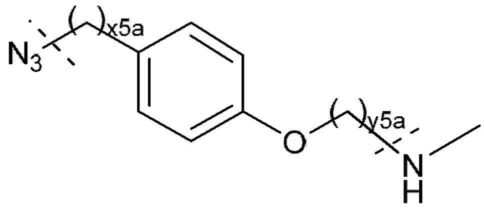 | x5a=0-2<br>y5a=2-4 |
| (L2-5b-p2) | 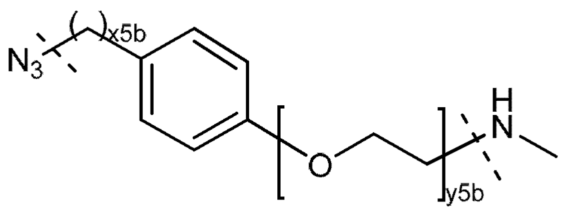 | x5b=0-2<br>y5b=1-3 |

[3-5] The structure described in [3-3], in which $-L^2-$ in Formula (HB-II) is selected from the group consisting of partial structural formulae [in each formula, the outsides of the broken lines at both ends are not included] shown in the following table:

[Table 9]

| No. | _L2_ |
|---|---|
| (L2-3-X1) | 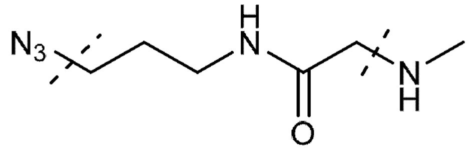 |
| (L2-3-X2) | 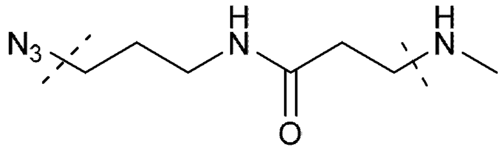 |
| (L2-4-X) | 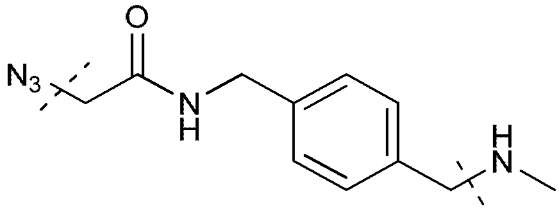 |
| (L2-5-X) | 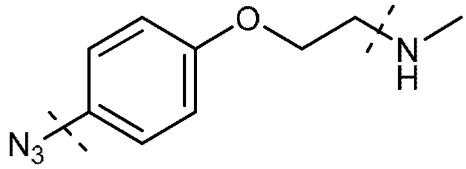 |

[3-6] The structure described in [3-3] to [3-5], in which -$L^1$- in Formula (HB-I) is selected from the group consisting of partial structural formulae [in each formula, the outsides of the broken lines at both ends are not included] shown in the following table:

[Table 10]

| No. | -$L^1$- |
|---|---|
| (L1-3-X1) | 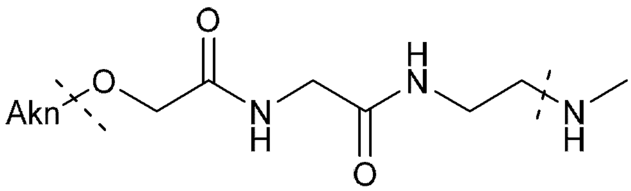 |
| (L1-3-X2) | 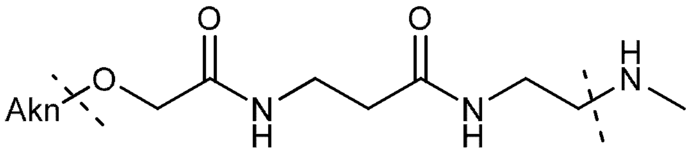 |
| (L1-4-X1) | 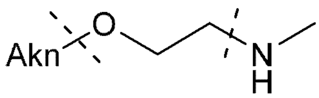 |
| (L1-4-X2) | 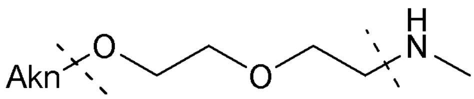 |

(continued)

| No. | -$L^1$- |
|---|---|
| (L1-5-X) | 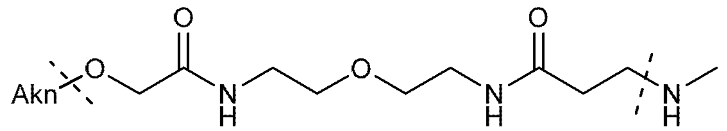 |
| (L1-6-X) | 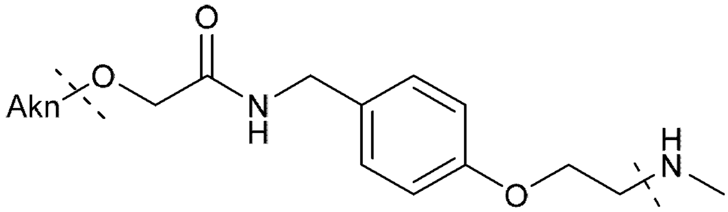 |
| (L1-7-X) | 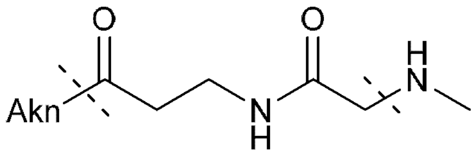 |
| (L1-8-X1) | 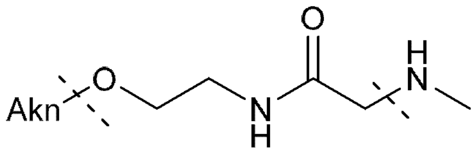 |
| (L1-8-X2) | 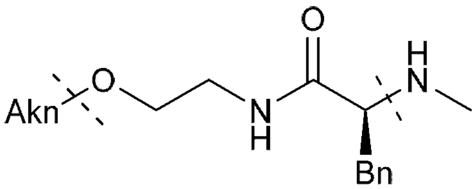 |

[4] The structure described in [1] or [2], in which the chemically crosslinked alginic acid is obtained by forming a crosslink between a chemically modified alginic acid derivative represented by Formula (HA-I) and a chemically modified alginic acid derivative represented by Formula (HA-II) using both alginic acid derivatives:

[chemically modified alginic acid derivative represented by Formula (HA-I)]
a chemically modified alginic acid derivative represented by Formula (HA-I) below:

[C19]

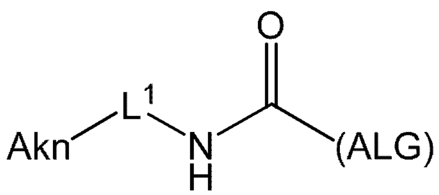

(HA-I)

[in Formula (HA-I), (ALG) represents alginic acid; -NHCO- represents an amide bond through an arbitrary carboxyl group of the alginic acid; -$L^1$- represents a divalent linker selected from the group of the partial structural formulae (in each formula, the outsides of the broken lines at both ends are not included)]:

[C20]

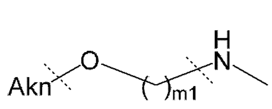

(LN-1) m1=2-6

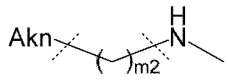

(LN-2) m2=1-6

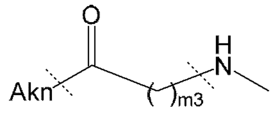

(LN-3) m3=1-6

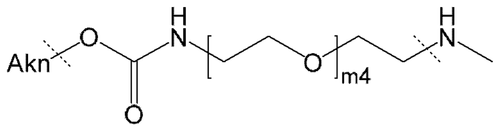

(LN-4) m4=1-6

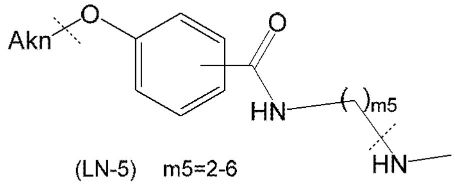

(LN-5) m5=2-6

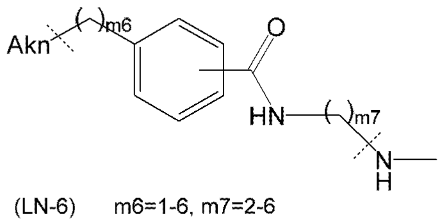

(LN-6) m6=1-6, m7=2-6

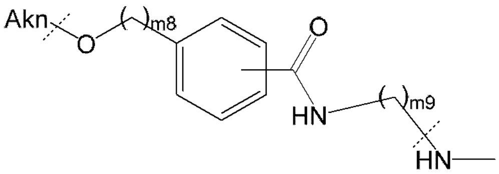

(LN-7) m8=1-6, m9=2-6

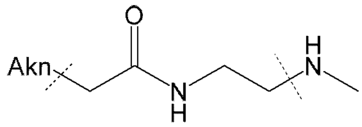

(LN-8)

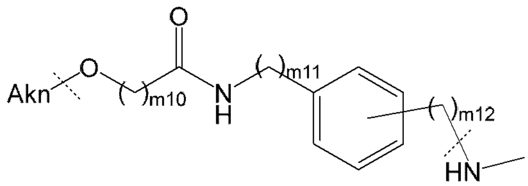

(LN-9) m10=1-4, m11=1-6,m12=1-6

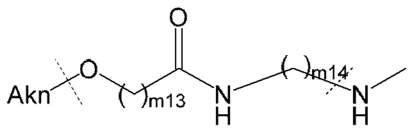

(LN-10) m13=1-4, m14=2~6

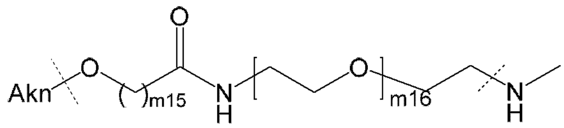

(LN-11) m15=1-4, m16=1-6

Akn represents a cyclic alkyne group selected from the group of the following partial structural formulae [in each formula, the right side of the broken line is not included]:

[C21]

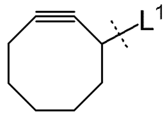 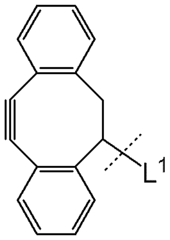 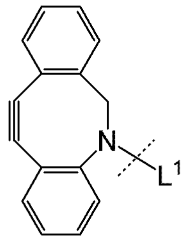 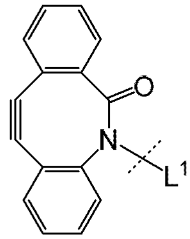

(AK-1) (AK-2) (AK-3) (AK-4)

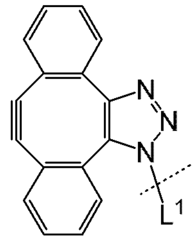 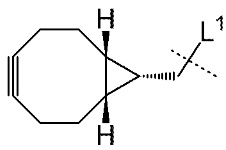 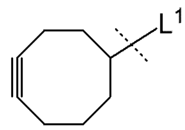 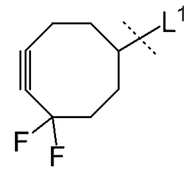

(AK-5) (AK-6) (AK-7) (AK-8)

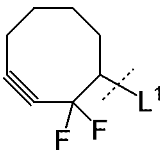 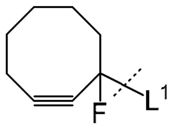 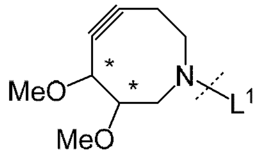 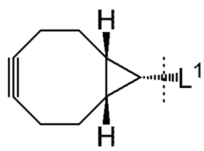

(AK-9) (AK-10) (AK-11) (AK-12)

asterisks indicate chiral centers];

[chemically modified alginic acid derivative represented by Formula (HA-II)]

a chemically modified alginic acid derivative represented by Formula (HA-II) below:

[C22]

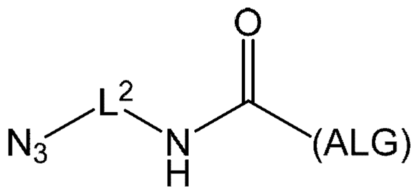 (HA-II)

[in Formula (HA-II), (ALG) represents alginic acid; -NHCO- represents an amide bond through an arbitrary carboxyl group of the alginic acid; -$L^2$- represents a divalent linker selected from the group of the partial structural formulae [in each formula, the outsides of the broken lines at both ends are not included]]:

[C23]

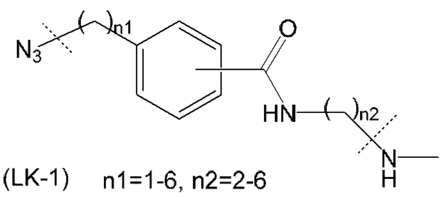

(LK-1) n1=1-6, n2=2-6

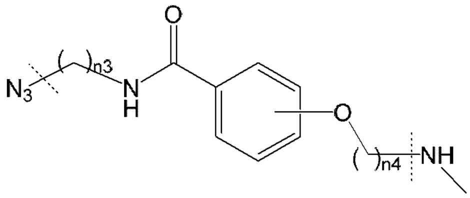

(LK-2) n3=2-6, n4=2-6

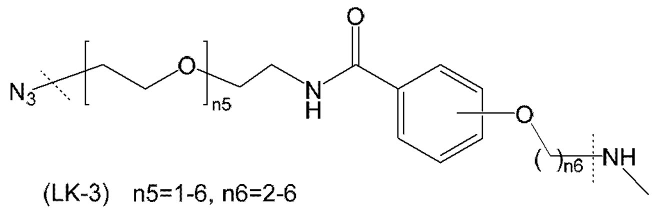

(LK-3) n5=1-6, n6=2-6

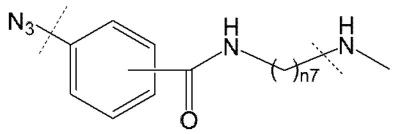

(LK-4) n7=2-6

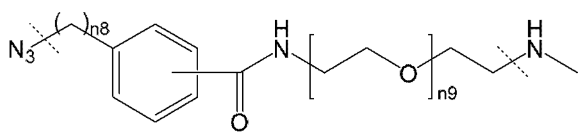

(LK-5) n8=1-4, n9=1-6

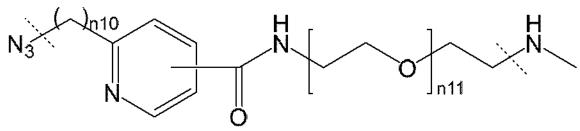

(LK-6) n10=1-4, n11=1-6

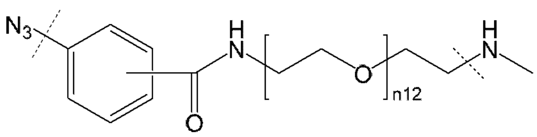

(LK-7) n12=1-6

[5] The structure described in [1], in which the chemically crosslinked alginic acid is a chemically crosslinked alginic acid in which an arbitrary carboxyl group of a first alginic acid and an arbitrary carboxyl group of a second alginic acid are crosslinked through a group represented by Formula (H-III-L) below:

[C24]

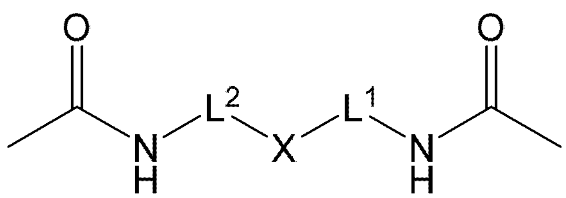

(H-III-L)

[in Formula (H-III-L), -CONH- and -NHCO- at both ends represent amide bonds through arbitrary carboxyl groups of the alginic acid;

$-L^1-$ is a divalent linker bonding to a cyclic alkyne group [Akn];

$-L^2-$ is a divalent linker bonding to an azide group;

X is a divalent group having a cyclic group that is generated from a 3+2 cycloaddition reaction between the azide group bonding to $L^2$ and the cyclic alkyne group (Akn) bonding to $L^1$].

[5-1] The structure described in [1], in which the chemically crosslinked alginic acid is a chemically crosslinked alginic acid in which an arbitrary carboxyl group of a first alginic acid and an arbitrary carboxyl group of a second alginic acid are crosslinked through a group represented by Formula (H-III-L) below:

[C25]

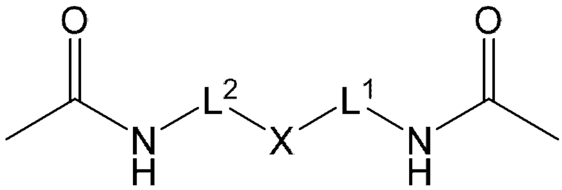

(H-III-L)

[in Formula (H-III-L), -CONH- and -NHCO- at both ends represent amide bonds through arbitrary carboxyl groups of the alginic acid;
-$L^1$- is the same as the definition in [2-1];
-$L^2$- is the same as the definition in [2-1];
X is a cyclic group (in each formula, the outsides of the broken lines at both ends are not included) selected from the group of partial structural formulae shown in the following table:

[Table 11-1]

| No. | X | No. | X |
|---|---|---|---|
| (TZ-1) | 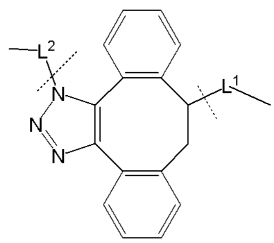 | (TZ-1-r) | 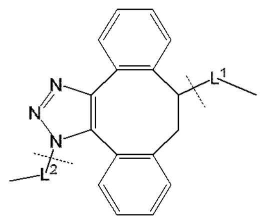 |
| (TZ-2) | 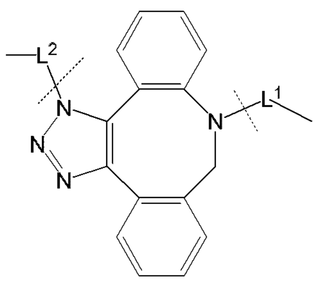 | (TZ-2-r) | 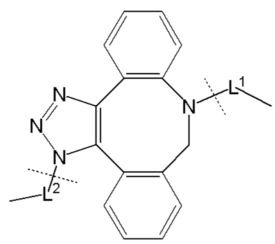 |
| (TZ-3) | 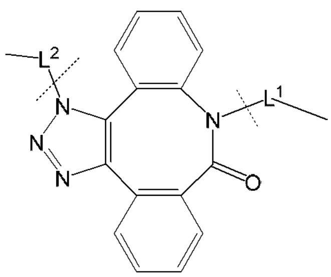 | (TZ-3-r) | 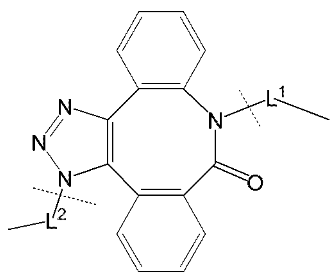 |

(continued)

| No. | X | No. | X |
|---|---|---|---|
| (TZ-4) | 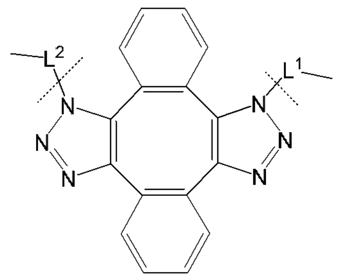 | (TZ-4-r) | 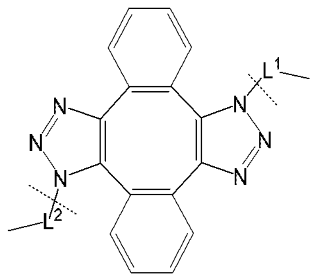 |
| (TZ-5) | 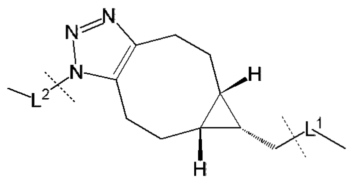 | (TZ-5-r) | 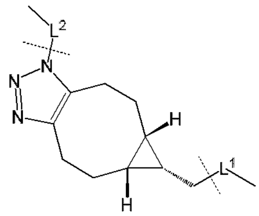 |
| (TZ-6) | 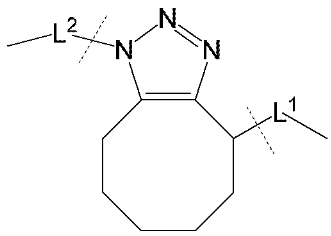 | (TZ-6-r) | 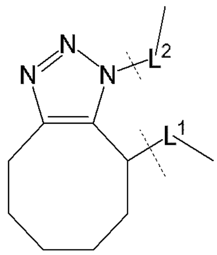 |

[Table 11-2]

| No. | X | No. | X |
|---|---|---|---|
| (TZ-7) | 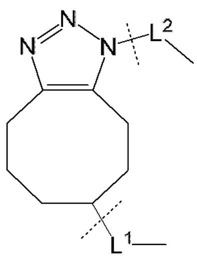 | (TZ-7-r) | 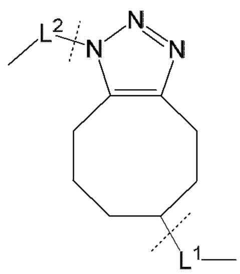 |
| (TZ-8) | 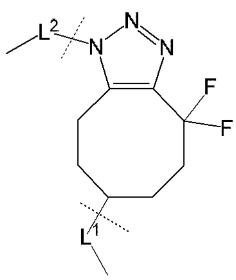 | (TZ-8-r) | 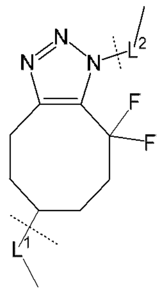 |

(continued)

| No. | X | No. | X |
|---|---|---|---|
| (TZ-9) | 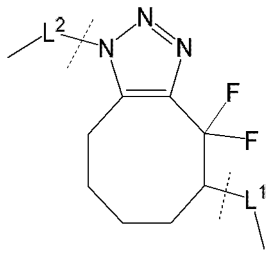 | (TZ-9-r) | 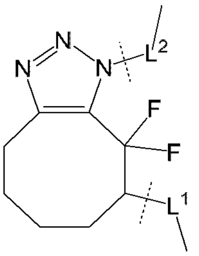 |
| (TZ-10) | 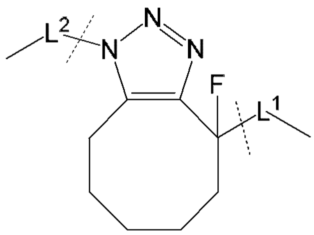 | (TZ-10-r) | 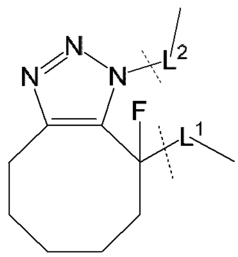 |
| (TZ-11) | 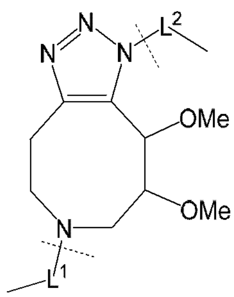 | (TZ-11-r) | 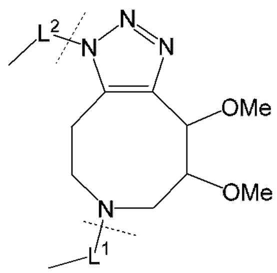 |
| (TZ-12) | 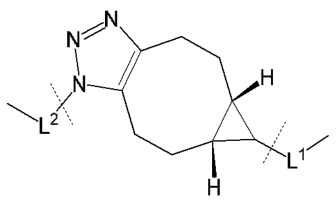 | (TZ-12-r) | 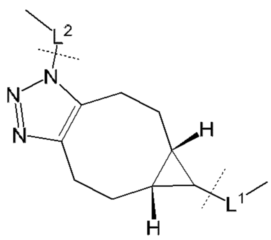 |

[5-2] The structure described in [5-1], in which the chemically crosslinked alginic acid is a chemically crosslinked alginic acid in which the combination of -X-$L^1$- in Formula (H-III-L) is selected from formulae in the following table:

[Table 12]

| -X- | -$L^1$- |
|---|---|
| (TZ-6) or (TZ-6-r) | Any one linker selected from the group of (LN-4-X), (LN-9-X), (L1-1-X), (L1-2-X), (L1-3-X1), (L1-3-X2), (L1-4-X1), (L1-4-X2), (L1-5-X), (L1-6-X), (L1-8-X1), (L1-8-X2), (LN-3-X1), (LN-3-X2) or (L1-7-X) |
| (TZ-2) or (TZ-2-r) | Any one linker selected from the group of (LN-3-X1), (LN-3-X2) or (L1-7-X) |
| (TZ-5) or (TZ-5-r) | Any one linker selected from the group of (LN-4-X), (LN-9-X), (L1-1-X), (L1-2-X), (L1-3-X1), (L1-3-X2), (L1-4-X1), (L1-4-X2), (L1-5-X), (L1-6-X), (L1-8-X1), (L1-8-X2), (LN-3-X1), (LN-3-X2) or (L1-7-X) |

(here, -$L^1$- in the table is the same as the definition in [2-2], and each reference symbol represented by -X- is the same as the partial structural formulae [in each formula, the outsides of the broken lines at both ends are not included] shown

in the table in [5-1]), and - $L^2$- is selected from structural formulae in [2-2].

[5-3] The structure described in [5-2], in which -x- in Formula (H-III-L) is (TZ-2) or (TZ-6).

[5-4] The structure described in [5-1], in which -X- in Formula (H-III-L) is (TZ-6) and -$L^1$- is (LN-3) or (LN-9) or -X- is (TZ-2 ) and -$L^1$- is (LN-3), and -$L^2$- is (LK-2), (LK-4), (LK-5) or (LK -7) in the table in [2-2].

[5-5] The structure described in [5-2], in which -X- in Formula (H-III-L) is (TZ-6) and -$L^1$- is (LN-3-X1) or (LN-9X) in the table in [2-2] or -X- is (TZ-2) and -$L^1$- is (LN-3-X1) and -$L^2$- is (LK-2-X), (LK-4-X), (LK-5-X1) or (LK-7-X1) in the table in [2-2].

[5-6] The structure described in [5], in which -$L^1$- in Formula (H-III-L) is selected from the partial structural formulae [in each formula, the outsides of the broken lines at both ends are not included] shown in the table in [2-14], -$L^2$- is selected from the partial structural formulae [in each formula, the outsides of the broken lines at both ends are not included] shown in the table in [2-14], and -X- is selected from the partial structural formulae [in each formula, the outsides of the broken lines at both ends are not included] shown in the table in [5-1].

[5-7] The structure described in [5], in which -$L^1$- in Formula (H-III-L) is selected from the partial structural formulae [in each formula, the outsides of the broken lines at both ends are not included] shown in the table in [2-15], -$L^2$- is selected from the partial structural formulae [in each formula, the outsides of the broken lines at both ends are not included] shown in the table in [2-15], and -X- is selected from the partial structural formulae [in each formula, the outsides of the broken lines at both ends are not included] shown in the table in [5-1].

[6-1] The structure described in [5-1], in which the chemically crosslinked alginic acid is a chemically crosslinked alginic acid in which an arbitrary carboxyl group of a first alginic acid and an arbitrary carboxyl group of a second alginic acid are crosslinked through a group represented by Formula (HB-III-L) below:

[C26]

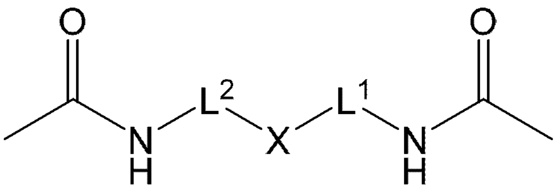

(HB-III-L)

[in Formula (HB-III-L), -CONH- and -NHCO- at both ends represent amide bonds through arbitrary carboxyl groups of the alginic acid;

-$L^1$- represents a linker selected from the group consisting of partial structural formulae (LN-3), (LN-9) and (L1-3) to (L1-10) in the partial structural formulae of -$L^1$-shown in the table in [2-1] [in each formula, the outsides of the broken lines at both ends are not included];

-$L^2$- represents a linker selected from the group consisting of partial structural formulae (LK-2) and (L2-1) to (L2-9b) in the partial structural formulae of -$L^2$- shown in the table in [2-1] [in each formula, the outsides of the broken lines at both ends are not included];

-X- is the same meaning as the partial structural formulae [in each formula, the outsides of the broken lines at both ends are not included] shown in the table in [5-1], however, a group for which -$L^1$- is (LN-3) or (LN-9) and -$L^2$- is (LK-2) is excluded].

[6-2] The structure described in [6-1], in which -$L^1$- in Formula (HB-III-L) is selected from the partial structural formulae [in each formula, the outsides of the broken lines at both ends are not included] shown in the table in [3-2], -$L^2$- is selected from the partial structural formulae [in each formula, the outsides of the broken lines at both ends are not included] shown in the table in [3-2], and -X- is selected from the partial structural formulae [in each formula, the outsides of the broken lines at both ends are not included] shown in the table in [5-1].

[6-3] The structure described in [6-2], in which -$L^2$- in Formula (HB-III-L) is selected from the partial structural formulae [in each formula, the outsides of the broken lines at both ends are not included] shown in the table in [3-3].

[6-4] The structure described in [6-3], in which -$L^2$- in Formula (HB-III-L) is selected from the partial structural formulae [in each formula, the outsides of the broken lines at both ends are not included] shown in the table in [3-4].

[6-5] The structure described in [6-3], in which -$L^2$- in Formula (HB-III-L) is selected from the partial structural formulae [in each formula, the outsides of the broken lines at both ends are not included] shown in the table in [3-5].

[6-6] The structure described in [6-3] to [6-5], in which -$L^1$- in Formula (HB-III-L) is selected from the partial structural formulae [in each formula, the outsides of the broken lines at both ends are not included] shown in the table in [3-6].

[7] The structure described in [1], in which the chemically crosslinked alginic acid is a chemically crosslinked alginic acid

in which an arbitrary carboxyl group of a first alginic acid and an arbitrary carboxyl group of a second alginic acid are crosslinked through a group represented by Formula (HA-III-L) below:

[C27]

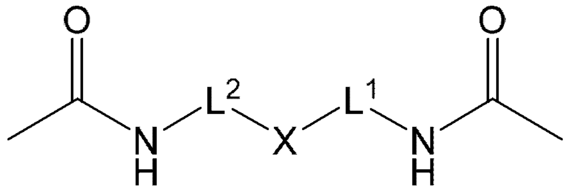

(HA-III-L)

[in Formula (HA-III-L), -CONH- and -NHCO- at both ends represent amide bonds through arbitrary carboxyl groups of the alginic acid;
$-L^1-$ is the same as the definition in [4];
$-L^2-$ is the same as the definition in [4];
X is the same as the definition in [5-1].

[8-1] The structure described in [1], in which the chemically crosslinked alginic acid is a chemically crosslinked alginic acid that is obtained by light exposure of a chemically modified alginic acid derivative into which a photoreactive group represented by Formula (C-I) has been introduced:

[chemically modified alginic acid derivative into which the photoreactive group represented by Formula (C-I) has been introduced]
a chemically modified alginic acid derivative in which a photoreactive group represented by Formula (C-1) below [in the formula, the right outside of the broken line is not included] is introduced into one or more arbitrary carboxyl groups of alginic acid.

[C28]

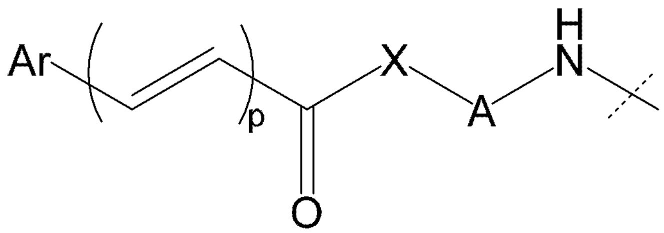

(C-I)

[in Formula (C-I),

Ar represents a $C_{6-10}$ aryl group (in the $C_{6-10}$ aryl group, one to three groups arbitrarily selected from a hydroxyl group, a cyano group, a nitro group, a halogen atom, a $C_{1-6}$ alkyl group, a halogenated $C_{1-6}$ alkyl group, a $C_{1-6}$ alkoxy group, and an $-NR^AR^B$ group ($R^A$ and $R^B$ in the $-NR^AR^B$ group are each independently a group selected from a hydrogen atom, a $C_{1-6}$ alkyl group, a $C_{2-7}$ alkanoyl group or a $C_{1-6}$ alkylsulfonyl group (here, $-NH_2$, $-NH(C_{1-6}$ alkyl group) and $-N(C_{1-6}$ alkyl group$)_2$ are excluded)) may be substituted with a hydrogen atom on a ring, in a case where the $C_{1-6}$ alkyl group and the $C_{1-6}$ alkoxy group or two $C_{1-6}$ alkoxy groups are included adjacent to each other in the $C_{6-10}$ aryl group as substituents, the $C_{1-6}$ alkyl group and the $C_{1-6}$ alkoxy group or the two $C_{1-6}$ alkoxy groups may bond to each other between carbon atoms in alkyl groups of the respective groups, from which one arbitrary hydrogen atom has been eliminated, or between carbon atoms in the respective alkyl groups of the two $C_{1-6}$ alkoxy groups, from which one arbitrary hydrogen atom has been eliminated, to form a cyclic ether or, in a case where the $C_{1-6}$ alkoxy group and an $-NHR^G$ group ($R^G$ in the $-NHR^G$ group is a $C_{2-7}$ alkanoyl group or a $C_{1-6}$ alkylsulfonyl group) are included adjacent to each other in the $C_{6-10}$ aryl group as substituents, carbon atoms in the alkyl group of the $C_{1-6}$ alkoxy group, from which one arbitrary hydrogen atom has been eliminated, and nitrogen atoms in the $-NHR^G$ group, from which one hydrogen atom has been eliminated, may bond to each other to form a 3-N-($C_{2-7}$ alkanoyl)oxazolidine ring, a 3-N-($C_{1-6}$ alkylsulfonyl)ox-

azolidine ring, a 4-N-($C_{2-7}$ alkanoyl)morpholine ring, a 4-N-($C_{1-6}$ alkylsulfonyl) morpholine ring, a 4-N-($C_{2-7}$ alkanoyl)-1,4-oxazepane ring or a 4-N-($C_{1-6}$ alkylsulfonyl)-1,4-oxazepane ring);
p represents an integer of 1 or 2;
-X- represents -O-;
-A- is Formulae (AL-1) to (AL-4) [in each formula, the outsides of the broken lines at both ends are not included]:

[C29]

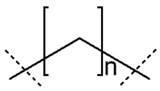

(AL-1)

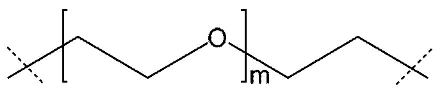

(AL-2)

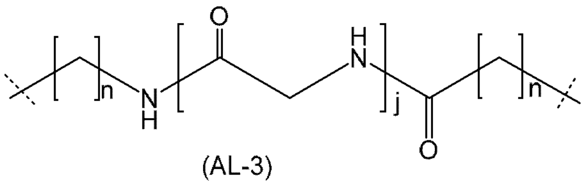

(AL-3)

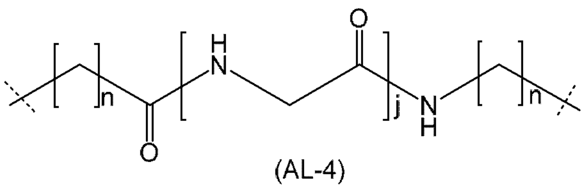

(AL-4)

(in Formulae (AL-1) to (AL-4), n represents an integer of 1 to 18; m represents an integer of 1 to 9; j represents an integer of 0 to 9;
a plurality (for example, one to 10 or one to five) of hydrogen atoms in a methylene group ($-CH_2-$) in Formulae (AL-1) to (AL-4) may be substituted with groups selected from a halogen atom, a hydroxyl group, a $C_{1-6}$ alkyl group, a hydroxy $C_{1-6}$ alkyl group, a thiol $C_{1-6}$ alkyl group, a $C_{1-6}$ alkylthio $C_{1-6}$ alkyl group, a carboxy $C_{1-6}$ alkyl group, an $-NR^aR^b$ group, an $(R^aR^bN)$-$C_{1-6}$ alkyl group, an $(R^aR^bN)C(=O)$-$C_{1-6}$ alkyl group (in the $-NR^aR^b$ group, the $(R^aR^bN)$-$C_{1-6}$ alkyl group or the $(R^aR^bN)C(=O)$-$C_{1-6}$ alkyl group, $R^a$ and $R^b$ are each independently a group selected from a hydrogen atom, a $C_{1-6}$ alkyl group, a $C_{2-7}$ alkanoyl group or a $C_{1-6}$ alkylsulfonyl group), a guanidino $C_{1-6}$ alkyl group, a $C_{7-16}$ aralkyl group, a hydroxy $C_{6-10}$ aryl $C_{1-6}$ alkyl group or a heteroaryl $C_{1-6}$ alkyl group; in a case where two hydrogen atoms in the methylene group ($-CH_2-$) in Formulae (AL-1) to (AL-4) are substituted with the $C_{1-6}$ alkyl groups, the $C_{1-6}$ alkyl groups may bond to each other between carbon atoms of the respective alkyl groups of the respective $C_{1-6}$ alkyl groups, from which one arbitrary hydrogen atom has been eliminated, to form a $C_{3-8}$ cycloalkyl ring; the -NH- groups in Formula (AL-3) or Formula (AL-4) may form a non-aromatic heterocycle together with the substituent substituting an adjacent carbon atom)] (here, in Formula (C-I), in a case where Ar is a phenyl group, p is one, -A- is Formula (AL-1) and n is three, in the phenyl group of Ar, one to three groups arbitrarily selected from a group of substituents that may substitute hydrogen atoms on the Ar ring substitute the hydrogen atoms on the phenyl ring).

[8-2] The structure described in [8-1], comprising a chemically crosslinked alginic acid that is obtained by light exposure of, "the chemically modified alginic acid derivative into which the photoreactive group has been introduced" in which, in Formula (C-I),

Ar is a phenyl group (in the phenyl group, one to three groups arbitrarily selected from a group of a cyano group, a fluorine atom, a trifluoromethyl group and a methoxy group may substitute hydrogen atoms on a ring, in a case where two methoxy groups are included adjacent to each other as substituents in the phenyl group, the methoxy groups may bond to each other between carbon atoms of the methyl groups of the respective groups of the two methoxy groups, from which one arbitrary hydrogen atom has been eliminated, to form a 1,4 dioxane ring) (here, in Formula (C-I), in a case where Ar is a phenyl group, p is one, -A- is Formula (AL-1) and n is three, in the phenyl group of Ar, one to three groups arbitrarily selected from a group of substituents that may substitute hydrogen atoms on the phenyl group substitute the hydrogen atoms on the phenyl group);
p is an integer of 1 or 2;
-X- is -O-;
-A- is (AL-1) or (AL-2) [in each formula, the outsides of the broken lines at both ends are not included] in the embodiment [8-1].

[8-3] The structure described in [8-2], comprising a chemically crosslinked alginic acid that is obtained by light exposure of "the chemically modified alginic acid derivative into which the photoreactive group has been introduced" in which, in Formula (C-I),

Ar is a phenyl group, a 4-fluorophenyl group, a 4-(trifluoromethyl)phenyl group, a 4-methoxyphenyl group, a 4-cyanophenyl group or a 2,3-dihydrobenzo[b][1,4]dioxinyl group.

[8-4] The structure described in [8-2], comprising a chemically crosslinked alginic acid that is obtained by light exposure of a chemically modified alginic acid derivative into which a photoreactive group has been introduced that is selected from partial structural formulae [in each formula, the right side of the broken line is not included] shown below in Formula (C-I),

[C30]

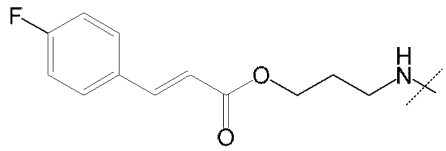
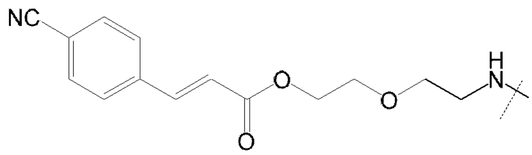
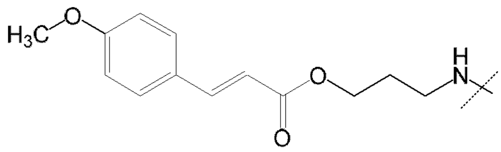
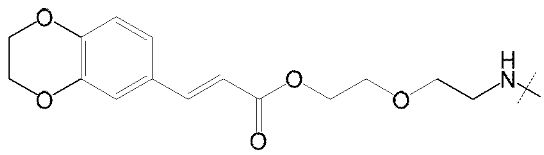
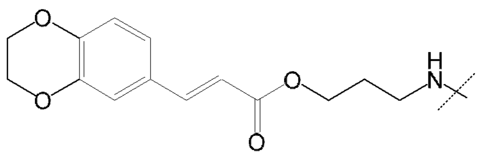
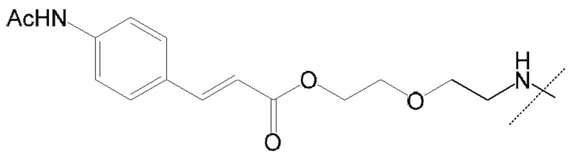
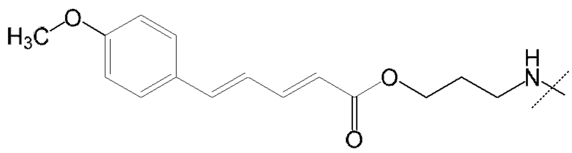
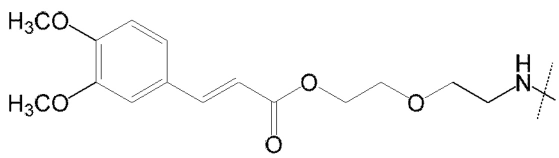
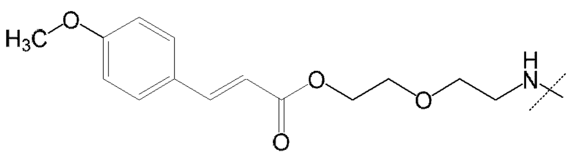
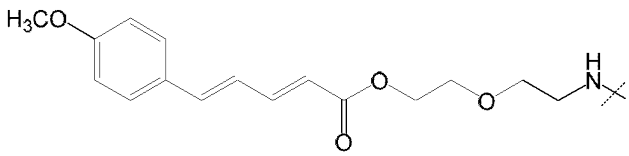

[8-5] The structure described in any one of [8-1] to [8-4], in which the light used for exposure is light selected from an ultraviolet ray or an LED light.

[9-1] The structure described in [1], in which the chemically crosslinked alginic acid is a chemically crosslinked alginic acid in which an arbitrary carboxyl group of a first alginic acid and an arbitrary carboxyl group of a second alginic acid are crosslinked through a group represented by Formula (C-II-L-1) or Formula (C-II-L-2) below:

[C31]

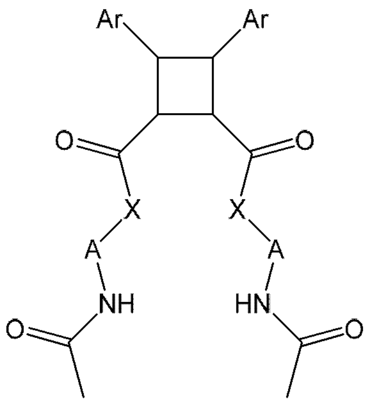

(C-II-L-1)

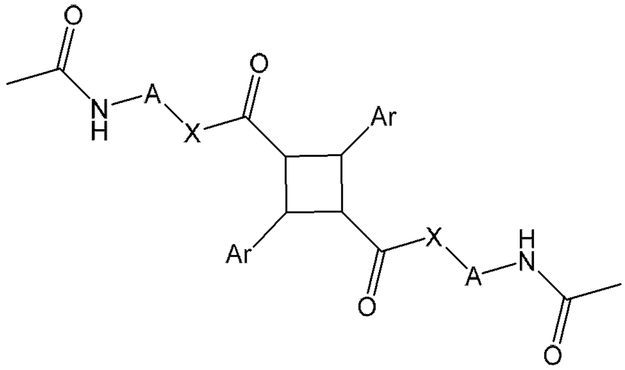

(C-II-L-2)

[in Formula (C-II-L-1) or Formula (C-II-L-2), -CONH- and -NHCO- at both ends represent amide bonds through an arbitrary carboxyl group of alginic acid; -A-, -X- and Ar are the same as the definitions in any one of [8-1] to [8-3]].

[9-2] The structure described in [1], in which the chemically crosslinked alginic acid is a chemically crosslinked alginic acid in which an arbitrary carboxyl group of a first alginic acid and an arbitrary carboxyl group of a second alginic acid are crosslinked through a group represented by Formula (C-II-L-3) below:

[C32]

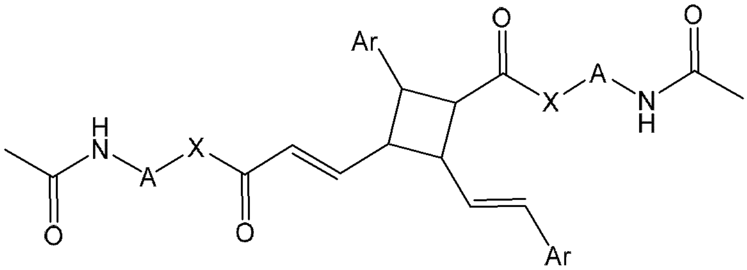

(C-II-L-3)

[in Formula (C-II-L-3), -CONH- and -NHCO- at both ends represent amide bonds through an arbitrary carboxyl group of alginic acid; -A-, -X- and Ar are the same as the definitions in any one of [8-1] to [8-3]].

[10-1] A method for manufacturing the structure comprising a pharmacological ingredient embedded in a chemically crosslinked alginic acid in the core layer, which is described in [2], comprising steps (a) to (c) below.

step (a): a step of bringing a solution of a chemically modified alginic acid derivative comprising a pharmacological ingredient [a mixture of the derivative represented by Formula (H-I) and the derivative represented by Formula (H-II)] into contact with a solution having a divalent metal ion,

step (b): a step of bringing gel obtained in the step (a) into contact with a solution comprising a cationic polymer to coat the gel with the cationic polymer, and

step (c): a step of bringing a manufactured product obtained in the step (b) into contact with a solution comprising an anionic polymer to further coat the gel with the anionic polymer.

[10-2] The method for manufacturing the structure described in [10-1], further comprising a step (d) below:

step (d): a step of performing a chelate treatment on a structure obtained in the step (c) with a chelating agent.

[10-3] The method for manufacturing the structure described in [10-1] or [10-2], in which the structure is the structure described in any one of [2-1] to [7].

[11-1] A method for manufacturing the structure comprising a pharmacological ingredient embedded in a chemically crosslinked alginic acid, comprising steps (a) to (c) below and light exposure in any stage after the step (a).

step (a): a step of bringing a solution of a chemically modified alginic acid derivative comprising a pharmacological ingredient into contact with a solution having a divalent metal ion to gelate,

step (b): a step of bringing gel obtained in the step (a) into contact with a solution comprising a cationic polymer to

coat the gel with the cationic polymer, and
step (c): a step of bringing a manufactured product obtained in the step (b) into contact with a solution comprising an anionic polymer to further coat the gel with the anionic polymer.

[11-2] The method for manufacturing the structure described in [11-1], further comprising a step (d) below:
step (d): a step of performing a chelate treatment on a structure obtained in the step (c) with a chelating agent.
[11-3] The method for manufacturing the structure described in [11-1] or [11-2], in which the structure is the structure described in any one of [8-1] to [9-2].
[12-1] The structure described in any one of [1] to [9-2], in which the anionic polymer is alginic acid or a chemically crosslinked alginic acid.
[12-2] The structure described in any one of [1] to [9-2], in which the anionic polymer is alginic acid.
[12-3] The structure described in any one of [1] to [9-2], in which the anionic polymer is the same chemically crosslinked alginic acid as one used in the core layer.
[12-4] The structure described in any one of [1] to [9-2], in which the anionic polymer is selected from chemically crosslinked alginic acids having a structural formula shown in any one of [1] to [9-2].
[12-5] The structure described in any one of [1] to [9-2] or [12-1] to [12-4], in which the pharmacological ingredient embedded in the chemically crosslinked alginic acid in the core layer is a cell. As a certain embodiment, the structure in which the cell is an insulin-secreting cell. As a separate embodiment, the structure described in any one of [1] to [9-2] or [12-1] to [12-4], in which the pharmacological ingredient is a bioactive substance-producing cell, as a separate embodiment, the structure described in any one of [1] to [9-2] or [12-1] to [12-4], in which the pharmacological ingredient is a physiologically active substance-producing cell, and, as a separate embodiment, the structure described in any one of [1] to [9-2] or [12-1] to [12-4], in which the pharmacological ingredient is a physiologically active natural substance-producing cell.
[12-6] The structure described in any one of [1] to [9-2] or [12-1] to [12-5], in which the cationic polymer is poly-L-ornithine or poly-L-lysine.
[12-7] The structure described in any one of [1] to [9-2] or [12-1] to [12-6], in which the cationic polymer is poly-L-omithine.
[12-8] The structure described in any one of [1] to [9-2] or [12-1] to [12-7], in which the shape of the structure is a bead, a sheet or a fiber. Preferably, the structure described in any one of [1] to [9-2] or [12-1] to [12-7], in which the shape is a bead. As a separate embodiment, the structure described in any one of [1] to [9-2] or [12-1] to [12-7], in which the shape is a sheet. As a still separate embodiment, the structure described in any one of [1] to [9-2] or [12-1] to [12-7], in which the shape is a fiber.
[12-9] The structure described in any one of [1] to [9-2] or [12-1] to [12-8] manufactured using alginic acid or a chemically modified alginic acid derivative having a weight-average molecular weight measured by gel filtration chromatography of 100,000 Da to 3,000,000 Da.
[12-10] The structure described in any one of [1] to [9-2] or [12-1] to [12-9] for an in vivo use.
[12-11] A medical material comprising, as an effective ingredient, a pharmacological ingredient encapsulated in the structure described in any one of [1] to [9-2] or [12-1] to [12-9].
[12-12] The medical material described in [12-11] that is used for a wound dressing, a postoperative adhesion barrier, a sustained drug release substrate, a cell transplantation substrate, a prosthetic material, a formulation coating material or a bio ink for bioprinter. As the prosthetic material, an implant or an artificial organ is preferable.
[13-1] The manufacturing method described in any one of [10-1] to [11-3] for manufacturing a structure, in which the anionic polymer is alginic acid or a chemically crosslinked alginic acid.
[13-2] The manufacturing method described in any one of [10-1] to [11-3] for manufacturing a structure, in which the anionic polymer is alginic acid.
[13-3] The manufacturing method described in any one of [10-1] to [11-3] for manufacturing a structure, in which the anionic polymer is the same chemically crosslinked alginic acid as one used in the core layer.
[13-4] The manufacturing method described in any one of [10-1] to [11-3] for manufacturing the structure described in any one of [1] to [9-2], in which the anionic polymer is selected from chemically crosslinked alginic acids described in any one of [1] to [9-2].
[13-5] The manufacturing method described in any one of [10-1] to [11-3] or [13-1] to [13-4] for manufacturing a structure, in which the pharmacological ingredient embedded in the chemically crosslinked alginic acid in the core layer is a cell. As a certain embodiment, the method for manufacturing the structure, in which the cell is an insulin-secreting cell.
[13-6] The manufacturing method described in any one of [10-1] to [11-3] or [13-1] to [13-5] for manufacturing the structure, in which the cationic polymer is poly-L-ornithine or poly-L-lysine.
[13-7] The manufacturing method described in any one of [10-1] to [11-3] or [13-1] to [13-6] for manufacturing the structure, in which the cationic polymer is poly-L-ornithine.
[13-8] The manufacturing method described in any one of [10-1] to [11-3] or [13-1] to [13-7] for manufacturing the

structure, in which the shape of the structure is a bead, a sheet or a fiber. Preferably, the manufacturing method described in any one of [10-1] to [11-3] or [13-1] to [13-7] for manufacturing the structure, in which the shape of the structure is a bead. As a separate embodiment, the manufacturing method described in any one of [10-1] to [11-3] or [13-1] to [13-7] for manufacturing the structure, in which the shape of the structure is a sheet. As a still separate embodiment, the manufacturing method described in any one of [10-1] to [11-3] or [13-1] to [13-7] for manufacturing the structure, in which the shape of the structure is a fiber.

[13-9] The manufacturing method described in any one of [10-1] to [11-3] or [13-1] to [13-8] for manufacturing a structure manufactured using alginic acid or a chemically modified alginic acid derivative having a weight-average molecular weight measured by gel filtration chromatography of 100,000 Da to 3,000,000 Da.

As a structure of a preferable embodiment of the present invention, the following examples are exemplified.

[14-1] The structure described in [1] to [7], in which the pharmacological ingredient is a bioactive substance-producing cell, the cationic polymer is poly-L-ornithine or poly-L-lysine, the anionic polymer is alginic acid or the same chemically crosslinked alginic acid as one used in the core layer, and the shape of the structure is a bead or a sheet.

[14-2] A medical material comprising, as an effective ingredient, the pharmacological ingredient encapsulated in the structure described in [1] to [7], in which the pharmacological ingredient is a bioactive substance-producing cell, the cationic polymer is poly-L-ornithine or poly-L-lysine, and the anionic polymer is alginic acid or the same chemically crosslinked alginic acid as one used in the core layer.

[14-3] The structure described in [1] to [7] that is used to be detained in vivo to exhibit the function of the pharmacological ingredient, in which the cationic polymer is poly-L-ornithine or poly-L-lysine, and the anionic polymer is alginic acid or the same chemically crosslinked alginic acid as one used in the core layer.

[14-4] A method for treating a patient in a human being or an animal by detaining in vivo the structure described in [1] to [7], in which the pharmacological ingredient is a bioactive substance-producing cell, the cationic polymer is poly-L-ornithine or poly-L-lysine, and the anionic polymer is alginic acid or the same chemically crosslinked alginic acid as one used in the core layer. Particularly, a treatment method in which the structure is used for wound dressing, postoperative adhesion prevention, sustained drug release, cell transplantation or a prosthetic material for such as an implant or an artificial organ.

[Advantageous Effects of Invention]

The present invention provides a novel structure. Preferably, the structure exhibits at least one of the following effects.

(1) The structure exhibits a high physical strength even under conditions where there is no intermolecular crosslinking of alginic acid by a divalent metal ion compared with a similar structure in which alginic acid is used as a core layer (hereinafter, abbreviated as a conventional structure).
(2) The structure can be produced to have a larger size than the conventional structure.
(3) It is also possible to produce large structures with a free shape, such as a flat plate or a fiber, and these structures are capable of maintaining structural stability for a long period of time.
(4) In a case where cells are encapsulated in the core layer, the structure enables the cells to survive for a long period of time.
(5) The structure can be used for the purpose of storing, culturing or the like cells or the like in the core layer.
(6) The structure can also be used for the transplantation or the like of pancreatic islets or device cells for cell transplantation.
(7) The structure has biocompatibility and thus can be applied to a variety of in vivo uses.

[Brief Description of Drawings]

[Fig. 1]
Fig. 1 is a view showing the evaluation of the stability of a chemically crosslinked alginic acid.
[Fig. 2]
Fig. 2 is a view showing the evaluation of the stability of the chemically crosslinked alginic acid under EDTA.
[Fig. 3]
Fig. 3 is a view showing the evaluation of the stability of a chemically crosslinked alginic acid.
[Fig. 4]
Fig. 4 is a view showing the evaluation of the stability of the chemically crosslinked alginic acid under EDTA.
[Fig. 5]

Fig. 5 is a view showing the evaluation of the stability of a chemically crosslinked alginic acid.
[Fig. 6]
Fig. 6 is a view showing the evaluation of the stability of the chemically crosslinked alginic acid under EDTA.
[Fig. 7]
Fig. 7 is a view showing the evaluation of the permeability of a chemically crosslinked alginic acid.
[Fig. 8]
Fig. 8 is a view showing the evaluation of the permeability of a chemically crosslinked alginic acid.
[Fig. 9]
Fig. 9 is a view showing the biocompatibility evaluation of a chemically crosslinked alginic acid.
[Fig. 10]
Fig. 10 is views showing the evaluation of the stability of a structure according to Example A-6 after a chelate treatment (a) and after one day of shaking (b).
[Fig. 11]
Fig. 11 is views showing the evaluation of the stability of a structure according to Example A-3 after a chelate treatment (a) and after one day of shaking (b).
[Fig. 12]
Fig. 12 is views showing the evaluation of the stability of a structure according to Example A-2 after a chelate treatment (a) and after one day of shaking (b).
[Fig. 13]
Fig. 13 is a view showing the evaluation of the stability of a structure according to Example D-1 after one day of shaking.
[Fig. 14]
Fig. 14 is a view showing the evaluation of the stability of a structure according to Example D-2 after one day of shaking.
[Fig. 15]
Fig. 15 is a view showing the evaluation of the stability of a structure according to Example D-3 after one day of shaking.
[Fig. 16]
Fig. 16 is a view showing the evaluation of the stability of a structure according to Example D-4 after one day of shaking.
[Fig. 17]
Fig. 17 is a view showing the evaluation of the stability of structures according to Example D(2)-1 and Example D(2)-2 after one day of shaking.

[Description of Embodiments]

Hereinafter, each embodiment of the present invention will be described in more detail.

1. Multilayer structure using chemically crosslinked alginic acid

In the present specification, "structure" is a multilayer structure using chemically crosslinked alginic acid that is capable of encapsulating a pharmacological ingredient inside. A structure to be provided herein is preferably a three-layer structure comprising a core layer in which a pharmacological ingredient is embeded, a cationic polymer layer and an anionic polymer layer, and, unless particularly limited, "structure" means a structure comprising a core layer comprising a pharmacological ingredient embedded in a chemically crosslinked alginic acid, a cationic polymer layer coating the core layer, and an anionic polymer layer coating the cationic polymer layer.

In the present specification, "a chemical crosslinking reaction is performed" or "a chemical crosslinking reaction is applied" means that a chemical crosslink (chemical bond) is formed between a chemically modified alginic acid derivative represented by Formula (H-I), Formula (HA-I) or Formula (HB-I) and a chemically modified alginic acid derivative represented by Formula (H-II), Formula (HA-II) or Formula (HB-II) by performing a Huisgen reaction by using these chemically modified alginic acid derivatives or that a chemical crosslink (chemical bond) is formed between chemically modified alginic acid derivatives represented by Formula (C-I) by performing a photocyclization reaction by light exposure to the chemically modified alginic acid derivatives represented by Formula (C-I) into which a photoreactive group has been introduced.

A chemically crosslinked alginic acid configuring the structure of the present invention will be described below in detail but is an alginic acid in which a chemical crosslink has been formed by performing a crosslinking reaction between "chemically modified alginic acid derivatives" in which a reactive group has been condensed into an arbitrary carboxyl group of alginic acid by using the chemically modified alginic acid derivatives.

On the other hand, it is known that, when a sugar polymer such as alginic acid comes into contact with a divalent metal ion, an ionic crosslinking is formed between two carboxyl groups, and a hydrogel is formed. The chemically crosslinked alginic acid configuring the structure of the present invention also has a similar property. Therefore, in the chemically crosslinked alginic acid configuring the structure of the present invention, two states of (i) a state where a

crosslink (ionic crosslinking) by a divalent metal ion is not formed (for example, a state where a bond with a divalent metal ion is removed by making a chelating agent such as EDTA present) and (ii) a state where both a chemical crosslink and an ionic crosslinking have been formed and a state where these states are present together can be present. When the structure of the present invention is jointly used with an ionic crosslinking, a strong gel can be produced and used, and it is possible to hold a stable structure even in a state where there is no ionic crosslinking. Unless particularly otherwise described, in the case of being mentioned in the present specification, "chemically crosslinked alginic acid" include all chemically crosslinked alginic acids in any state.

1-1. Core layer comprising pharmacological ingredient embedded in chemically crosslinked alginic acid

Examples of a pharmacological ingredient that can be encapsulated in the core layer include, in addition to substances having a pharmacological activity or a biological activity, that is, bioactive substances, cells and microorganisms

The pharmacological ingredient that is encapsulated in the core layer needs to be at least one kind of a pharmacological ingredient, but a plurality of kinds of pharmacological ingredients may be encapsulated at the same time, and, in the case of a plurality of kinds of pharmacological ingredients, the pharmacological ingredients each may have an independent activity, the pharmacological ingredients may interact with each other having an activity or the pharmacological ingredients may be a combination of a main ingredient and an auxiliary ingredient of an activity. In addition, the pharmacological ingredients may be a combination of a plurality of kinds of bioactive substances, a combination of a plurality of kinds of cells, a combination of a plurality of kinds of microorganisms or a combination of one kind or a plurality of kinds of bioactive substances, cells or microorganisms.

The bioactive substance that can be encapsulated in the core layer is not particularly limited, bioactive substances that are used as drugs are preferable, and examples thereof include small molecule drugs, medium molecule drugs such as peptides and oligonucleic acids, biopharmaceuticals such as proteins, bioactive substances and the like. In the present specification, examples of the bioactive substance include, in addition to bioactive substances that living bodies originally have (bioactive natural substances), substances obtained by modifying or altering bioactive substances, substances that activate or inhibit bioactivity and the like, naturally occurring substances and substances produced in a genetically engineered manner or chemically synthesized substances are also included, and prodrugs thereof are also included. Specific examples thereof include vitamins, coenzymes, hormones, antibiotics, neurotransmitters, cytokines, enzymes, growth factors, antibodies and other in vivo factors, and more specific examples of the in vivo factors include insulin, dopamine, factor VIII, factor IX and the like.

The cell that can be encapsulated in the core layer is not particularly limited, cells capable of secreting bioactive substances (bioactive substance-producing cells) are preferable, not only natural cells but also cells on which an artificial modifying operation has been performed are included, and cell masses composed of a plurality of cells are also included. Examples of the cell that can be encapsulated in the core layer include cells for transplantation. Examples of the cells for transplantation include cells capable of secreting hormone such as insulin, and specific examples thereof include insulin-secreting cells, pancreatic islets, pancreatic islet cells and the like. In addition, examples of other cells capable of secreting bioactive substances include a dopamine-secreting cell, a pituitary cell, a growth hormone-secreting cell, a parathyroid cell, a nerve growth factor-secreting cell, a blood coagulation factor-secreting cell, a hepatocyte, a parathyroid cell, an erythropoietin-secreting cell, a norepinephrine-secreting cell and the like. Bioactive substance-producing cells (physiologically active substance-producing cells) are preferable. A separate embodiment is bioactive natural substance-producing cells.

"Insulin-secreting cell" means a cell having an insulin-secreting function and, for example, means a β cell that secrete insulin in cells constituing a pancreatic islet. In addition, "insulin-secreting cell" may be a cell given an insulin-secreting function by differentiation, maturation, alteration or the like, and, for example, cells having an insulin-secreting function obtained by differentiating a stem cell such as an iPS cell, an ES cell or a somatic stem cell (for example, a mesenchymal stem cell), cells having an insulin-secreting function obtained by maturing a juvenile cell or a progenitor cell and cells given an insulating-secreting function by genetic recombination also can be included. Here, the differentiation or maturation of the cell includes the culture of the cell, that is, cells obtained by differentiation or maturation may include cells obtained by culturing.

"Pancreatic islet" is a cell mass composed of an average of approximately 2000 pancreatic islet cells, which is also referred to as a separate name of islets of Langerhans. The pancreatic islet is composed of five kinds of cells: an α-cell that secretes glucagon, a β-cell that secretes insulin, a δ-cell that secretes somatostatin, an ε-cell that secretes ghrelin, and a PP (pancreatic polypeptide) cell that secretes pancreatic polypeptide.

In the present specification, "pancreatic islet cell" may be a cell comprising at least one kind of cell of the above-described five kinds of cells constituing the pancreatic islet, but preferably comprises at least the β-cell. In several embodiments, the pancreatic islet cell may be a mixture comprising all of the α-cell, the β-cell, the δ-cell, the ε-cell and the PP cell and may be a cell contained in the pancreatic islet.

In addition, "pancreatic islet cell" may be a cell that has become a pancreatic islet cell by differentiation, maturation, alteration or the like. In this case, "pancreatic islet cell" may also include, for example, a pancreatic islet cell obtained by differentiating a stem cell such as an iPS cell, an ES cell or a somatic stem cell (for example, a mesenchymal stem cell) and a pancreatic islet cell obtained by maturing a juvenile cell or a progenitor cell.

In the case of use for transplantation, "insulin-secreting cell" or "pancreatic islet (comprising the pancreatic islet cell)" preferably has viability and functions favorable enough to recover the patient’s morbidity when transplanted into a patient. Examples of the functions of the insulin-secreting cell, the pancreatic islet or the pancreatic islet cell include secretion of insulin, and it is preferable that glucose responsiveness be maintained even after transplantation.

Donors of "insulin-secreting cell", "pancreatic islet" or "pancreatic islet cell" are animals, preferably vertebrates and more preferably mammals, specific examples thereof include humans, pigs, monkeys, rats, mice and the like, and human or pig is still more preferable. In several aspects, the donors of "insulin-secreting cell", "pancreatic islet" or "pancreatic islet cell" are pigs from the viewpoint of donor shortage elimination. "Insulin-secreting cell", "pancreatic islet" or "pancreatic islet cell" may be any of a pancreatic islet or pancreatic islet cell obtained from an animal, which is a donor, or an insulin-secreting cell or pancreatic islet cell obtained from a donor-derived cell and may be, for example, an insulin-secreting cell or pancreatic islet cell differentiated from a human-derived ES cell or iPS cell.

In a case where "insulin-secreting cell", "pancreatic islet" or "pancreatic islet cell" is derived from a pig, insulin-secreting cells or pancreatic islet cells obtained from an adult porcine islet, a fetal, neonatal or perinatal porcine islet or the pancreatic islet are exemplary examples. The pancreatic islet may be used after being appropriately cultured, and a pancreatic islet obtained by maturing a fetal, neonatal or perinatal porcine islet may be used.

Examples of the blood coagulation factor-secreting cell include factor VIII-secreting cells and factor IX-secreting cells.

As other cells for transplantation, animal cells are preferable, vertebrate-derived cells are more preferable, mammalian-derived cells are still more preferable, and human-derived cells or porcine-derived cells are particularly preferable. The kind of the vertebrate-derived cells (particularly, human-derived cells) may be any of stem cells (for example, pluripotent stem cells (pluripotent cells) or somatic stem cells), progenitor cells or mature cells. As the pluripotent stem cells, it is possible to use, for example, embryonic stem (ES) cells, germ stem (GS) cells or induced pluripotent stem (iPS) cells. As the somatic stem cells, it is possible to use, for example, mesenchymal stem cells (MSC), hematopoietic stem cells, amniotic cells, cord blood cells, bone marrow-derived cells, cardiac muscle stem cells, adipose-derived stem cells or neural stem cells. It is also possible to use cells obtained by the differentiation induction of these stem cells.

As the progenitor cells and the mature cells, it is possible to use, for example, cells derived from skin, dermis, epidermis, muscle, myocardium, nerve, bone, cartilage, endothelium, brain, epithelium, heart, kidney, liver, spleen, oral cavity, cornea, bone marrow, cord blood, amniotic membrane or hair. As the human-derived cells, it is possible to use, for example, ES cells, iPS cells, MSCs, chondrocytes, osteoblasts, osteoprogenitor cells, mesenchymal cells, myoblasts, cardiomyocytes, cardiac myoblasts, nerve cells, hepatocytes, fibroblasts, corneal endothelial cells, vascular endothelial cells, corneal epithelial cells, amniotic cells, cord blood cells, bone marrow-derived cells or hematopoietic stem cells. In addition, cells may be derived from any of autologous cells or allogeneic cells.

A preferable aspect of the pharmacological ingredient that can be encapsulated in the core layer is a substance having a pharmacological activity or a biological activity in vivo or a cell or microorganism from which the above-described substance is produced. A certain aspect is a bioactive substance-producing cell, a separate aspect is a physiologically active substance-producing cell, and a separate aspect is a bioactive natural substance-producing cell.

The microorganisms that can be encapsulated in the core layer are not particularly limited, a microorganism capable of secreting bioactive substances is preferable, and examples thereof include aerobe, anaerobe and the like.

"Chemically crosslinked alginic acid" that is used in the core layer will be described in detail in "5. Chemically crosslinked alginic acid (core layer and anionic polymer layer)."

In addition, to the core layer of the structure of one aspect of the present invention, it is also possible to add an additive other than the pharmacological ingredient. For example, in a case where the pharmacological ingredient is a bioactive substance or the like, there are cases where it is effective to use an additive on which formulation for controlling slow release has been performed or an additive supported by a carrier. In the case of a cell, there are cases where it is effective to use a substance for maintaining the viability or functions of the cell, and a substance that uniformly disperse the pharmacological ingredient in the core layer is also added. The structure of the present invention includes both an aspect in which these are included and an aspect in which these are not included. Examples of the additive other than the pharmacological ingredient include polymer substances other than alginic acid, and, as specific components, examples of biocompatible polymer substances include polylactic acid (PLA), polylactic-co-glycolic acid (PLGA), gelatin, collagen, hyaluronic acid and the like. In addition, for example, in a case where a cell or a microorganism is used as the pharmacological ingredient, there are also cases where a culture medium or culture fluid ingredient or a formulation ingredient that is commonly used as a drug is contained.

1-2. Cationic polymer layer coating core layer

The cationic polymer layer is a layer that coats the surface of the core layer by an electrostatic interaction with the chemically crosslinked alginic acid in the core layer. The cationic polymer that forms the cationic polymer layer is not particularly limited as long as the anionic polymer is capable of forming a film by an electrostatic interaction with the chemically crosslinked alginic acid, and examples thereof include polymers having a basic functional group such as an amino group or an imino group. Specific examples thereof include polysaccharides such as chitosan and glycol chitosan, polyamino acids such as polylysine (preferably poly-L-lysine (PLL)), polyornithine (preferably poly-L-ornithine (PLO)), polyarginine (preferably poly-L-arginine) and polyhistidine (preferably poly-L-histidine), proteins such as protamine, collagen and gelatin, synthetic polymers such as polyethyleneimine, polyvinylamine, polyvinylpyridine, polyvinylimidazole and polyallylamine, and the like. The cationic polymer is preferably chitosan, PLL or PLO, more preferably PLL or PLO and still more preferably PLO. As PLO, a commercially available product can be used, and it is possible to procure, for example, poly-L-ornithine hydrochloride and poly-L-ornithine hydrobromide (Sigma-Aldrich).

In a case where PLO is used as the cationic polymer, the weight-average molecular weight of PLO is preferably 500 to 300,000, more preferably 1,000 to 200,000 and still more preferably 5,000 to 100,000 and is 10,000 to 50,000 as a certain aspect. The weight-average molecular weight of PLO can be measured by gel permeation chromatography (GPC).

1-3. Anionic polymer layer coating cationic polymer layer

The anionic polymer layer is a layer that coats the outside of the cationic polymer layer by an electrostatic interaction with the cationic polymer. The anionic polymer that forms the anionic polymer layer is not particularly limited as long as the anionic polymer is capable of forming a film by an electrostatic interaction with the cationic polymer, and examples thereof include polymers having an acidic functional group such as a carboxyl group or a sulfate group. Specific examples thereof include polysaccharides such as alginic acid, polygalacturonic acid, pectin, pectic acid, carboxymethylcellulose, carrageenan, hyaluronic acid and chondroitin sulfate, synthetic polymers such as polyacrylic acid and polystyrene sulfonic acid, and chemically modified products thereof, and chemically crosslinked products thereof are also included. In addition, the anionic polymer needs to be capable of forming a film by an electrostatic interaction with the cationic polymer and may be a wholly neutral polymer. The anionic polymer is preferably a layer composed of a biocompatible anionic polymer. In addition, depending on the intended use or the environment of where the structure is to be used, it is preferable to select an anionic polymer or chemical modifier having an appropriate property, and, for example, in a case where adhesion is concerned, it is possible to select an anionic polymer having low cell adhesion or cell proliferation.

In several aspects, the anionic polymer layer is alginic acid or a chemically crosslinked alginic acid. "Alginic acid" mentioned herein is alginic acid (for example, sodium alginate) to be mentioned in "2. Alginic acid (regarding alginic acid of raw material that is used to synthesize chemically modified alginic acid derivative and alginic acid that is used in anionic polymer layer)" to be described below. In addition, "chemically crosslinked alginic acid" is a chemically crosslinked alginic acid to be mentioned in "5. Chemically crosslinked alginic acid (core layer and anionic polymer layer)" to be described below. The anionic polymer layer is preferably alginic acid.

1-4. Shape and the like of structure

The structure to be provided herein may have a variety of shapes. The shape of the structure may be, for example, a bead, a sheet, a fiber or the like and may be a shape obtained by combining these shapes or a three-dimensionally deformed shape. In addition, the shape may be a free shape molded with a 3D printer or the like.

The size of the structure is not particularly limited as long as the size is large enough to maintain the shape, and it is preferable to select an appropriate size depending on the intended use, where the structure is to be used or the like. The diameter of the core layer of the structure is preferably less than the outer diameter of the structure and 50% or more.

In addition, the thickness of the cationic polymer layer in the structure is not particularly limited as long as the thickness is large enough to form and maintain the layer, and it is preferable to select an appropriate thickness depending on the kind of the cationic polymer or the intended use, where to be used or the like of the structure. The thickness is 0.01 to 1000 μm as a certain aspect, 0.1 to 200 μm as a separate aspect and 1 to 100 μm as a still separate aspect. For example, in a case where PLO is used as the cationic polymer, the thickness of the cationic polymer is 0.1 to 200 μm as a certain aspect and 1 to 100 μm as a separate aspect.

In addition, the thickness of the anionic polymer layer in the structure is not particularly limited as long as the thickness is large enough to form and maintain the layer, and it is preferable to select an appropriate thickness depending on the kind of the anionic polymer or the intended use, where to be used or the like of the structure. The thickness is

0.01 to 1000 μm as a certain aspect, 0.1 to 200 μm as a separate aspect and 1 to 100 μm as a still separate aspect. For example, in a case where alginic acid or a chemically modified alginic acid is used as the anionic polymer, the thickness of the anionic polymer is 0.1 to 200 μm in a certain aspect and 1 to 100 μm in a separate aspect.

In a case where a bead shape is given to the structure, the shape of the structure is not particularly limited as long as the shape is spherical, and the structure is also referred to as a capsule in some cases. The bead shape is not limited to spheres having a symmetric structure and may be an asymmetric structure or a deformed shape, and examples thereof include an ellipsoid, a prolate spheroid (oblong ellipsoid), a bean shape and a marble shape.

In a case where a bead shape is given to the structure, the diameter (hereinafter, the major axis or the maximum diameter in the case of a non-sphere) is preferably 0.01 to 20 mm and more preferably 0.1 to 10 mm. The diameter is 1 to 8 mm or 1 to 6 mm as a certain aspect. The diameter is 2 to 6 mm, 3 to 6 mm or 4 to 6 mm as a separate aspect.

In addition, in a case where a bead shape is given to the structure, the diameter of the core layer is preferably 0.01 to 20 mm and more preferably 0.1 to 10 mm. The diameter is 1 to 8 mm or 1 to 6 mm as a certain aspect. In addition, the diameter of the core layer is preferably less than the diameter of the structure and 50% or more.

In addition, in a case where a bead shape is given to the structure, the thickness of the cationic polymer is 0.1 to 200 μm as a certain aspect and 1 to 100 μm as a separate aspect.

In addition, in a case where a bead shape is given to the structure, the thickness of the anionic polymer is 0.1 to 200 μm as a certain aspect and 1 to 100 μm as a separate aspect.

In a case where a sheet shape is given to the structure, the shape of the structure is not particularly limited as long as the shape is a flat plate shape. The flat plate means a flat plate, indicates a plate shape having an almost uniform thickness and a wide area, but the sheet shape as the structure, that is, the flat plate shape may not have a uniform thickness and may be an inclined structure that is thick on one side but thin on the other side. In addition, the sheet shape may be a shape obtained by deforming the flat plate-shaped structure, and examples thereof include a mesh shape, a honeycomb shape, a perforated shape and a tubular shape. Examples of the sheet shape include polygonal, such as triangular, square or pentagonal, or circular flat plate shapes. In the present specification, in a case where a sheet shape is given to the structure, the three-layer structure is not a structure in which the core layer, the cationic polymer layer and the anionic polymer layer are simply laminated in this order, but a structure in which the upper surface, lower surface and side surfaces of the core layer are coated with the cationic polymer layer and, furthermore, the upper surface, lower surface and side surfaces of the cationic polymer layer are coated with the anionic polymer layer and a structure molded by cutting the sheet-shaped structure obtained by performing coating as described above is also included in the structure.

In a case where a sheet shape is given to the structure, the thickness is preferably 0.1 to 10 mm and more preferably 0.2 to 5 mm.

In addition, in a case where a sheet shape is given to the structure, the thickness of the core layer is preferably 0.1 to 10 mm and more preferably 0.2 to 5 mm. In addition, the thickness of the core layer of the structure is preferably less than the thickness of the structure and 50% or more.

In addition, in a case where a sheet shape is given to the structure, the thickness of the cationic polymer is 0.1 to 200 μm as a certain aspect and 1 to 100 μm as a separate aspect.

In addition, in a case where a sheet shape is given to the structure, the thickness of the anionic polymer is 0.1 to 200 μm as a certain aspect and 1 to 100 μm as a separate aspect.

The thicknesses of the cationic polymer layer and the anionic polymer layer mentioned herein refer to the thickness of any one of the upper surface or lower surface of the sheet-shaped structure.

In addition, the sheet-shaped structure preferably has a thickness that is almost constant throughout the entire plate shape. A variation in the thickness in the plate-shaped structure is preferably within ±20%.

In several aspects, for the sheet-shaped structure, for example, when the flat plate shape is expressed with length, width and thickness, the length is 1 to 200 mm, the width is 1 to 200 mm, and the thickness of 0.2 to 5 mm. In several separate aspects, the sheet-shaped structure has, for example, an area indicated by length × width of 1 to 40000 $mm^2$ and a thickness of 0.2 to 5 mm. In these aspects, the structure also may have a shape of a circle, a square, a hexagon, an octagon or the like.

In a case where a fiber shape is given to the structure, the shape of the structure is not particularly limited as long as the shape is a fibrous elongated shape. The fiber shape is not limited to a shape in which the cross-sectional shape in a direction perpendicular to the central axis is a circle and may be an asymmetric shape or a deformed shape, and examples thereof also include shapes in which the cross section has a polygonal shape such as a triangular shape, a square shape or a pentagonal shape or an elliptical shape. A shape having a circular cross-sectional shape is preferable. In addition, a structure molded in a ring shape by coupling both ends of fibers, a structure made into a chain shape by coupling a plurality of the rings, a structure having a fiber bundle shape or a structure in which fibers are coupled in a sheet shape are also included.

In a case where a fiber shape is given to the structure, the diameter (hereinafter, the major axis or the maximum diameter in the case of a non-sphere) of the cross section is preferably 0.01 to 20 mm, more preferably 0.1 to 10 mm,

still more preferably 0.2 to 5 mm and particularly preferably 0.2 to 2 mm.

In addition, in a case where a fiber shape is given to the structure, the diameter of the cross section of the core layer is preferably 0.01 to 20 mm, more preferably 0.1 to 10 mm, still more preferably 0.2 to 5 mm and particularly preferably 0.2 to 2 mm. In addition, the diameter of the cross section of the core layer is preferably less than the diameter of the cross section of the structure and 50% or more.

In addition, in a case where a fiber shape is given to the structure, the thickness of the cationic polymer is 0.1 to 200 μm as a certain aspect and 1 to 100 μm as a separate aspect.

In addition, in a case where a fiber shape is given to the structure, the thickness of the anionic polymer is 0.1 to 200 μm as a certain aspect and 1 to 100 μm as a separate aspect.

In addition, in a case where a fiber shape is given to the structure, the length is not limited and is, for example, 10 mm to 50 m as a certain aspect, 10 mm to less than 30 cm as a separate aspect and 30 cm to 50 m as a still separate aspect.

In the present specification, a numerical range expressed using "to" indicates a range comprising numerical values before and after "to" as the minimum value and the maximum value, respectively.

2. Alginic acid (regarding alginic acid of raw material that is used to synthesize chemically modified alginic acid derivative and alginic acid that is used in anionic polymer layer)

Alginic acid mentioned in the present specification means at least one alginic acid selected from the group consisting of alginic acid, alginate ester and salts thereof (for example, sodium alginate) (referred to as "alginic acids" in some cases). Alginic acid that is used may naturally occur or may be a synthetic product, but is preferably a naturally-occurring alginic acid. Alginic acids that are preferably used are polymers that are bioabsorbable polysaccharides that are extracted from brown algae such as lessonia, macrocystis, laminaria, ascophyllum, durvillia, kajime, arame and kelp and contain two kinds of linearly polymerized uronic acids, such as D-mannuronic acid (M) and L-guluronic acid (G). More specifically, the alginic acids are block copolymers in which a homopolymer block of D-mannuronic acid (MM fraction), a homopolymer block of L-guluronic acid (GG fraction) and a block in which D-mannuronic acid and L-guluronic acid are arranged (M/G fractions) arbitrarily bond to one another.

Alginic acid is one kind of natural polysaccharide that is manufactured by being extracted from brown algae seaweed and purified and is a polymer in which D-mannuronic acid (M) and L-guluronic acid (G) are polymerized. The composition rate (M/G ratio) of D-mannuronic acid to L-guluronic acid in alginic acid, that is, the gel strength varies mainly with the kind of a creature from which alginic acid is derived such as seaweed, is also affected by the habitat of the creature or seasons, and covers a high range from a high G type where the M/G ratio is approximately 0.2 to a high M type where the M/G ratio is approximately 5. The physicochemical properties of alginic acid vary with the M/G ratio of alginic acid, how M and G are arranged and the like, and there are cases where preferable uses vary. The gelation ability of alginic acids and the properties of formed gel are affected by the M/G ratio, and it is known that, ordinarily, the gel strength becomes high in a case where the G ratio is high. Additionally, the M/G ratio also affects the hardness, fragility, water absorption, flexibility and the like of the gel. Therefore, as alginic acid that is used in the present invention, it is preferable to use alginic acid having an appropriate M/G ratio or an appropriate viscosity depending on the final intended use.

As industrial methods for manufacturing alginic acid, there are an acid method, a calcium method and the like, and, in the present invention, alginic acid manufactured by any method can be used. The quantitative value of alginic acid by the HPLC method is made by purification to be preferably within a range of 80 to 120 mass%, more preferably within a range of 90 to 110 mass% and still more preferably within a range of 95 to 105 mass%. In the present invention, alginic acid having a quantitative value by the HPLC method within the above-described range will be referred to as high-purity alginic acid. Alginic acid or a salt thereof that is used in the present invention is preferably high-purity alginic acid. As a commercially available product, it is possible to purchase and use, for example, KIMICA ALGIN series made commercially available by KIMICA Corporation, preferably, high-purity food and pharmaceutical grade alginic acid. The commercially available product can also be used after being further purified as appropriate. For example, it is preferable to perform a low endotoxin treatment. As a purification method or a low endotoxin treatment method, it is possible to adopt, for example, a method described in Japanese Patent Application Publication No. 2007-75425.

In the present specification, "alginate ester" and "alginate salt" that are used are not particularly limited, but need to have no functional group that impairs a reaction with a reaction reagent for performing ionic crosslinking or chemical crosslinking to cause such a reaction. Examples of the alginate ester preferably include propylene glycol alginate and the like.

In the present specification, examples of alginate include monovalent salts of alginic acid and divalent salts of alginic acid. The monovalent salts of alginic acid are salts made by ion-exchanging a hydrogen ion in carboxylic acid of D-mannuronic acid or L-guluronic acid in alginic acid with a monovalent metal ion such as $Na+$ or $K+$. Preferably, sodium alginate, potassium alginate, ammonium alginate and the like are exemplified, sodium alginate or potassium alginate is

preferable, and sodium alginate is more preferable. Preferable examples of the divalent salts of alginic acid include calcium alginate, magnesium alginate, barium alginate, strontium alginate and the like.

In a case where alginate is used as a configuration ingredient of the structure of the present invention, the alginate is calcium alginate as a certain embodiment and is sodium alginate as a separate embodiment.

In a case where alginate is used as a raw material for manufacturing the chemically modified alginic acid derivative of the present invention or is used in a process for manufacturing the structure of the present invention, there are cases where "monovalent metal salt of alginic acid" is preferably used. Specific examples of the monovalent metal salt of alginic acid include sodium alginate, potassium alginate and the like, and sodium alginate is particularly preferable.

In the present specification, there will be cases where alginic acid is expressed as (ALG)-COOH wherein (ALG) indicates alginic acid and -COOH indicates one arbitrary carboxyl group of alginic acid.

As alginic acid that is used in the present invention, alginic acid having an appropriate weight-average molecular weight depending on the final intended use is preferably used. For example, the weight-average molecular weight of alginic acid to be used is preferably 10,000 to 10,000,000, more preferably 100,000 or more and 5,000,000 or less and still more preferably 150,000 or more and 3,000,000 or less.

In several embodiments, alginic acid refers to sodium alginate. As the sodium alginate, it is possible to use commercially available sodium alginate. For example, it is possible to use sodium alginates of A-1, A-2, A-3, B-1, B-2 and B-3 shown in the following table (sales agency: MOCHIDA PHARMACEUTICAL CO., LTD.). The viscosity, weight-average molecular weight and M/G ratio of an aqueous solution of 1 w/w% of each sodium alginate are shown in the following table.

[Table 13]

| Sodium alginate | 1 w/w% viscosity (mPa·s) | Weight-average molecular weight | | M/G ratio |
|---|---|---|---|---|
| | | GPC | GPC-MALS | |
| A-1 | 10 to 40 | 300,000 to 700,000 | 60, 000 to 130,000 | 0.5 to 1.8 |
| A-2 | 60 to 150 | 700,000 to 1, 400, 000 | 130,000 to 200,000 | |
| A-3 | 300 to 600 | 1,400,000 to 2, 000, 000 | 200,000 to 400, 000 | |
| B-1 | 10 to 40 | 150,000 to 800, 000 | 60, 000 to 130, 000 | 0. 1 to 0. 5 |
| B-2 | 70 to 150 | 800, 000 to 1,500,000 | 130,000 to 200,000 | |
| B-3 | 400 to 600 | 1, 500, 000 to 2, 500, 000 | 200,000 to 350, 000 | |

Individual physical property values of the sodium alginates A-1, A-2, A-3, B-1, B-2 and B-3 were measured by a variety of methods to be described below. The measurement methods are not limited to the following methods, and there are cases where individual physical property values may differ from the above-described values depending on the measurement method. One preferable embodiment where sodium alginate that is used to manufacture the structure of the present invention is not limited is "A-2, A-3, B-2 and B-3" and a separate embodiment is "A-2 and B-2." An embodiment where there is a case where a chelate treatment is performed on the structure is "A-2 and A-3" and a still separate embodiment is "A-2."

[Measurement of viscosity of sodium alginate]

The viscosity was measured according to The Japanese Pharmacopoeia (16$^{th}$ edition) using a rotational viscometer method (cone-plate rotating viscometer). Specific measurement conditions are a described below. A sample solution was prepared using Milli-Q water. As a measurement device, a cone-plate rotational viscometer (viscotester RheoStress 600 (Thermo HAAKE GmbH) sensor: 35/1) was used. The rotating speed was set to 1 rpm at the time of measuring the 1 w/w% sodium alginate solution. As the readout time, the average value from one minute after the beginning to two minutes when the measurement was performed for two minutes was used. The average value of three times of measurement was used as the measurement value. The measurement temperature was set to 20°C.

[Measurement of weight-average molecular weight of sodium alginate]

The weight-average molecular weight was measured by two kinds of measurement methods of (1) gel permeation chromatography (GPC) and (2) GPC-MALS. The measurement conditions are as described below.

[Pretreatment method]

A solution obtained by adding an eluent to the sample, dissolving the sample and then filtering the sample with a 0.45 μm membrane filter was used as a measurement solution.

(1) Gel permeation chromatography (GPC) measurement

[Measurement conditions (relative molecular weight distribution measurement)]

Column: TSKgel GMPW-XL × 2 + G2500PW-XL (7.8 mm I. D. × 300 mm × three)
Eluent: 200 mM sodium nitrate aqueous solution
Flow rate: 1.0 mL/min
Concentration: 0.05%
Detector: RI detector
Column temperature: 40°C
Injection amount: 200 μL
Molecular weight standard: Standard pullulan, glucose

(2) GPC-MALS measurement

[Refractive index increment (dn/dc) measurement (measurement conditions)]

Differential refractometer: Optilab T-rEX
Measurement wavelength: 658 nm
Measurement temperature: 40°C
Solvent: 200 mM sodium nitrate aqueous solution
Sample concentration: 0.5 to 2.5 mg/mL (five concentrations)

[Measurement conditions (absolute molecular weight distribution measurement)]

Column: TSKgel GMPW-XL × 2 + G2500PW-XL (7.8 mm I. D. × 300 mm × three)
Eluent: 200 mM sodium nitrate aqueous solution
Flow rate: 1.0 mL/min
Concentration: 0.05%
Detector: RI detector, light scattering detector (MALS)
Column temperature: 40°C
Injection amount: 200 μL

In the present specification, there will be cases where Da (Dalton) is added as the unit to the molecular weights of alginic acid, chemically modified alginic acid derivatives, and chemically crosslinked alginic acid.

The composition rates (M/G ratio) of D-mannuronic acid to L-guluronic acid in alginic acids vary mainly with the kind of a creature from which alginic acid is derived such as seaweed, are also affected by the habitat of the creature or seasons, and cover a high range from a high G type where the M/G ratio is approximately 0.2 to a high M type where the M/G ratio is approximately 5. The gelation ability of alginic acids and the properties of produced gel are affected by the M/G ratio, and it is known that, ordinarily, the gel strength becomes high in a case where the G ratio is high. Additionally, the M/G ratio also affects the hardness, fragility, water absorption, flexibility and the like of the gel. The M/G ratios of alginic acids that are used and salts thereof are normally 0.1 to 4.0, 0.1 to 3.0 in certain embodiments, 0.1 to 2.0 in certain embodiments, 0.5 to 1.8 in certain embodiments and 0.8 to 1.2 in certain embodiments. In addition, the M/G ratios are 0.1 to 0.5 in other embodiments.

Alginic acid is a high-molecular-weight polysaccharide, it is difficult to accurately determine the molecular weight; however, ordinarily, the weight-average molecular weight is within a range of 1,000 to 10,000,000, preferably 10,000 to 8,000,000 and more preferably 20,000 to 3,000,000. It is known that, in the measurement of the molecular weights of

naturally-occurring high-molecular-weight substances, values may differ depending on measurement methods.

In the case of specifying the molecular weight of the chemically modified alginic acid derivative or alginic acid or a salt thereof in the present specification, unless particularly otherwise described, the molecular weight is the weight-average molecular weight that is calculated by size exclusion chromatography (SEC). As alginic acid or a salt thereof that is used in the present invention, it is desirable to use alginic acid or salt thereof having an appropriate molecular weight distribution depending on the final intended use.

For example, depending on the measurement conditions of gel permeation chromatography (GPC) or gel filtration chromatography (both are also collectively referred to as size exclusion chromatography (SEC)) to be described in the following examples, the molecular weight is preferably 100,000 to 5,000,000 and more preferably 150,000 to 3,000,000. In addition, in certain embodiments, the molecular weight is preferably within a range of 500,000 to 3,000,000, more preferably 1,000,000 to 2,500,000 and still more preferably 1,000,000 to 2,000,000.

In addition, according to, for example, the GPC-MALS (SEC-MALS) method, it is possible to measure the absolute weight-average molecular weight. The weight-average molecular weight (absolute weight-average molecular weight) measured by the GPC-MALS method is preferably 10,000 or more, more preferably 50,000 or more and still more preferably 60,000 or more and is preferably 1,000,000 or less, more preferably 800,000 or less, still more preferably 700,000 or less and especially preferably 500,000 or less. A preferable range thereof is 10,000 to 1,000,000, more preferably 50,000 to 800,000 and still more preferably 60,000 to 500,000.

Normally, in the case of calculating the molecular weights of high-molecular-weight polysaccharides by a method in which the above-described SEC or SEC-MALS is used, a measurement error of approximately 10% to approximately 30% may be caused. For example, the fluctuation of the value may be caused within a range of 350,000 to 650,000 when the molecular weight is 500,000 and within a range of 700,000 to 1,300,000 when the molecular weight is 1,000,000. In the present specification, in a case where the molecular weight is expressed with "approximately" in the measurement, the temperature may include up to the numerical value $\pm$ 10% and up to the numerical value $\pm$ 20% in certain embodiments.

Here, ordinarily, naturally-occurring high-molecular-weight substances are aggregates of molecules having a variety of molecular weights, not a single molecular weight, and are thus measured to have a molecular weight distribution with a certain constant width. A typical measurement method is gel filtration chromatography. Examples of typical information of a molecular weight distribution that is obtained by gel filtration chromatography include the weight-average molecular weight (Mw), the number-average molecular weight (Mn) and the dispersion ratio (Mw/Mn).

The weight-average molecular weight is a property where contribution of high-molecular-weight substances having a large molecular weight to the average molecular weight is emphasized and is represented by the following formula.

$$Mw = \Sigma(WiMi)/W = \Sigma(HiMi)/\Sigma(Hi)$$

The number-average molecular weight is calculated by dividing the total weight of high-molecular-weight substances by the total number of the high-molecular-weight substances.

$$Mn = W/\Sigma Ni = \Sigma(MiNi)/\Sigma Ni = \Sigma(Hi)/\Sigma(Hi/Mi)$$

Here, W is the total weight of the high-molecular-weight substances, Wi is the weight of the $i^{th}$ high-molecular-weight substance, Mi is the molecular weight in the $i^{th}$ elution time, Ni is the number of the molecular weights Mi and Hi is the height at the $i^{th}$ elution time.

It is known that, in the measurement of the molecular weights of naturally-occurring high-molecular-weight substances, values may differ depending on measurement methods (examples of hyaluronic acid: Chikako Yomota et. al. Bull. Natl. Health Sci., Vol. 117, pp. 135 to 139 (1999) and Chikako Yomota et. al. Bull. Natl. Inst. Health Sci., Vol. 121, pp. 30 to 33 (2003)). Regarding the measurement of the molecular weight of alginic acid, there is a publication where a method for calculating the molecular weight from the intrinsic viscosity and a method for calculating the molecular weight by SEC-MALLS (size exclusion chromatography with multiple angle laser light scattering detection) are described (ASTM F2064-00 (2006), published by ASTM International). In the present invention, as the weight-average molecular weight, it is possible to use a value calculated from a calibration curve obtained by measuring the molecular weights by such a normal method as described in the above-described publication, for example, size exclusion chromatography (SEC), and using pullulan as a standard substance.

In addition, in the present invention, as the weight-average molecular weight, it is possible to use an absolute molecular weight measured by such a normal method as described in the above-described publication, for example, size exclusion chromatography (SEC)-MALS. Measurement of the molecular weights of alginic acids can be measured

according to the normal methods.

In the case of specifying the molecular weight of alginic acid or a salt thereof in the present specification, unless particularly otherwise described, the molecular weight is the weight-average molecular weight that is calculated by gel filtration chromatography. As typical conditions in the case of using gel filtration chromatography in the measurement of the molecular weight, it is possible to adopt conditions in the present examples to be described below. As a column, for example, a Superose 6 Increase 10/300 GL column (GE Healthcare Corporation) can be used, as a developing solvent, for example, a 10 mmol/L phosphate buffer solution containing 0.15 mol/L of NaCl (pH: 7.4) can be used, and, as molecular weight standards, blue dextran, thyroglobulin, ferritin, aldolase, conalbumin, ovalbumin, ribonuclease A and aprotinin can be used.

The viscosity of alginic acid that is used in the present specification is not particularly limited, but is preferably 10 mPa s to 1000 mPa s and more preferably 50 mPa s to 800 mPa·s in the case of measuring the viscosity as an aqueous solution of 1 w/w% alginic acids.

Measurement of the viscosity of an aqueous solution of alginic acid can be measured according to a normal method. For example, the viscosity can be measured using a coaxial double cylinder rotational viscometer, a single cylinder rotational viscometer (Brookfield viscometer), a cone-plate rotational viscometer (cone plate viscometer) or the like of the rotational viscometer method. Preferably, the viscosity measurement method in The Japanese Pharmacopoeia ($16^{th}$ edition) is desirably followed. More preferably, a cone-plate viscometer is used.

Immediately after being extracted from brown algae, alginic acids have a large molecular weight and a high viscosity, but the molecular weight becomes small, and the viscosity becomes low in the process of drying by heat, purification or the like. Alginic acids having different molecular weights can be manufactured by a method such as the management of conditions such as the temperature in the manufacturing process, selection of brown algae, which serves as a raw material, or the fractionation of the molecular weight in the manufacturing steps. Furthermore, it is also possible to produce alginic acids having intended molecular weights by mixing alginic acids with a different lot of alginic acids having different molecular weights or viscosities.

Alginic acid that is used in the present specification is alginic acid on which a low endotoxin treatment has not been performed in several embodiments or alginic acid on which a low endotoxin treatment has been performed in several different embodiments. A low endotoxin refers to the fact that the endotoxin level is so low that, substantially, inflammation or fever is not caused. More preferably, alginic acid on which a low endotoxin treatment has been performed is desirable.

The low endotoxin treatment can be performed by a well-known method or an equivalent method thereto. For example, the low endotoxin treatment can be performed by Suga et al.'s method in which sodium hyaluronate is purified (for example, refer to Japanese Patent Application Publication No. H09-324001 or the like), Yoshida et al.'s method in which (β1,3-glucan is purified (for example, refer to Japanese Patent Application Publication No. H08-269102 or the like), William et al.'s method in which a biopolymer salt such as alginate or gellan gum is purified (for example, refer to Japanese Translation of PCT Application No. 2002-530440 or the like), James et al.'s method in which polysaccharide is purified (for example, refer to WO 93/13136 or the like), Lewis et al.'s method (for example, refer to the specification of US Patent No. 5589591 or the like), Herman Frank et al.s' method in which alginate is purified (for example, refer to Appl Microbiol Biotechnol (1994) 40: 638 to 643 or the like) or the like or an equivalent method thereto. The low endotoxin treatment is not limited thereto and can be performed by a well-known method such as washing, filtration with a filter (an endotoxin removal filter, a charged filter or the like), ultrafiltration, purification using a column (an endotoxin adsorption affinity column, a gel filtration column, an ion exchange resin column or the like), adsorption into a hydrophobic substance, resin, activated carbon or the like, an organic solvent treatment (extrusion with an organic solvent, precipitation and sedimentation by addition of an organic solvent or the like), a surfactant treatment (for example, refer to Japanese Patent Application Publication No. 2005-036036 or the like) or an appropriate combination thereof. A well-known method such as centrifugation may be appropriately combined with a step of these treatments. It is desirable to select the method as appropriate in accordance with the kind of alginic acid.

The endotoxin level can be confirmed by a well-known method and can be measured by, for example, a method in which a limulus reagent (LAL) is used, a method in which an ENDOSPECY (registered trademark) ES-24S set (Seikagaku Corporation) is used or the like.

A method for treating the endotoxin that is used is not particularly limited, and, as a result, in the case of performing endotoxin measurement with a limulus reagent (LAL), the endotoxin content in alginic acids is preferably 500 endotoxin units (EU)/g or less, more preferably 100 EU/g or less, especially preferably 50 EU/g or less and particularly preferably 30 EU/g or less. In the present invention, "substantially comprising no endotoxin" means that the endotoxin value measured by an endotoxin test in The Japanese pharmacopoeia is within the above-described numerical range. Sodium alginate on which the low endotoxin treatment has been performed can be procured from, for example, commercially available products such as Sea Matrix (registered trademark) (Mochida Pharmaceutical Co., Ltd.) and PRONOVA™ UP LVG (FMC BioPolymer).

3. Chemically modified alginic acid derivative

3-1. Chemically modified alginic acid derivative (Huisgen type)

In several embodiments, the chemically modified alginic acid derivatives in the present specification are derivatives in which a reactive group in a Huisgen reaction or a complementary reactive group of the above-described reactive group has been introduced into one or more arbitrary carboxyl groups of alginic acid through an amide bond and a divalent linker. More specifically, the chemically modified alginic acid derivatives are a chemically modified alginic acid derivative represented by Formula (H-I) below:

[C33]

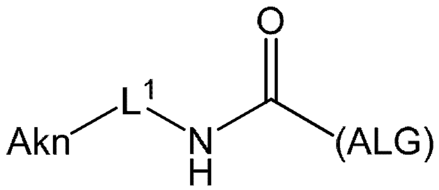

(H-I)

[in Formula (H-I), (ALG) represents alginic acid; -NHCO- represents an amide bond through an arbitrary carboxyl group of the alginic acid; $-L^1-$ is a divalent linker that bonds to a cyclic alkyne group (Akn)] and a chemically modified alginic acid derivative represented by Formula (H-II) below:

[C34]

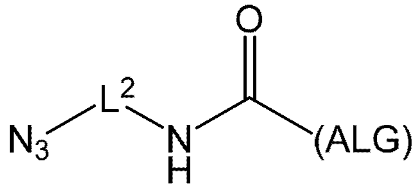

(H-II)

[in Formula (H-II), (ALG) represents alginic acid; -NHCO- represents an amide bond through an arbitrary carboxyl group of the alginic acid; $-L^2-$ is a divalent linker that bonds to an azide group].

As the divalent linker ($-L^1-$ or $-L^2-$), an arbitrary linear group can be used as long as a reaction between a reactive group and a reactive group that is complementary with the above-described reactive group is not impaired. Specific examples thereof include linear alkylene groups ($-(CH_2)_n-$, n = 1 to 30) (a plurality of (for example, one to 10 or one to five) $-CH_2-$'s in the group may be substituted by groups such as -C(=O)-, -CONH-, -O-, -NH-, -S-, a benzene ring, a heterocycle (a five or six-membered aromatic heterocycle or a five or six-membered non-aromatic heterocycle such as a pyridine ring, a piperidine ring or a piperazine ring), a plurality of (for example, one to 10 or one to five) hydrogen atoms in the $-CH_2-$ may be substituted by groups selected from groups of an oxo group (=O), $C_{1-6}$ alkyl groups (for example, groups such as a methyl group, an ethyl group, an n-propyl group and an iso-propyl group), halogen atoms (for example, a fluorine atom, a chlorine atom, a bromine atom, an iodine atom and the like), a hydroxyl group (-OH) and the like), but the divalent linker is not limited thereto.

The cyclic alkyne group (Akn) is a 7 to 10-membered saturated hydrocarbon ring in which one bond configuring the ring is a triple bond and is preferably an 8-membered cyclic alkyne group. In the cyclic alkyne group, furthermore, one or two ring groups selected from a benzene ring, a three to eight-membered hydrocarbon ring or an aromatic heterocycle such as a pyridine ring is optionally condensed, and $-CH_2-$ in the group is optionally substituted with one or two groups selected from -C(=O)-, -CO-NH-, -O-, -NH- and -S-. In addition, hydrogen atoms in the $-CH_2-$ may be substituted with one or two groups selected from an oxo group (=O), a $C_{1-6}$ alkyl group (for example, a methyl group, an ethyl group, an n-propyl group, an iso-propyl group or the like), a halogen atom (for example, a fluorine atom or a chlorine atom), a hydroxyl group (-OH) and the like. However, the cyclic alkyne group is not limited thereto.

A certain embodiment of the chemically modified alginic acid derivative that is used to manufacture the structure of the embodiment [2] is a compound for which, in Formula (H-I),

$-L^1-$ is $-(CH_2)_{n1}-$ (here, n1 is 1 to 50, $-CH_2-$ in the group is optionally substituted with one to 10 groups selected from

-CO-, -CONH-, -NHCO-, -O-CONH-, -NHCO-O-, -O- and - NH- or a benzene ring, and a hydrogen atom in the $-CH_2-$ is optionally substituted with a $C_{1-3}$ alkyl group or a $C_{1-3}$ alkyl group substituted with a phenyl group),

Akn is a compound that is an eight-membered cyclic alkyne group (here, the cyclic alkyne group is optionally an eight-membered cyclic group in which, furthermore, one or two benzene rings, cyclopropane rings or 1,2,3-triazole rings are optionally condensed and optionally bond to $-L^1-$ in a condensed ring, and $-CH_2-$ in the group is optionally substituted with one or two groups selected from -C(=O)-, -CONH- and -NH-, and a hydrogen atom in - $CH_2-$ is optionally substituted with one or two groups selected from a $C_{1-3}$ alkyl group, a fluorine atom, a hydroxyl group, a $C_{1-3}$ alkyloxy group or the like), and

a compound for which, in Formula (H-II), $-L^2-$ is $-(CH_2)_{n2}-$ (here, n2 is 1 to 50, $-CH_2-$in the group is optionally substituted with one to 10 groups selected from -CONH-, -NHCO-, -O- and -NH-, a benzene ring or a pyridine ring, and a hydrogen atom in the $-CH_2-$ is optionally substituted with a $C_{1-3}$ alkyl group).

Here, in the compound, a preferable embodiment of n1 is 1 to 20, and a more preferable embodiment is 1 to 15 (for example, 2 to 15). In addition, as a preferable embodiment of $-L^1-$, in $-(CH_2)_{n1}-$, $-CH_2-$ is a group that may be substituted with one to five groups, more preferably one to three groups, selected from -CO-, -CONH-, -NHCO-, -O-CONH-, -NHCO-O-, -O- and -NH-. Akn is preferably an eight-membered cyclic alkyne group in which one or two benzene rings are optionally condensed and $-CH_2-$ in the eight-membered cyclic alkyne group is optionally substituted with -NH-. n2 is 1 to 20 as a preferable embodiment and 1 to 15 (for example, 2 to 15) as a more preferable embodiment. In addition, as a preferable embodiment of $-L^2-$, in $-(CH_2)_{n2}-$, $-CH_2-$ is a group that may be substituted with one to five groups, more preferably one to three groups, selected from - CONH-, -NHCO-, -O- and -NH-.

A preferable embodiment of the chemically modified alginic acid derivative that is used to manufacture the structure of the embodiment [2] is a compound for which, in Formula (H-I), $-L^1-$ is $-(CH_2)_{n1}-$ (here, n1 is 1 to 15 (for example, 2 to 15), $-CH_2-$ in the group is optionally substituted with one to five groups selected from -CO-, -CONH-, -NHCO-, -O-CONH-, -NHCO-O-, -O- and -NH- or a benzene ring, and a hydrogen atom in the $-CH_2-$ is optionally substituted with a $C_{1-3}$ alkyl group or a $C_{1-3}$ alkyl group substituted with a phenyl group),

Akn is a compound that is an eight-membered cyclic alkyne group (here, the cyclic alkyne group is optionally an eight-membered cyclic group in which, furthermore, one or two benzene rings are optionally condensed or $-CH_2-$ in the eight-membered cyclic alkyne group is substituted with -NH-), and

a compound for which, in Formula (H-II), $-L^2-$ is $-(CH_2)_{n2}-$ (here, n2 is 1 to 15 (for example, 2 to 15), and $-CH_2-$ in the group is optionally substituted with one to five groups selected from -CONH-, -NHCO-, -O- and -NH- or a benzene ring).

A certain embodiment of the chemically modified alginic acid derivative is a compound for which each definition of $-L^1-$ or Akn in Formula (H-I) is the same as any definition in the embodiments [2-1] to [2-5].

In addition, a certain embodiment of the chemically modified alginic acid derivative is a compound for which the definition of $-L^2-$ in Formula (H-II) is the same as any definition in the embodiments [2-1] to [2-5].

In addition, in several embodiments, the chemically modified alginic acid derivative is a chemically modified alginic acid derivative represented by Formula (HA-I) below:

[C35]

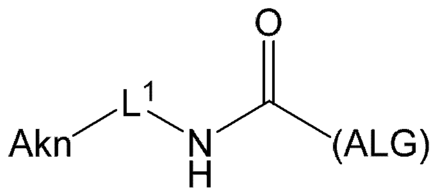

(HA-I)

[in Formula (HA-I), the definitions of (ALG), $-L^1-$ and Akn are the same as the definitions in the embodiment [4]] and a chemically modified alginic acid derivative represented by Formula (HA-II) below:

[C36]

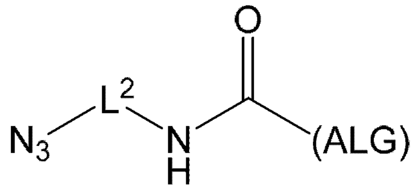

(HA-II)

[in Formula (HA-II), the definitions of (ALG) and -$L^2$- are the same as the definitions in the embodiment [4]].

In addition, in several embodiments, the chemically modified alginic acid derivative is a chemically modified alginic acid derivative represented by Formula (HB-I) below:

[C37]

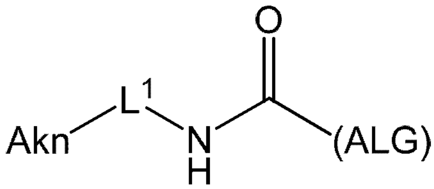

(HB-I)

[in Formula (HB-I), the definitions of (ALG), -$L^1$- and Akn are the same as any definitions in the embodiments [3-1] to [3-6]] and a chemically modified alginic acid derivative represented by Formula (HB-II) below:

[C38]

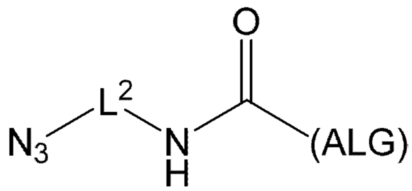

(HB-II)

[in Formula (HB-II), the definitions of (ALG) and -$L^2$- are the same as any definitions in the embodiments [3-1] to [3-6]].

In the -NH-CO- groups of the chemically modified alginic acid derivatives represented by Formula (H-I) to Formula (HB-II), it is possible to substitute the hydrogen atom in the imino group (-NH-) into a methyl group to produce a -N(Me)-CO- group.

The bonding style of the linker (-L1- or -L2-) and the chemically modified alginic acid derivatives in the chemically modified alginic acid derivatives represented by Formula (H-I) to Formula (HB-II) is a -NH-CO- bond or -N(Me)-CO-; preferably a -NH-CO- bond.

The chemically modified alginic acid derivatives represented by Formula (H-I) to Formula (HB-II), which are chemically modified alginic acid derivatives in the present specification, can be manufactured by, for example, a method of the following formula (for the detail, refer to 4. Method for synthesizing chemically modified alginic acid derivative).

[C39]

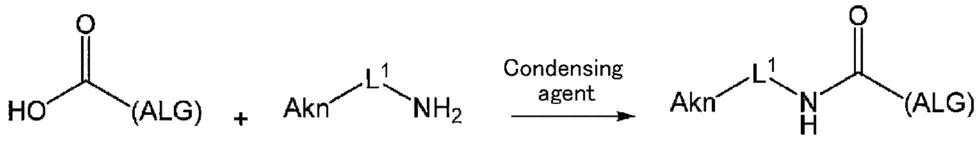


Alginic acid (AM-1) (H-I), (HA-I), (HB-I)

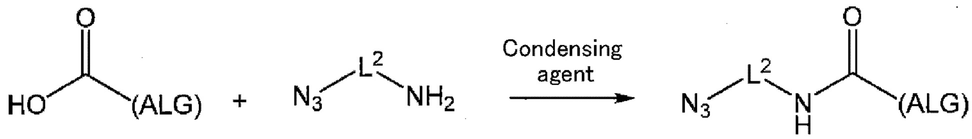


Alginic acid (AM-2) (H-II), (HA-II), (HB-II)

The weight-average molecular weights of the chemically modified alginic acid derivatives represented by Formula (H-I) to Formula (HB-II) of the present specification are 100,000 Da to 3,000,000 Da, preferably 300,000 Da to 2,500,000 Da and more preferably 500,000 Da to 2,000,000 Da. The molecular weights of the two alginic acid derivatives can be obtained by a method to be described below.

In the present specification, the Akn-$L^1$-NH- group in Formula (H-I), Formula (HA-I) or Formula (HB-I) does not need to bond to all carboxyl groups in the alginic acid constituting unit, and the $N_3$-$L^2$-NH- group in Formula (H-II), Formula (HA-II) or Formula (HB-II) does not need to bond to all carboxyl groups in the alginic acid constituting unit.

In the present specification, in a case where the Akn-$L^1$-NH- group in Formula (H-I), Formula (HA-I) or Formula (HB-I) is referred to as the reactive group, the $N_3$-$L^2$-NH- group in Formula (H-II), Formula (HA-II) or Formula (HB-II) becomes the complementary reactive group. In addition, conversely, in a case where the $N_3$-$L^2$-NH- group in Formula (H-II), Formula (HA-II) or Formula (HB-II) is referred to as the reactive group, the Akn-$L^1$-NH-group in Formula (H-I), Formula (HA-I) or Formula (HB-I) becomes the complementary reactive group.

In the present specification, the introduction rate of the reactive group or the complementary reactive group is each 0.1% to 30% or 1% to 30%, preferably 2% to 20% and more preferably 3% to 10%.

The introduction rate of the reactive group or the complementary reactive group is a value expressing the number of uronic acid monosaccharide units into which each reactive group has been introduced in uronic acid monosaccharide units, which are the repeating units of alginic acids in percentage. In the present specification, unless particularly otherwise described, "%" that is used for the introduction rate of the reactive group or the complementary reactive group in the chemically modified alginic acid derivatives (Formula (H-I) to Formula (HB-II)) means "mol%." The introduction rate of the reactive group or the complementary reactive group can be obtained by a method described below in the examples to be described below.

In the present specification, the cyclic alkyne group (Akn) in Formula (H-I), Formula (HA-I) or Formula (HB-I) and the azide group in Formula (H-II), Formula (HA-II) or Formula (HB-II) form a triazole ring by the Huisgen reaction, whereby a crosslink is formed.

3-1-1. Huisgen reaction

The Huisgen reaction (1,3-dipolar cycloaddition) is a condensation reaction between compounds having a terminal azide group and a terminal alkyne group as shown in the following formula. As a result of the reaction, a disubstituted 1,2,3-triazole ring can be obtained with an efficient yield, and a surplus by-product is not generated, which is a characteristic. It is conceivable that a 1,4- or 1,5-disubstituted triazole ring can be generated by the reaction, and it is possible to obtain a triazole ring regioselectively using a copper catalyst.

[C40]

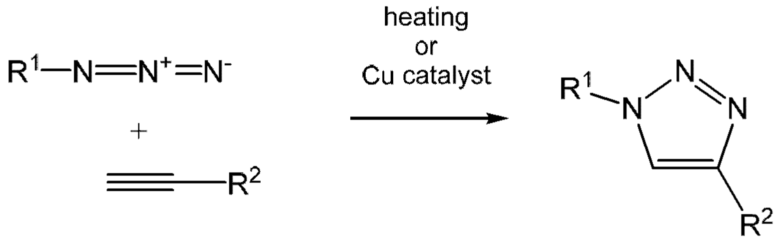


In addition, the Huisgen reaction where no copper catalyst is used has been reported by Wittig and Krebs. That is, the Huisgen reaction is a reaction where a cycloadduct can be obtained simply by mixing cyclooctyne and phenyl azide (in the following formula, $R^3$ is phenyl). In the present reaction, since the triple bond of cyclooctyne is significantly distorted, elimination of the distortion by a reaction with phenyl azide becomes a driving force, and the reaction progresses spontaneously, whereby no catalysts are required.

[C41]

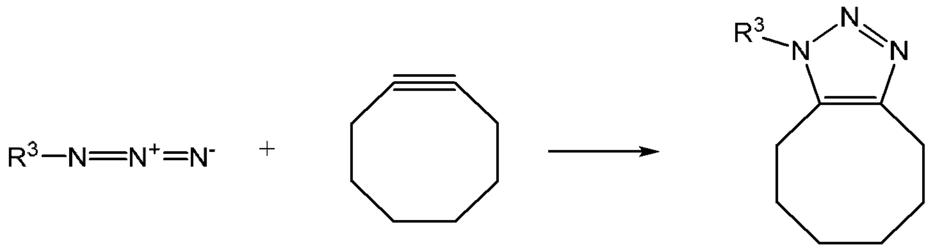

As described above, in the Huisgen reaction, it is possible to use an azide compound having a substituted primary azide, secondary azide, tertiary azide, aromatic azide or the like and a compound having a terminal or cyclic alkyne group that is a complementary reactive group of the azide group. In addition, in the Huisgen reaction, since almost only the azide group and the alkyne group react, it is possible to substitute a variety of functional groups (for example, an ester group, a carboxyl group, an alkenyl group, a hydroxyl group, an amino group and the like) into the reaction substrate.

In several embodiments, in order to easily and efficiently form a crosslink by a 1,2,3-triazole ring between alginic acid molecules within a short period of time without generating an undesirable by-product and using a copper catalyst to avoid cytotoxicity attributed to the copper catalyst, as the alkyne group in the Huisgen reaction, for example, the cyclic alkyne group (cyclooctyne group and the like).

In a preferable embodiment of a method for crosslinking the chemically modified alginic acid derivative, in the Huisgen reaction, an undesirable by-product is almost not formed. Therefore, it becomes possible to incorporate a variety of pharmacological ingredients, such as cells or bioactive substances, into the core layer of the structure of the present invention.

3-2. Chemically modified alginic acid derivative (cinnamic acid type)

A chemically modified alginic acid derivative into which a photoreactive group has been introduced is a chemically modified alginic acid derivative in which part of carboxyl groups of alginic acid have been substituted with photoreactive groups of Formula (C-I) below [in the formula, the right side of the broken line is not included]:

[C42]

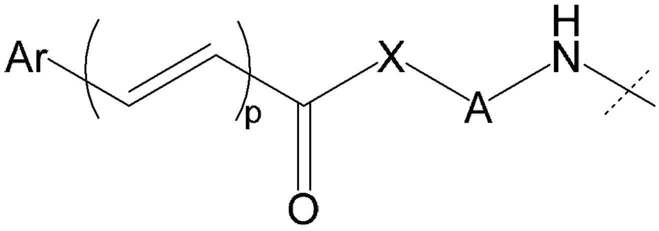

(C-I)

[the definitions of Ar, p, -X- and -A- are as described below, for example, the same as the definitions in the embodiment [8-1], preferably, the photoreactive groups described in [8-2] or [8-3], that is, a chemically modified alginic acid derivative having photoreactive groups in parts of carboxyl groups of alginic acid. A particularly preferable embodiment is the chemically modified alginic acid derivative described in [8-3]. The photoreactive group (referred to as "photocrosslinking group" in some cases) includes a portion where cyclization proceeds in a photoreaction (photoreactive portion). The photoreactive portion needs to be a portion where a dimerization reaction (cyclization reaction) or a polymerization reaction occurs by light irradiation, and specific examples thereof include a cinnamic acid moiety, a substituted cinnamic acid moiety, a phenylpenta-2,4-dienoic acid moiety, a substituted phenylpenta-2,4-dienoic acid moiety, a heterocyclic substituted acrylic acid moiety and the like. An alkene portion in Formula (C-I) is shown as a trans form as one form and may be a bonding style where a dimerization reaction (cyclization reaction) or a polymerization reaction occurs by light irradiation, in the case of p = 1, the alkene portion may be a trans form or a cis form, in the case of p = 2, the alkene portion may be a bonding style where a trans form and a cis form are combined together, and photoreactive groups satisfying what has been described above are considered to be included in Formula (C-I).

Among these photoreactive portions, a portion having a vinylene group capable of forming a cyclobutane ring by a dimerization reaction is preferable, and, for example, a cinnamic acid moiety, a substituted cinnamic acid moiety, a phenylpenta-2,4-dienoic acid moiety, a substituted phenylpenta-2,4-dienoic acid moiety, and a heterocyclic substituted acrylic acid moiety are preferable.

In addition, a spacer for holding the photoreactive portion and alginic acid at a certain distance by bonding to both may bond to the photoreactive group. A derivative in which a spacer bonds to the photoreactive portion such as a cinnamic acid moiety, a substituted cinnamic acid moiety, a phenylpenta-2,4-dienoic acid moiety, a substituted phenylpenta-2,4-dienoic acid moiety or a heterocyclic substituted acrylic acid moiety is most preferable as the photoreactive group (photolinking group).

Here, "the photoreactive group has been introduced" means that one or more arbitrary carboxyl groups of alginic acid form an azide bond with a terminal amino group of a linker in the photoreactive group, whereby the one or more arbitrary carboxyl groups of alginic acid and the photoreactive group bond to each other through the linker.

3-2-1. Photoreactive portion and linker

Here, there will be cases where, in Formula (C-I), which is the photoreactive group, a partial structural formula (C-I-S) [in the formula, the right side of the broken line is not included]:

[C43]

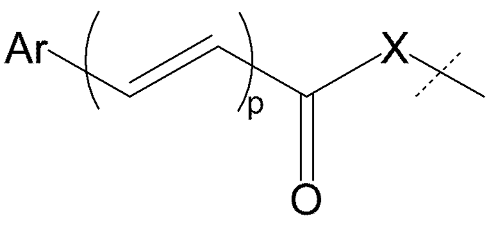

(C-I-S)

is referred to as "photoreactive portion", and there will be cases where Formula -A-below [in the formula, the outsides of the broken lines are not included]:

[C44]

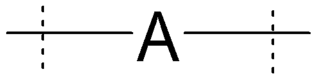

(A)

is referred to as "linker."

In the photoreactive portion, Ar is a $C_{6-10}$ aryl group (in the $C_{6-10}$ aryl group, one to three groups arbitrarily selected from a hydroxyl group, a cyano group, a nitro group, a halogen atom, a $C_{1-6}$ alkyl group, a halogenated $C_{1-6}$ alkyl group, a $C_{1-6}$ alkoxy group, and a - $NR^AR^B$ group ($R^A$ and $R^B$ in the $-NR^AR^B$ group are each independently a group selected from a hydrogen atom, a $C_{1-6}$ alkyl group, a $C_{2-7}$ alkanoyl group or a $C_{1-6}$ alkylsulfonyl group (here, $-NH_2$, $-NH(C_{1-6}$ alkyl group), $-N(C_{1-6}$ alkyl group$)_2$ and $-N(C_{1-6}$ alkyl group$)_2$ are excluded)) may be substituted with a hydrogen atom on a ring, in a case where the $C_{1-6}$ alkyl group and the $C_{1-6}$ alkoxy group or two $C_{1-6}$ alkoxy groups are included adjacent to each other in the $C_{6-10}$ aryl group as substituents, the $C_{1-6}$ alkyl group and the $C_{1-6}$ alkoxy group may bond to each other between carbon atoms in alkyl groups of the respective groups, from which one arbitrary hydrogen atom has been eliminated, or between carbon atoms in the respective alkyl groups of the two $C_{1-6}$ alkoxy groups, from which one arbitrary hydrogen atom has been eliminated, to form a cyclic ether or, in a case where the $C_{1-6}$ alkoxy group and an $-NHR^G$ group ($R^G$ in the $-NHR^G$ group is a $C_{2-7}$ alkanoyl group or a $C_{1-6}$ alkylsulfonyl group) are included adjacent to each other in the $C_{6-10}$ aryl group as substituents, carbon atoms in the alkyl group of the $C_{1-6}$ alkoxy group, from which one arbitrary hydrogen atom has been eliminated, and nitrogen atoms in the $-NHR^G$ group, from which one hydrogen atom has been eliminated, may bond to each other to form a 3-N-($C_{2-7}$ alkanoyl)oxazolidine ring, a 3-N-($C_{1-6}$ alkylsulfonyl)oxazolidine ring, a 4-N-($C_{2-7}$ alkanoyl)morpholine ring, a 4-N-($C_{1-6}$ alkylsulfonyl) morpholine ring, a 4-N-($C_{2-7}$ alkanoyl)-1,4-oxazepane ring or a 4-N-($C_{1-6}$ alkylsulfonyl)-1,4-oxazepane ring);

As the linker (-A-), an arbitrary linear group can be used as long as the photoreaction of the photoreactive portion is not impaired. Specifically, Formulae (AL-1) to (AL-4) [in each formula, the outsides of the broken lines at both ends are not included]:

[C45]

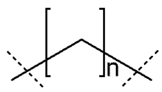

(AL-1)

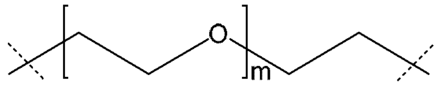

(AL-2)

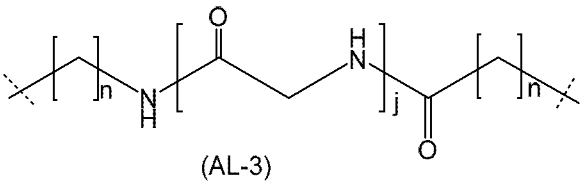

(AL-3)

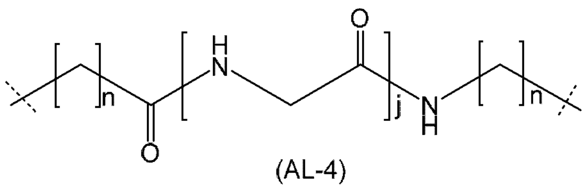

(AL-4)

(in Formulae (AL-1) to (AL-4), n represents an integer of 1 to 18; m represents an integer of 1 to 9; j represents an integer of 0 to 9;

a plurality (for example, one to 10 or one to five) of hydrogen atoms in a methylene group ($-CH_2-$) in Formulae (AL-1) to (AL-4) may be substituted with groups selected from a halogen atom, a hydroxyl group, a $C_{1-6}$ alkyl group, a hydroxy $C_{1-6}$ alkyl group, a thiol $C_{1-6}$ alkyl group, a $C_{1-6}$ alkylthio $C_{1-6}$ alkyl group, a carboxy $C_{1-6}$ alkyl group, a $-NR^aR^b$ group, a $(R^aR^bN)$-$C_{1-6}$ alkyl group, a $(R^aR^bN)C(=O)$-$C_{1-6}$ alkyl group (in the $-NR^aR^b$ group, the $(R^aR^bN)$-$C_{1-6}$ alkyl group or the $(R^aR^bN)C(=O)$-$C_{1-6}$ alkyl group, $R^a$ and $R^b$ are each independently a group selected from a hydrogen atom, a $C_{1-6}$ alkyl group, a $C_{2-7}$ alkanoyl group or a $C_{1-6}$ alkylsulfonyl group), a guanidino $C_{1-6}$ alkyl group, a $C_{7-16}$ aralkyl group, a hydroxy $C_{6-10}$ aryl $C_{1-6}$ alkyl group and a heteroaryl $C_{1-6}$ alkyl group; in a case where two hydrogen atoms in the methylene group ($-CH_2-$) in Formulae (AL-1) to (AL-4) are substituted with the $C_{1-6}$ alkyl groups, the $C_{1-6}$ alkyl groups may bond to each other between carbon atoms of the respective $C_{1-6}$ groups, from which one arbitrary hydrogen atom has been eliminated, to form a $C_{3-8}$ cycloalkyl ring; the -NH- groups in Formula (AL-3) or Formula (AL-4) may form a non-aromatic heterocycle together with the substituent substituting an adjacent carbon atom). Here, in Formula (C-I), in a case where Ar is a phenyl group, p is one, -A- is Formula (AL-1) and n is three, in the phenyl group of Ar, one to three groups arbitrarily selected from a group of substituents that may

substitute hydrogen atoms on the Ar ring substitute the hydrogen atoms on the phenyl ring.

The -X- represents -O-, -NH- or -N($C_{1-6}$ alkyl)- and is preferably -O-.

The p represents an integer of 1 or 2.

In the present specification, unless particularly otherwise described, examples of "halogen atom" include a fluorine atom, a chlorine atom, a bromine atom, an iodine atom and the like.

In the present specification, unless particularly otherwise described, "halogenation" in "halogenated $C_{1-6}$ alkyl group" and the like means having several, preferably one to five, "halogen atoms" described above as substituents.

In the present specification, unless particularly otherwise described, examples of "$C_{1-6}$ alkyl group" include groups such as methyl, ethyl, propyl, isopropyl, butyl, isobutyl, sec-butyl, tert-butyl, pentyl, isopentyl, neopentyl and hexyl.

In the present specification, unless particularly otherwise described, "halogenated $C_{1-6}$ alkyl group" means a group in which "$C_{1-6}$ alkyl" has been arbitrarily substituted with several, preferably one to five, halogen atoms, and examples thereof include groups such as fluoromethyl, difluoromethyl, trifluoromethyl, 2,2,2-trifluoroethyl, 1,1,2,2-tetrafluoroethyl and pentafluoroethyl.

In the present specification, unless particularly otherwise described, "$C_{1-6}$ alkoxy group" represents alkoxy in which "$C_{1-6}$ alkyl" has bonded to an oxygen atom, and examples thereof include groups such as methoxy, ethoxy, propoxy, isopropoxy, butoxy, isobutoxy, sec-butoxy, tert-butoxy, pentyloxy and hexyloxy.

In the present specification, unless particularly otherwise described, examples of "$C_{6-10}$ aryl group" include groups such as phenyl, 1-naphthyl, 2-naphthyl, indanyl, indenyl and 1,2,3,4-tetrahydronaphthyl.

In the present specification, unless particularly otherwise described, "$C_{3-8}$ cycloalkyl ring" means a cyclic saturated hydrocarbon ring (including monocyclic type or polycyclic type) having 3 to 8 carbon atoms, and examples thereof include rings such as cyclopropane, cyclobutane, cyclopentane, cyclohexane, cyclopentane and cyclooctane.

In the present specification, unless particularly otherwise described, "non-aromatic heterocycle" means "three to 14-membered saturated or unsaturated non-aromatic heterocycle."

In the present specification, unless particularly otherwise described, "three to 14-membered saturated or unsaturated non-aromatic heterocycle" means a three to 14-membered saturated or unsaturated heterocycle containing one to four hetero atoms selected from an oxygen atom, a sulfur atom and a nitrogen atom.

In the present specification, unless particularly otherwise described, examples of "non-aromatic heterocycle" include rings such as aziridine, azetidine, pyrrolidine, pyrazolidine, oxazolidine, thiazolidineisoxazolidine, isothiazolidine, imidazolidine, piperidine, piperazine, morpholine, thiomorpholine, oxazepane, diazepane, thiazepane, oxazocane, diazocane, thiazocane and oxazine.

In the present specification, unless particularly otherwise described, "five or six-membered non-aromatic heterocycle" is five-membered or six-membered heterocycles among the "non-aromatic heterocycles", and a certain embodiment is piperidine or piperazine.

In the present specification, unless particularly otherwise described, "heteroaryl" and "aromatic heterocycle" groups mean monocyclic, polycyclic or condensed ring-type (here, in the case of polycyclic or condensed ring-type, the heteroaryl may be partially hydrogenated) five to 14-membered, preferably five to eight-membered, more preferably five to seven-membered and still more preferably five or six-membered, heteroaryl having one to five, preferably one to three, heteroatoms selected from the group consisting of a nitrogen atom, a sulfur atom and an oxygen atom.

In the present specification, unless particularly otherwise described, "five or six-membered heteroaryl" and "five or six-membered aromatic heterocycle" groups are five or six-membered heteroaryl comprising one to four hetero atoms selected from a nitrogen atom, a sulfur atom and an oxygen atom, and, unless particularly otherwise described, "five or six-membered heteroaryl" means a monovalent group that is generated by removing an arbitrary hydrogen atom from the heteroaryl ring. Examples thereof include groups such as pyrrolyl, furyl, thienyl, imidazolyl, pyrazolyl, oxazolyl, isoxazolyl, thiazolyl, isothiazolyl, 1,2,3-triazolyl, 1,2,4-triazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, tetrazolyl, pyridyl, pyridazinyl, pyrimidinyl, pyrazinyl, 1,2,3- triazinyl, 1,2,4-triazinyl, 1,3,5-triazinyl, 2H-1,2,3-thiadiazinyl, 4H-1,2,4-thiadiazinyl, 6H-1,3,4-thiadiazinyl, pyridazine-3(2H)-one, pyrimidin-2(1H)-one, pyrazin-2(1H)-one and pyridin-2(1H)-one.

In the present specification, unless particularly otherwise described, "heteroaryl $C_{1-6}$ alkyl group" means a group obtained by substituting an arbitrary hydrogen atom of the "heteroaryl group" with the "$C_{1-6}$ alkyl group", and examples thereof include groups such as a 2-pyridylmethyl group, a 4-imidazolylmethyl group and a 3-indolylmethyl group.

In several embodiments, the photoreactive group (photolinking group portion) absorbs light (for example, the wavelength is near 180 to 650 nm) and is photodimerizable, which enables the photoreactive group to dimerize (for example, dimerize to a truxillic acid derivative).

In addition, the introduction of a linker makes a photocrosslinking reaction proceed even in a case where the introduction rate of the photoreactive group (photocrosslinking group) is low. The photocrosslinking reaction makes the chemically modified alginic acid derivative form a three-dimensional network structure through the photocrosslinking

group. A preferable chemically modified alginic acid derivative is a chemically modified alginic acid derivative having improved stability after photocrosslinking.

The chemically modified alginic acid derivative into which the reactive group represented by Formula (C-I) has been introduced in the present specification can be manufactured by, for example, a method of the following formula by substituting the photoreactive group into part of carboxyl groups of alginic acids and thereby introducing the reactive group into the alginic acids. In the present specification, both "(ALG)" and "(alginic acid)" indicate alginic acid, in structural formulae, there are cases where the amide bond (-NHCO-) through an arbitrary carboxyl group of alginic acid is expressed differently as "(ALG)" and "(alginic acid)"; however, it is common in all cases that the amide bond is present between the linker and alginic acid. That is, -NHCO-(ALG) and -NH-(alginic acid) represent the same structure.

[C46]

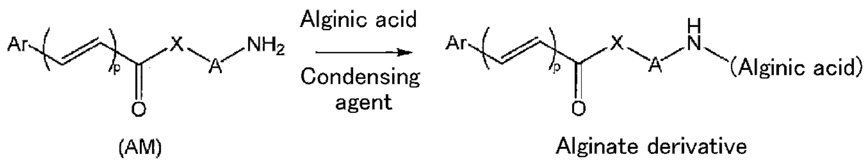


The weight-average molecular weight of the novel chemically modified alginic acid derivative into which the photoreactive group represented by Formula (C-I) has been introduced is 100,000 Da to 3,000,000 Da, preferably 300,000 Da to 2,500,000 Da and more preferably 500,000 Da to 2,000,000 Da. The molecular weight of the alginic acid derivative can be obtained by the same method as for the above-described alginic acids.

In the present specification, the photoreactive group represented by Formula (C-I) does not need to bond to all carboxyl groups in the alginic acid constituting unit.

In the present specification, the introduction rate of the photoreactive group represented by Formula (C-I) in the chemically modified alginic acid derivative is preferably 0.5% to 30%, more preferably 0.5% to 20% and still more preferably 1.0% to 15%.

The introduction rate of the photoreactive group represented by Formula (C-I) is a value expressing the number of uronic acid monosaccharide units into which the photoreactive group represented by Formula (C-I) has been introduced in uronic acid monosaccharide units, which are the repeating units of alginic acids in percentage. In the present specification, unless particularly otherwise described, "%" that is used for the introduction rate of the photoreactive group represented by Formula (C-I) in the chemically modified alginic acid derivative means "mol%." The introduction rate of the photoreactive group represented by Formula (C-I) can be obtained by a method described below in the examples to be described below.

4. Method for synthesizing chemically modified alginic acid derivative

The chemically modified alginic acid derivative (Huisgen type) represented by Formula (HA-I) or Formula (HA-II) can be manufactured with reference to PCT/JP2019/023478 (filed on June 13, 2019). In addition, the chemically modified alginic acid derivatives (cinnamic acid type) represented by Formula (C-I) can be manufactured with reference to PCT/JP2019/007655 (filed on February 27, 2019).

The chemically modified alginic acid derivative (Huisgen type (B)) represented by Formula (HB-I) or Formula (HB-II) can each be manufactured by a condensation reaction between an amine derivative (AM-1) represented by $H_2N-L^1-Akn$ (in the formula, $L^1$ and Akn are the same as the definitions in the embodiment [3-1]) or an amine derivative (AM-2) represented by $H_2N-L^2-N_3$ (in the formula, $L^2$ is the same as the definition in the embodiment [3-1]) and an arbitrary carboxyl group of alginic acids using a condensing agent.

[C47]

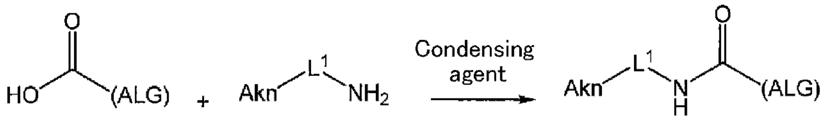


Alginic acid (AM-1) (HB-I)

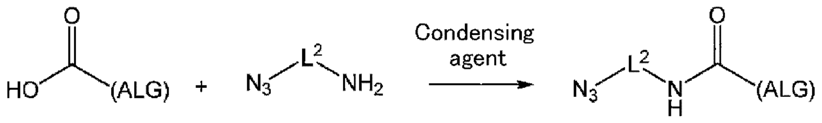


Alginic acid (AM-2) (HB-II)

[Manufacturing method of chemically modified alginic acid derivative of Formula (HB-I)]

The chemically modified alginic acid derivative of Formula (HB-I) can be manufactured by performing a condensation reaction at a temperature of 0°C to 50°C using a 0.5 wt% to 1 wt% alginic acid aqueous solution and an amine represented by Formula (AM-1) according to a method described in, for example, "Experimental Chemistry 5th Edition 16, Synthesis IV of Organic Compounds, Carboxylic Acids and Derivatives, Esters, pp. 35 to 70, Acid Amides and Acid Imides, pp. 118 to 154, Amino Acids/Peptides, pp. 258 to 283, 2007, Maruzen" in the presence of a condensing agent selected from 1,3-dicyclohexylcarbodiimide (DCC), 1-ethyl-3-(3-dimethylaminopropyl)carbodiimide hydrochloride (WSC/HCl), benzotriazol-1-yloxytris(dimethylamino)phosphonium hexafluorophosphate (BOP reagent), bis(2-oxo-3-oxazolidinyl)phosphinic chloride (BOP-Cl), 2-chloro-1,3-dimethylimidazolinium hexafluorophosphate (CIP) or 4-(4,6-dimethoxy-1,3,5-triazin-2-yl)-4-methylmorpholinium chloride (DMT-MM) in a solvent mixture of a solvent selected from ether solvents such as tetrahydrofuran and 1,4-dioxane, alcohol-based solvents such as methanol, ethanol and 2-propanol, polar solvents such as N,N-dimethylformamide and the like, which is as much as alginic acid is not precipitated, and water in the presence or absence of an inorganic base such as sodium bicarbonate or sodium carbonate or an organic base such as triethylamine or pyridine.

[Manufacturing method of chemically modified alginic acid derivative of Formula (HB-II)]

The chemically modified alginic acid derivative of Formula (HB-II) can be manufactured by performing a reaction according to the [manufacturing method of chemically modified alginic acid derivative of Formula (HB-1)] using a 0.5 wt% to 1 wt% alginic acid aqueous solution and an amine represented by Formula (AM-2).

In the above-described manufacturing method of the chemically modified alginic acid derivative of Formula (HB-I) or the chemically modified alginic acid derivative of Formula (HB-II), the introduction rate of the amine represented by Formula (AM-1) or Formula (AM-2) can be adjusted by appropriately selecting and combining reaction conditions of the following (i) to (v) and the like in consideration of the properties and the like of the amine. (i) An increase or decrease in the equivalent of the condensing agent, (ii) an increase or decrease in the reaction temperature, (iii) the extension or shortening of the reaction time, (iv) the adjustment of the concentration of alginic acid in the reaction substrate, (v) the addition of an organic solvent that is mixed with water to increase the solubility of the amine of Formula (AM-1) or Formula (AM-2) and the like.

Hereinafter, a method for manufacturing, among the amines represented by Formula (AM-1) or Formula (AM-2), more specific amines will be described.

In each of the following manufacturing methods, the definitions of m1, n1, m2a, n2a, p2a, m2b, n2b, p2b, m3, n3, p3, m4a, n4a, m4b, n4b, m5a, n5a, p5a, q5a, m5b, n5b, p5b, q5b, m6a, n6a, p6a, m6b, n6b, p6b, m7, n7, m8a, n8a, m8b, n8b, m9a, n9a, p9a, m9b, n9b, p9b, m10, n10, p10, x1, x2, x2a, y2a, x2b, y2b, x3, y3, x4, y4, z4, x5a, y5a, x5b, y5b, x6a, y6a, z6a, v6a, x6b, y6b, z6b, v6b, x7a, y7a, z7a, x7b, y7b, z7b, x8a, y8a, z8a, x8b, y8b, z8b, x9a, y9a, z9a, x9b, y9b and z9b are the same definitions as described in the embodiment [3-1]; $P^1$ is a protective group of an amino group selected from a -C(O)O-tert Bu group, a -C(O)O-Bn group, a $-C(O)CH_3$ group, a $-C(O)CF_3$ group, a $-SO_2Ph$, a $-SO_2PhMe$ group, a $-SO_2Ph(NO_2)$ group and the like; E is a leaving group such as a halogen atom (a fluorine atom, a chlroine atom, a bromine atom, an iodine atom or the like), a -OTs group or a -OMs group.

In addition, in each of the following manufacturing methods, the protection and deprotection the protection and deprotection of the protective group $P^1$ can be performed according to methods well known by publications, for example, a deprotection method described in "Protective Groups in Organic Synthesis 4th Edition, 2007, John Wiley & Sons, Greene et al".

[Manufacturing method AM-A] Method for manufacturing amine represented by Formula (AM-1-B 1):

[C48]

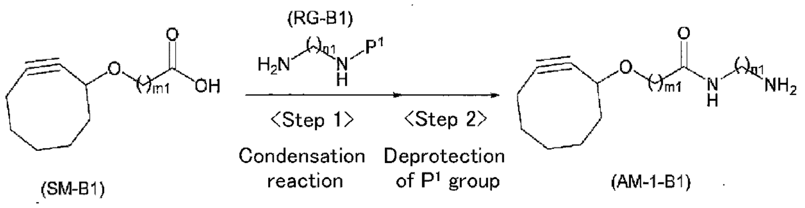


An amine represented by Formula (AM-1-B1) or a salt thereof can be manufactured by performing the same condensation reaction as in the [manufacturing method of chemically modified alginic acid derivative of Formula (HB-I)] and subsequently deprotecting a protective group $P^1$ using a compound of Formula (SM-B1) and a compound of Formula (RG-B1) [the compound of Formula (SM-B1) and the compound of Formula (RG-B1) are commercially available compounds or compounds that can be manufactured from commercially available compounds by a manufacturing method well known by publications].

[Manufacturing method AM-B] Method for manufacturing amines represented by Formula (AM-1-B2a) and Formula (AM-1-B2b):

[C49]

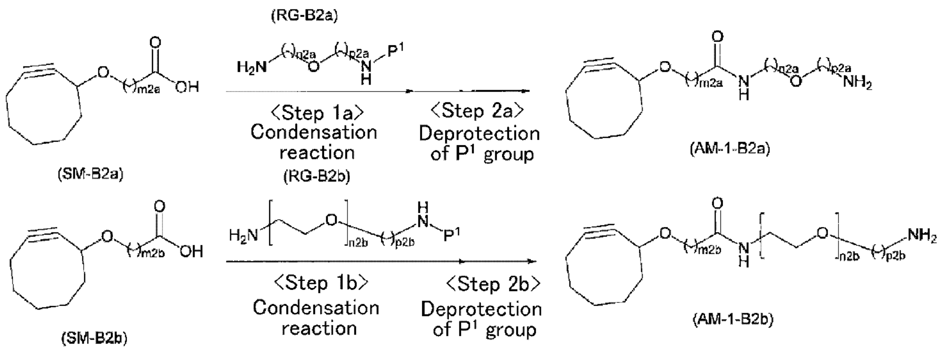


An amine represented by Formula (AM-1-B2a) or a salt thereof can be manufactured by causing a reaction in the same manner as in [Manufacturing method AM-A] using a compound of Formula (SM-B2a) and a compound of Formula (RG-B2a) [the compound of Formula (SM-B2a) and the compound of Formula (RG-B2b) are commercially available compounds or compounds that can be manufactured from commercially available compounds by a manufacturing method well known by publications]. Similarly, an amine represented by Formula (AM-1-B2b) or a salt thereof can be manufactured by performing a reaction in the same manner using a compound of Formula (SM-B2b) and a compound of Formula (RG-B2b) [the compound of Formula (SM-B2b) and the compound of Formula (RG-B2b) are commercially available compounds or compounds that can be manufactured from commercially available compounds by a manufacturing method well known by publications].

[Manufacturing method AM-C] Method for manufacturing amine represented by Formula (AM-1-B3):

[C50]

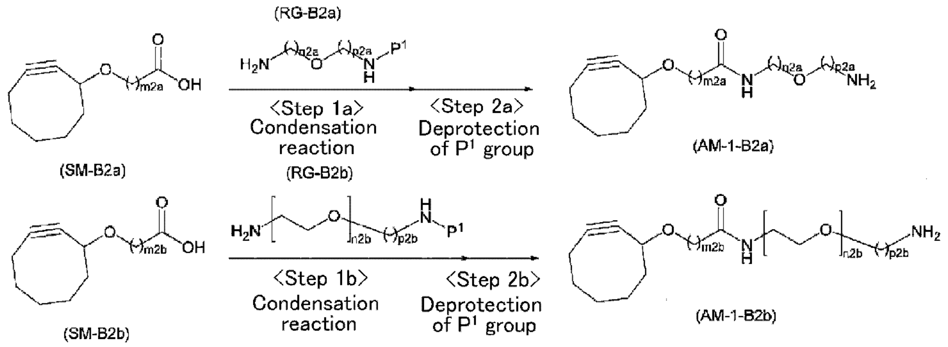


An amine represented by Formula (AM-1-B3) or a salt thereof can be manufactured by performing a reaction according to the above-described synthesis scheme (a reaction in each step is according to the reaction described in [Manufacturing method AM-A]) using a compound of Formula (SM-B3) [the compound of Formula (SM-B3) is a commercially available compound or a compound that can be manufactured from a commercially available compound by a manufacturing method well known by publications]. In the scheme, the compounds of Formula (RG-B3), Formula (RG-B3-1) and Formula (RG-B3-2) are commercially available compounds or compounds that can be manufactured from commercially available compounds by a manufacturing method well known by publications.

[Manufacturing method AM-D] Method for manufacturing amines represented by Formula (AM-1-B5a) and Formula (AM-1-B5b):

[C51]

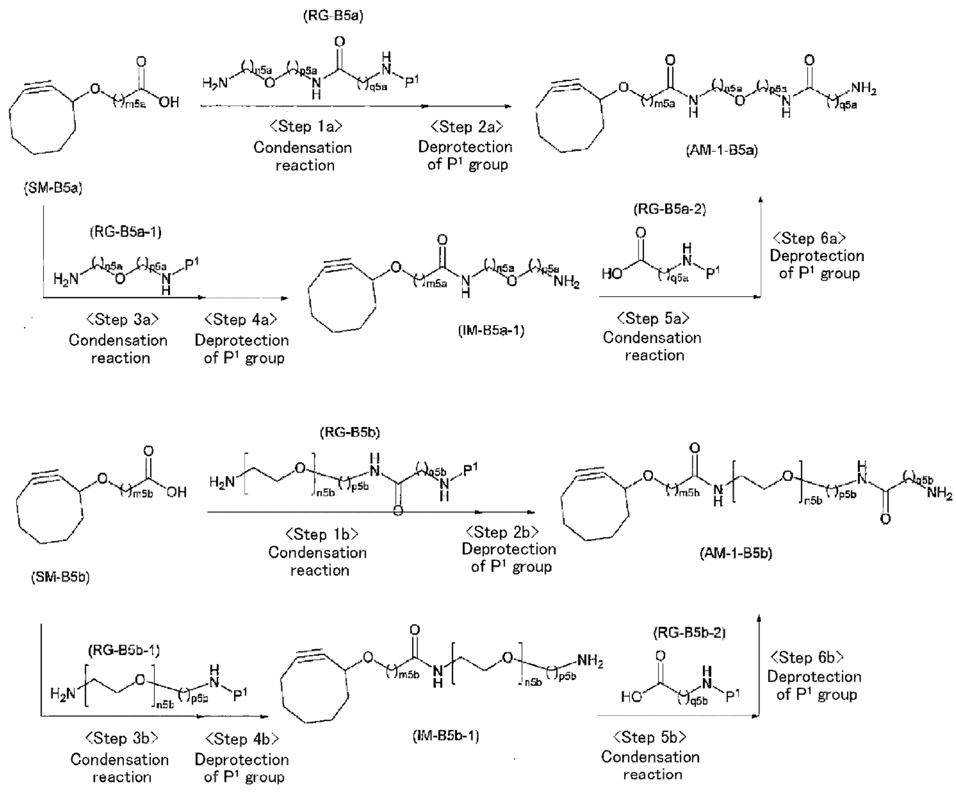


Amines represented by Formula (AM-1-B5a) and Formula (AM-1-B5b) or salts thereof can be manufactured

by performing a reaction according to the above-described synthesis scheme (a reaction in each step is according to the reaction described in [Manufacturing method AM-A]). In the scheme, the compounds of Formula (SM-B5a), Formula (RG-B5a), Formula (RG-B5a-1), Formula (RG-B5a-2), Formula (SM-B5b), Formula (RG-B5b), Formula (RG-B5b-1) and Formula (RG-B5b-2) are commercially available compounds or compounds that can be manufactured from commercially available compounds by a manufacturing method well known by publications.

[Manufacturing method AM-E] Method for manufacturing amines represented by Formula (AM-1-B6a) and Formula (AM-1-B6b):

[C52]

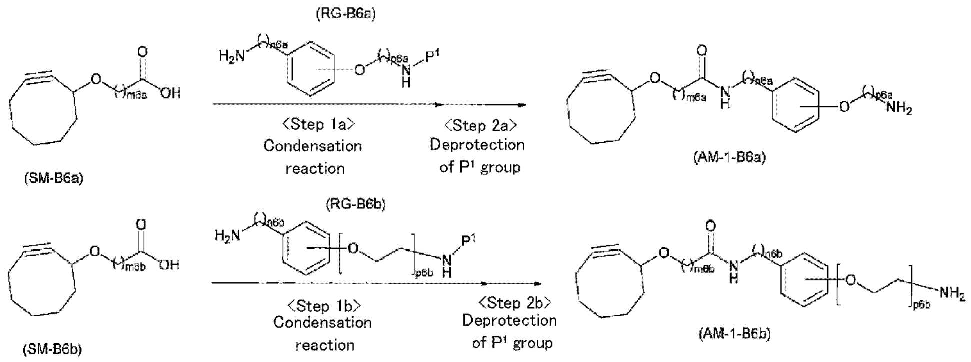


Amines represented by Formula (AM-1-B6a) and Formula (AM-1-B6b) or salts thereof can be manufactured by performing a reaction according to the above-described synthesis scheme (a reaction in each step is according to the reaction described in [Manufacturing method AM-A]). In the scheme, the compounds of Formula (SM-B6a), Formula (RG-B6a), Formula (SM-B6b) and Formula (RG-B6b) are commercially available compounds or compounds that can be manufactured from commercially available compounds by a manufacturing method well known by publications.

[Manufacturing method AM-F] Method for manufacturing amine represented by Formula (AM-1-B 10):

[C53]

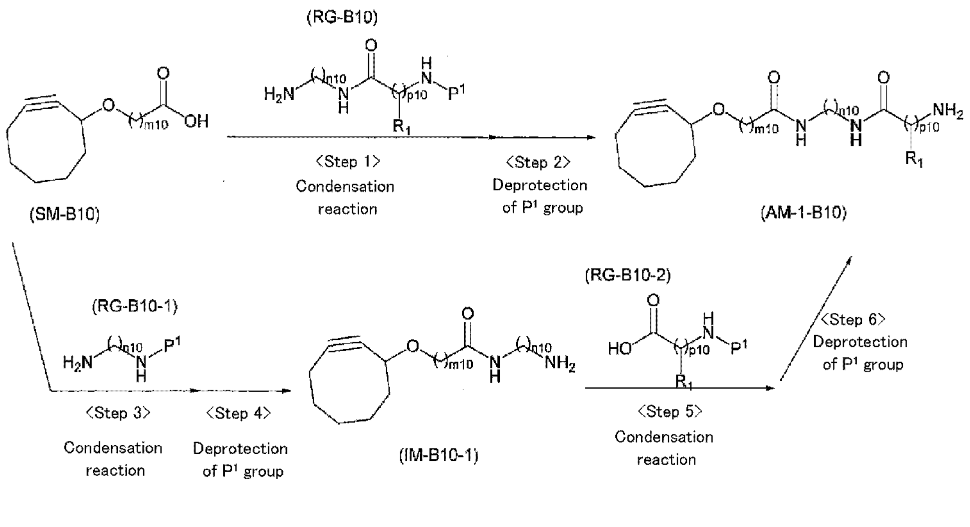


An amine represented by Formula (AM-1-B10) or a salt thereof can be manufactured by performing a reaction

according to the above-described synthesis scheme (a reaction in each step is according to the reaction described in [Manufacturing method AM-A]). In the scheme, the compounds of Formula (SM-B10), Formula (RG-B10), Formula (RG-B10-1) and Formula (RG-B 10-2) are commercially available compounds or compounds that can be manufactured from commercially available compounds by a manufacturing method well known by publications.

[Manufacturing method AM-G] Method for manufacturing amines represented by Formula (AM-1-B4a) and Formula (AM-1-B4b):

[C54]

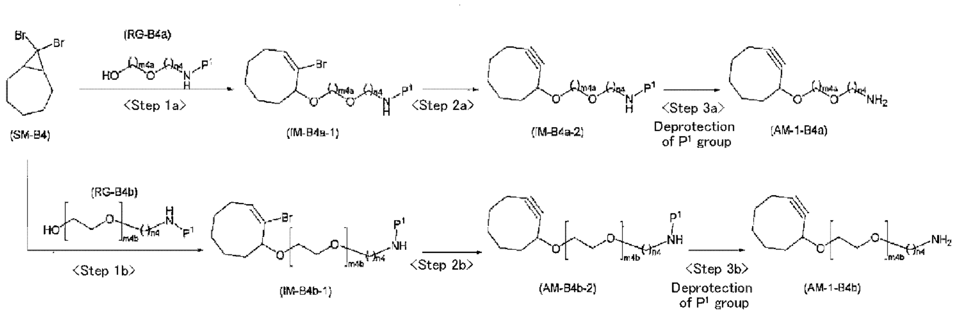


An amine represented by Formula (AM-1-B4a) or a salt thereof can be manufactured by <step 1a> performing a reaction in the presence of a reagent such as silver trifluoromethanesulfonate or $AgClO_4$ in a solvent that does not get involved in the reaction, such as toluene or dichloromethane, to obtain a compound of Formula (IM-B4a-1), <step 2a>, subsequently, performing a debromination reaction using a base such as sodium hydride or NaOMe to obtain a compound of Formula (IM-B4a-2) and <step 3a>, furthermore, deprotecting a protective group $P^1$ using a compound of Formula (SM-B4) and a compound of Formula (RG-B4a) [the compound of Formula (SM-B4) and the compound of Formula (RG-B4a) are commercially available compounds or compounds that can be manufactured from commercially available compounds by a manufacturing method well known by publications] according to a method well known by publications, for example, the method described in "Journal of the America Chemical Society, 126 (46), pp. 15046 to 15047, 2004" or the like.

An amine represented by Formula (AM-1-B4b) or a salt thereof can be manufactured by performing a reaction in the same manner according to the above-described scheme using Formula (RG-B4b) instead of Formula (RG-B4a).

[Manufacturing method AM-H] Method for manufacturing amines represented by Formula (AM-1-B8a) and Formula (AM-1-B8b):

[C55]

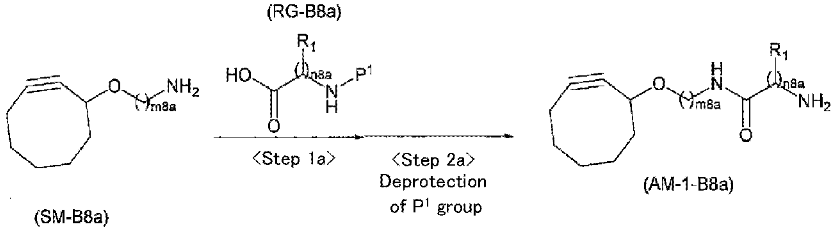


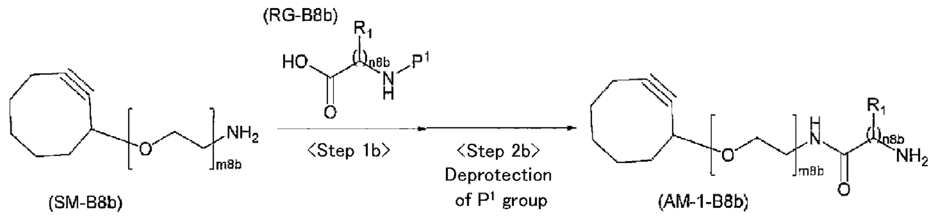


Amines represented by Formula (AM-1-B8a) and Formula (AM-1-B8b) or salts thereof can be manufactured by performing a reaction according to the above-described synthesis scheme (a reaction in each step is according to the reaction described in [Manufacturing method AM-A]). In the scheme, the compound of Formula (SM-B8a) or Formula (SM-B8b) is a commercially available compound or a compound that can be manufactured according to the reaction described in [Manufacturing method AM-G], and the compound of Formula (RG-B8a) or Formula (RG-B8b) is a commercially available compound or a compound that can be manufactured from a commercially available compound by a manufacturing method well known by publications.

[Manufacturing method AM-J] Method for manufacturing amine represented by Formula (AM-1-B7):

[C56]

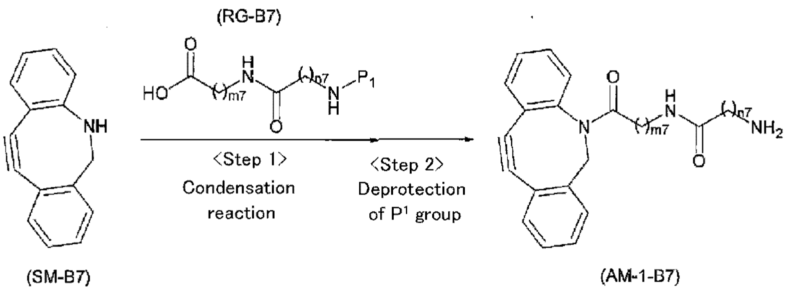


An amine represented by Formula (AM-1-B7) or a salt thereof can be manufactured by performing the same condensation reaction as in the [manufacturing method of chemically modified alginic acid derivative of Formula (HB-I)] and subsequently deprotecting a protective group $P^1$ using a compound of Formula (SM-B7) and a compound of Formula (RG-B7) [the compound of Formula (SM-B7) and the compound of Formula (RG-B7) are commercially available compounds or compounds that can be manufactured from commercially available compounds by a manufacturing method well known by publications].

[Manufacturing method AM-J-2] Method for manufacturing amine represented by Formula (AM-1-B7):

[C57]

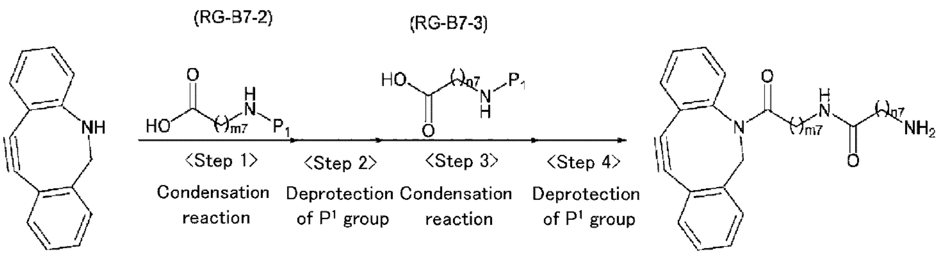


(SM-B7) (AM-1-B7)

An amine represented by Formula (AM-1-B7) or a salt thereof can be manufactured by performing the same condensation reaction as in the [manufacturing method of chemically modified alginic acid derivative of Formula (HB-I)] using a compound of Formula (SM-B7) and a compound of Formula (RG-B7-2) [the compound of Formula (SM-B7) and the compound of Formula (RG-B7-2) are commercially available compounds or compounds that can be manufactured by a manufacturing method well known by publications] (<step 1> and <step 2>), subsequently, deprotecting a protective group $P^1$, subsequently, performing the same condensation reaction as in the <step 1> using a compound of Formula (RG-B7-3) [which is a commercially available compound or a compound that can be manufactured from a commercially available product by a manufacturing method well known by publications] and, subsequently, deprotecting the protective group $P^1$.

[Manufacturing method AM-K] Method for manufacturing amines represented by Formula (AM-1-B9a) and Formula (AM-1-B9b):

[C58]

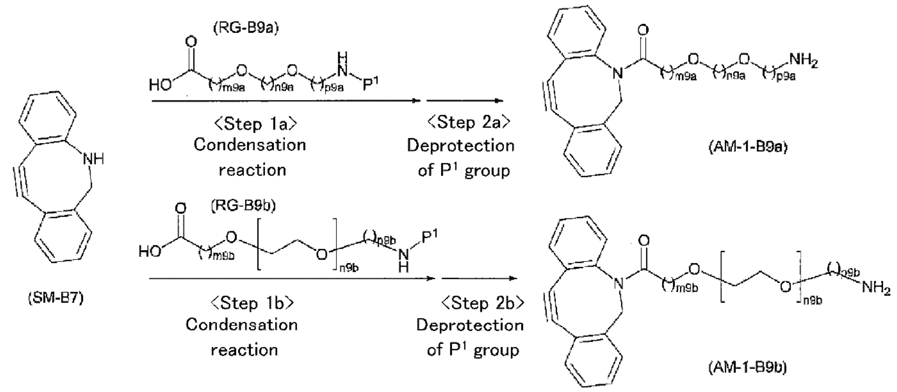


Amines represented by Formula (AM-1-B9a) and Formula (AM-1-B9b) or salts thereof can be manufactured by performing a reaction according to the above-described synthesis scheme (a reaction in each step is according to the reaction described in [Manufacturing method AM-J]). In the scheme, the compound of Formula (SM-B7), Formula (RG-B9a) or Formula (RG-B9b) is a commercially available compound or a compound that can be manufactured from a commercially available compound by a manufacturing method well known by publications.

[Manufacturing method AM-L] Method for manufacturing amine represented by Formula (AM-2-Z 1):

[C59]

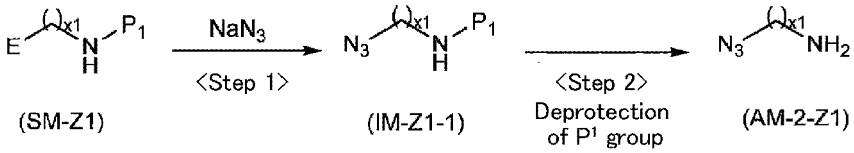


(SM-Z1) (IM-Z1-1) (AM-2-Z1)

An amine represented by Formula (AM-2-Z1) or a salt thereof can be manufactured by reacting $NaN_3$ in a solvent that does not get involved in a reaction such as dimethyl sulfoxide and then deprotecting the protective group $P^1$ using the compound of (SM-Z1) [the compound of Formula (SM-Z1) is a commercially available compound or a compound that can be manufactured from a commercially available compound by a manufacturing method well known by publications] according to a method well known by publications, for example, a method described in "Organometallics, 29 (23), pp. 6619 to 6622; 2010" or the like.

The amine represented by Formula (AM-2-Z1) or a salt thereof can also be procured as a commercially available product.

[Manufacturing method AM-M] Method for manufacturing amines represented by Formula (AM-2-Z2a) and Formula (AM-2-Z2b):

[C60]

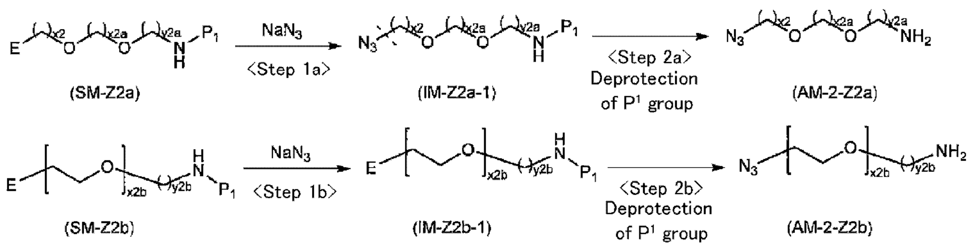


(SM-Z2a) (IM-Z2a-1) (AM-2-Z2a)

(SM-Z2b) (IM-Z2b-1) (AM-2-Z2b)

An amine represented by Formula (AM-2-Z2a) or Formula (AM-2-Z2b) or a salt thereof can be manufactured by reacting $NaN_3$ to introduce an azide group and then deprotecting the protective group $P^1$ in the same manner as in [Manufacturing method AM-L] using a compound of Formula (SM-Z2a) and a compound of Formula (SM-Z2b) [the compound of Formula (SM-Z2a) and the compound of Formula (SM-Z2b) are commercially available compounds or compounds that can be manufactured from commercially available compounds by a manufacturing method well known by publications]. The amine represented by Formula (AM-2-Z2a) or Formula (AM-2-Z2b) or a salt thereof can also be procured as a commercially available product.

[Manufacturing method AM-N] Method for manufacturing amine represented by Formula (AM-2-Z3):

[C61]

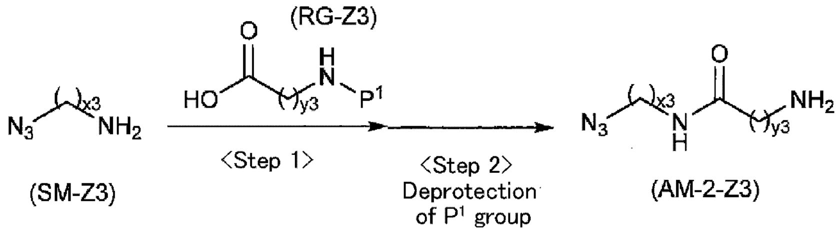


An amine represented by Formula (AM-2-Z3) or a salt thereof can be manufactured by performing the same condensation reaction as in the [manufacturing method of chemically modified alginic acid derivative of Formula (HB-I)] and subsequently deprotecting a protective group $P^1$ using a compound of Formula (SM-Z3) and a compound of Formula (RG-Z3) a compound of [the compound of Formula (SM-Z3) and the compound of Formula (RG-Z3) are commercially available compounds or compounds that can be manufactured from commercially available compounds by a manufacturing method well known by publications].

[Manufacturing method AM-O] Method for manufacturing amine represented by Formula (AM-2-Z4):

[C62]

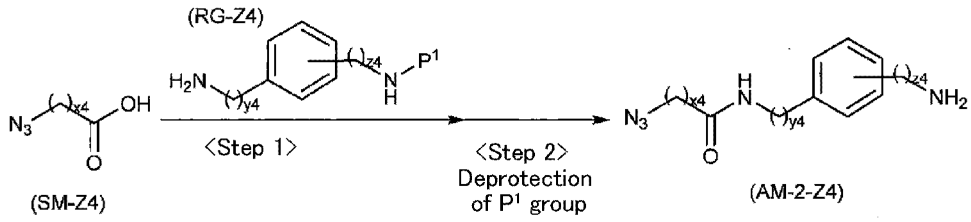


An amine represented by Formula (AM-2-Z4) or a salt thereof can be manufactured by performing the same condensation reaction as in the [manufacturing method of chemically modified alginic acid derivative of Formula (HB-I)] and subsequently deprotecting a protective group $P^1$ using a compound of Formula (SM-Z4) and a compound of Formula (RG-Z4) [the compound of Formula (SM-Z4) and the compound of Formula (RG-Z4) are commercially available compounds or compounds that can be manufactured from commercially available compounds by a manufacturing method well known by publications].

[Manufacturing method AM-P] Method for manufacturing amines represented by Formula (AM-2-Z5a) and Formula (AM-2-Z5b):

[C63]

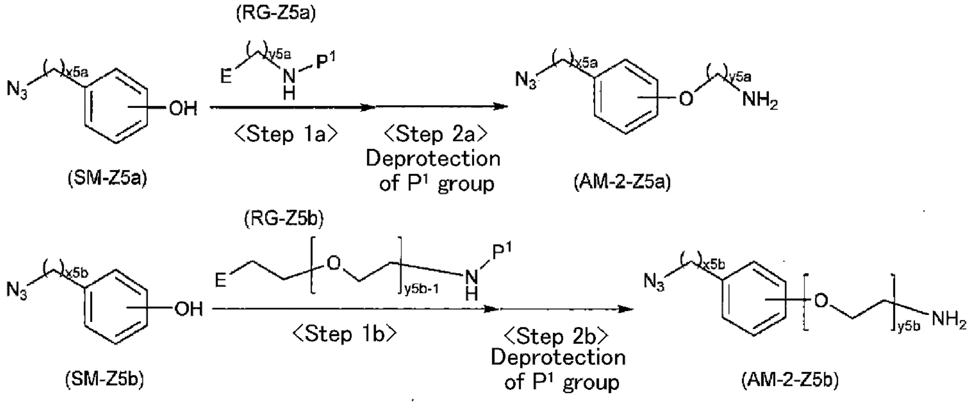


A compound into which a side chain had been introduced is obtained by causing a reaction in a solvent that does not get involved in the reaction such as tetrahydrofuran, N,N-dimethylformamide or N-methylpyrrolidone, dimethylsulfoxide in the presence of a base such as sodium hydride or potassium carbonate using a compound of Formula (SM-Z5a) and a compound of Formula (RG-Z5a) [the compound of Formula (SM-Z5a) and the compound of Formula (RG-Z5a) are commercially available compounds or compounds that can be manufactured from commercially available compounds by a manufacturing method well known by publications]. Subsequently, the protective groups $P^1$ are deprotected, whereby an amine represented by Formula (AM-2-Z5a) or a salt thereof can be manufactured.

Similarly, an amine represented by Formula (AM-2-Z5b) or a salt thereof can be manufactured by performing a reaction in the same manner using a compound of Formula (SM-Z5b) and a compound of Formula (RG-Z5b) [the compound of Formula (SM-Z5b) and the compound of Formula (RG-Z5b) are commercially available compounds or compounds that can be manufactured from commercially available compounds by a manufacturing method well known by publications].

Amines represented by Formula (AM-2-Z6a), Formula (AM-2-Z6b), Formula (AM-2-Z7a), Formula (AM-2-Z7b), Formula (AM-2-Z8a), Formula (AM-2 -Z8b), Formula (AM-2-Z9a) and Formula (AM-2-Z9b) and salts thereof can be manufactured by a manufacturing method shown in the following scheme according to the [Manufacturing method AM-A] to [Manufacturing method AM-P].

[C64]

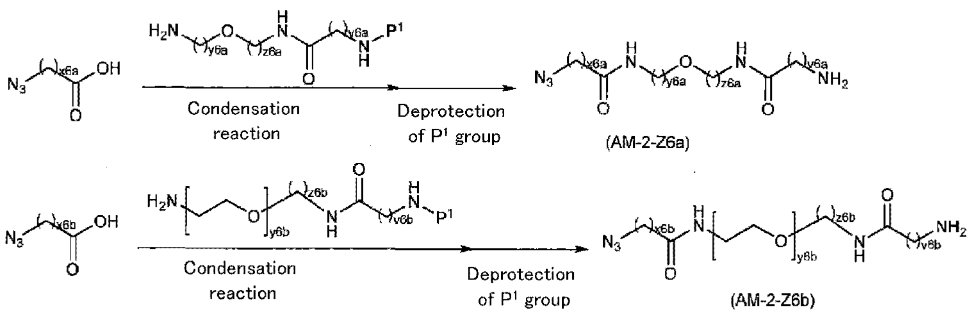

[C65]

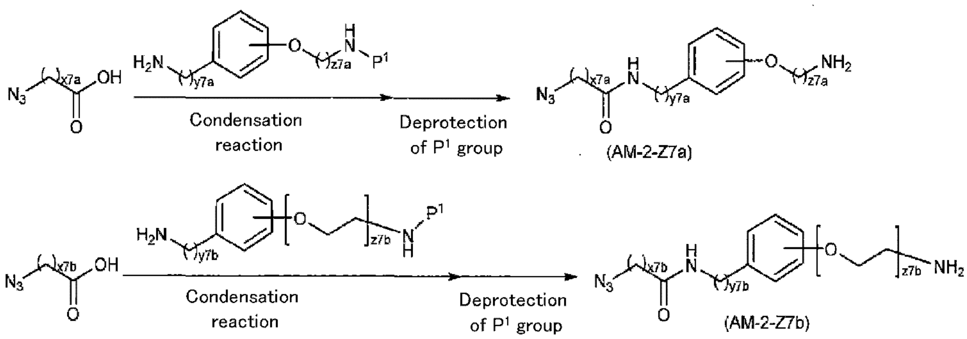

(AM-2-Z7a)

(AM-2-Z7b)

[C66]

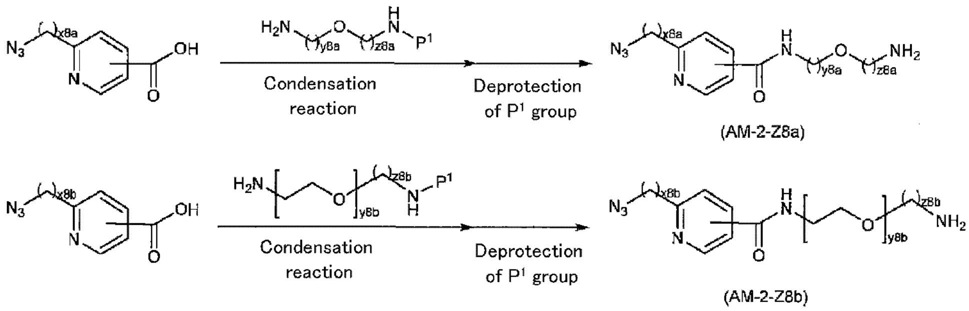

(AM-2-Z8a)

(AM-2-Z8b)

[C67]

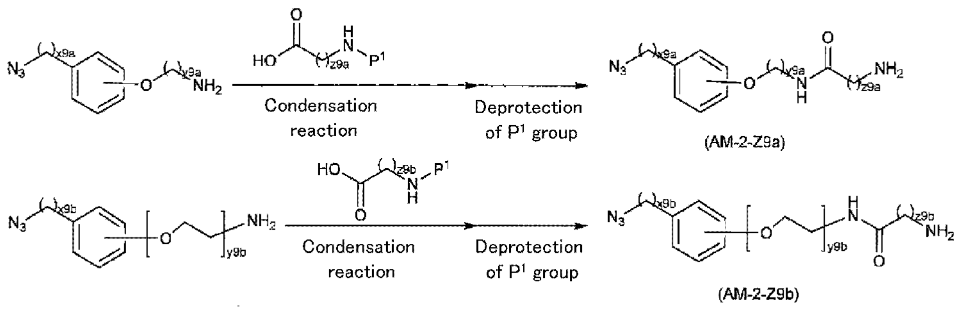

(AM-2-Z9a)

(AM-2-Z9b)

[Manufacturing method AM-T]

A method for manufacturing amines represented by Formula (AM-1-T1) and Formula (AM-1-T2):

[C68]

(RG-T-1) (RG-T-2)

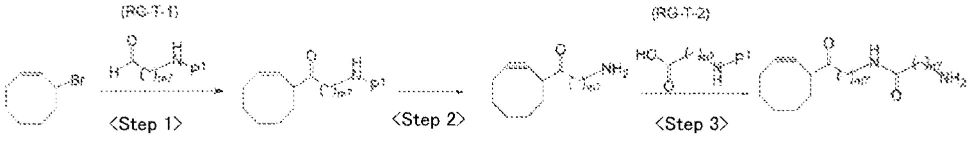

(SM-T) (AM-T-1) (AM-1-T1) (AM-1-T2)

<Step 1> A compound of Formula (IM-T-1) is obtained by performing a grignard reaction and subsequently performing an oxidation reaction according to a method well known by publications, for example, the method described in WO2004/035017 or the like using a compound of Formula (SM-T) and a compound of Formula (RG-T-1) [the compound of Formula (SM-T) and the compound of Formula (RG-T-1) are commercially available compounds or compounds that can be manufactured from commercially available compounds by a manufacturing method well known by publications].

<Step 2> An amine represented by Formula (AM-1-T1) or a salt thereof is manufactured by protecting a carbonyl group of the compound of Formula (IM-T-1) (for example, an acetal group or the like), then, adding bromine to a cyclooctene ring, then, performing a debromination reaction using a base such as tert-BuOK and subsequently deprotecting a protective group of the carbonyl group and a protective group $P^1$.

<Step 3> An amine represented by Formula (AM-1-T2) or a salt thereof is manufactured by performing a condensation reaction and deprotecting the protective group $P^1$ using the amine represented by Formula (AM-1-T1) or the salt thereof and a compound of Formula (RG-T-2) [the compound of Formula (RG-T-2) is a commercially available compound or a compound that can be manufactured from a commercially available compound by a manufacturing method well known by publications].

[Manufacturing method AM-U]

A method for manufacturing amines represented by Formula (AM-1-U1) and Formula (AM-1-U2):

[C69]

(RG-T-1) (RG-T-2)

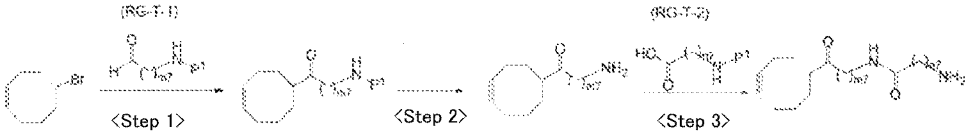


(SM-U) (IM-U-1) (AM-1-U1) (AM-1-U2)

Amines represented by Formula (AM-1-U1) and Formula (AM-1-U2) or salts thereof are manufactured by substituting the compound of Formula (SM-T) into a compound of Formula (SM-U) [the compound of Formula (SM-U) is a commercially available compound or a compound that can be manufactured from a commercially available compound by a manufacturing method well known by publications] in the [Manufacturing method AM-T] and performing a reaction according to the method described in [Manufacturing method AM-T].

An amine having a corresponding linker or a salt thereof is manufactured by substituting aldehyde that is used in <Step 1> of the [Manufacturing method AM-T] and [Manufacturing method AM-U] into aldehyde of Formula (RG-T-3) or Formula (RG-T-4) below [the compound of Formula (RG-T-3) and the compound of Formula (RG-T-4) are commercially available compounds or compounds that can be manufactured from commercially available compounds by a manufacturing method well known by publications] and performing the step.

[C70]

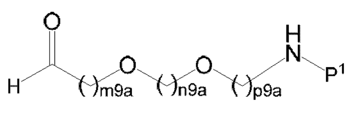

(RG-T-3)

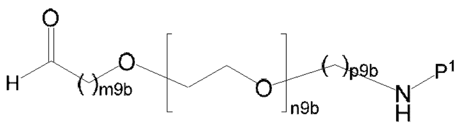

(RG-T-4)

[Manufacturing method AM-V]

A method for manufacturing amines represented by Formula (AM-1-V1) and Formula (AM-1-V2):

[C71]

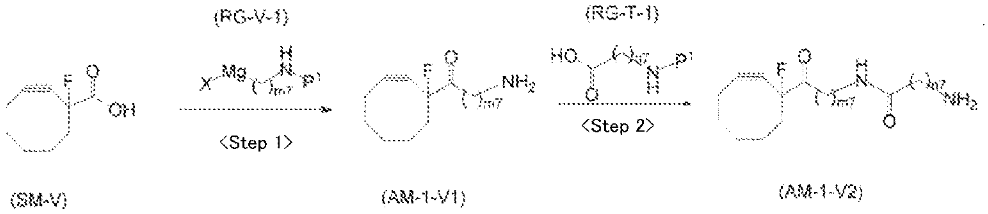


<Step 1> An amine represented by Formula (AM-1-V1) or a salt thereof is manufactured by converting a compound of Formula (SM-V) [the compound of Formula (SM-V) is a commercially available compound or can be manufactured by a method well known by publication, for example, the method described in Bioorganic & Medicinal Chemistry, 23 (22), pp. 7150 to 7157, 2015 or the like] into an acid chloride according to a normal method, then, performing a grignard reaction using a compound of Formula (RG-V-1) [the compound of Formula (RG-V-1) is a commercially available compound or a compound that can be manufactured from a commercially available compound by a method well known by publication] and, subsequently, deprotecting the protective group $P^1$.

<Step 2> An amine represented by Formula (AM-1-V2) or a salt thereof is manufactured by performing a condensation reaction using the compound of Formula (AM-1-V1) or the salt thereof and the compound of Formula (RG-T-1) and deprotecting the protective group $P^1$.

An amine having a corresponding linker or a salt thereof is manufactured by substituting the compound that is used in <step 1> of the [Manufacturing method AM-V] into a compound of the following formula [each compound is a commercially available compound or can be manufactured from a commercially available compound by a method well known by publication] and performing a reaction.

[C72]

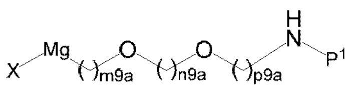

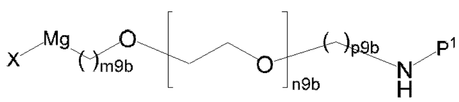

[Manufacturing method AM-W]

A method for manufacturing amines represented by Formula (AM-1-W1) and Formula (AM-1-W2):

[C73]

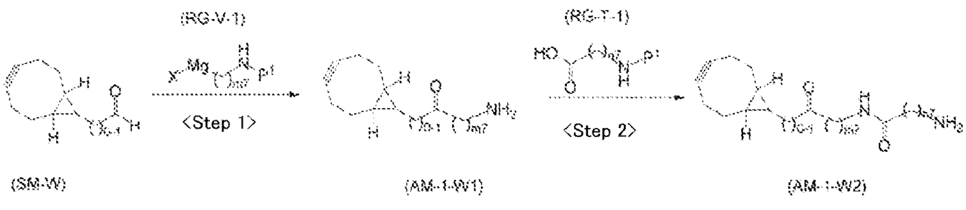


Amines represented by Formula (AM-1-W1) and Formula (AM-1-W2) or salts thereof are manufactured by substituting the compound of Formula (SM-V) into a compound of Formula (SM-W) [the compound of Formula (SM-W) is a commercially available compound or a compound that can be manufactured from a commercially available compound by a manufacturing method well known by publications] in the [Manufacturing method AM-V] and performing a reaction according to the method described in [Manufacturing method AM-V].

An amine having a corresponding linker or a salt thereof is manufactured by substituting the compound that is used in <step 1> of the [Manufacturing method AM-W] into a compound of the following formula [each compound is a commercially available compound or can be manufactured from a commercially available compound by a method well known by publication] and performing a reaction.

[C74]

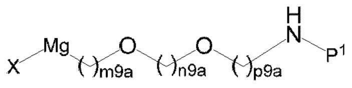

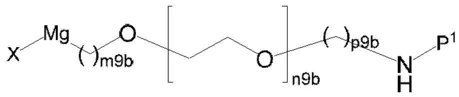

Regarding an amine into which an alkyne group has been introduced (Akn-$L^1$-$NH_2$) or an amine into which an azide group has been introduced ($N_3$-$L^2$-$NH_2$), which is used to manufacture the chemically modified alginic acid derivative represented by Formula (HB-I) or Formula (HB-II), it is possible to manufacture desired amines by appropriately combining each reaction described in the [Manufacturing method AM-A] to [Manufacturing method AM-P] or the like, methods well known by publications, for example, "Experimental Chemistry 5th Edition, each book, 2007, Maruzen", "Comprehensive Organic Transformations, A Guide to Functional Group Preparations, 3rd Edition (Edited by Richard C. Larock), 2018", "Strategic Applications of Named Reactions in Organic Synthesis, (Edited by Laszlo Kurti, Barbara Czako), Academic Press, 2005" and the like.

In the present specification, there are cases where the amine represented by Formula (AM-1) or Formula (AM-2) (also comprising a subordinate formula of each formula) forms a pharmaceutically acceptable salt (for example, an acid addition salts). Such a salt is not particularly limited as long as the salt is pharmaceutically acceptable, and examples thereof include salts with an inorganic acid, salts with an organic acid, salts with an acidic amino acid and the like. Preferable examples of the salts with an inorganic acid include salts with hydrochloric acid, hydrobromic acid, hydroiodic acid, nitric acid, sulfuric acid, phosphoric acid or the like. Preferable examples of the salts with an organic acid include salts with an aliphatic monocarboxylic acid such as formic acid, acetic acid, trifluoroacetic acid, propionic acid, butyric acid, valeric acid, enanthic acid, capric acid, myristic acid, palmitic acid, stearic acid, lactic acid, sorbic acid or mandelic acid, salts with an aliphatic dicarboxylic acid such as oxalic acid, malonic acid, succinic acid, fumaric acid, maleic acid, malic acid or tartaric acid, salts with an aliphatic tricarboxylic acids such as citric acid, salts with an aromatic monocarboxylic acid such as benzoic acid or salicylic acid, salts with an aromatic dicarboxylic acid such as phthalic acid, salts with an organic carboxylic acid such as cinnamic acid, glycolic acid, pyruvic acid, oxylic acid, salicylic acid or N-acetylcysteine, salts with an organic sulfonic acid such as methanesulfonic acid, benzenesulfonic acid or p-toluenesulfonic acid and acid addition salts with an acidic amino acid such as aspartic acid or glutamic acid. Preferable examples of the salts with an acidic amino acid include salts with aspartic acid, glutamic acid or the like. Among these, pharmaceutically acceptable salts are preferable.

The salt can be obtained by, according to a normal method, for example, mixing the compound of the present invention and a solution containing an appropriate amount of an acid or a base to form an intended salt and then performing fractional filtration or distilling away the solvent mixture. As a review regarding salts, Handbook of Pharmaceutical Salts: Properties, Selection, and Use, Stahl & Wermuth (Wiley-VCH, 2002) has been published, and the present book includes detailed description.

In the present specification, the amine represented by Formula (AM-1) or Formula (AM-2) (also comprising a subordinate formula of each formula) or a salt thereof is capable of forming a solvate with a solvent such as water, ethanol or glycerol.

In the present specification, unless particularly otherwise described, in a case where a cyclic group has a variable substituent as a substituent, it means that the variable substituent does not bond to a specific carbon atom of the cyclic group. For example, it means that a variable substituent Rs in Formula A below is capable of substituting any of carbon atoms i, ii, iii, iv and v in Formula A.

In the present specification, in a case where chiral carbon is present in the linker (-$L^1$-or -$L^2$-) in the chemically modified alginic acid derivative represented by Formula (HB-I) or Formula (HB-II), it means that each optical isomer thereof is also included.

[C75]

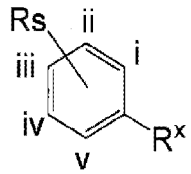

Formula A

For example, in the case of Formula (L1-8a-M) below in a case where -$L^1$- in Formula (HB-I) is Formula (L1-8a),

m8a is two, n8a is one and $R^1$ is Me (in the formula, both outsides of the broken lines are not included):

[C76]

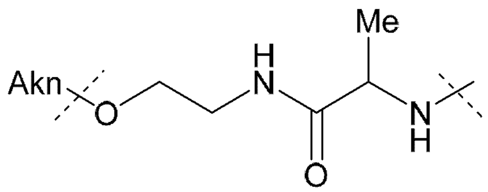

(L1−8a−M)

it means that linkers represented by Formula (L1-8a-M-S) below in which the configuration of carbon into which an $R^1$ group substitutes is an S system and Formula (L1-8a-M-R) below in which the configuration of carbon into which a benzyl group substitutes is an R system (in all formulae, both outsides of the broken lines are not included):

[C77]

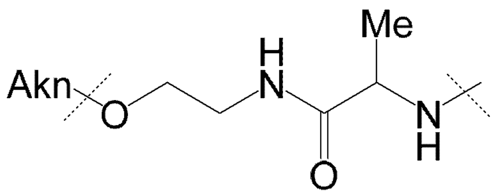

(L1-8a-M)

:

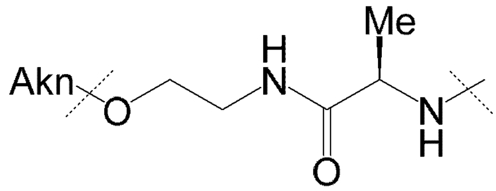

(L1-8a-M-R)

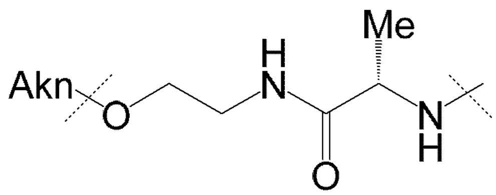

(L1-8a-M-S)

are included.

In a case where chiral carbon is present in the linker ($-L^1-$ or $-L^2-$) in the chemically modified alginic acid derivative represented by Formula (HB-I) or Formula (HB-II) (in the case of an optically active substance), in a step of synthesizing the amine derivative (AM-1) corresponding to Formula (HB-I) or Formula (HB-II), it is possible to separate each optically active substance from a racemate thereof by normal optical resolution means (separation method) or, in a step of synthesizing Formula (AM-1) or Formula (AM-2), which is an amine derivative corresponding to Formula (HB-I), it is possible to selectively synthesize one of optical isomers using asymmetric synthesis and to synthesize each optical active substance.

5. Chemically crosslinked alginic acid (core layer and anionic polymer layer)

Among the chemically crosslinked alginic acids configuring the structure of the present invention, as the chemically crosslinked alginic acid that is used in the core layer and the chemically crosslinked alginic acid that is used in the anionic polymer layer, chemically crosslinked alginic acids that are manufactured by performing a chemical crosslinking reaction using the chemically modified alginic acid derivative described in the section "3. Chemically modified alginic acid derivative" are exemplified. Specifically, the chemically crosslinked alginic acids are the chemically crosslinked alginic acid used to manufacture the structures described in the embodiments [1] to [9-2]. In a case where a chemically crosslinked alginic acid is used in the anionic polymer layer, the chemically crosslinked alginic acid may be the same structure as or a different structure from the chemically crosslinked alginic acid that is used in the core layer. For example, it is also possible to use the chemically crosslinked alginic acid crosslinked by the Huisgen-type reaction, which is used

to manufacture the structures described in the embodiments [2] to [7], as the chemically crosslinked alginic acid that is used in the core layer and to use the chemically crosslinked alginic acid crosslinked with the photocrosslinked alginic acid (transcutaneous acid type), which is used to manufacture the structures described in the embodiments [8-1] to [9-2], as the chemically crosslinked alginic acid that is used in the anionic polymer layer. As a separate embodiment, in a case where the chemically crosslinked alginic acid crosslinked by the Huisgen-type reaction, which is used to manufacture the structures described in the embodiments [2] to [7], is used as both the chemically crosslinked alginic acid that is used in the core layer and the chemically crosslinked alginic acid that is used in the anionic polymer layer, it is also possible to use chemically crosslinked alginic acids having different structures in the core layer and the anionic polymer layer, and it is possible to select, for example, chemically crosslinked alginic acids for which the crosslinking reaction rates are different from each other in the individual layers.

As the chemically crosslinked alginic acid configuring the structure of the present invention, as described in "1. Multilayer structure using chemically crosslinked alginic acid", any state of (i) a state where a crosslink (ionic crosslinking) by a divalent metal ion is not formed (for example, a state where a bond with a divalent metal ion is removed by making a chelating agent such as EDTA present) and (ii) a state where both a chemical crosslink and an ionic crosslinking have been formed can be used. For example, in a state where both a chemical crosslink and an ionic crosslinking have been formed, since the structural stability is high, there are cases where the state is advantageous at the time of embodying the structure in living bodies. In this case, the divalent metal ion is not particularly limited, examples thereof include a calcium ion, a magnesium ion, a barium ion, a strontium ion, a zinc ion and the like, a calcium ion or a barium ion is preferable, and a calcium ion is more preferable. A separate preferable embodiment is a barium ion.

The solution containing the divalent metal ion is not particularly limited, examples thereof include solutions containing a calcium ion (for example, aqueous solutions such as a calcium chloride aqueous solution, a calcium carbonate aqueous solution, a calcium gluconate aqueous solution) and solutions containing a barium ion (for example, aqueous solutions such as a barium chloride aqueous solution), and a calcium chloride aqueous solution is preferable. The divalent metal ion concentration (for example, the calcium ion or barium ion concentration) of the solution containing the divalent metal ion is not particularly limited and is, for example, within a range of 1 mM to 1 M or a range of 5 mM to 500 mM and more preferably 20 mM to 100 mM. In the present specification, there are case where divalent metal ion aqueous solutions are simply referred to as divalent metal ion solutions, and, unless particularly otherwise described, the divalent metal ion solutions are aqueous solutions.

A solvent that is used to prepare the solution of the chemically modified alginic acid derivative or a solvent that is used to prepare the solution or the like containing the divalent metal ion is not particularly limited, examples of each independently include tap water, pure water (for example, distilled water, ion-exchanged water, RO water, RO-EDI water and the like), ultrapure water (MilliQ water), a culture medium (that is, a cell culture medium (or a culture fluid)), phosphate buffered saline (PBS), physiological saline and the like, and ultrapure water is preferable.

In a case where the chemically crosslinked alginic acid accompanies an ionic crosslinking, the ionic crosslinking reaction is instant and reversible whereas the reaction of the chemical crosslink relatively slowly progresses under relatively mild conditions and is irreversible. When the chemical crosslink and the ionic crosslinking are appropriately combined using these properties, it becomes possible to efficiently produce the structure of the present invention. For example, the ionic crosslinking reaction makes it easy to instantly produce a bead or sheet-shaped structure from the solution mixture of the chemically modified alginic acid derivatives. Incidentally, in the present invention, since chemical crosslinking (Huisgen reaction) is used for the structural strengthening of the structure (for example, acquisition of long-term stability or the like), even in a case where, after the production of the structure of the present invention through both chemical crosslinking and ionic crosslinking, the divalent metal ion in the chemically crosslinked alginic acid, which has been incorporated by the ionic crosslinking, is gradually and reversibly discharged and only crosslinks by chemical bonds remains, it is possible to stably and continuously use the structure.

5-1. Chemically crosslinked alginic acid (Huisgen type)

A chemically crosslinked alginic acid of a certain embodiment can be obtained by mixing the chemically modified alginic acid derivatives of Formula (H-I) and Formula (H-II) and performing the Huisgen reaction. Chemically crosslinked alginic acids of several separate embodiments can be obtained by mixing the chemically modified alginic acid derivatives of Formula (HA-I) and Formula (HA-II) and performing the Huisgen reaction. Chemically crosslinked alginic acids of several separate embodiments can be obtained by mixing the chemically modified alginic acid derivatives of Formula (HB-I) and Formula (HB-II) and performing the Huisgen reaction.

A chemically crosslinked alginic acid of a certain embodiment forms a three-dimensional network structure through a chemical crosslink (a crosslink by a triazole ring that is formed of an alkyne group and an azide group). A preferable chemically modified alginic acid derivative is a derivative that improves the stability of the chemically crosslinked alginic acid after crosslinking. The physical properties of the chemically crosslinked alginic acid can be adjusted with, for example, the introduction rate of the reactive group in the chemically modified alginic acid, which is used as a raw

material.

Chemically crosslinked alginic acids of several embodiments are chemically crosslinked alginic acids crosslinked through a group represented by Formula (H-III-L) below:

[C78]

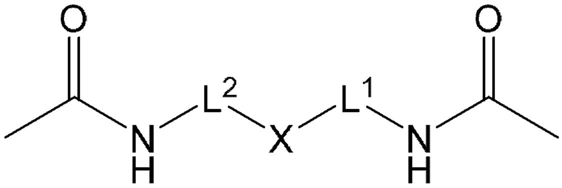

(H-III-L)

[in Formula (H-III-L), -CONH- and -NHCO- at both ends represent amide bonds through an arbitrary carboxyl group of alginic acid; $-L^1-$, $-L^2-$ and X are each the same as the combination of any of the embodiments [5] to [5-7]].

Chemically crosslinked alginic acids of several embodiments are chemically crosslinked alginic acids crosslinked through a group represented by Formula (HA-III-L) below:

[C79]

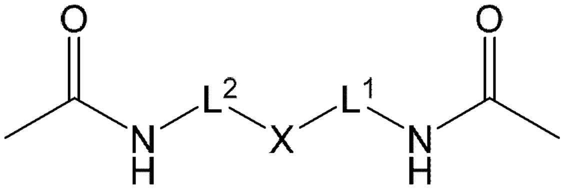

(HA-III-L)

[in Formula (HA-III-L), -CONH- and -NHCO- at both ends represent amide bonds through an arbitrary carboxyl group of alginic acid; $-L^1-$, $-L^2-$ and X are the same as the definitions in the embodiment [7]].

In addition, chemically crosslinked alginic acids of several embodiments are chemically crosslinked alginic acids crosslinked through a group represented by Formula (HB-III-L) below:

[C80]

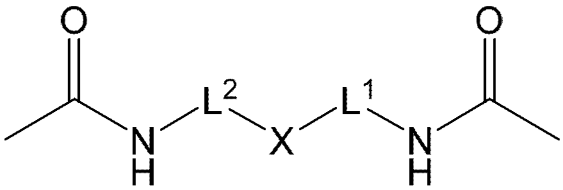

(HB-III-L)

[in Formula (HB-III-L), -CONH- and -NHCO- at both ends represent amide bonds through an arbitrary carboxyl group of alginic acid; $-L^1-$, $-L^2-$ and X are each the same as the combination of any of the embodiments [6-1] to [6-6]].

In a certain embodiment, the chemically crosslinked alginic acids of Formula (H-III-L), Formula (HA-III-L) and Formula (HB-III-L) can be each manufactured by mixing the chemically modified alginic acid derivatives of Formula (H-I) and Formula (H-II), Formula (HA-I) and Formula (HA-II), and Formula (HB-I) and Formula (HB-II).

In several embodiments, at the time of preparing the chemically crosslinked alginic acid, the mixing ratio between the chemically modified alginic acid derivative of Formula (H-I), Formula (HA-1) or Formula (HB-I) and the chemically modified alginic acid derivative of Formula (H-II), Formula (HA-II) or Formula (HB-II) is, for example, 1: 1.0 to 4.0, 1:1.0 to 3.0, 1:1.0 to 2.0, 1:1.0 to 1.5 or 1: 1 and preferably 1:1.0 to 3.0 in terms of the weight ratio between the derivative of Formula (H-I), Formula (HA-1) or Formula (HB-1) and the derivative of Formula (H-II), Formula (HA-II) or Formula (HB-11).

In several embodiments, at the time of preparing the chemically crosslinked alginic acid, the mixing ratio between the chemically modified alginic acid derivative of Formula (H-II), Formula (HA-II) or Formula (HB-II) and the chemically modified alginic acid derivative of Formula (H-I), Formula (HA-1) or Formula (HB-I) is, for example, 1: 1.0 to 4.0, 1:1.0 to 3.0, 1:1.0 to 2.0, 1:1.0 to 1.5 or 1:1 in terms of the weight ratio between the derivative of Formula (H-II), Formula (HA-II) or Formula (HB-II) and the derivative of Formula (H-I), Formula (HA-1) or Formula (HB-I).

In several embodiments, at the time of preparing the chemically crosslinked alginic acid, the mixing ratio between the chemically modified alginic acid derivative of Formula (H-I), Formula (HA-1) or Formula (HB-I) and the chemically modified alginic acid derivative of Formula (H-II), Formula (HA-II) or Formula (HB-II) is more preferably, for example, 1:1.0 to 4.0, 1:1.0 to 3.0, 1:1.0 to 2.0, 1:1.0 to 1.5 or 1:1 and preferably 1:1.0 to 3.0 in terms of the ratio between the introduction rates (mol%) of the reactive group of the chemically modified alginic acid derivative of Formula (H-I), Formula (HA-1) or Formula (HB-1) and the chemically modified alginic acid derivative of Formula (H-II), Formula (HA-II) or Formula (HB-11).

In several embodiments, at the time of preparing the chemically crosslinked alginic acid, the mixing ratio between the chemically modified alginic acid derivative of Formula (H-II), Formula (HA-II) or Formula (HB-II) and the chemically modified alginic acid derivative of Formula (H-I), Formula (HA-1) or Formula (HB-I) is more preferably, for example, 1:1.0 to 4.0, 1:1.0 to 3.0, 1:1.0 to 2.0, 1:1.0 to 1.5 or 1:1 in terms of the ratio between the introduction rates (mol%) of the reactive group of the chemically modified alginic acid derivative of Formula (H-II), Formula (HA-II) or Formula (HB-II) and the chemically modified alginic acid derivative of Formula (H-I), Formula (HA-1) or Formula (HB-I).

In the chemically crosslinked alginic acid, all of the carboxyl groups in the alginic acid constituting unit do not need to have the crosslink of Formula (HA-III-L) or Formula (HB-III-L). The introduction rate (also referred to as the crosslink rate) of the crosslink represented by Formula (HA-III-L) or formula (HB-III-L) in the chemically crosslinked alginic acid is, for example, within a range of approximately 0.1% to approximately 80%, approximately 0.3% to approximately 60%, approximately 0.5% to approximately 30% or approximately 1.0% to approximately 10%.

The concentrations of the chemically modified alginic acid derivatives represented by Formulae (H-1) to (HB-II) in the Huisgen reaction for obtaining the chemically crosslinked alginic acid are normally approximately 1 to 500 mg/mL and preferably within a range of approximately 5 to 100 mg/mL. The concentrations are within a range of 5 to 50 mg/mL as a certain embodiment and within a range of 5 to 20 mg/mL as a separate embodiment.

Regarding the reaction temperature of the Huisgen reaction (the temperature at the time of producing the chemically crosslinked alginic acid), normally, the outside temperature is approximately 4°C to approximately 60°C and preferably approximately 15°C to approximately 37°C.

5-2. Photocrosslinked alginic acid (cinnamic acid type)

Several embodiments of the chemically crosslinked alginic acid configuring the structure of the present invention are chemically crosslinked alginic acids that are obtained by light exposure of a chemically modified alginic acid derivative into which a photoreactive group (photocrosslinking group) has been introduced, for example, a chemically modified alginic acid derivative into which the group represented by Formula (C-I) has been introduced with light (also referred to as photocrosslinked alginic acids). In the photocrosslinked alginic acid, the chemically modified alginic acid derivative forms a three-dimensional network structure through the photocrosslinking group (crosslink by a cyclobutane ring that is formed by a photoreaction). A preferable chemically modified alginic acid derivative is a derivative that improves the stability of the chemically crosslinked alginic acid after crosslinking.

The light used for the irradiation is not particularly limited as long as the light acts on the photoreactive group (photocrosslinking group) and causes reactions of polymerization, dimerization and the like (preferably a photodimerization reaction (photocyclization reaction)). As the light used for the irradiation, for example, ultraviolet light, LED light (LED = light emitting diode), visible light, infrared light, electron beam and the like can be used. Among these lights for the irradiation, visible light, LED light or ultraviolet light is preferable and ultraviolet light is more preferable. The light wavelength of a light source that is used is preferably within a range of 180 to 650 nm and more preferably within a range of 300 to 410 nm.

In several embodiments, specific light amount and wavelength for causing the photocrosslinking reaction are as described below. That is, the illumination of HLR100T-2 (manufactured by SEN LIGHTS Corporation) is 170 mW/cm$^2$ (at a distance of 50 mm from a light source), the main wavelength is 365 nm and the irradiation time is 10 minutes. The exposure amount is 25 J/cm$^2$ in irradiation from a distance of 100 mm from the light source.

In several embodiments, in the photocrosslinked alginic acid, a photoreactive portion (alkene part portion) of the photoreactive group represented by Formula (C-I) is dimerized by a photocyclization reaction, and the photocrosslinked alginic acid has a chemical crosslink in which a cyclobutane ring (truxillic acid derivative) represented by, for example, Formula (C-II-L-1) to (C-II-L-3):

[C81]

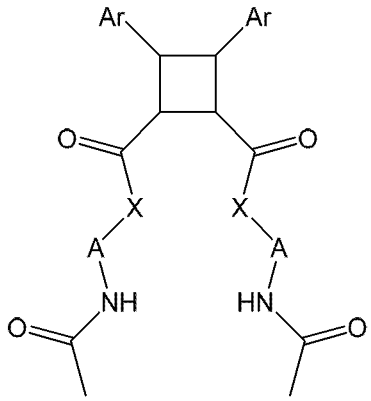

(C-II-L-1)

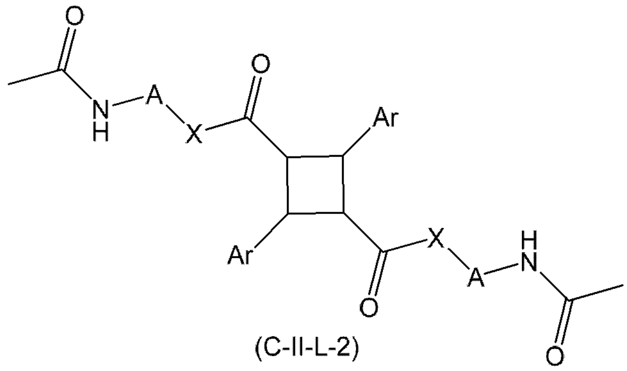

(C-II-L-2)

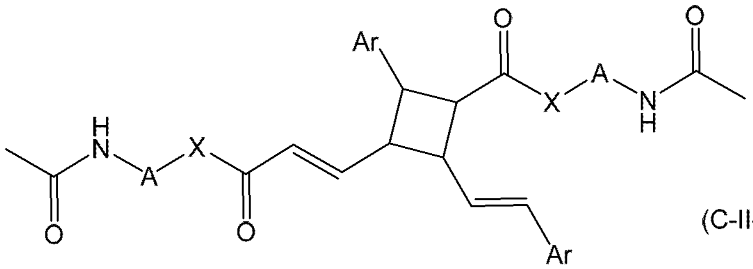

(C-II-L-3)

[in Formula (C-II-L-1) to Formula (C-II-L-3), -CONH- and -NHCO- at both ends represent amide bonds through an arbitrary carboxyl group of alginic acid; -A-, -X- and Ar are the same as the definitions in the embodiments [9-1] and [9-2]] is formed.

In the photocrosslinked alginic acid, all of the carboxyl groups in the alginic acid constituting unit do not need to have the above-described crosslink. The introduction rate (also referred to as the crosslink rate) of the crosslink represented by the above formula in the chemically crosslinked alginic acid is, for example, within a range of 0.1% to 80%, 0.3% to 60%, 0.5% to 30% or 1.0% to 10%.

The concentration of the chemically modified alginic acid derivative for obtaining the photocrosslinked alginic acid is, for example, within a range of 1 to 500 mg/mL or 5 to 100 mg/mL.

The temperature at which the photocrosslinking reaction is performed is normally room temperature (approximately 0°C to approximately 35°C).

A reaction solvent or a reaction solution that is used in the photocrosslinking reaction is not particularly limited, examples thereof include tap water, pure water (for example, distilled water, ion-exchanged water, RO water, RO-EDI water and the like), ultrapure water (MilliQ water), a culture medium (that is, a cell culture medium (or a culture fluid)), phosphate buffered saline (PBS), physiological saline and the like, and ultrapure water is preferable.

6. Method for manufacturing structure (multilayer structure in which chemically crosslinked alginic acid is used in core layer)

The present invention also relates to a method for manufacturing a structure. In the present specification, a method for manufacturing a structure comprising a pharmacological ingredient embedded in a chemically crosslinked alginic acid in a core layer, comprising the following steps (a) to (c), is provided.

step (a): a step of bringing a solution of a chemically modified alginic acid derivative comprising a pharmacological ingredient into contact with a solution having a divalent metal ion to gelate,

step (b): a step of bringing gel obtained in the step (a) into contact with a solution comprising a cationic polymer to coat the gel with the cationic polymer, and

step (c): a step of bringing a manufactured product obtained in the step (b) into contact with a solution comprising an anionic polymer to further coat the gel with the anionic polymer.

In the method for manufacturing a structure, in "step (a): a step of bringing a solution of a chemically modified alginic acid derivative comprising a pharmacological ingredient into contact with a solution having a divalent metal ion to gelate", a solution of a chemically modified alginic acid derivative, in which a pharmacological ingredient has been suspended, is brought into contact with a divalent metal ion solution and caused to gelate. When the solution of the

chemically modified alginic acid derivative is brought into contact with the solution containing a divalent metal ion, chemical crosslinking also proceeds at the same time as ionic crosslinking proceeds, and gel can be produced. In the step (a), specifically, first, a variety of pharmacological ingredients described in the "1-1. Core layer comprising pharmacological ingredient embedded in chemically crosslinked alginic acid" are suspended or dissolved in a solution comprising a chemically modified alginic acid derivative. At this time, it is also possible to add an additive other than the pharmacological ingredients, and, for example, in the case of a bioactive substance or the like, there are cases where it is effective to use an additive on which formulation for controlling slow release has been performed or an additive supported by a carrier. In the case of a cell, there are cases where it is effective to use a substance for maintaining the viability or functions of the cell, and a substance that uniformly disperse the pharmacological ingredient in the core layer is also added. Examples of the additive other than the pharmacological ingredient include polymer substances other than alginic acid, and, specifically, examples of biocompatible polymer substances include polylactic acid (PLA), polylactic-co-glycolic acid (PLGA), gelatin, collagen, hyaluronic acid and the like. For example, in a case where a cell or a microorganism is used as the pharmacological ingredient, there are also cases where a culture medium or culture fluid ingredient or a formulation ingredient that is commonly used as a drug is contained.

Examples of the chemically modified alginic acid derivative include the compounds represented by Formula (H-I) and Formula (H-II), the compounds represented by Formula (HA-I) and Formula (HA-II), the compounds represented by Formula (HB-I) and Formula (HB-II) or the compound represented by Formula (C-I). In the step (a), for example, an aqueous solution of 0.1 to 5 wt% of the chemically modified alginic acid derivative or a physiological saline solution is produced, and a necessary amount of the variety of pharmacological ingredients are appropriately suspended in the solution.

In the step (a), it is possible to use a combination of a solution of the chemically modified alginic acid derivative represented by Formula (H-I) and a solution of the chemically modified alginic acid derivative represented by Formula (H-11). In several embodiments, it is possible to use a combination of a solution of the chemically modified alginic acid derivative represented by Formula (HA-1) and a solution of the chemically modified alginic acid derivative represented by Formula (HA-II). In several separate embodiments, it is possible to use a combination of a solution of the chemically modified alginic acid derivative represented by Formula (HB-1) and a solution of the chemically modified alginic acid derivative represented by Formula (HB-II). These solutions and solutions obtained by mixing the pharmacological ingredients with the above-described solutions are not mixed together and are separately produced. In the case of using each of the combinations of the solutions, the pharmacological ingredients may be mixed with only one solution or may be mixed with both.

Here, "contact" means that a certain solution (for example, the solution of the chemically modified alginic acid derivative) is immersed in or added to another solution (for example, the solution containing a divalent metal ion), that a certain solution (for example, the solution of the chemically modified alginic acid derivative) is sprayed or blown to another solution (for example, the solution containing a divalent metal ion) or the like.

Examples of the solution containing "divalent metal ion", which is used in the structure, include solutions containing a calcium ion, a barium ion, a zinc ion, a strontium ion or the like. A solution containing a calcium ion or a barium ion is preferable, and a solution containing a calcium ion is more preferable. A calcium chloride aqueous solution is preferable. A separate preferable embodiment is a barium chloride aqueous solution.

The solution containing a divalent metal ion can be obtained by, for example, dissolving a salt of a divalent metal ion in a solvent. Examples of the salt of a divalent metal ion include calcium chloride, barium chloride, strontium chloride and the like. Examples of the solvent include water and physiological saline.

The amount of the solution containing a divalent metal ion used is desirably adjusted as appropriate depending on the amount of the chemically modified alginic acid derivative used, the molecular weight or the like.

One of the characteristics of the structure of the present invention is that the degree of freedom at the time of molding is high compared with those of conventional structures for which alginic acid is used, and, for example, a method as described below makes it possible to manufacture structures having a variety of shapes. The shape of the core layer accounting for the majority of the volume has the largest influence on the shape of the entire structure.

For example, in order to produce a spherical structure, a method in which the solution of the chemically modified alginic acid derivative mixed with the pharmacological ingredient is added dropwise to the divalent metal ion solution such as a calcium chloride aqueous solution is used. At this time, a change in the diameter of a conduit through which the solution is added dropwise or the dropwise addition rate makes it possible to manufacture spherical bodies having a variety of sizes.

As one method for producing a fiber-like structure, the solution of the chemically modified alginic acid derivative mixed with the pharmacological ingredient is slowly discharged into the divalent metal ion solution through an introduction pipe equipped with a syringe or the like at the front end. At this time, the discharged solution sequentially gelates, whereby a filamentous structure can be manufactured. When the size of the introduction pipe is changed or the discharge rate is controlled using a syringe pump or the like, it becomes possible to manufacture structures having a variety of sizes.

Additionally, the use of a template and a semipermeable membrane also makes it possible to manufacture

structures having an arbitrary shape. For example, in a case where a flat plate-shaped structure needs to be manufactured, a template is produced using a material that does not affect reactions and is easy to process, such as silicon rubber, and the solution of the chemically modified alginic acid derivative mixed with the pharmacological ingredient is caused to flow into the template. When the semipermeable membrane is used in a part of this template, the divalent metal ion solution is infiltrated into the template through this semipermeable membrane, and the content becomes gel. In this method, it becomes possible to manufacture structures having a variety of shapes by designing the template in a desired shape.

In addition, the use of a 3D printer makes it possible to mold a free shape, and, for example, a method in which the solution of the chemically modified alginic acid derivative mixed with the pharmacological ingredient is used as a bio ink and brought into contact with the divalent metal ion solution is used.

A high degree of freedom of molding in the structure of the present invention has been achieved using a chemically crosslinked alginic acid that can be easily manufactured while maintaining a high strength of the structure.

Next, in "step (b): a step of bringing gel obtained in the step (a) into contact with a solution comprising a cationic polymer to coat the gel with the cationic polymer", a solution comprising a cationic polymer, for example, PLL or PLO is brought into contact with the gel obtained in the step (a). It is preferable to bring a solution comprising PLO into contact with the gel obtained in the step (a). This makes the surface of the molded product obtained in the step (a) coated with the cationic polymer by an interaction between the chemically crosslinked alginic acid and the cationic polymer. As the cationic polymer, cationic polymers mentioned in the "1-2. Cationic polymer layer coating core layer" are preferably used.

The concentration of the cationic polymer at the time of being reacted with the chemically crosslinked alginic acid in the core layer is, for example, 0.02 to 0.2 w/w% or 0.05 to 0.1 w/w% in the case of using PLO as the cationic polymer.

In addition, the temperature at the time of reacting the chemically crosslinked alginic acid in the core layer and the cationic polymer is, for example, room temperature. The reaction time is, for example, one minute to 45 minutes or one minute to 30 minutes.

Next, in "step (c): a step of bringing a manufactured product obtained in the step (b) into contact with a solution comprising an anionic polymer to further coat the gel with the anionic polymer", the manufactured product obtained in the step (b) is brought into contact with a solution comprising an anionic polymer. The solution comprising an anionic polymer mentioned in "1-3. Anionic polymer layer coating cationic polymer layer" is preferably brought into contact with the manufactured product obtained in the step (b), a chemically modified alginic acid derivative or alginic acid is more preferably brought into contact with the manufactured product obtained in the step (b), and alginic acid is still more preferably brought into contact. "Chemically modified alginic acid derivative" mentioned herein is the chemically modified alginic acid mentioned in the "2. Chemically modified alginic acid derivative." In addition, "alginic acid" is alginic acid (for example, sodium alginate) mentioned in the "2. Alginic acid (regarding alginic acid of raw material that is used to synthesize chemically modified alginic acid derivative and alginic acid that is used in anionic polymer layer)." This makes the surface of the manufactured product coated with the cationic polymer further coated with the chemically modified alginic acid derivative or alginic acid.

Here, in a case where the chemically modified alginic acid derivative is used as the anionic polymer, the same one as the chemically modified alginic acid derivative that is used in the core layer may be used or a different one may be used. In addition, as the treatment conditions of the step (c), almost the same conditions as those used in the step (b) are used.

The concentration of the anionic polymer at the time of being reacted with the cationic polymer layer is, for example, 0.01 to 4 w/w% in the case of using alginic acid or the chemically modified alginic acid derivative as the anionic polymer.

In several embodiments, next, a step (d) may be included. In "step (d): a step of performing a chelate treatment on a structure obtained in the step (c) with a chelating agent", a chelating agent is added to the structure manufactured by the operations up to the step (c), thereby migrating the ionically crosslinked divalent metal ion toward the chelating agent. Examples of the chelating agent include sodium citrate, ethylenediaminetetraacetic acid (EDTA) and the like. The amount of the chelating agent used is desirably adjusted as appropriate depending on the amount of the chemically modified alginic acid derivative used or the like.

Among the structures of the present invention, for structures for which the chemically crosslinked alginic acid is photocrosslinked alginic acid (transcutaneous acid type), in the "method for manufacturing a structure comprising a pharmacological ingredient embedded in a chemically crosslinked alginic acid, comprising the steps (a) to (c)", a solution of the chemically modified alginic acid derivative represented by Formula (C-I) is used as the solution of the chemically modified alginic acid derivative in the step (a), and a chemical crosslinking reaction is performed by light exposure of the chemically modified alginic acid derivative with light in any stage after the step (a).

More specifically, for example, the solution of the chemically modified alginic acid derivative into which the photoreactive group represented by Formula (C-I) has been introduced is added dropwise to the solution containing a divalent metal ion, thereby obtaining a specific structure in which an ionic crosslink (crosslink that is partially formed by the divalent metal ion) has been formed. Subsequently, the structure is irradiated with light to perform photocrosslinking

(chemical crosslinking: cyclobutane ring (truxillic acid derivative)), whereby photocrosslinked alginic acid, which is a specific structure, can be obtained. The irradiation with light may be performed in any stage after the step (a) and can also be performed after the step (c).

The light used for the irradiation is not particularly limited as long as the light acts on the photoreactive group (photocrosslinking group) and causes reactions, for example, polymerization, dimerization and the like (preferably a photodimerization reaction (photocyclization reaction)). As the light used for the irradiation, for example, ultraviolet light, LED light (LED = light emitting diode), visible light, infrared light, electron beam and the like can be used. Among these lights for the irradiation, visible light, LED light or ultraviolet light is preferable and ultraviolet light is more preferable. The light wavelength of a light source that is used is preferably within a range of 180 to 650 nm and more preferably within a range of 300 to 410 nm.

In several embodiments, specific light amount and wavelength for causing the photocrosslinking reaction are as described below. That is, the illumination of HLR100T-2 (manufactured by SEN LIGHTS Corporation) is 170 mW/$cm^2$ (at a distance of 50 mm from a light source), the main wavelength is 365 nm and the irradiation time is 10 minutes. The exposure amount is 25 J/$cm^2$ in irradiation from a distance of 100 mm from the light source.

7. Physical properties of structure (multilayer structure) of present invention and chemically crosslinked alginic acid

[Method for confirming stability of structure]

The stability of the structure of the present invention can be confirmed by, for example, the following testing method. More specifically, the stability can be confirmed by methods to be described in the following examples.

(1) Chelate treatment: On the structure of the present invention, the step (d) mentioned in "6. Method for manufacturing structure (multilayer structure in which chemically crosslinked alginic acid is used in core layer)", that is, "the step of performing a chelate treatment on a structure obtained in the step (c) with a chelating agent" is performed, thereby releasing ionic crosslinking in the chemically crosslinked alginic acid or alginic acid. Specifically, in a case where the structure of the present invention has been treated with an aqueous solution of sodium citrate or ethylenediaminetetraacetic acid (EDTA), it is possible to confirm a structure capable of holding the shape of the structure as "stable structure."

The structure of the present invention is capable of holding the shape even under conditions where no ionic crosslinking is present by the chemical crosslink of the chemically crosslinked alginic acid in the core layer and an electrostatic interaction among individual layers of the core layer, the cationic polymer layer and the anionic polymer layer. The present test makes it possible to confirm, for example, long-term stability in a case where the structure has been detained in vivo.

(2) Shaking test: The structures on which the chelate treatment has been performed are suspended in phosphate buffered saline (PBS), these are shaken for a certain time, and structure capable of maintaining the structure and structure incapable of maintaining the structure are confirmed, whereby the physical strength can be measured. Examples of a specific testing method include a method to be described in the following examples.

Several advantages of the structure of the present invention are attributed to the fact that the chemically crosslinked alginic acid configuring the structure has optimal properties for the function of the structure of the present invention. For example, the chemically crosslinked alginic acid that is used in the present invention has high physical stability (abbreviated as gel stability) and releases the pharmacological ingredient from the core layer and thus has appropriate permeability even in a gel form. This gel permeability can be confirmed by measuring a gel permeability as described below.

[Method for measuring gel stability]

Phosphate buffered saline is added to the chemically crosslinked alginic acid put into a container, and the concentration ($\mu$g/mL) of alginic acid leaked into PBS is measured. A value expressed in percentage of a value obtained by dividing the measured alginic acid concentration by the concentration of all alginic acid obtained by decomposing the chemically crosslinked alginic acid is regarded as a collapse rate. The gel stability can be obtained by, specifically, a method to be described in the following examples.

In the present specification, the gel collapse rate of the chemically crosslinked alginic acid is preferably 0% to 90%, more preferably 0% to 70% and still more preferably 0% to 50%. The stability of the chemically crosslinked alginic acid means that, as the concentration of alginic acid that leaks into an aqueous solution decreases, that is, the gel collapse rate decreases, the stability increases.

[Method for measuring gel permeability)

A chemically crosslinked alginic acid containing fluorescein isothiocyanate-dextran is produced, this is put into a container, physiological saline is added thereto, and the concentration of dextran leaked into the physiological saline is measured. A value expressed in percentage of a value obtained by dividing the measured dextran concentration by the concentration of all dextran obtained by decomposing the chemically crosslinked alginic acid containing fluorescein isothiocyanate-dextran is the gel permeability. The gel permeability can be obtained by, specifically, a method to be described in the following examples.

The gel permeability of the chemically crosslinked alginic acid after 24 hours from the addition of the physiological saline is, for example, preferably 0% to 90%, more preferably 0% to 70% and still more preferably 0% to 50% in a case where dextran having a molecular weight of 2,000,000 is contained. In addition, in a case where dextran having a molecular weight of 150,000 is contained, for example, if the intended use of the chemically crosslinked alginic acid is the release and production of protein or antibody, the gel permeability is preferably 1% to 100%, more preferably 10% to 100% and still more preferably 30% to 100%. In addition, if the intended use is immune septum, the gel permeability is preferably 0% to 90%, more preferably 0% to 70% and still more preferably 0% to 50%.

Regarding the permeability of the chemically crosslinked alginic acid, it means that, as thepermeability decreases, the permeability of the contents or a substance outside the gel is lower, and it means that, as the permeability increases, the permeability of the contents or a substance outside the gel is higher.

The permeability of the gel can be adjusted with the molecular weight or concentration of alginic acid that is used, the kind or introduction rate of the crosslinking group that is introduced into alginic acid, the kind or concentration of the divalent metal ion that is used for gelation or a combination thereof.

8. Biocompatibility of structure

In the present specification, biocompatibility means a property of not causing an interaction between the structure and living bodies, a local reaction of a tissue adjacent to the structure or a reaction such as a systemic reaction, and having such a property is referred to as having biocompatibility. It has been confirmed that the chemically modified alginic acid derivative or chemically crosslinked alginic acid configuring the structure of the present invention has, similar to alginic acid, favorable biocompatibility (for example, regarding the chemically crosslinked alginic acid, refer to PCT/JP2019/023478 and PCT/JP2019/007655).

In addition, among chemically crosslinked alginic acids, regarding the chemically crosslinked alginic acids that are manufactured using the chemically modified alginic acid derivatives represented by Formula (HB-I) and Formula (HB-II), the biocompatibility can be confirmed in examples regarding biocompatibility to be described below.

9. Uses of structure

Examples of a preferable use of the structure include medical materials such as a wound dressing, a postoperative adhesion barrier, a sustained drug release substrate, a cell transplantation substrate, a prosthetic material (an implant, an artificial organ or the like), a formulation coating material. In addition, the structure is used as a bio ink that is used in bio printers, and the use of a molded structure in the above-described uses can be exemplified. The structure is preferably used in vivo.

10. Method for using structure

As a method for transplanting the structure into living bodies, incision and placement, injection, endoscopy, and laparoscope can be used.

Transplantation sites are not particularly limited, examples thereof include subcutaneous, intraperitoneal, intrahepatic, intramuscular, omental, subrenal and the like, and the structure is preferably transplanted into subcutaneous or intraperitoneal.

All publications, published publications, patent publications and other patent literature cited in the present specification are regarded as being incorporated by reference into the present specification.

In addition, the objective, characteristics, advantages and ideas of the present invention are clear to a person skilled in the art from the description of the present specification, and a person skilled in the art should be able to easily perform the present invention based on the description of the present specification. Embodiments, specific examples and the like for performing the invention show preferable embodiments of the present invention, are shown for exemplification or description, and do not limit the present invention thereto. It is clear to a person skilled in the art that a variety of modifications based on the description of the present specification are possible within the intention and scope of the present invention that are disclosed in the present specification.

Examples

Next, in order to describe the present invention in more detail, examples and test examples will be described, but these examples are simply examples and test examples, do not limit the present invention, and may be modified without departing from the scope of the present invention.

In the measurement of nuclear magnetic resonance spectrum (NMR), JEOL JNM-ECX 400 FT-NMR (JEOL, Ltd.) was used. Liquid chromatography-mass spectrometry (LC-Mass) was measured by the following method. [UPLC] Waters AQUITY UPLC system and BEH C18 column (2.1 mm × 50 mm, 1.7 μm) (Waters Corporation) were used, and mobile phases and gradient conditions of acetonitrile:0.05% trifluoroacetate aqueous solution = 5:95 (0 minutes) to 95:5 (1.0 minute) to 95:5 (1.6 minutes) to 5:95 (2.0 minutes) were used.

In $^1$H-NMR data, regarding the patterns of NMR signals, s means singlet, d means doublet, t means triplet, q means quadruplet, m means multiplet, br means broad, J means the coupling constant, Hz means hertz, $CDCl_3$ means deuterated chloroform, DMSO-$d_6$ means deuterated dimethyl sulfoxide, and $D_2O$ means deuterated oxide. Regarding signals that were broad bands and thus could not be confirmed such as protons of a hydroxyl group (OH), an amino group ($NH_2$) and a carboxyl group (COOH), data was not entered in the $^1$H-NMR data.

In LC-Mass data, M means the molecular weight, RT means the retention time, $[M + H]^+$ and $[M + Na]^+$ mean molecular ionic peaks.

"Room temperature" in the examples normally indicates temperatures from approximately 0°C to approximately 35°C.

The reactive substituent introduction rate (mol%) in the examples is considered to indicate the proportion of the mole number of a reactive substituent introduced in the mole number of a monosaccharide (guluronic acid and mannuronic acid) unit that constitutes alginic acid calculated from $^1$H-NMR ($D_2O$).

In the examples, as sodium alginate into which a reactive group or a complementary reactive group was to be introduced, sodium alginates showing physical property values shown in Table 13 were used.

Table 15 shows the physical property values (specifically, reactive group introduction rate (mol%), molecular weight and weight-average molecular weight (10,000 Da)) of alginic acid derivatives into which a reactive group had been introduced that were obtained in (Example 1) to (Example 20).

Table 16-1 to Table 16-5 show $^1$H-NMR's of intermediates in (Example 1) to (Example 20), and Table 17 shows LCM-Mass's of the intermediates in (Example 1) to (Example 20).

(Example 1)

Synthesis of 3-azidopropylamino group-introduced alginic acid (EX1-A2):

[C82]

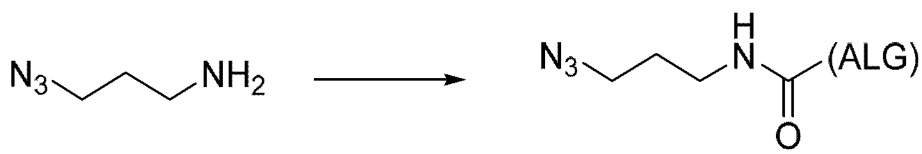

1-1 EX1-A2

4-(4,6-Dimethoxy-1,3,5-triazin-2-yl)-4-methylmorpholinium chloride (DMT-MM) (56 mg), an ethanol (2 mL) solution of commercially available 3-azidopropylamine [CAS REGISTRYNO.: 88192-19-2] (1-1, 5.1 mg) and 1-molar aqueous sodium bicarbonate solution (50 μL) were added to a sodium alginate (manufactured by MOCHIDA PHARMACEUTICAL CO., LTD.: A-2) aqueous solution (20 mL) prepared to 1 wt%. The components were stirred at 30°C for three hours, and then sodium chloride (0.2 g) and ethanol (40 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX1-A2 (187 mg) as a white solid.

(Example 2)

Synthesis of 2-(2-(2-azidoethoxy)ethoxy)ethane-1-amino group-introduced alginic acid (EX2-A2):

[C83]

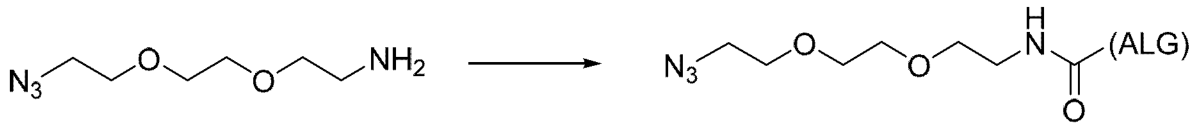

2-1 EX2-A2

4-(4,6-Dimethoxy-1,3,5-triazin-2-yl)-4-methylmorpholinium chloride (DMT-MM) (55.83 mg) and 1-molar aqueous sodium bicarbonate solution (252.17 µL) were added to a sodium alginate (manufactured by MOCHIDA PHARMACEUTICAL CO., LTD.: A-2) aqueous solution (10.9 mL) prepared to 1 wt% under ice cooling and stirring. Subsequently, an ethanol (1 mL) and water (1 mL) solution of commercially available 2-(2-(2-azidoethoxy)ethoxy)ethan-1-amine [CAS REGISTRYNO.: 166388-57-4] (2-1, 26.36 mg) was added thereto and stirred at room temperature for 15 hours, and then sodium chloride (100 mg) and ethanol (21.8 mL) were sequentially added thereto and stirred at room temperature for 30 minutes. An obtained precipitation was filtered, washed with ethanol and dried under reduced pressure, thereby obtaining a title compound EX2-A2 (99 mg) as a white solid.

(Example 3)

Synthesis of 2-amino-N-(3-azidopropyl) acetamide group-introduced alginic acid (EX3-A2):

[C84]

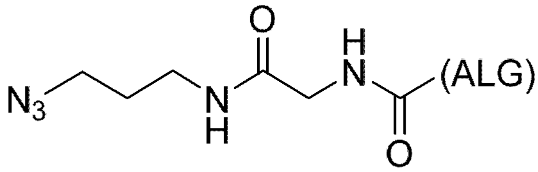

EX3-A2

<Step 1>

Synthesis of tert-butyl (2-((3-azidopropyl)amino)-2-oxoethyl)carbamate (3-2):

[C85]

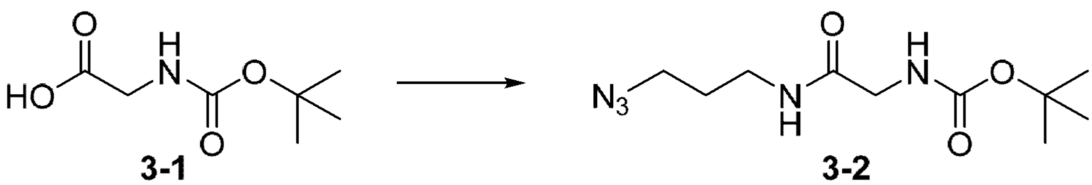

3-1 3-2

4-(4,6-Dimethoxy-1,3,5-triazin-2-yl)-4-methylmorpholinium chloride (DMT-MM) (197 mg) was added to an ethanol (2 mL) solution of commercially available 3-azidopropylamine [CAS REGISTRY NO.: 88192-19-2] (1-1, 41 μL) and N-(tert-butoxycarbonyl)glycine [CAS REGISTRY NO.: 4530-20-5] (3-1, 100 mg) and stirred at room temperature for 18 hours. Water was added to a reaction liquid, and an organic layer was extracted with ethyl acetate and then sequentially washed with water and brine. The organic layer was dried with anhydrous sodium sulfate and then concentrated under reduced pressure. An obtained oily substance was dissolved in methyl-tert-butyl ether (10 mL) and sequentially washed with saturated aqueous sodium bicarbonate solution, water and brine. The organic layer was dried with anhydrous sodium sulfate and then concentrated under reduced pressure, thereby obtaining a title compound 3-2 (95 mg) as a colorless oily substance.

<Step 2>

Synthesis of 2-amino-N-(3-azidopropyl)acetamide hydrochloride (3-3):

[C86]

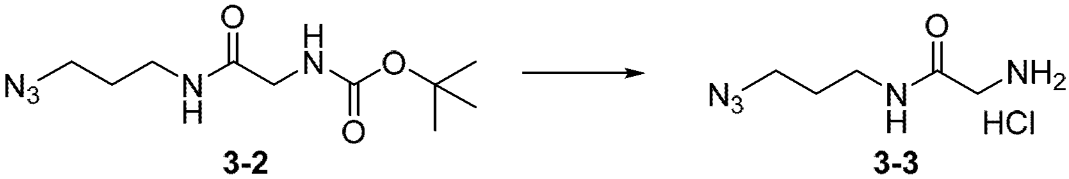

3-2 3-3

4N-hydrogen chloride/1,4-dioxane (665 μL) was added to the compound (3-2, 95 mg) obtained in <Step 1> of (Example 3) under ice water cooling and then stirred at room temperature for one hour. Diisopropyl ether (2.0 mL) was added to a reaction liquid and concentrated under reduced pressure. An obtained oily substance was decant-washed with methyl-tert-butyl ether and then concentrated under reduced pressure, thereby obtaining a title compound 3-3 (62 mg) as a colorless gummy substance.

<Step 3>

Synthesis of 2-amino-N-(3-azidopropyl) acetamide group-introduced alginic acid (EX3-A2):

[C87]

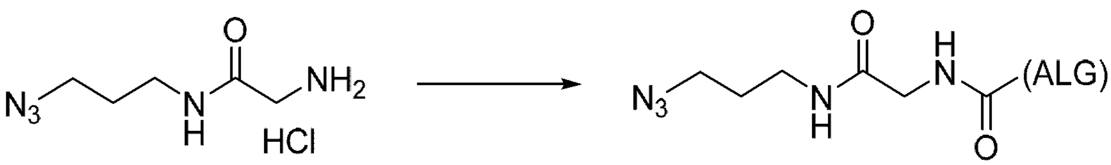

3-3 EX3-A2

4-(4,6-Dimethoxy-1,3,5-triazin-2-yl)-4-methylmorpholinium chloride (DMT-MM) (56 mg), an ethanol (2 mL) solution of the compound obtained in <Step 2> of (Example 3) (3-3, 10.6 mg) and 1-molar aqueous sodium bicarbonate solution (76 μL) were added to a sodium alginate (manufactured by MOCHIDA PHARMACEUTICAL CO., LTD.: A-2) aqueous solution (20 mL) prepared to 1 wt%. The components were stirred at 30°C for three hours, and then sodium chloride (0.2 g) and ethanol (40 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX3-A2 (207 mg) as a white solid.

(Example 4)

Synthesis of 3-amino-N-(3-azidopropyl)propanamide group-introduced alginic acid (EX4-A2):

[C88]

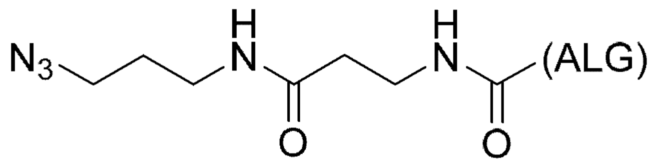

**EX4-A2**

<Step 1>

Synthesis of tert-butyl (3-((3-azidopropyl)amino)-3-oxopropyl)carbamate (4-2):

[C89]

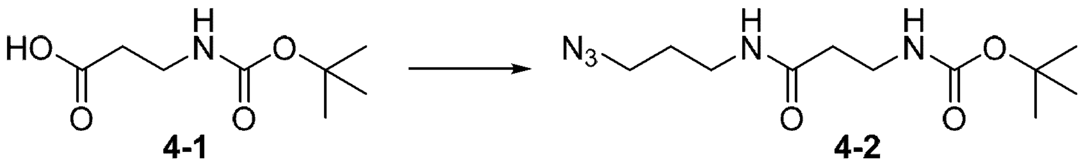

**4-1** **4-2**

4-(4,6-Dimethoxy-1,3,5-triazin-2-yl)-4-methylmorpholinium chloride (DMT-MM) (146 mg) was added to an ethanol (2 mL) solution of commercially available 3-azidopropylamine [CAS REGISTRY NO.: 88192-19-2] (1-1, 38 μL) and N-(tert-butoxycarbonyl)-β-alanine [CAS REGISTRY NO.: 3303-84-2] (4-1, 100 mg) and stirred at room temperature for 18 hours. Water was added to a reaction liquid, and an organic layer was extracted with ethyl acetate and then sequentially washed with water and brine. The organic layer was dried with anhydrous sodium sulfate and then concentrated under reduced pressure, thereby obtaining a title compound 4-2 (124 mg) as a white waxy substance.

<Step 2>

Synthesis of 3-amino-N-(3-azidopropyl)propanamide hydrochloride (4-3):

[C90]

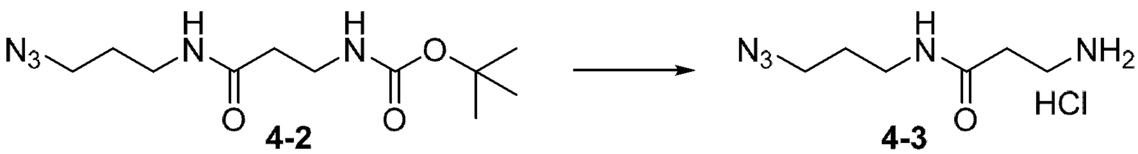

**4-2** **4-3**

4N-Hydrogen chloride/1,4-dioxane (868 μL) was added to the compound (4-2, 124 mg) obtained in <Step 1> of (Example 4) under ice water cooling and then stirred at room temperature for one hour. Diisopropyl ether (2.6 mL) was added to a reaction liquid and concentrated under reduced pressure. An obtained oily substance was decant-washed with methyl-tert-butyl ether and then concentrated under reduced pressure, thereby obtaining a title compound 4-3 (93 mg) as a colorless gummy substance.

<Step 3>

Synthesis of 3-amino-N-(3-azidopropyl)propanamide group-introduced alginic acid (EX4-A2):

[C91]

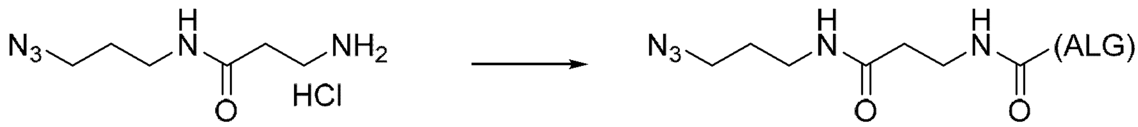

4-3 EX4-A2

4-(4,6-Dimethoxy-1,3,5-triazin-2-yl)-4-methylmorpholinium chloride (DMT-MM) (56 mg), an ethanol (2 mL) solution of the compound obtained in <Step 2> of (Example 4) (4-3, 12.6 mg) and 1-molar aqueous sodium bicarbonate solution (76 μL) were added to a sodium alginate (manufactured by MOCHIDA PHARMACEUTICAL CO., LTD.: A-2) aqueous solution (20 mL) prepared to 1 wt%. The components were stirred at 30°C for three hours, and then sodium chloride (0.2 g) and ethanol (40 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX4-A2 (211 mg) as a white solid.

(Example 5)

Synthesis of N-(4-(aminomethyl)benzyl)-2-azidoacetamide group-introduced alginic acid (EX5-A2):

[C92]

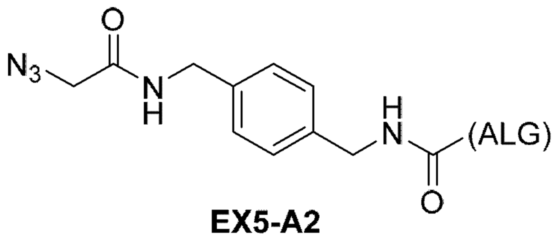

EX5-A2

<Step 1>

Synthesis of tert-butyl (4-((2-azidoacetamido)methyl)benzyl)carbamate (5-2):

[C93]

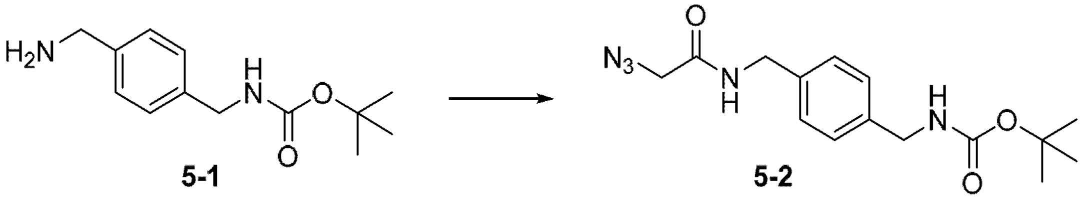

5-1 5-2

A methylene chloride (1.0 mL) solution of azidoacetic chloride prepared in the same manner as in a method disclosed in Organic Letters (2017), 19(23), 6400 to 6403 from commercially available 2-azidoacetic acid [CAS REGISTRY NO.: 18523-48-3] (41 μL) was added to a methylene chloride (1.0 mL) solution of commercially available 1-(N-tert-butytoxycarbonyl-aminomethyl)-4-(aminomethyl)benzene [CAS REGISTRY NO.: 108468-00-4] (5-1, 100 mg) and triethylamine (118 μL) under ice water cooling and stirred at room temperature for 2.5 hours. Ethyl acetate (20 mL) and water (5 mL) were added to a reaction liquid, the liquid was separated, and then an organic layer was sequentially washed with water, saturated aqueous sodium bicarbonate solution, water and brine. An insoluble matter was removed, and a filtrate was dried with anhydrous sodium sulfate and then concentrated under reduced pressure. A residue was triturated with methyl-tert-butyl ether/n-heptane. An obtained solid was filtered, thereby obtaining a title compound 5-2 (91 mg) as a thin beige solid.

<Step 2>

Synthesis of N-(4-(aminomethyl)benzyl)-2-azidoacetamide hydrochloride (5-3):

[C94]

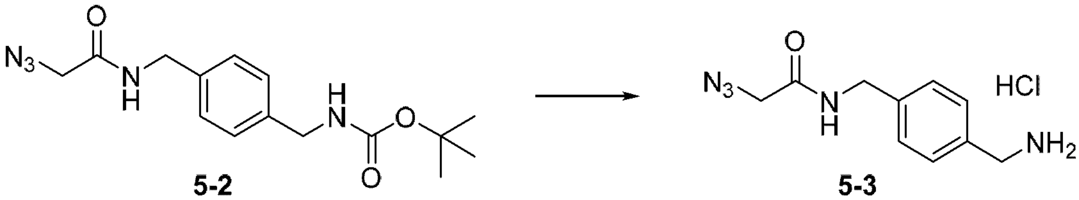

5-2 5-3

4N-Hydrogen chloride/1,4-dioxane (637 μL) was added to the compound (5-2, 91 mg) obtained in <Step 1> of (Example 5) under ice cooling, 1,4-dioxane (627 μL) was added thereto and then stirred at room temperature for 3.5 hours. Diisopropyl ether (3.8 mL) was added to a reaction liquid and stirred for 10 minutes. An obtained solid was filtered, thereby obtaining a title compound 5-3 (62 mg) as a beige solid.

<Step 3>

Synthesis of N-(4-(aminomethyl)benzyl)-2-azidoacetamide group-introduced alginic acid (EX5-A2):

[C95]

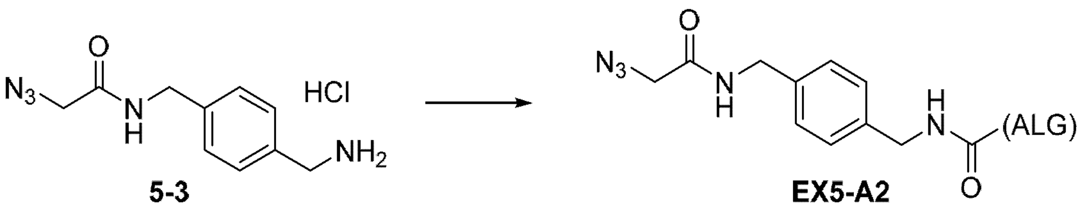

5-3 EX5-A2

4-(4,6-Dimethoxy-1,3,5-triazin-2-yl)-4-methylmorpholinium chloride (DMT-MM) (70 mg), the compound obtained in <Step 2> of (Example 5) (5-3, 16.1 mg) and 1-molar aqueous sodium bicarbonate solution (95 μL) were added to a sodium alginate (manufactured by MOCHIDA PHARMACEUTICAL CO., LTD.: A-2) aqueous solution (25 mL) prepared to 1 wt%. The components were stirred at 30°C for three hours, and then sodium chloride (0.25 g) and ethanol (50 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX5-A2 (239 mg) as a white solid.

(Example 6)

Synthesis of 2-(4-azidophenoxy)ethane-1-amino group-introduced alginic acid (EX6-A2):

[C96]

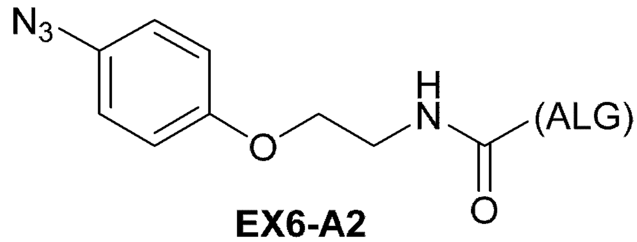

EX6-A2

<Step 1>

Synthesis of tert-butyl (2-(4-azidophenoxy)ethyl)carbamate (6-3):

[C97]

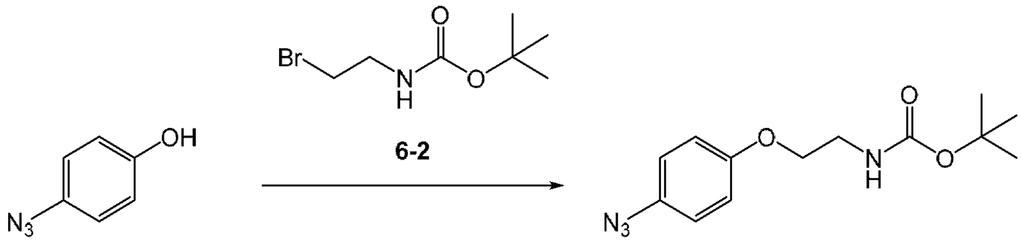

6-2

6-1

6-3

Potassium carbonate (0.61 g) was added to a mixture of commercially available 4-azidophenol [CAS REGISTRYNO.: 24541-43-3] (6-1, 0.3 g), commercially available tert-butyl (2-bromoethyl)carbamate [CAS REGISTRY NO.: 39684-80-5] (6-2, 0.6 g) and N-methylpyrrolidone (3 mL). After a reaction mixture was stirred at 80°C for six hours 30 minutes and cooled to room temperature, water (10 mL) and methyl tert-butyl ether (20 mL) were added thereto. A generated suspension was filtered with Celite, and a residue was washed with methyl tert-butyl ether (5 mL) twice. A filtrate was separated, and an organic layer was concentrated under reduced pressure, thereby obtaining a crude product. This crude product was dissolved in methyl tert-butyl ether (20 mL), sequentially washed with a 1 N-sodium hydroxide aqueous solution (5 mL) twice, water (5 mL) twice and brine (5 mL) and dried with anhydrous sodium sulfate. The organic layer was filtered and then concentrated under reduced pressure, thereby obtaining a title compound 6-3 (0.411 g) as a purple oily substance.

<Step 2>

Synthesis of 2-(4-azidophenoxy)ethane-1-amine hydrochloride (6-4):

[C98]

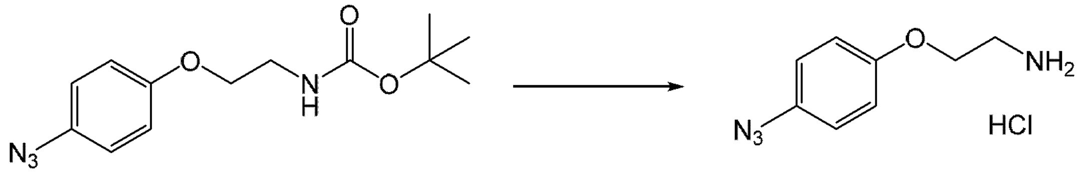

6-3

6-4

4N-Hydrogen chloride/1,4-dioxane (2.87 mL) was added to a mixture of the compound (6-3, 0.41 g) obtained in <Step 1> of (Example 6) and 1,4-dioxane (2.87 mL) under water cooling and stirring and then stirred at room temperature for 18 hours. Diisopropyl ether (40 mL) was added to a reaction liquid, and a suspension was stirred at room temperature for 30 minutes. A precipitate was filtered, and a recovered solid was dried under reduced pressure, thereby obtaining a title compound 6-4 (0.2834 g) as a light purple solid.

<Step 3>

Synthesis of 2-(4-azidophenoxy)ethane-1-amino group-introduced alginic acid (EX6-A2):

[C99]

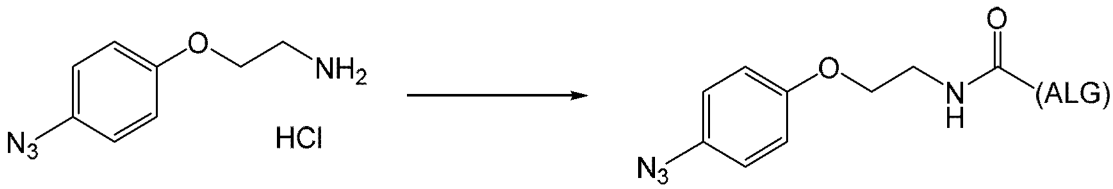

6-4

EX6-A2

4-(4,6-Dimethoxy-1,3,5-triazin-2-yl)-4-methylmorpholinium chloride (DMT-MM) (91.52 mg) and 1-molar aqueous sodium bicarbonate solution (68.63 μL) were added to a sodium alginate (manufactured by MOCHIDA PHARMACEUTICAL CO., LTD.: A-2) aqueous solution (29.66 mL) prepared to 1 wt% at room temperature. Subsequently, a water (1 mL) and ethanol (1 mL) solution of the compound (6-4, 14.73 mg) obtained in <Step 2> of (Example 6) was added thereto at room temperature and stirred at the same temperature for 42 hours, and then sodium chloride (300 mg) and ethanol (59.3 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX6-A2 (269 mg) as a pink solid.

(Example 7)

Synthesis of N-(2-aminoethyl)-2-(cyclooct-2-yn-1-yloxy)acetamide group-introduced alginic acid (EX7-B2):

[C100]

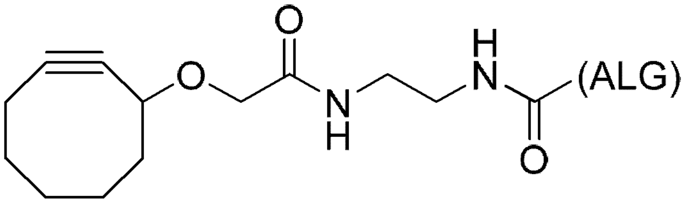

EX7-B2

<Step 1>

Synthesis of tert-butyl (2-(2,2,2-trifluoroacetamido)ethyl)carbamate (7-2):

[C101]

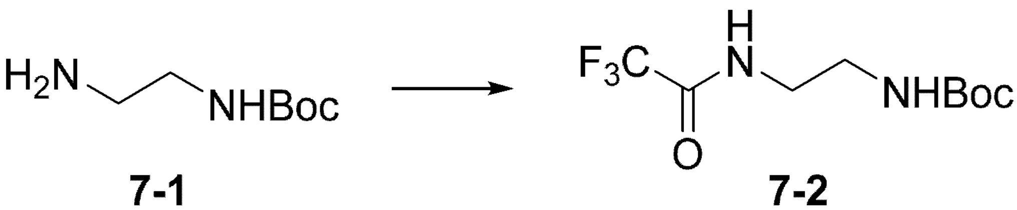

7-1 7-2

Ethyl trifluoroacetate (2.24 mL) was added dropwise to a tetrahydrofuran (12.0 mL) solution of commercially available tert-butyl (2-aminoethyl)carbamate (7-1, 3.00 g, [CAS REGISTRY NO.: 57260-73-8]). A reaction mixture was stirred at room temperature for 14.5 hours. A reaction liquid was concentrated under reduced pressure, and tert-butyl methyl ether (5 mL) and heptane (25 mL) were added to a residue and triturated. A solid was filtered and then washed with heptane, thereby obtaining a title compound 7-2 (4.36 g) as a white solid.

<Step 2>

Synthesis of N-(2-aminoethyl)-2,2,2-trifluoroacetamide hydrochloride (7-3):

[C102]

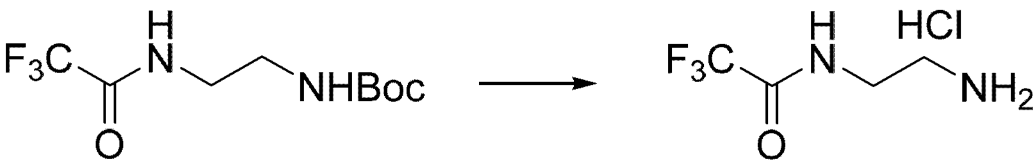

7-2 7-3

The compound 7-2 (0.50 g) obtained in <Step 1> of (Example 7) was suspended in 1,4-dioxane (3.0 mL). 4N-Hydrogen chloride/1,4-dioxane (7.0 mL) was added thereto and stirred at room temperature for three hours under ice water cooling. Diisopropyl ether (30.0 mL) was added to a reaction liquid and stirred at room temperature for 50 minutes. A solid was filtered, washed with diisopropyl ether and then dried under reduced pressure, thereby obtaining a title compound 7-3 (0.70 g) as a white solid.

<Step 3>

Synthesis of N-(2-(2-(cyclooct-2-yn-1-yloxy)acetamido)ethyl)-2,2,2-trifluoroacetamide (7-5):

[C103]

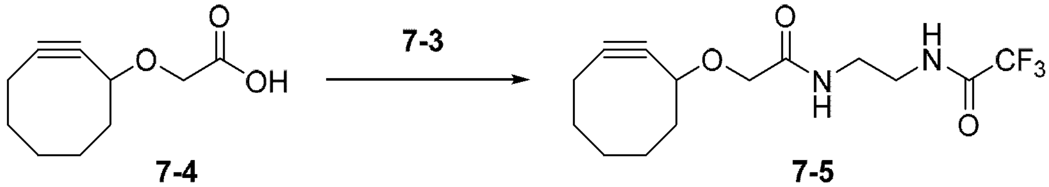


7-4 7-5

4-(4,6-Dimethoxy-1,3,5-triazin-2-yl)-4-methylmorpholinium chloride (DMT-MM) (1.09 g), the compound 7-3 (380 mg) obtained in <Step 2> of (Example 7) and triethylamine (321 μL) were added to an ethanol (2 mL) solution of carboxylic acid (7-4, 300 mg) synthesized according to a method well known by a publication (Org. Process Res. Dev. (2018) 22: 108 to 110) and stirred at 30°C for three hours, and then triethylamine (229 μL) was added thereto and stirred at the same temperature for one hour. The components were further stirred at room temperature for 15.5 hours, water (10 mL) and ethyl acetate (50 mL) were added thereto, liquid was separated, and a water layer was extracted with ethyl acetate (10 mL). An organic layer was sequentially washed with 0.5N-citric acid, water and brine, dried with anhydrous sodium sulfate and then concentrated under reduced pressure. tert-Butyl methyl ether was added to a residue, an insoluble matter was removed, and a filtrate was concentrated and then purified by silica gel column chromatography (10%-ethyl acetate/n-heptane to 40% ethyl acetate/n-heptane), thereby obtaining a title compound 7-5 (322 mg) as a white solid.

<Step 4>

Synthesis of N-(2-aminoethyl)-2-(cyclooct-2-yn-1-yloxy)acetamide (7-6):

[C104]

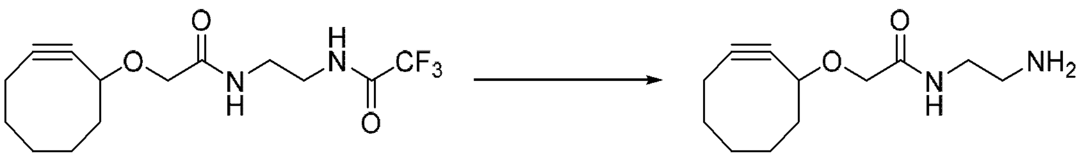

7-5 7-6

A water (1.6 mL) solution of potassium carbonate (278 mg) was added to a methanol (4.8 mL) solution of the compound 7-5 (322 mg) obtained in <Step 3> of (Example 7) and stirred at room temperature for 7.5 hours. A reaction liquid was concentrated under reduced pressure, water (3 mL) was added thereto, and then the reaction liquid was saturated with sodium chloride. A water layer was extracted with ethyl acetate (30 mL, 10 mL × 3), dried with anhydrous sodium sulfate and then concentrated under reduced pressure, thereby obtaining a title compound 7-6 (238 mg) as a colorless oily substance.

<Step 5>

Synthesis of N-(2-aminoethyl)-2-(cyclooct-2-yn-1-yloxy)acetamide group-introduced alginic acid (EX7-B2):

[C105]

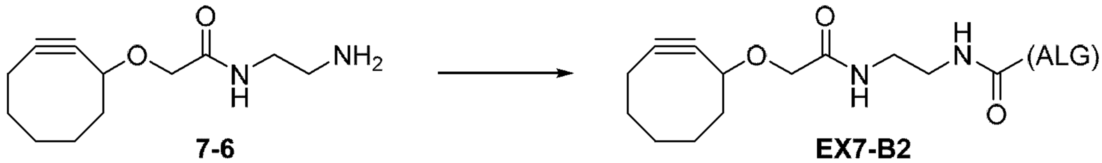

7-6 EX7-B2

4-(4,6-Dimethoxy-1,3,5-triazin-2-yl)-4-methylmorpholinium chloride (DMT-MM) (335 mg), an ethanol (12 mL) solution of the compound 7-6 (68 mg) obtained in <Step 4> of (Example 7) and 1-molar aqueous sodium bicarbonate solution (303 μL) were sequentially added to a sodium alginate (manufactured by MOCHIDA PHARMACEUTICAL CO., LTD.: B-2) aqueous solution (120 mL) prepared to 1 wt% at room temperature under stirring and stirred at 30 °C for three hours. Sodium chloride (1.2 g) was added to a reaction liquid, then, ethanol (240 mL) was added thereto and stirred for 1.5 hours. An obtained precipitation was filtered, washed with ethanol (20 mL × 5) and dried under reduced pressure. An obtained solid was dissolved in water, lyophilized, thereby obtaining a title compound EX7-B2 (1.16 g) as a white solid.

(Example 8)

Synthesis of N-(2-(2-aminoethoxy)ethyl)-2-(cyclooct-2-yn-1-yloxy)acetamide group-introduced alginic acid (EX8-A2):

[C106]

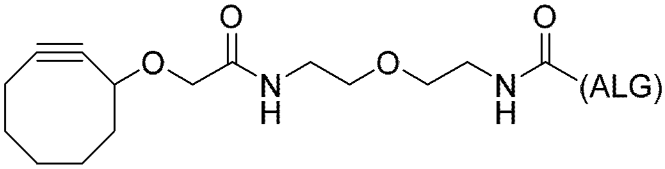

**EX8-A2**

<Step 1>

Synthesis of tert-butyl (2-(2-(2,2,2-trifluoroacetamido)ethoxy)ethyl)carbamate (8-2):

[C107]

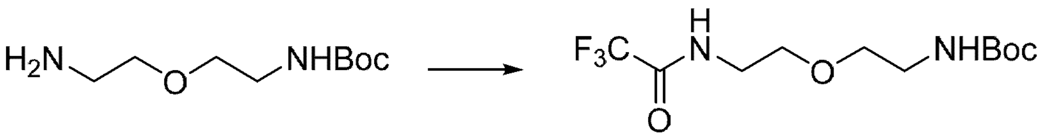

**8-1** **8-2**

Ethyl trifluoroacetate (0.6 mL) was added dropwise to a tetrahydrofuran (4.0 mL) solution of tert-butyl (2-(2-aminoethoxy)ethyl)carbamate (8-1, 1.0 g, [CAS REGISTRY NO.: 127828-22-2]). A reaction mixture was stirred at room temperature for 3.5 hours and concentrated under reduced pressure, thereby obtaining a title crude compound 8-2 (1.5 g) as a colorless oily substance.

<Step 2>

Synthesis of N-(2-(2-aminoethoxy)ethyl)-2,2,2-trifluoroacetamide hydrochloride (8-3):

[C108]

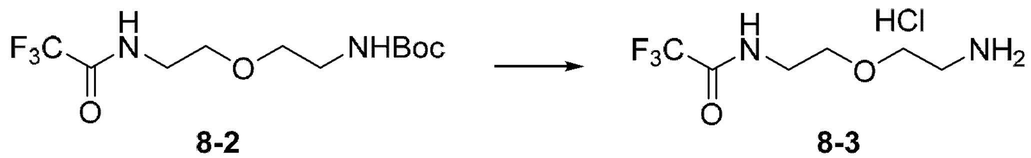

8-2 8-3

A 4N-hydrogen chloride/1,4-dioxane solution (10.3 mL) was added to the compound 8-2, (1.5 g) obtained in <Step 1> of (Example 8) under ice water cooling and stirred at room temperature for one hour. Diisopropyl ether (30 mL) was added to a reaction liquid and stirred at room temperature for 30 minutes. A solvent was distilled away under reduced pressure, an azeotrope of the reaction liquid was produced with diisopropyl ether and then dried under reduced pressure, thereby obtaining a title compound 8-3 (1.3 g) as a colorless oily substance.

<Step 3>

Synthesis of N-(2-(2-(2-(cyclooct-2-yn-1-yloxy)acetamido)ethoxy)ethyl)-2,2,2-trifluoroacetamide (8-4):

[C109]

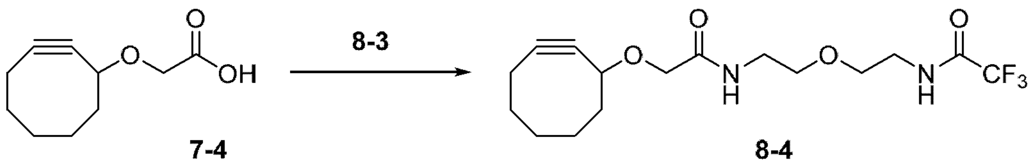


7-4 8-4

Carboxylic acid (7-4, 300 mg) synthesized according to a method well known by a publication (Org. Process Res. Dev. (2018) 22: 108 to 110) and the compound 8-3 (443 mg) obtained in <Step 2> of (Example 8) were dissolved in acetonitrile (6.0 mL). O-(7-azabenzotriazol-1-yl)-N,N,N',N'-tetramethyluronium hexafluorophosphate (0.75 g) and N,N-diisopropylethylamine (920 μL) were added thereto and stirred at room temperature for 2.5 hours. Ethyl acetate (20 mL) and water (10 mL) were added to a reaction liquid, and a liquid was separated. An organic layer was sequentially washed with water (10 mL) and brine (5 mL), dried with anhydrous sodium sulfate and then concentrated under reduced pressure. A residue was purified by silica gel column chromatography (50% ethyl acetate/n-heptane to 70% ethyl acetate/n-heptane), thereby obtaining a title compound 8-4 (469 mg) as a colorless gummy substance.

<Step 4>

Synthesis of N-(2-(2-aminoethoxy)ethyl)-2-(cyclooct-2-yn-1-yloxy)acetamide (8-5):

[C110]

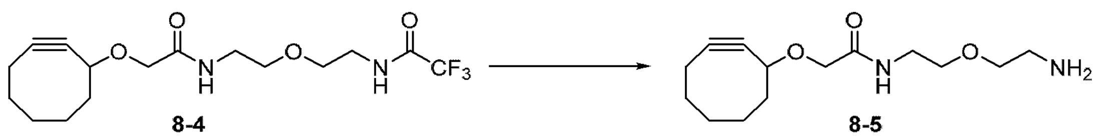

8-4 8-5

A water (0.99 mL) solution of potassium carbonate (103 mg) was added to a methanol (3.0 mL) solution of the compound 8-4 (220 mg) obtained in <Step 3> of (Example 8) and stirred at room temperature for 4.5 hours. Methanol was distilled away under reduced pressure, water (2 mL) was added thereto, and the solution mixture was saturated with salt. The solution mixture was extracted with ethyl acetate (15 mL, 10 mL × 4) and dried with anhydrous sodium sulfate, and then a solvent was distilled away under reduced pressure. A residue was dissolved in ethyl acetate (10 mL), and an insoluble matter was filtered out and then concentrated under reduced pressure, thereby obtaining a title crude compound 8-5 (140 mg) as a light yellow gummy substance.

<Step 5>

Synthesis of N-(2-(2-aminoethoxy)ethyl)-2-(cyclooct-2-yn-1-yloxy)acetamide group-introduced alginic acid (EX8-A2):

[C111]

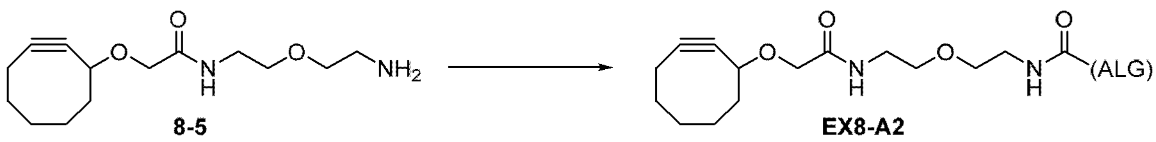

4-(4,6-Dimethoxy-1,3,5-triazin-2-yl)-4-methylmorpholinium chloride (DMT-MM) (112 mg), an ethanol (4.0 mL) solution of the compound 8-5 (30 mg) obtained in <Step 4> of (Example 8) and 1-molar aqueous sodium bicarbonate solution (101 μL) were sequentially added to a sodium alginate (manufactured by MOCHIDA PHARMACEUTICAL CO., LTD.: A-2) aqueous solution (40 mL) prepared to 1 wt% at room temperature under stirring and stirred at 30°C for three hours. Sodium chloride (0.4 g) was added to a reaction liquid, and then ethanol (80 mL) was added thereto and stirred for 30 minutes. An obtained precipitation was filtered, washed with ethanol and dried under reduced pressure. An obtained solid was dissolved in water, lyophilized, thereby obtaining a title compound EX8-A2 (410 mg) as a white solid.

(Examples 9a and 9b)

Syntheses of N-(2-aminoethyl)-2-(2-(cyclooct-2-yn-1-yloxy)acetamide)acetamide group-introduced alginic acids (EX9a-A2 and EX9b-B2):

[C112]

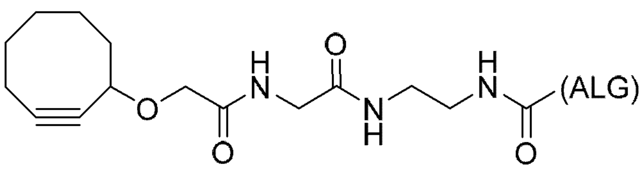

**EX9a-A2**
**EX9b-B2**

<Step 1>

Synthesis of tert-butyl (2-oxo-2-((2-(2,2,2-trifluoroacetamido)ethyl)amino)ethyl)carbamate (9-1):

[C113]

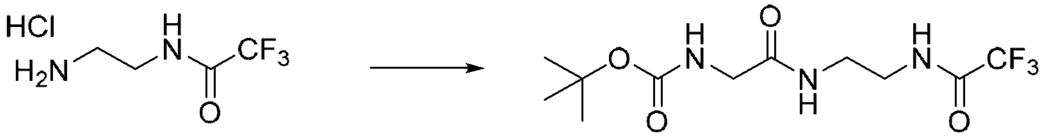

7-3

9-1

N-(tert-Butoxycarbonyl)glycine (91 mg, [CAS REGISTRY NO.: 4530-20-5]) and the compound (7-3, 100 mg) obtained in <Step 2> of (Example 7) were dissolved in acetonitrile (3.0 mL). O-(7-azabenzotriazol-1-yl)-N,N,N',N'-tetramethyluronium hexafluorophosphate (217 mg) and N,N-diisopropylethylamine (281 μL) were added thereto and stirred at room temperature for 3.5 hours. Ethyl acetate (15 mL) and water (5 mL) were added to a reaction liquid, liquid was separated, and then an organic layer was sequentially washed with water and brine. The organic layer was dried with anhydrous sodium sulfate and then concentrated under reduced pressure. A residue was purified by column chromatography (elution solvent: 40% ethyl acetate/n-heptane → ethyl acetate), thereby obtaining a title compound 9-1 (180 mg) as thin beige amorphous.

<Step 2>

Synthesis of N-(2-(2-aminoacetamido)ethyl)-2,2,2-trifluoroacetamide hydrochloride (9-2):

[C114]

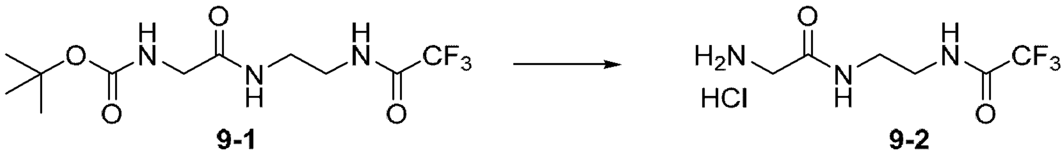

9-1

9-2

4N-Hydrogen chloride/1,4-dioxane (1.2 mL) was added to the compound (9-1, 180 mg) obtained in <Step 1> of (Example 9) under ice water cooling and then stirred at room temperature for 0.8 hours. Diisopropyl ether (3.6 mL) was added to a reaction liquid and stirred for 30 minutes. An obtained solid was filtered, thereby obtaining a title compound 9-2 (114 mg) as a white solid.

<Step 3>

Synthesis of N-(2-(2-(2-(cyclooct-2-yn-1-yloxy)acetamido)acetamido)ethyl)-2,2,2-trifluoroacetamide (9-3):

[C115]

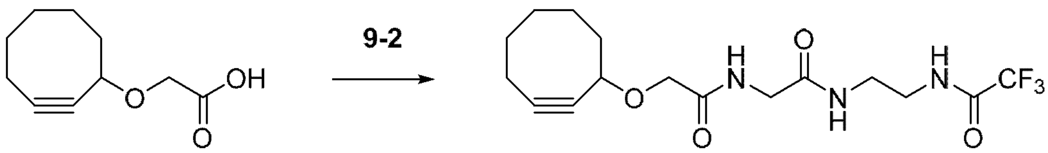


7-4

9-3

Ethanol (1.6 mL), 4-(4,6-dimethoxy-1,3,5-triazin-2-yl)-4-methylmorpholinium chloride (DMT-MM) (219 mg) and triethylamine (67 μL) were added to carboxylic acid (7-4, 80 mg) synthesized according to a method well known by a publication (Org. Process Res. Dev. (2018) 22: 108 to 110) and the compound (9-2, 110 mg) obtained in <Step 2> of (Example 9) and stirred at room temperature for three hours. Water (3.2 mL) was added to a reaction liquid and stirred at room temperature for 30 minutes, and then a solid was filtered and washed with water. Ethyl acetate/ethanol (1/1, 10

mL) was added to the obtained solid, and an insoluble matter was removed. A filtrate was concentrated under reduced pressure, thereby obtaining a title compound 9-3 (101 mg) as a white solid.

<Step 4>

Synthesis of N-(2-aminoethyl)-2-(2-(cyclooct-2-yn-1-yloxy)acetamide)acetamide (9-4):

[C116]

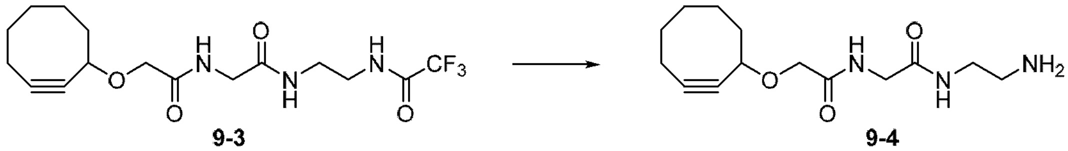

9-3 9-4

A water (0.3 mL) solution of potassium carbonate (59 mg) was added to a methanol (1.8 mL) solution of the compound (9-3, 60 mg) obtained in <Step 3> of (Example 9) and stirred at room temperature for four hours. A reaction liquid was concentrated under reduced pressure, then, water (2 mL) was added thereto, and the reaction liquid was saturated with sodium chloride. An extracted layer was extracted with ethyl acetate (15 mL, 10 mL × 4) and concentrated under reduced pressure. Ethyl acetate (10 mL) and ethanol (1 mL) were added to a residue, and an insoluble matter was removed. An obtained filtrate was concentrated under reduced pressure, thereby obtaining a title compound 9-4 (49 mg) as a colorless gummy substance.

<Step 5-1>

Synthesis of N-(2-aminoethyl)-2-(2-(cyclooct-2-yn-1-yloxy)acetamide)acetamide group-introduced alginic acid (EX9a-A2):

[C117]

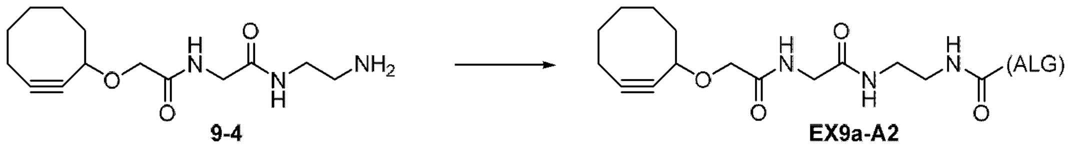

9-4 EX9a-A2

4-(4,6-Dimethoxy-1,3,5-triazin-2-yl)-4-methylmorpholinium chloride (DMT-MM) (106 mg), an ethanol (3.8 mL) solution of the compound (9-4, 30.3 mg) obtained in <Step 4> of (Example 9) and 1-molar aqueous sodium bicarbonate solution (96 μL) were added to a sodium alginate (manufactured by MOCHIDA PHARMACEUTICAL CO., LTD.: A-2) aqueous solution (38 mL) prepared to 1 wt%. The components were stirred at 30°C for 3.2 hours, and then sodium chloride (0.38 g) and ethanol (76 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX9a-A2 (381 mg) as a white solid.

<Step 5-2>

Synthesis of N-(2-aminoethyl)-2-(2-(cyclooct-2-yn-1-yloxy)acetamide)acetamide group-introduced alginic acid (EX9b-B2):

[C118]

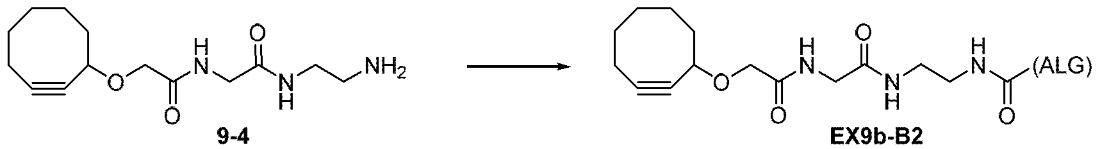

9-4

EX9b-B2

4-(4,6-Dimethoxy-1,3,5-triazin-2-yl)-4-methylmorpholinium chloride (DMT-MM) (64 mg), the compound (9-4, 18.2 mg) obtained in <Step 4> of (Example 9) and 1-molar aqueous sodium bicarbonate solution (58 μL) were added to a sodium alginate (manufactured by MOCHIDA PHARMACEUTICAL CO., LTD.: B-2) aqueous solution (38 mL) prepared to 1 wt%. The components were stirred at 30°C for 3.2 hours, and then sodium chloride (0.38 g) and ethanol (76 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX9b-B2 (366 mg) as a white solid.

(Example 10)

Synthesis of N-(2-aminoethyl)-3-(2-(cyclooct-2-yn-1-yloxy)acetamide)propanamide group-introduced alginic acid (EX10-A2):

[C119]

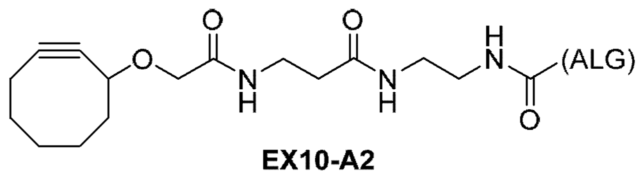

EX10-A2

<Step 1>

Synthesis of tert-butyl (3-oxo-3-((2-(2,2,2-trifluoroacetamido)ethyl)amino)propyl)carbamate (10-1):

[C120]

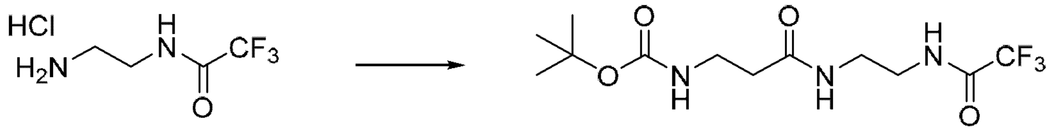

7-3

10-1

Commercially available N-(tert-butoxycarbonyl)-β-alanine (113 mg, [CAS REGISTRY NO.: 3303-84-2]) and the compound (7-3, 110 mg) obtained in <Step 2> of (Example 7) were dissolved in acetonitrile (3.3 mL). O-(7-azabenzotriazol-1-yl)-N,N,N',N'-tetramethyluronium hexafluorophosphate (261 mg) and N,N-diisopropylethylamine (319 μL) were added thereto and stirred at room temperature for three hours. Ethyl acetate (15 mL) and water (5 mL) were added to a reaction liquid, liquid was separated, and then an organic layer was sequentially washed with water and brine. The organic layer was dried with anhydrous sodium sulfate, then, concentrated under reduced pressure and triturated with tert-butyl methyl ether (20 mL). A solid was filtered and dissolved in ethyl acetate (20 mL). The organic layer was sequentially washed with 1N-citric acid, water and brine, then, dried with anhydrous sodium sulfate and then concentrated under reduced pressure. A residue was triturated with tert butyl methyl ether (10 mL), and then a solid was filtered, thereby obtaining a title compound 10-1 (80 mg) as a white solid.

<Step 2>

Synthesis of 3-amino-N-(2-(2,2,2-trifluoroacetamido)ethyl)propanamide hydrochloride (10-2):

[C121]

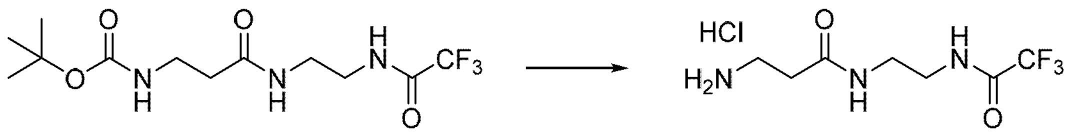

10-1 10-2

4N-Hydrogen chloride/1,4-dioxane (1.1 mL) was added to the compound (10-1, 80 mg) obtained in <Step 1> of (Example 10) under ice water cooling and then stirred at room temperature for two hours. Diisopropyl ether (3.4 mL) was added to a reaction liquid and stirred for 1.5 hours. An obtained solid was filtered, thereby obtaining a title compound 10-2 (61 mg) as a white solid.

<Step 3>

Synthesis of 3-(2-(cyclooct-2-yn-1-yloxy)acetamide)-N-(2-(2,2,2-trifluoroacetamido)ethyl)propanamide (10-3):

[C122]

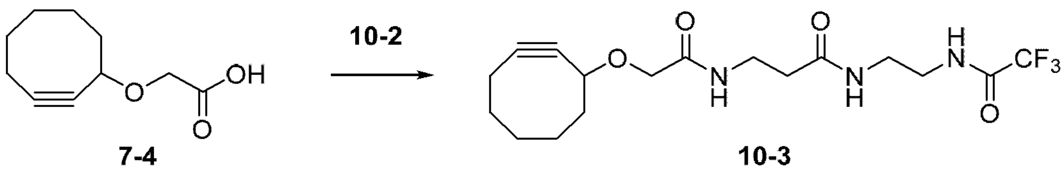


7-4 10-3

Ethanol (1.2 mL), 4-(4,6-dimethoxy-1,3,5-triazin-2-yl)-4-methylmorpholinium chloride (DMT-MM) (115 mg) and triethylamine (39 μL) were added to carboxylic acid (7-4, 44 mg) synthesized according to a method well known by a publication (Org. Process Res. Dev. (2018) 22: 108 to 110) and the compound (10-2, 61 mg) obtained in <Step 2> of (Example 10) and stirred at room temperature for two hours. Water (3.7 mL) was added to a reaction liquid, and an organic layer was extracted with ethyl acetate (15 mL, 5 mL). The organic layer was sequentially washed with water and brine, dried with anhydrous sodium sulfate and then concentrated under reduced pressure. tert-Butyl methyl ether (10 mL) was added to the obtained solid, triturated and filtered. The obtained solid was purified by column chromatography (80% ethyl acetate/n-heptane → 20% methanol/ethyl acetate), thereby obtaining a title compound 10-3 (60 mg) as a light yellow solid.

<Step 4>

Synthesis of N-(2-aminoethyl)-3-(2-(cyclooct-2-yn-1-yloxy)acetamide)propanamide (10-4):

[C123]

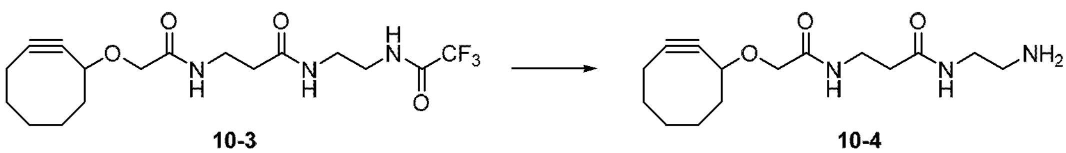

10-3 10-4

A water (0.3 mL) solution of potassium carbonate (42 mg) was added to a methanol (3.0 mL) solution of the compound (10-3, 60 mg) obtained in <Step 3> of (Example 10) and stirred at room temperature for three hours, then, furthermore, a water (0.3 mL) solution of potassium carbonate (42 mg) was added thereto and stirred at room temperature for 16.5 hours. A reaction liquid was concentrated under reduced pressure, then, brine (2 mL) was added thereto, and, furthermore, the reaction liquid was saturated with sodium chloride. A layer was extracted with ethyl acetate (15 mL, 10 mL × 4), and the extracted layer was dried with anhydrous sodium sulfate and then concentrated under reduced pressure. Ethyl acetate (5 mL) and several droplets of methanol were added to a residue, and an insoluble matter was removed. An obtained filtrate was concentrated under reduced pressure, thereby obtaining a title compound 10-4 (31 mg) as a colorless oily substance.

<Step 5>

Synthesis of N-(2-aminoethyl)-3-(2-(cyclooct-2-yn-1-yloxy)acetamide)propanamide group-introduced alginic acid (EX10-A2):

[C124]

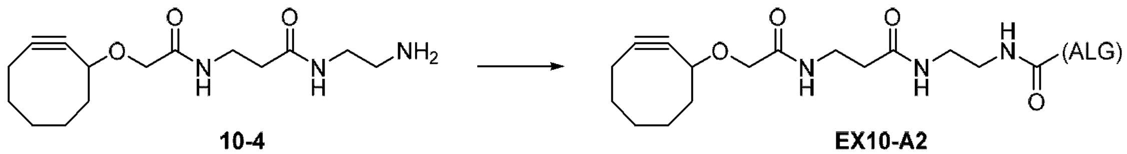

10-4 EX10-A2

4-(4,6-Dimethoxy-1,3,5-triazin-2-yl)-4-methylmorpholinium chloride (DMT-MM) (114 mg), an ethanol (4.1 mL) solution of the compound (10-4, 30.5 mg) obtained in <Step 4> of (Example 10) and 1-molar aqueous sodium bicarbonate solution (103 μL) were added to a sodium alginate (manufactured by MOCHIDA PHARMACEUTICAL CO., LTD.: A-2) aqueous solution (41 mL) prepared to 1 wt%. The components were stirred at 30°C for three hours, and then sodium chloride (0.41 g) and ethanol (82 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX10-A2 (406 mg) as a white solid.

(Examples 11a and 11b)

Syntheses of 2-(cyclooct-2-yn-1-yloxy)ethane-1-amino group-introduced alginic acids (EX11a-A2 and EX11b-B2):

[C125]

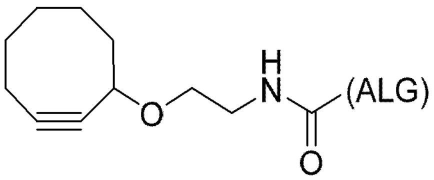

**EX11a-A2**
**EX11b-B2**

<Step 1>

Synthesis of (E)-N-(2-((2-bromocyclooct-2-en-1-yl)oxy)ethyl)-2,2,2-trifluoroacetamide (11-3):

[C126]

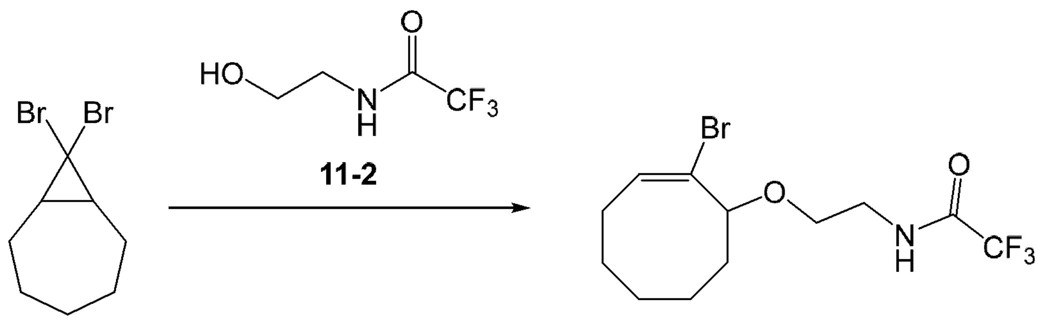

11-2

11-1 11-3

Dichloromethane (2 mL) was added to a mixture of a dibromo form (11-1, 1 g) synthesized according to a method well known by a publication (Org. Process Res. Dev. (2018) 22: 108 to 110) and an alcohol form (11-2, 5.28 g) synthesized according to a method well known by a publication (WO 2015/140807) at room temperature. A reaction container was protected from light by being wrapped with an aluminum foil while holding the inner temperature at room temperature. Subsequently, silver trifluoromethanesulfonate (1.92 g) was added thereto once at room temperature and stirred at the same temperature for one hour. After the stirring, brine (5 mL) was added thereto under ice cooling, a precipitated silver salt was removed by Celite filtration, and a residue was washed with methyl tert-butyl ether (10 mL). A filtrate was separated, and an organic layer was washed with water (5 mL) twice. After that, the organic layer was dried with anhydrous sodium sulfate, filtered and then concentrated under reduced pressure, thereby obtaining a crude product. This crude product was purified by silica gel column chromatography (n-heptane/ethyl acetate), thereby obtaining a fraction of a compound 11-3 (0.46 g).

<Step 2>

Synthesis of 2-(cyclooct-2-yn-1-yloxy)ethan-1-amine (11-4):

[C127]

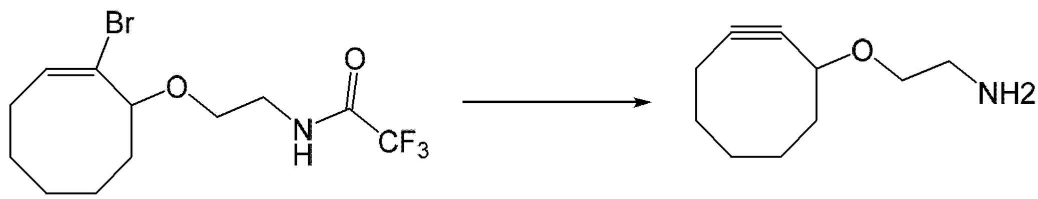

11-3 11-4

A 28% sodium methoxide methanol solution (1.82 mL) was added to a mixture of a fraction comprising the compound (11-3, 0.46 g) obtained in <Step 1> of (Example 11) and dimethyl sulfoxide (1.38 mL) under water cooling and stirring and stirred at room temperature for 16 hours. Water (10 mL) was added thereto to stop the reaction, and methanol was concentrated under reduced pressure. A generated solution was extracted with methyl tert-butyl ether (10 mL) three times. An organic layer was dried with anhydrous sodium sulfate, filtered and concentrated under reduced pressure, thereby obtaining a crude product of a title compound 11-4 (0.196 g) as a brown oily substance.

<Step 3-1>

Synthesis of 2-(cyclooct-2-yn-1-yloxy)ethane-1-amino group-introduced alginic acid (EX11a-A2):

[C128]

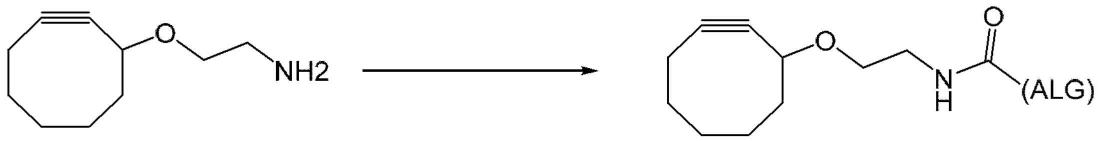

11-4 EX11a-A2

4-(4,6-Dimethoxy-1,3,5-triazin-2-yl)-4-methylmorpholinium chloride (DMT-MM) (213.55 mg) was added to a sodium alginate (manufactured by MOCHIDA PHARMACEUTICAL CO., LTD.: A-2) aqueous solution (69.2 mL) prepared to 1 wt% at room temperature. Subsequently, a water (1 mL) and ethanol (1 mL) solution of the compound (11-4, 26.78 mg) obtained in <Step 2> of (Example 11) was added thereto at room temperature and stirred at the same temperature for 24 hours, and then sodium chloride (700 mg) and ethanol (138.4 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX11a-A2 (661 mg) as a white solid.

<Step 3-2>

Synthesis of 2-(cyclooct-2-yn-1-yloxy)ethane-1-amino group-introduced alginic acid (EX11b-B2):

[C129]

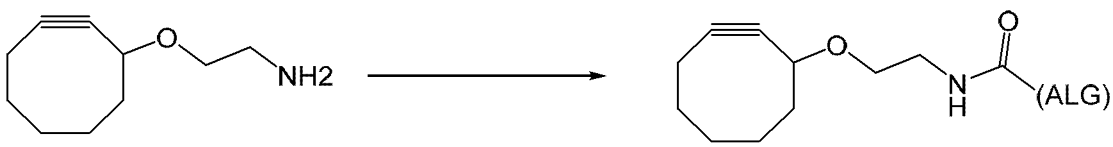

11-4 EX11b-B2

The same operation as <Step 3-1> of (Example 11) was performed using a sodium alginate (manufactured by MOCHIDA PHARMACEUTICAL CO., LTD.: B-2) aqueous solution (70.1 mL) prepared to 1 wt%, 4-(4,6-dimethoxy-1,3,5-triazin-2-yl)-4-methylmorpholinium chloride (DMT-MM) (216.4 mg) and the compound (11-4, 27.14 mg) obtained in <Step 2> of (Example 11), thereby obtaining a title compound EX11b-B2 (648 mg) as a white solid.

(Example 12)

Synthesis of 2-(2-(cyclooct-2-yn-1-yloxy)ethoxy)ethane-1-amino group-introduced alginic acid (EX12-A2):

[C130]

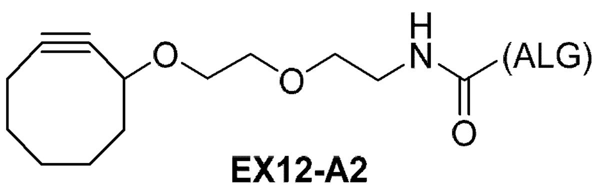

EX12-A2

<Step 1>

Synthesis of 2,2,2-trifluoro-N-(2-(2-hydroxyethoxy)ethyl)acetamide (12-2):

[C131]

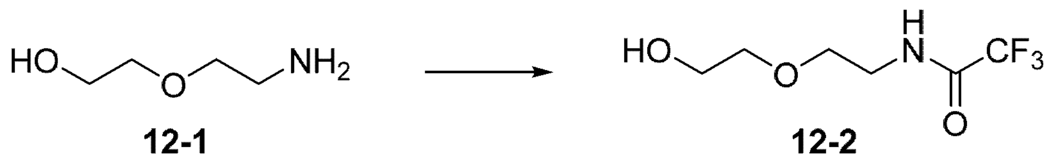

2,2,2-Ethyl trifluoroacetate (2.5 mL) was added dropwise to a tetrahydrofuran (8.0 mL) solution of commercially available 2-(2-aminoethoxy) ethanol [CAS REGISTRY NO.: 929-06-6] (12-1, 2.0 mL) for five minutes and then stirred at room temperature for 20 hours. A reaction liquid was concentrated under reduced pressure, and then ethyl acetate (30 mL) and water (10 mL) were added thereto, and a liquid was separated. A water layer was extracted with ethyl acetate (10 mL), and a matching organic layer was sequentially washed with water and brine. The organic layer was dried with anhydrous sodium sulfate and then concentrated under reduced pressure, thereby obtaining a title compound 12-2 (3.7 g) as a colorless oily substance.

<Step 2>

Synthesis of (E)-N-(2-(2-((2-bromocyclooct-2-en-1-yl)oxy)ethoxy)ethyl)-2,2,2-trifluoroacetamide (12-3):

[C132]

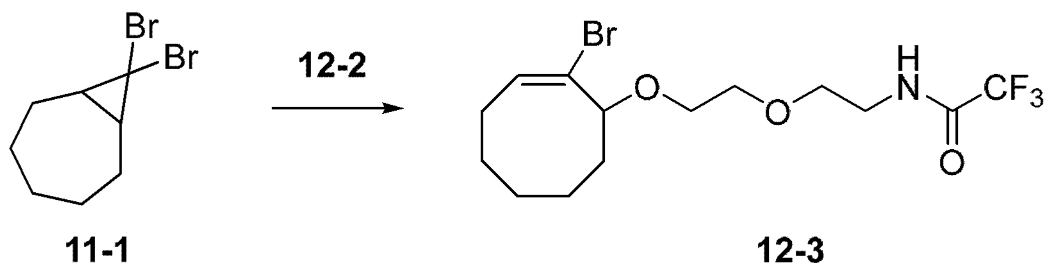


A dibromo form (11-1, 0.30 g) synthesized according to a method well known by a publication (Org. Process Res. Dev. (2018) 22: 108 to 110) was dissolved in methylene chloride (0.54 mL), and the compound (12-2, 1.86 g) obtained in <Step 1> of (Example 12) and silver trifluoromethanesulfonate (0.52 g) were added thereto while light was shielded with an aluminum foil. The components were stirred at room temperature for 1.5 hours while light was shielded, and saturated aqueous sodium bicarbonate solution (2.0 mL) and brine (3.0 mL) were sequentially added to a reaction liquid under ice water cooling. A solid was removed with Celite and washed with tert-butyl methyl ether (10 mL × 3). A filtrate was separated, and an organic layer was sequentially washed with water and brine. The organic layer was dried with anhydrous sodium sulfate and then concentrated under reduced pressure, thereby obtaining a title compound 12-3 (424 mg) as a thin brown oily substance.

<Step 3>

Synthesis of N-(2-(2-(cyclooct-2-yn-1-yloxy)ethoxy)ethyl)-2,2,2-trifluoroacetamide (12-4):

[C133]

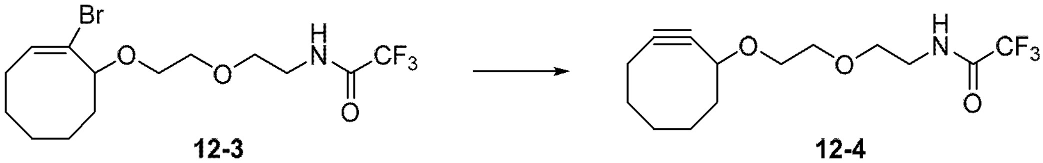

12-3 12-4

The compound (12-3, 100 mg) obtained in <Step 2> of (Example 12) was dissolved in tetrahydrofuran (0.7 mL) and N,N-dimethylformaldehyde (0.7 mL). 60% sodium hydroxide (21 mg) was added thereto under ice water cooling and stirred at the same temperature for three hours. 60% sodium hydroxide (10 mg) was added thereto, 60% sodium hydroxide (10 mg) was further added thereto at room temperature for one hour and stirred at room temperature for 20 hours. Water (3 mL) were added thereto, and an organic layer was extracted with ethyl acetate (15 mL, 10 mL) and then sequentially washed with water and brine. The organic layer was dried with anhydrous sodium sulfate and then concentrated under reduced pressure. A residue was purified by column chromatography (n-heptane → 50% ethyl acetate/n-heptane), thereby obtaining a title compound 12-4 (37 mg) as a colorless oily substance.

<Step 4>

Synthesis of 2-(2-(cyclooct-2-yn-1-yloxy)ethoxy)ethan-1-amine (12-5):

[C134]

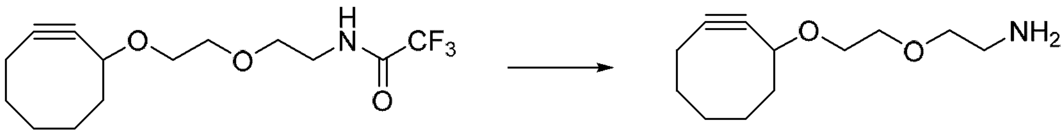

12-4 12-5

A water (185 μL) solution of potassium carbonate (50 mg) was added to a methanol (555 μL) solution of the compound (12-4, 37 mg) obtained in <Step 3> of (Example 12) and stirred at room temperature for 17 hours. A reaction liquid was concentrated under reduced pressure, then, water (1 mL) was added thereto, and the reaction liquid was saturated with sodium chloride. A layer was extracted with ethyl acetate (10 mL × 4), and the extracted layer was dried with anhydrous sodium sulfate and then concentrated under reduced pressure. Ethyl acetate (10 mL) and several droplets of methanol were added to a residue, and an insoluble matter was removed. An obtained filtrate was concentrated under reduced pressure, thereby obtaining a title compound 12-5 (30 mg) as a colorless oily substance.

<Step 5>

Synthesis of 2-(2-(cyclooct-2-yn-1-yloxy)ethoxy)ethane-1-amino group-introduced alginic acid (EX12-A2):

[C135]

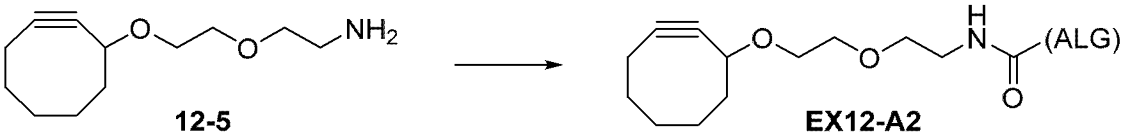

12-5 EX12-A2

4-(4,6-Dimethoxy-1,3,5-triazin-2-yl)-4-methylmorpholinium chloride (DMT-MM) (145 mg), an ethanol (5.2 mL) solution of the compound (12-5, 29 mg) obtained in <Step 4> of (Example 12) and 1-molar aqueous sodium bicarbonate solution (131 μL) were added to a sodium alginate (manufactured by MOCHIDA PHARMACEUTICAL CO., LTD.: A-2)

aqueous solution (52 mL) prepared to 1 wt%. The components were stirred at 30°C for 3.2 hours, and then sodium chloride (0.52 g) and ethanol (104 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX12-A2 (522 mg) as a white solid.

(Example 13)

Synthesis of 3-amino-N-(2-(2-(2-(cyclooct-2-yn-1-yloxy)acetamido)ethoxy)ethyl)propanamide group-introduced alginic acid (EX13-A2):

[C136]

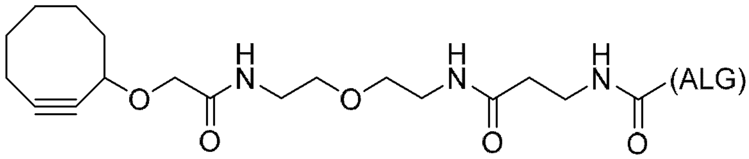

**EX13-A2**

<Step 1>

Synthesis of 3-(2,2,2-trifluoroacetamido)propanoic acid (13-2):

[C137]

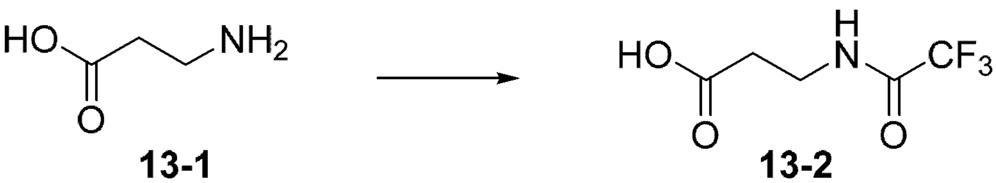

**13-1** **13-2**

Commercially available β-alanine [CAS REGISTRY NO.: 107-95-9] (13-1, 2.0 g) was dissolved in methanol (40.0 mL), and triethylamine (3.3 mL) was added thereto. Ethyl 2,2,2-trifluoroacetate (3.4 mL) was added dropwise for five minutes under water cooling and then stirred at room temperature for 20.5 hours. A reaction liquid was concentrated under reduced pressure, water (20 mL) was added thereto, and the pH was adjusted to four with 1N-hydrochloric acid. An organic layer was extracted with ethyl acetate (100 mL × 2, 50 mL) and washed with brine. The organic layer was dried with anhydrous sodium sulfate and then concentrated under reduced pressure, thereby obtaining a title compound 13-2 (2.9 g) as a white solid.

<Step 2>

Synthesis of tert-butyl (2-(2-(3-(2,2,2-trifluoroacetamido)propanamido)ethoxy)ethyl)carbamate (13-3):

[C138]

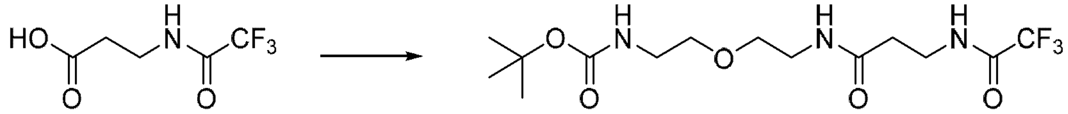

13-2

13-3

4-(4,6-Dimethoxy-1,3,5-triazin-2-yl)-4-methylmorpholinium chloride (DMT-MM) (897 mg) was added to an ethanol (4.0 mL) solution of the compound (13-2, 400 mg) obtained in <Step 1> of (Example 13) and tert-butyl (2-(2-aminoethoxy)ethyl)carbamate (8-1, 441 mg, [CAS REGISTRY NO.: 127828-22-2]) and stirred for 3.5 hours. Water (5 mL) were added to a reaction liquid, and an organic layer was extracted with ethyl acetate (20 mL, 10 mL) and then sequentially washed with water and brine. The organic layer was dried with anhydrous sodium sulfate and then concentrated under reduced pressure, and a residue was purified by column chromatography (30% ethyl acetate/n-heptane → ethyl acetate), thereby obtaining a title compound 13-3 (451 mg) as a colorless oily substance.

<Step 3>

Synthesis of N-(2-(2-aminoethoxy)ethyl)-3-(2,2,2-trifluoroacetamide)propanamide hydrochloride (13-4):

[C139]

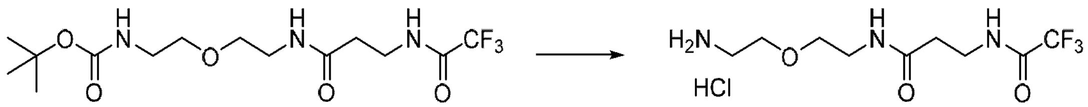

13-3

13-4

4N-hydrogen chloride/1,4-dioxane (3.16 mL) was added to the compound (13-3, 451 mg) obtained in <Step 2> of (Example 13) under ice water cooling and stirred at room temperature for three hours. Diisopropyl ether (6.4 mL) was added to a reaction liquid and concentrated under reduced pressure, thereby obtaining a title compound 13-4 (433 mg) as a colorless gummy substance.

<Step 4>

Synthesis of N-(2-(2-(2-(cyclooct-2-yn-1-yloxy)acetamido)ethoxy)ethyl-3-(2,2,2-trifluoroacetamido)propanamide (13-5):

[C140]

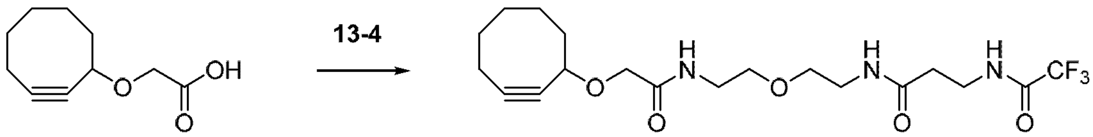


7-4

13-5

Ethanol (1.7 mL), 4-(4,6-dimethoxy-1,3,5-triazin-2-yl)-4-methylmorpholinium chloride (DMT-MM) (253 mg) and triethylamine (102 μL) were added to carboxylic acid (7-4, 111 mg) synthesized according to a method well known by a publication (Org. Process Res. Dev. (2018) 22: 108 to 110) and the compound (13-4, 215 mg) obtained in <Step 3> of (Example 13) and stirred at room temperature for 21 hours. Water (5 mL) was added to a reaction liquid, and an organic layer was extracted with ethyl acetate (15 mL). The organic layer was sequentially washed with water and brine, dried with anhydrous sodium sulfate and then concentrated under reduced pressure. An obtained residue was purified by column chromatography (30% ethyl acetate/n-heptane → 15% methanol/ethyl acetate), thereby obtaining a title

compound 13-5 (35 mg) as a colorless oily substance.

<Step 5>

Synthesis of 3-amino-N-(2-(2-(2-(cyclooct-2-yn-1-yloxy)acetamido)ethoxy)ethyl)propanamide (13-6):

[C141]

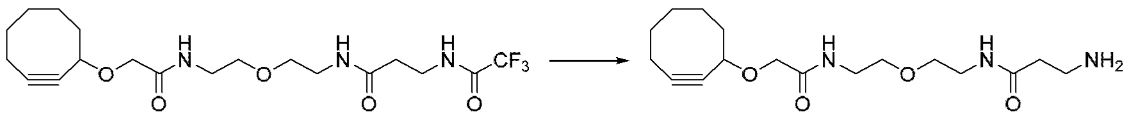

13-5 13-6

A water (175 μL) solution of potassium carbonate (33 mg) was added to a methanol (700 μL) solution of the compound (13-5, 35 mg) obtained in <Step 4> of (Example 13) and stirred at room temperature for 16.5 hours. A reaction liquid was concentrated under reduced pressure, then, water (2 mL) was added thereto, and the reaction liquid was saturated with sodium chloride. A layer was extracted with ethyl acetate (10 mL × 5), and the extracted layer was dried with anhydrous sodium sulfate and then concentrated under reduced pressure. Ethyl acetate (10 mL) and several droplets of methanol were added to a residue, and an insoluble matter was removed. An obtained filtrate was concentrated under reduced pressure, thereby obtaining a title compound 13-6 (24 mg) as a colorless gummy substance.

<Step 6>

Synthesis of 3-amino-N-(2-(2-(2-(cyclooct-2-yn-1-yloxy)acetamido)ethoxy)ethyl)propanamide group-introduced alginic acid (EX13-A2):

[C142]

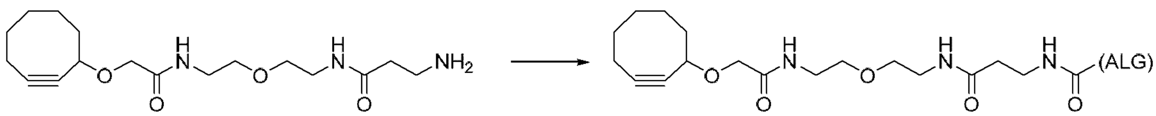

13-6 EX13-A2

4-(4,6-Dimethoxy-1,3,5-triazin-2-yl)-4-methylmorpholinium chloride (DMT-MM) (78 mg), an ethanol (2.8 mL) solution of the compound (13-6, 24 mg) obtained in <Step 5> of (Example 13) and 1-molar aqueous sodium bicarbonate solution (71 μL) were added to a sodium alginate (manufactured by MOCHIDA PHARMACEUTICAL CO., LTD.: A-2) aqueous solution (28 mL) prepared to 1 wt%. The components were stirred at 30°C for 3.5 hours, and then sodium chloride (0.28 g) and ethanol (56 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX13-A2 (272 mg) as a white solid.

(Example 14)

Syntheses of N-(4-(2-aminoethoxy)benzyl)-2-(cyclooct-2-yn-1-yloxy)acetamide group-introduced alginic acids (EX14a-A2 and EX14b-B2):

[C143]

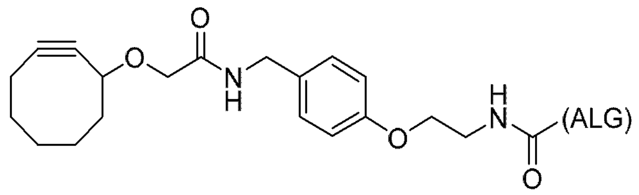

EX14a-A2
EX14b-B2

<Step 1>

Synthesis of N-(2-bromoethyl)-2,2,2-trifluoroacetamide (14-2):

[C144]

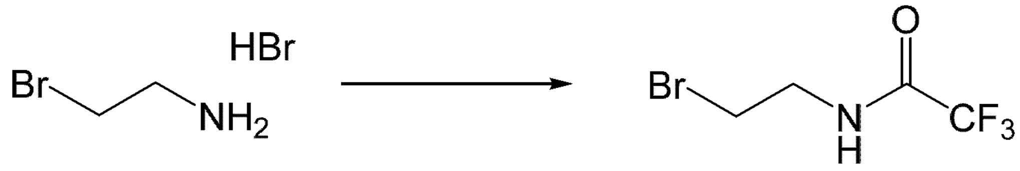

**14-1** **14-2**

Triethylamine (4.29 mL) was added to a methanol (30 mL) solution of 2-bromoethylamine hydrobromide [CAS REGISTRY NO.: 2576-47-8] (14-1, 3 g) under ice cooling and stirring. Ethyl trifluoroacetate (1.92 mL) was slowly added to this mixture at the same temperature and stirred at room temperature for 42 hours. After the end of a reaction, a reaction liquid was concentrated under reduced pressure, and water (10 mL) was added thereto. An organic layer was extracted with ethyl acetate (10 mL) three times, sequentially washed with water (5 mL) and brine (5 mL), dried with anhydrous sodium sulfate, filtered and then concentrated under reduced pressure, thereby obtaining a title compound 14-2 (2.457 g) as a light brown solid.

<Step 2>

Synthesis of tert-butyl (4-(2-(2,2,2-trifluoroacetamido)ethoxy)benzyl)carbamate (14-4):

[C145]

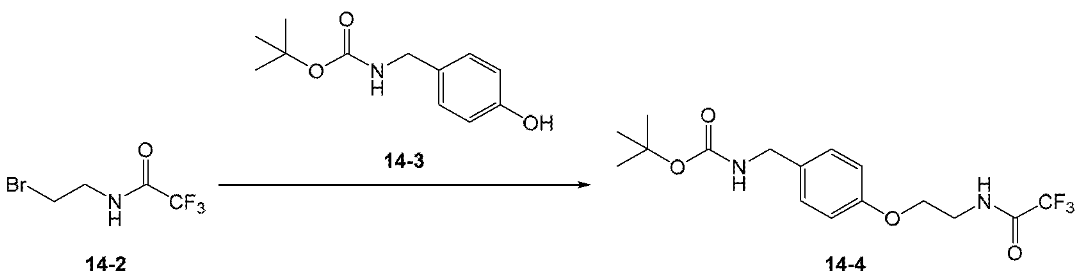

14-3

14-2 14-4

Potassium carbonate (0.45 g) was added to a mixture of commercially available tert-butyl (4-hydroxybenzyl)carbamate [CAS REGISTRY NO.: 149505-94-2] (14-3, 0.36 g), the compound (14-2, 0.46 g) obtained in <Step 1> of (Example 14), potassium iodide (0.35 g) and N-methylpyrrolidone (3.6 mL) at room temperature and stirred at 140°C for five hours. After the end of a reaction, a reaction product was cooled to room temperature and diluted with water (10 mL). An organic layer was extracted three times with methyl tert-butyl ether (10 mL), sequentially washed with a 1 N-sodium hydroxide aqueous solution (5 mL) twice, water (5 mL) and brine (5 mL) and dried with anhydrous sodium sulfate. The organic layer was filtered and then concentrated under reduced pressure, thereby obtaining a crude product. The obtained crude product was purified by silica gel column chromatography (n-heptane/ethyl acetate), and a title compound 14-4 (0.202 g) was obtained as white amorphous.

<Step 3>

Synthesis of N-(2-(4-(aminomethyl)phenoxy)ethyl)-2,2,2-trifluoroacetamide hydrochloride (14-5):

[C146]

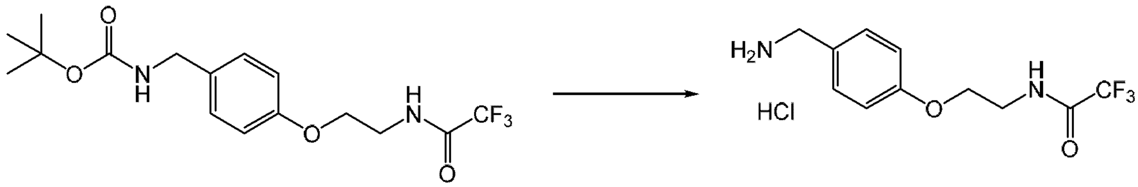

14-4

14-5

The compound (14-4, 0.2 g) obtained in <Step 2> of (Example 14) was used, and the same operation as in <Step 2> of (Example 6) was performed, thereby obtaining a title compound 14-5 (0.147 g) as a white solid.

<Step 4>

Synthesis of N-(2-(4-((2-(cyclooct-2-yn-1-yloxy)acetamido)methyl)phenoxy)ethyl)-2,2,2-trifluoroacetamide (14-6):

[C147]

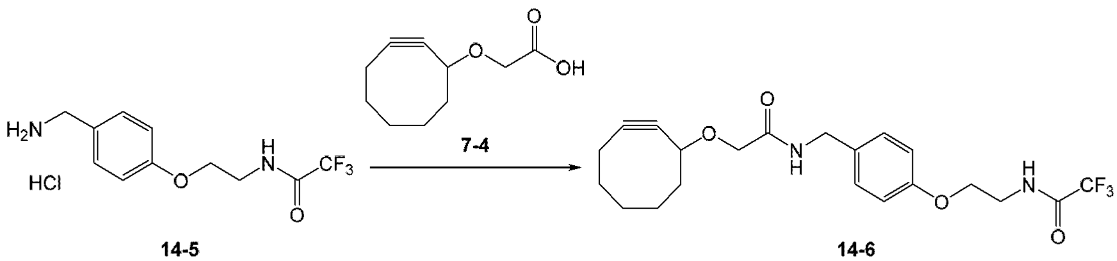

7-4

14-5

14-6

4-(4,6-Dimethoxy-1,3,5-triazin-2-yl)-4-methylmorpholinium chloride (DMT-MM) (137.22 mg) and triethylamine (38.25 μL) were added to a mixture of carboxylic acid (7-4, 50 mg) synthesized according to a method well known by a publication (Org. Process Res. Dev. (2018) 22: 108 to 110) and the compound (14-5, 81.96 mg) obtained in <Step 3> of (Example 14) and ethanol under ice cooling and stirring and stirred at room temperature for one hour 30 minutes. After the end of a reaction, water (2 mL) was added thereto, a suspension was stirred, and methyl tert-butyl ether (0.5 mL) was added thereto. A separated water layer was extracted with methyl tert-butyl ether (5 mL) twice, sequentially washed with water (5 mL) and brine (5 mL) and dried with anhydrous sodium sulfate. A dried organic layer was filtered and concentrated under reduced pressure. A crude product was purified by silica gel column chromatography (n-heptane/ethyl acetate), and a title compound 14-6 (99 mg) was obtained as white amorphous.

<Step 5>

Synthesis of N-(4-(2-aminoethoxy)benzyl)-2-(cyclooct-2-yn-1-yloxy)acetamide (14-7):

[C148]

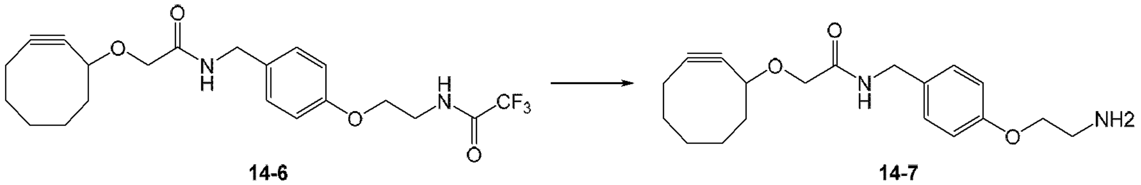

14-6 14-7

Potassium carbonate (64.17 mg) and water (495 μL) were added to a mixture of the compound (14-6, 99 mg) obtained in <Step 4> of (Example 14) and methanol (1485 μL) under water cooling and stirring and stirred at room temperature for 15 hours. After the end of a reaction, methanol was concentrated under reduced pressure, and a generated water layer was extracted with ethyl acetate (5 mL) three times. An organic layer was sequentially washed with water (5 mL) and brine (5 mL) and dried with anhydrous sodium sulfate. The dried organic layer was filtered and then concentrated under reduced pressure, thereby obtaining a crude product of a title compound 14-7 (68 mg) as a yellow oily substance.

<Step 6-1>

Synthesis of N-(4-(2-aminoethoxy)benzyl)-2-(cyclooct-2-yn-1-yloxy)acetamide group-introduced alginic acid (EX14a-A2):

[C149]

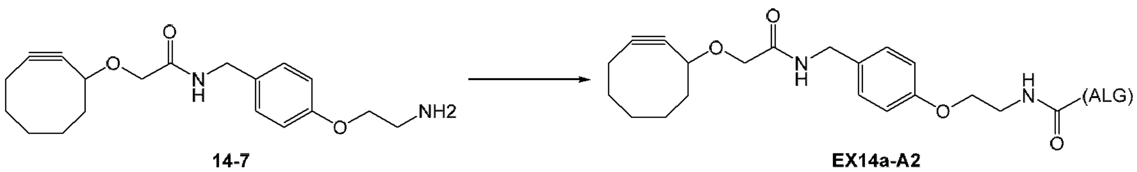

14-7 EX14a-A2

The same operation as <Step 3-1> of (Example 11) was performed using a sodium alginate (manufactured by MOCHIDA PHARMACEUTICAL CO., LTD.: A-2) aqueous solution (49.44 mL) prepared to 1 wt%, 4-(4,6-dimethoxy-1,3,5-triazin-2-yl)-4-methylmorpholinium chloride (DMT-MM) (152.54 mg) and the compound (14-7, 37.79 mg) obtained in <Step 5> of (Example 14), thereby obtaining a title compound EX14a-A2 (479 mg) as a white solid.

<Step 6-2>

Synthesis of N-(4-(2-aminoethoxy)benzyl)-2-(cyclooct-2-yn-1-yloxy)acetamide group-introduced alginic acid (EX14b-B2):

[C150]

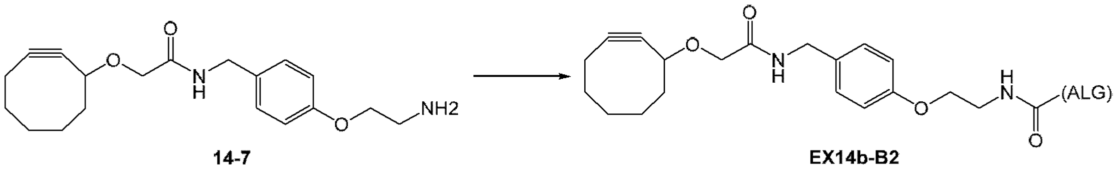

14-7 EX14b-B2

The same operation as <Step 3-1> of (Example 11) was performed using a sodium alginate (manufactured by MOCHIDA PHARMACEUTICAL CO., LTD.: B-2) aqueous solution (40.08 mL) prepared to 1 wt%, 4-(4,6-dimethoxy-1,3,5-triazin-2-yl)-4-methylmorpholinium chloride (DMT-MM) (123.66 mg) and the compound (14-7, 30.64 mg) obtained

in <Step 5> of (Example 14), thereby obtaining a title compound EX14b-B2 (356 mg) as a white solid.

(Example 15)

Synthesis of 2-amino-N-[3-(11,12-didehydrodibenz[b,f]azocin-5(6H)-yl)-3-oxopropyl]acetamide group-introduced alginic acid (EX15-A2):

[C151]

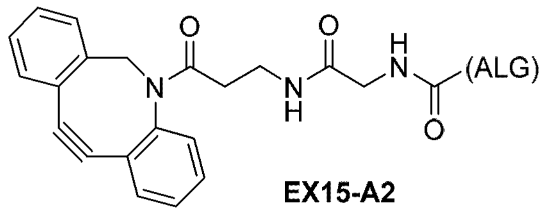

EX15-A2

<Step 1>

Synthesis of (9H-fluoren-9-yl)methyl-N-[3-(11,12-didehydrodibenz[b,f]azocin-5(6H)-yl)-3-oxopropyl]acetamido-2-carbamate group (15-2):

[C152]

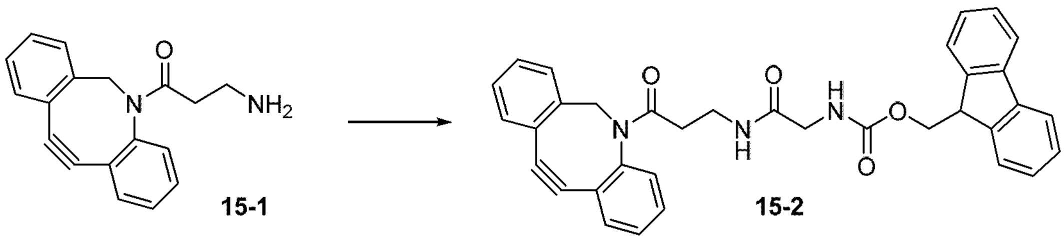

15-1 15-2

Commercially available 3-amino-1-(11,12-didehydrodibenz[b,f]azocin-5(6H)-yl)-1-propanone [CAS REGISTRY NO.: 1255942-06-3] (15-1, 50 mg) and N-[(9H-fluoren-9-ylmethoxy)carbonyl]glycine [CAS REGISTRY NO.: 29022-11-5] (54 mg) were dissolved in acetonitrile (1.5 mL). O-(7-azabenzotriazol-1-yl)-N,N,N',N'-tetramethyluronium hexafluorophosphate (76 mg) and N,N-diisopropylethylamine (70 μL) were added thereto and stirred at room temperature for 4.5 hours. Ethyl acetate (15 mL) and water (5 mL) were added to a reaction liquid, liquid was separated, and then an organic layer was sequentially washed with water and brine. The organic layer was dried with anhydrous sodium sulfate, then, concentrated under reduced pressure and purified by column chromatography, thereby obtaining a title compound 15-2 (63 mg) as thin beige amorphous.

<Step 2>

Synthesis of 2-amino-N-[3-(11,12-didehydrodibenz[b,f]azocin-5(6H)-yl)-3-oxopropyl]acetamide (15-3):

[C153]

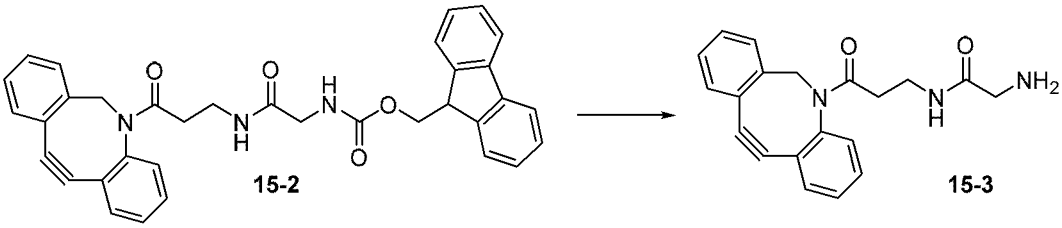

15-2

15-3

An N,N-dimethylformamide (315 μL) solution of piperidine (56 μL) was added to the compound (15-2, 63 mg) obtained in <Step 1> of (Example 15), and sales were expanded at room temperature for 30 minutes. Ethyl acetate (15 mL) and water (5 mL) were added to a reaction liquid, liquid was separated, and then an organic layer was sequentially washed with water and brine. The organic layer was dried with anhydrous sodium sulfate and then concentrated under reduced pressure. tert-Butyl methyl ether (5 mL) was added to the obtained solid, triturated and then filtered, thereby obtaining a title compound 15-3 (10 mg) as thin beige solid. The title compound 15-3 (11 mg) was additionally recovered from the filtrate and obtained as a light yellow gummy substance.

<Step 3>

Synthesis of 2-amino-N-[3-(11,12-didehydrodibenz[b,f]azocin-5(6H)-yl)-3-oxopropyl]acetamide group-introduced alginic acid (EX15-A2):

[C154]

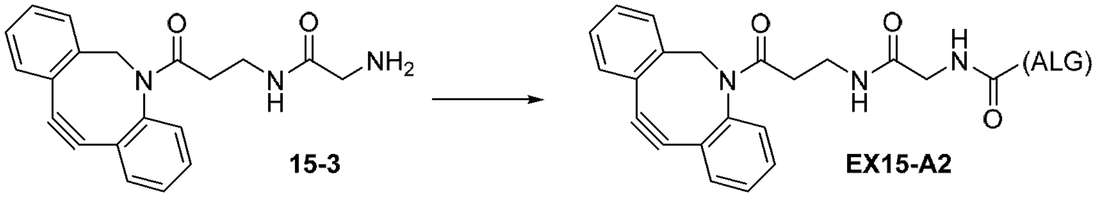

15-3

EX15-A2

4-(4,6-Dimethoxy-1,3,5-triazin-2-yl)-4-methylmorpholinium chloride (DMT-MM) (106 mg), an ethanol (1.9 mL) solution of the compound (15-3, 21 mg) obtained in <Step 2> of (Example 15) and 1-molar aqueous sodium bicarbonate solution (48 μL) were added to a sodium alginate (manufactured by MOCHIDA PHARMACEUTICAL CO., LTD.: A-2) aqueous solution (19 mL) prepared to 1 wt%. The components were stirred at 30°C for three hours, and then sodium chloride (0.19 g) and ethanol (38 mL) were sequentially added thereto and stirred at room temperature for 30 minutes. An obtained precipitation was filtered, washed with ethanol and dried under reduced pressure. An obtained solid was dissolved in water, then, lyophilized, thereby obtaining a title compound EX15-A2 (188 mg) as a white solid.

(Example 16)

Synthesis of 2-amino-N-(2-(cyclooct-2-yn-1-yloxy)ethyl)acetamide group-introduced alginic acid (EX16-A2):

[C155]

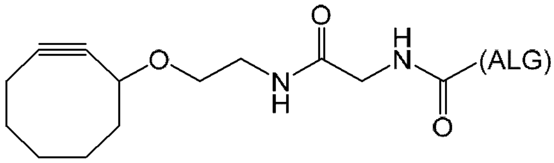

**EX16-A2**

<Step 1>

Synthesis of (2,2,2-trifluoroacetyl)glycine (16-2):

[C156]

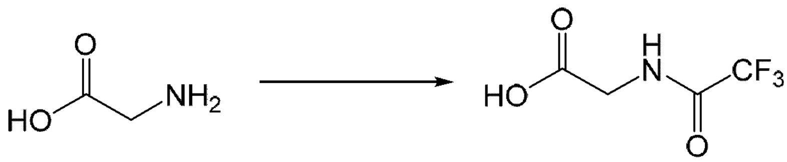

**16-1** **16-2**

Glycine (16-1, 2 g) was suspended in methanol (10 mL) and cooled to 4°C. Ethyl trifluoroacetate (3.5 mL) and triethylamine (3.71 mL) were added thereto at the same temperature and stirred at room temperature for 23 hours. After the end of a reaction, 1N-hydrochloric acid (20 mL) was slowly added thereto until the pH reached two, and a reaction product was extracted with ethyl acetate (10 mL) three times and sequentially washed with water (5 mL) and brine (5 mL). An organic layer was dried with anhydrous sodium sulfate, filtered and then concentrated under reduced pressure, thereby obtaining a light yellow oily substance. The obtained oily substance was dissolved in ethyl acetate (20 mL), and n-heptane (10 mL) was added thereto. This solution was concentrated under reduced pressure, thereby obtaining a title compound 16-2 (3.22 g) as white amorphous.

<Step 2>

Synthesis of N-(2-((2-(cyclooct-2-yn-1-yloxy)ethyl)amino)-2-oxoethyl)-2,2,2-trifluoroacetamide (16-3):

[C157]

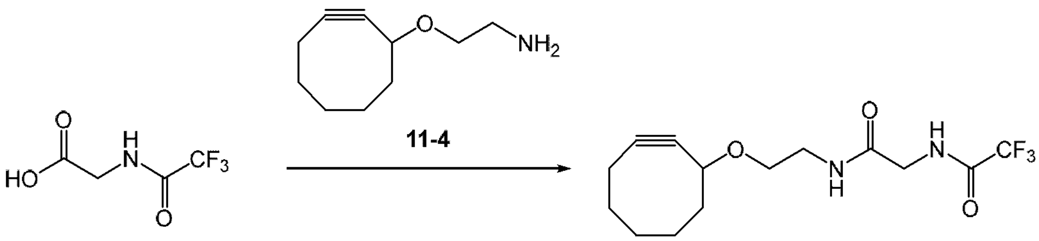


**16-2** **16-3**

Ethanol (1600 μL) and 4-(4,6-dimethoxy-1,3,5-triazin-2-yl)-4-methylmorpholinium chloride (DMT-MM) (239.21 mg) were added to a mixture of the compound 11-4 (80 mg) and the compound (16-2, 81.83 mg) obtained in <Step 1>

of (Example 16) under ice cooling and stirring and stirred at room temperature for three hours. Water (2 mL) was added thereto to stop a reaction, and an organic layer was extracted with methyl tert-butyl ether (5 mL) three times. The organic layer was sequentially washed with water (5 mL) and brine (5 mL) and dried with anhydrous sodium sulfate. The organic layer was filtered and then concentrated under reduced pressure, thereby obtaining a crude product. This crude product was triturated with n-heptane (10 mL), filtered and dried under reduced pressure, thereby obtaining a title compound 16-3 (95.1 mg) as a white solid.

<Step 3>

Synthesis of 2-amino-N-(2-(cyclooct-2-yn-1-yloxy)ethyl)acetamide (16-4):

[C158]

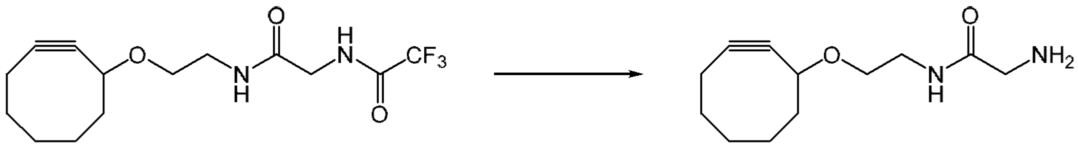

16-3 16-4

The same operation as in <Step 5> of (Example 14) was performed using the compound (16-3, 60 mg) obtained in <Step 2> of (Example 16), methanol (900 μL), potassium carbonate (51.78 mg) and water (300 μL), thereby obtaining a title compound 16-4 (15 mg) as a light yellow oily substance.

<Step 4>

Synthesis of 2-amino-N-(2-(cyclooct-2-yn-1-yloxy)ethyl)acetamide group-introduced alginic acid (EX16-A2):

[C159]

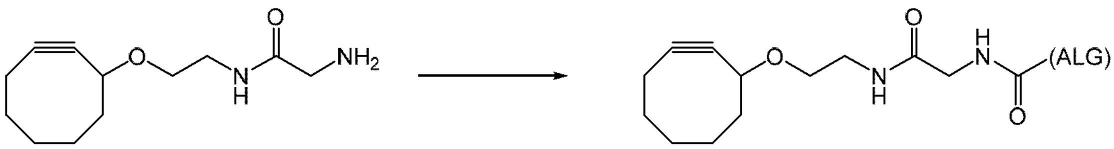

16-4 EX16-A2

The same operation as <Step 3-1> of (Example 11) was performed using a sodium alginate (manufactured by MOCHIDA PHARMACEUTICAL CO., LTD.: A-2) aqueous solution (29.66 mL) prepared to 1 wt%, 4-(4,6-Dimethoxy-1,3,5-triazin-2-yl)-4-methylmorpholinium chloride (DMT-MM) (91.52 mg) and the compound (16-4, 15 mg) obtained in <Step 3> of (Example 16), thereby obtaining a title compound EX16-A2 (279 mg) as a white solid.

(Example 17)

Synthesis of (2S)-2-amino-N-(2-(cyclooct-2-yn-1-yloxy)ethyl)-3-phenylpropanamide group-introduced alginic acid (EX17-A2):

[C160]

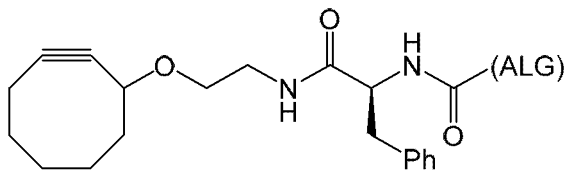

**EX17-A2**

<Step 1>

Synthesis of (2,2,2-trifluoroacetyl)-L-phenylalanine (17-2):

[C161]

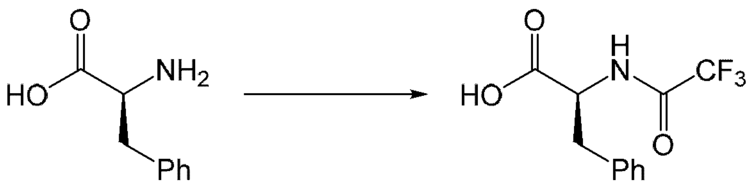

**17-1** **17-2**

L-phenylalanine [CAS REGISTRY NO.: 63-91-2] (17-1, 2 g) was dissolved in methanol (10 mL) and cooled to 4°C. Subsequently, ethyl trifluoroacetate (1.59 mL) and triethylamine (1.69 mL) were added thereto at the same temperature and stirred at room temperature for 16 hours. After the end of a reaction, 1N-hydrochloric acid (10 mL) was slowly added thereto until the pH reached one, and a suspension was stirred for 30 minutes. The suspension was filtered, and a recovered solid was dried under reduced pressure, thereby obtaining a title compound 17-2 (2.53 g) as a white solid.

<Step 2>

Synthesis of (2S)-N-(2-(cyclooct-2-yn-1-yloxy)ethyl)-3-phenyl-2-(2,2,2-trifluoroacetamide)propanamide (17-3):

[C162]

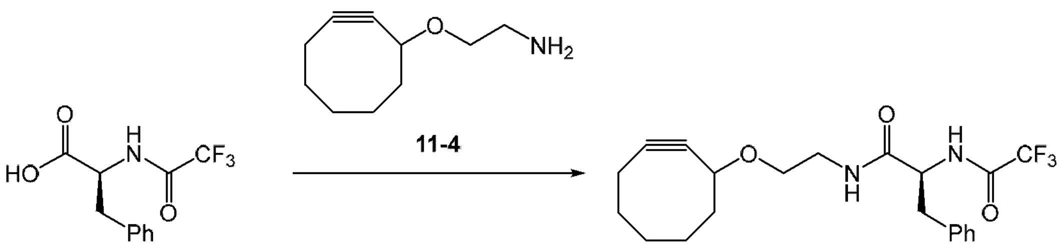

**11-4**

**17-2** **17-3**

Ethanol (1200 μL) and 4-(4,6-dimethoxy-1,3,5-triazin-2-yl)-4-methylmorpholinium chloride (DMT-MM) (179.41 mg) were added to a mixture of the compound 11-4 (60 mg) and the compound (17-2, 93.7 mg) obtained in <Step 1> of (Example 17) under ice cooling and stirring and stirred at room temperature for three hours. Water (2 mL) was added thereto to stop a reaction, and an organic layer was extracted with methyl tert-butyl ether (5 mL) three times. The organic layer was sequentially washed with water (5 mL) and brine (5 mL) and dried with anhydrous sodium sulfate. The organic

layer was filtered and then concentrated under reduced pressure, thereby obtaining a crude product. A crude product was purified by silica gel column chromatography (n-heptane/ethyl acetate), and a title compound 17-3 (57 mg) was obtained as white amorphous.

<Step 3>

Synthesis of (2S)-2-amino-N-(2-(cyclooct-2-yn-1-yloxy)ethyl)-3-phenylpropanamide (17-4):

[C163]

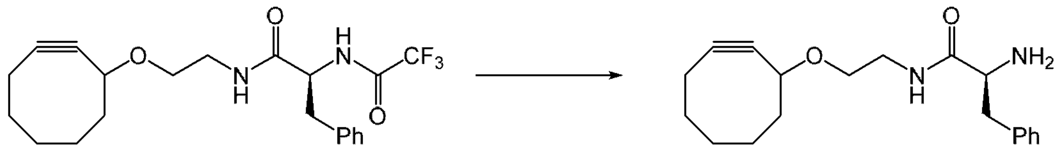

17-3 17-4

The same operation as in <Step 5> of (Example 14) was performed using the compound (17-3, 57 mg) obtained in <Step 2> of (Example 17), methanol (855 μL), potassium carbonate (38.39 mg) and water (285 μL), thereby obtaining a title compound 17-4 (35 mg) as a light yellow oily substance.

<Step 4>

Synthesis of (2S)-2-amino-N-(2-(cyclooct-2-yn-1-yloxy)ethyl)-3-phenylpropanamide group-introduced alginic acid (EX17-A2):

[C164]

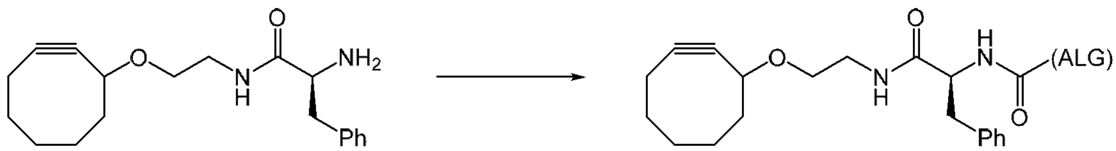

17-4 EX17-A2

The same operation as <Step 3-1> of (Example 11) was performed using a sodium alginate (manufactured by MOCHIDA PHARMACEUTICAL CO., LTD.: A-2) aqueous solution (47.46 mL) prepared to 1 wt%, 4-(4,6-dimethoxy-1,3,5-triazin-2-yl)-4-methylmorpholinium chloride (DMT-MM) (146.44 mg) and the compound (17-4, 34.53 mg) obtained in <Step 3> of (Example 17), thereby obtaining a title compound EX17-A2 (383 mg) as a white solid.

(Example 18)

Synthesis of 4-(2-aminoethoxy)-N-(3-azidopropyl)benzamide group-introduced alginic acid (EX18-A2):

[C165]

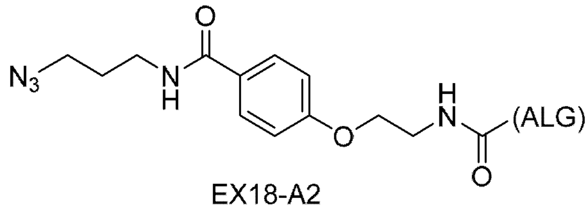

EX18-A2

<Step 1>

Synthesis of methyl 4-(2-((tert-butoxycarbonyl)amino)ethoxy)benzoate (18-2):

[C166]

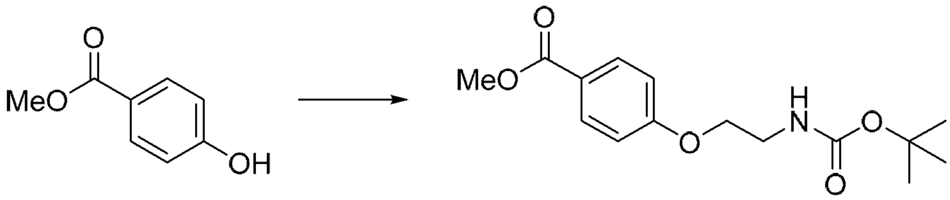

18-1 18-2

A diethyl azodicarboxylate (40% toluene solution, 1.92 mL) solution was added to a tetrahydrofuran (2.59 mL) solution of triphenylphosphine (0.96 g) under ice cooling and stirring and stirred at room temperature for 20 minutes. A tetrahydrofuran (1.1 mL) solution of commercially available 4-hydroxybenzoate (compound 18-1, 0.37 g) and 2-(tert-butoxycarbonyl)ethanolamine (0.39 g) was added to this solution under ice cooling and stirring and stirred at room temperature for 17 hours. A reaction liquid was concentrated under reduced pressure, a residue was purified by silica gel column chromatography (5% ethyl acetate/n-heptane to 40% ethyl acetate/n-heptane), thereby obtaining a mixture of a compound 18-1 and a compound 18-2. This mixture was dissolved in methyl tert-butyl ether (20 mL) and sequentially washed with a 1 N-sodium hydroxide aqueous solution (5 mL) twice and brine (5 mL). An organic layer was dried with anhydrous sodium sulfate, and then a solvent was distilled away under reduced pressure, thereby obtaining a title compound 18-2 (0.45 g) as a pink oily substance.

<Step 2>

Synthesis of 4-(2-aminoethoxy)-N-(3-azidopropyl)benzamide hydrochloride (compound 18-4):

[C167]

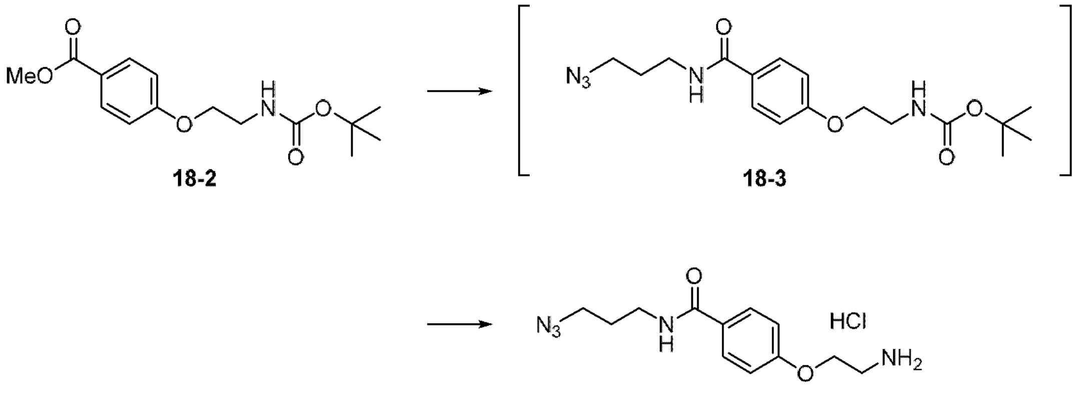

18-2 18-3

18-4

Lithium hydroxide monohydrate (0.25 g) was added to a methanol (4.4 mL) solution of the compound 18-2 (0.44 g) obtained in <Step 1> of (Example 18) and stirred at 60°C for three hours 30 minutes. 1N-hydrochloric acid (5 mL) was added to a reaction liquid, and an organic layer was extracted with ethyl acetate (10 mL) three times. The organic layer was sequentially washed with water (5 mL) and brine (5 mL) and dried with anhydrous sodium sulfate, and a solvent was distilled away under reduced pressure. A residue was dissolved in acetonitrile (4.4 mL), and 3-azidopropan-1-amine (0.15 g) and O-(7-azabenzotriazol-1-yl)-N,N,N',N'-tetramethyluronium hexafluorophosphate (0.57 g) were added thereto. Subsequently, N,N-diisopropylethylamine (0.52 mL) was added thereto under ice cooling and stirring and stirred at room temperature for five hours. Water (10 mL) was added to a reaction liquid, and an organic layer was extracted with ethyl acetate (15 mL) three times and dried with anhydrous sodium sulfate, and a solvent was distilled away under reduced pressure. The residue was purified by silica gel column chromatography (16% ethyl acetate/n-heptane to 100% ethyl acetate), thereby obtaining a fraction of a compound 18-3 (0.71 g).

4N-hydrogen chloride/1,4-dioxane (4.9 mL) was added to the fraction comprising the compound 18-3 (0.71 g) and stirred at room temperature for 20 minutes. Diisopropyl ether was added to the reaction liquid, and then a precipitate was filtered, thereby obtaining a title compound 18-4 (0.49 g) as a white solid.

<Step 3>

Synthesis of 4-(2-aminoethoxy)-N-(3-azidopropyl)benzamide group-introduced alginic acid (compound EX18-A2):

[C168]

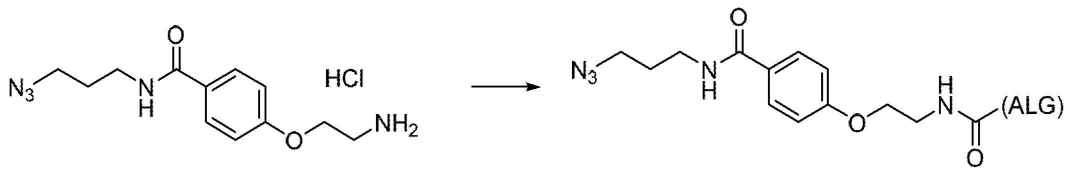

18-4 EX18-A2

The same operation as <Step 3-1> of (Example 11) was performed using a sodium alginate (manufactured by MOCHIDA PHARMACEUTICAL CO., LTD.: A-2) aqueous solution (19.6 mL) prepared to 1 wt%, 4-(4,6-Dimethoxy-1,3,5-triazin-2-yl)-4-methylmorpholinium chloride (DMT-MM) (50.19 mg), the compound 18-4 (54.37 mg) obtained in <Step 2> of (Example 18) and 1-molar aqueous sodium bicarbonate solution (181.4 µL) under ice cooling and stirring, thereby obtaining a title compound EX18-A2 (198 mg) as a white solid.

(Example 19)

Synthesis of 3-amino-1-(11,12-didehydrodibenz[b,f]azocin-5(6H)-yl)-1-propanone group-introduced alginic acid (EX19-A2):

[C169]

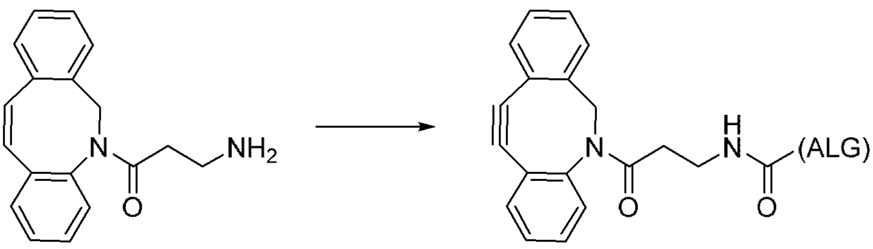

19-1 EX19-A2

The same operation as <Step 3-1> of (Example 11) was performed using a sodium alginate (manufactured by MOCHIDA PHARMACEUTICAL CO., LTD.: A-2) aqueous solution (43.6 mL) prepared to 1 wt%, 4-(4,6-dimethoxy-1,3,5-triazin-2-yl)-4-methylmorpholinium chloride (DMT-MM) (111.7 mg), 1-molar aqueous sodium bicarbonate solution (403.5 μL) and commercially available 3-amino-1-(11,12-didehydrodibenz[b,f]azocin-5(6H)-yl)-1-propanone [CAS REGISTRYNO.: 1255942-06-3] (19-1, 83.6 mg), thereby obtaining a title compound EX19-A2 (376 mg) as a light yellow solid.

(Example 20)

Syntheses of N-(4-(aminomethyl)benzyl)-2-(cyclooct-2-yn-1-yloxy)acetamido group-introduced alginic acids (EX20-B2 and EX20-A2):

[C170]

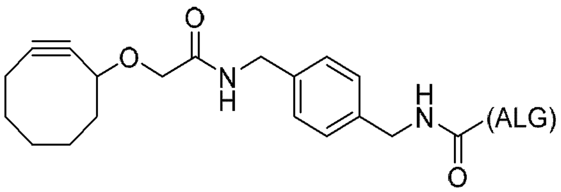

EX20-B2
EX20-A2

<Step 1>

Synthesis of tert-butyl (4-((2,2,2-trifluoroacetamido)methyl)benzyl)carbamate (compound 20-2):

[C171]

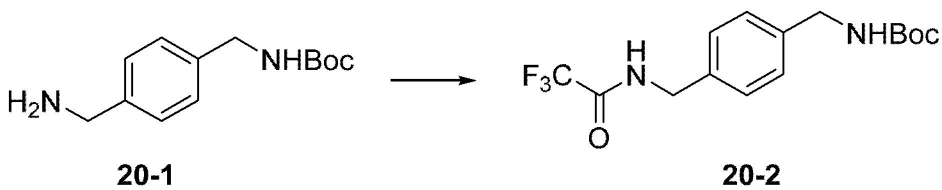

20-1 20-2

Ethyl trifluoroacetate (0.44 mL) was added dropwise to a mixture of tert-butyl (4-(aminomethyl)benzyl)carbamate (20-1, 0.67 g) synthesized with reference to a method well known by a publication (Bioorganic & Medicinal Chemistry (2003) 11: 4189-4206), triethylamine (0.39 mL) and methanol (6.67 mL) under ice cooling and stirring. A reaction mixture was heated to room temperature and stirred at the same temperature for five hours. A reaction was stopped with water (10 mL), and an organic layer was extracted with ethyl acetate (10 mL) three times. The recovered organic layer was washed with brine (5 mL) and dried with anhydrous sodium sulfate. The dried organic layer was filtered and then

concentrated, thereby obtaining a title crude compound 20-2 (0.67 g) as light yellow amorphous.

<Step 2>

Synthesis of N-(4-(aminomethyl)benzyl)-2,2,2-trifluoroacetamide hydrochloride (compound 20-3):

[C172]

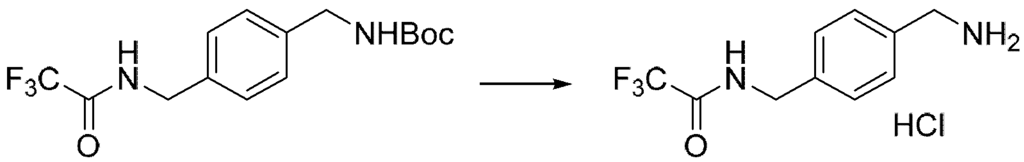

20-2 20-3

4N-Hydrogen chloride/1,4-dioxane (3.5 mL) was added to a 1,4-dioxane solution (3.5 mL) of the compound 20-2 (0.5 g) obtained in <Step 1> of (Example 20) under water cooling and stirring and stirred at room temperature for three hours. Diisopropyl ether (40 mL) was added to the reaction liquid, and then a precipitate was filtered, thereby obtaining a title compound 20-3 (0.4 g) as a white solid.

<Step 3>

Synthesis of N-(4-((2-(cyclooct-2-yn-1-yloxy)acetamido)methyl)benzyl)-2,2,2-trifluoroacetamide (compound 20-4):

[C173]

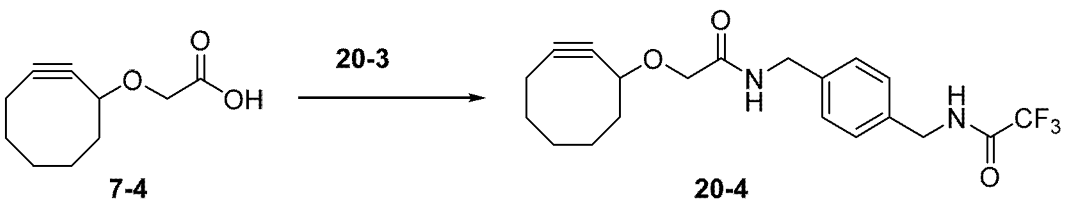


7-4 20-4

The compound 20-3 (0.26 g) obtained in <Step 2> of (Example 20) and N,N-diisopropylethylamine (0.51 mL) were added dropwise to an acetonitrile (1.7 mL) solution of carboxylic acid (7-4, 0.17 g) synthesized according to a method well known by a publication (Org. Process Res. Dev. (2018) 22: 108 to 110) and O-(7-azabenzotriazol-1-yl)-N,N,N',N'-tetramethyluronium hexafluorophosphate (0.26 g) and stirred at room temperature for one hour 30 minutes. Water (5 mL) was added thereto to stop a reaction, and an organic layer was extracted with ethyl acetate (5 mL) three times. The organic layer was washed with brine (3 mL) and then dried with anhydrous sodium sulfate. The dried organic layer was filtered, and a solvent was distilled away under reduced pressure. The residue was purified by silica gel column chromatography (12% ethyl acetate/n-heptane to 100% ethyl acetate), thereby obtaining a title compound 20-4 (0.19 g) as white amorphous.

<Step 4>

Synthesis of N-(4-(aminomethyl)benzyl)-2-(cyclooct-2-yn-1-yloxy)acetamide (compound 20-5):

[C174]

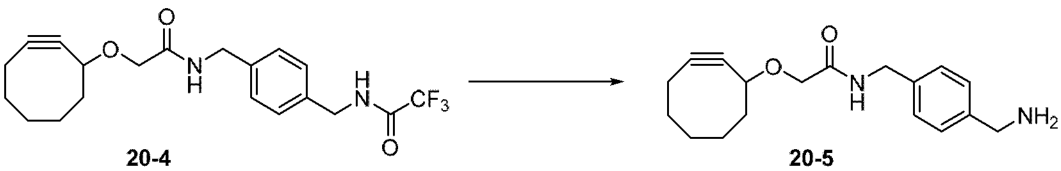

20-4

20-5

An aqueous solution (0.9 mL) of potassium carbonate (0.13 g) was added dropwise to a mixture of the compound 20-4 (0.18 g) obtained in <Step 3> of (Example 20) and methanol (1.8 mL) under ice cooling and stirring and stirred at room temperature for 17 hours 30 minutes. Methanol was distilled away under reduced pressure, and an organic layer was extracted with ethyl acetate (5 mL) three times. The organic layer was washed with brine (5 mL) and then dried with anhydrous sodium sulfate. The organic layer was filtered, and a solvent was distilled away under reduced pressure, thereby obtaining a title crude compound 20-5 (0.13 g) as a light yellow oily substance.

<Step 5>

Synthesis of N-(4-(aminomethyl)benzyl)-2-(cyclooct-2-yn-1-yloxy)acetamido group-introduced alginic acid (EX20-B2):

[C175]

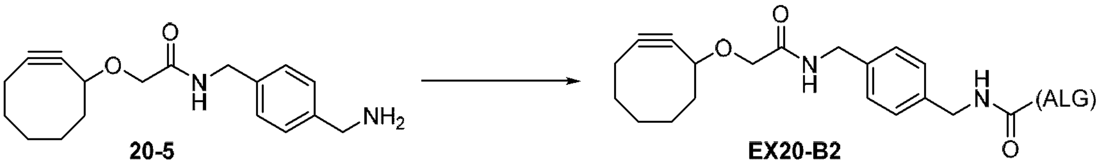

20-5

EX20-B2

The same operation as <Step 3-1> of (Example 11) was performed using a sodium alginate (manufactured by MOCHIDA PHARMACEUTICAL CO., LTD.: B-2) aqueous solution (50.9 mL) prepared to 1 wt%, 4-(4,6-dimethoxy-1,3,5-triazin-2-yl)-4-methylmorpholinium chloride (DMT-MM) (0.12 g) and an ethanol (3 mL) solution of the compound 20-5 (35 mg) obtained in <Step 4> of (Example 20), thereby obtaining a title compound EX20-B2 (521 mg) as a white solid.

<Step 5-2>

Synthesis of N-(4-(aminomethyl)benzyl)-2-(cyclooct-2-yn-1-yloxy)acetamido group-introduced alginic acid (EX20-A2):

[C176]

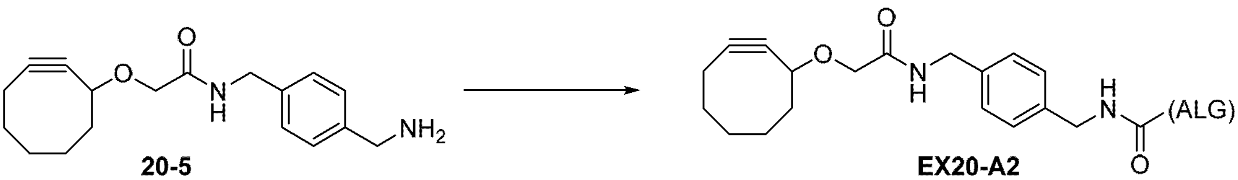

20-5

EX20-A2

The same operation as <Step 3-1> of (Example 11) was performed using a sodium alginate (manufactured by MOCHIDA PHARMACEUTICAL CO., LTD.: A-2) aqueous solution (250 mL) prepared to 2 wt%, 4-(4,6-Dimethoxy-1,3,5-triazin-2-yl)-4-methylmorpholinium chloride (DMT-MM) (279 mg), 1-molar aqueous sodium bicarbonate solution (252 μL) and an ethanol (25 mL) solution of the compound 20-5 (84 mg) obtained in <Step 4> of (Example 20), thereby obtaining a title compound EX20-A2 (4.57 g) as a white solid.

(Examples P1 to P7)

Alginic acid derivatives of (Example P1) to (Example P7) shown in the following table are manufactured according to the method described in the examples using corresponding amino compounds (which may be pharmaceutically acceptable salts thereof or solvates thereof) and alginic acid.

[Table 14]

| Example | Alginic acid derivative | Corresponding amino compound |
|---|---|---|
| P1 | 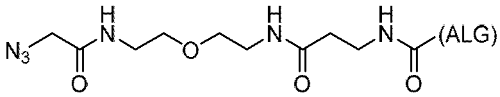 | 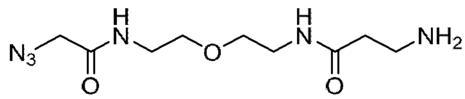 |
| P2 | 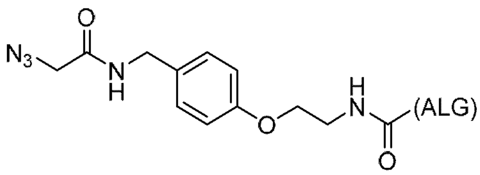 | 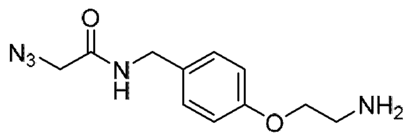 |
| P3 | 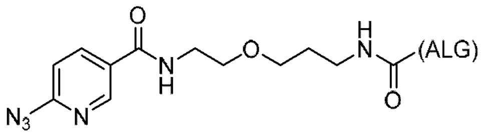 | 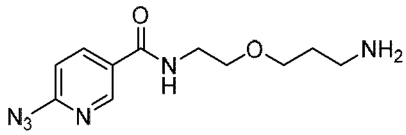 |
| P4 | 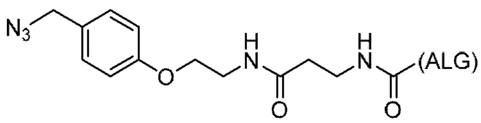 | 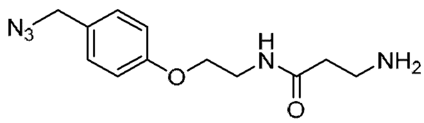 |
| P5 | 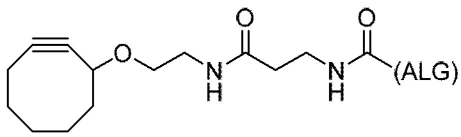 | 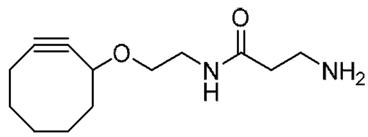 |
| P6 | 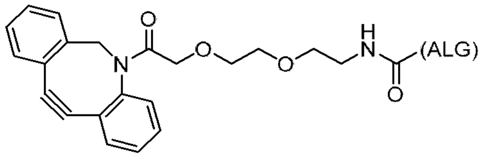 | 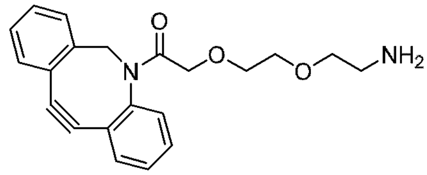 |
| P7 | 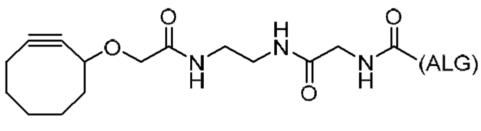 | 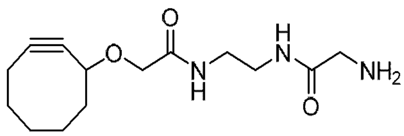 |

Physical property data of chemically modified alginic acid derivative

[Table 15]

| Compound | Measurement wavelength (nm) | Molecular weight (Da) | Weight-average molecular weight (Da) | Introduction rate of reactive group (mol%) (NMR integral ratio) |
|---|---|---|---|---|
| EX1-A2 | Differential refractometer | 14,000 to 2,660,000 | 1,370,000 | 3.2 |

(continued)

| Compound | Measurement wavelength (nm) | Molecular weight (Da) | Weight-average molecular weight (Da) | Introduction rate of reactive group (mol%) (NMR integral ratio) |
|---|---|---|---|---|
| EX2-A2 | Differential refractometer | 22,000 to 2,760,000 | 1,480,000 | 3.1 |
| EX3-A2 | Differential refractometer | 12,000 to 2,490,000 | 1,340,000 | 4.9 |
| EX4-A2 | Differential refractometer | 13,000 to 2,610,000 | 1,350,000 | 4.0 |
| EX5-A2 | 220 | 7,000 to 2,650,000 | 1,420,000 | 3.6 |
| EX6-A2 | 250 | 7,000 to 2,730,000 | 1,410,000 | 4.7 |
| EX7-B2 | Differential refractometer | 13,000 to 2,590,000 | 1,790,000 | 4.2 |
| EX8-A2 | Differential refractometer | 13,000 to 3,640,000 | 1,400,000 | 3.2 |
| EX9a-A2 | Differential refractometer | 13,000 to 2,660,000 | 1,370,000 | 5.0 |
| EX9b-B2 | Differential refractometer | 17,000 to 2,540,000 | 1,380,000 | 2.4 |
| EX10-A2 | Differential refractometer | 14,000 to 2,720,000 | 1,370,000 | 4.3 |
| EX11a-A2 | Differential refractometer | 15,000 to 2,530,000 | 1,350,000 | 3.4 |
| EX11b-B2 | Differential refractometer | 14,000 to 2,580,000 | 1,360,000 | 2.8 |
| EX12-A2 | Differential refractometer | 13,000 to 2,700,000 | 1,360,000 | 3.6 |
| EX13-A2 | Differential refractometer | 13,000 to 2,580,000 | 1,350,000 | 4.4 |
| EX14a-A2 | 220 | 8,000 to 2,650,000 | 1,420,000 | 4.6 |
| EX14b-B2 | 220 | 5,000 to 2,680,000 | 1,400,000 | 4.1 |
| EX15-A2 | 290 | 1,000 to 2,690,000 | 1,260,000 | 4.3 |
| EX16-A2 | Differential refractometer | 13,000 to 2,660,000 | 1,350,000 | 3.5 |
| EX17-A2 | Differential refractometer | 13,000 to 2,530,000 | 1,350,000 | 4.4 |
| EX18-A2 | 255 | 15,000 to 2,520,000 | 1,520,000 | 6.1 |
| EX19-A2 | 280 | 12,000 to 2,640,000 | 1,530,000 | 6.9 |

(continued)

| Compound | Measurement wavelength (nm) | Molecular weight (Da) | Weight-average molecular weight (Da) | Introduction rate of reactive group (mol%) (NMR integral ratio) |
|---|---|---|---|---|
| EX20-B2 | 215 | 19,000 to 2,830,000 | 1,380,000 | 4.5 |
| EX20-A2 | Differential refractometer | 36,000 to 2,520,000 | 1,380,000 | 0.7 |

NMR data of intermediate compound

[Table 16-1]

| Compound | NMR data (δ: ppm) |
|---|---|
| 3-2 | $CDCl_3$: 6.30(1H, brs), 5.07(1H, brs),3.77(2H, d, J = 6 Hz), 3.40-3.35(4H, m), 1.84-1.77(2H, m), 1.46 (9H, s) |
| 3-3 | DMSO-$d_6$: 8.47(1H, t, J = 5 Hz), 8.06(3H, brs), 3.52(2H, q, J = 6 Hz), 3.39(2H, t, J = 7 Hz), 3.18(2H, q, J = 6 Hz), 1.71-1.64(2H, m) |
| 4-2 | $CDCl_3$: 5.97(1H, brs), 5.12(1H, brs), 3.43-3.33(6H, m), 2.41(2H, t, J = 6 Hz), 1.83-1.76(2H, m), 1.44 (9H, s) |
| 4-3 | DMSO-$d_6$: 8.23(1H, t, J = 6 Hz), 7.94(3H, brs), 3.36(2H, t, J = 7 Hz), 3.12(2H, q, J = 6 Hz), 3.01-2.92 (2H, m), 2.47(2H, t, J = 7 Hz), 1.69-1.63(2H, m) |
| 5-2 | $CDCl_3$: 7.31-7.20(4H, m), 6.57(1H, brs), 4.84(1H, brs), 4.45(2H, d, J = 6 Hz), 4.30(2H, d, J = 6 Hz), 4.05(2H, s), 1.46(9H, s) |
| 5-3 | DMSO-$d_6$: 8.69(1H, t, J = 6 Hz), 8.20(3H, brs), 7.41(2H, d, J - 8 Hz), 7.30(2H, d, J = 8 Hz), 4.31(2H, d, J = 6Hz), 4.04-3.96(2H, m), 3.90(2H, s) |
| 6-3 | $CDCl_3$: 6.95 (2H, d, J = 9 Hz), 6.87 (2H, d, J = 9 Hz), 4.97 (1H, brs), 3.99 (2H, t, J = 5 Hz), 3.52 (2H, q, J = 5 Hz), 1.45 (9H, s) |
| 6-4 | DMSO-$d_6$: 8.11 (3H, brs), 7.09 (2H, d, J = 9 Hz), 7.03 (2H, t, J = 9 Hz), 4.15 (2H, t, J = 5 Hz), 3.22-3.16 (2H, m) |
| 7-2 | $CDCl_3$: 7.80(1H, brs), 4.93(1H, brs), 3.45(2H, q, J = 5 Hz), 3.41-3.34(2H, m), 1.44(9H, s) |
| 7-3 | DMSO-$d_6$: 9.56(1H, brs), 8.00(3H, brs), 3.45(2H, d, J = 6 Hz), 2.95(2H, d, J = 6 Hz) |
| 7-5 | $CDCl_3$: 7.95(1H, brs), 6.95(1H, brs), 4.28-4.23(1H, m),4.08(2H, d, J = 15 Hz), 3.91(1H, d, J = 15 Hz), 3.56-3.50(4H, m), 2.31-2.12(3H, m), 2.03-1.78(4H, m), 1.75-1.61(2H, m), 1.52-1.42(1H, m) |
| 7-6 | $CDCl_3$: 6.82(1H, brs), 4.28-4.22(1H, m), 4.06(1H, d, J = 15 Hz), 3.90(1H, d, J = 15 Hz), 3.40-3.31(2H, m), 2.86(2H, t, J = 6 Hz), 2.31-2.12(3H, m), 2.02-1.78(4H, m), 1.75-1.57(2H, m),1.52-1.41(1H, m) |
| 8-2 | $CDCl_3$: 7.01(1H, brs), 4.84 (1H, brs), 3.62-3.51(6H, m),3.31(2H, q, J = 5 Hz), 1.45(9H, s) |
| 8-3 | DMSO-$d_6$: 9.55(1H, brs), 8.05(3H, brs), 3.61(2H, t, J = 5 Hz), 3.54(2H, t, J = 6 Hz), 3.39(2H, q , J = 6 Hz), 3.00-2.91(2H, m) |
| 8-4 | DMSO-$d_6$: 9.45(1H, brs), 7.61(1H, t, J = 6 Hz), 4.29-4.25(1H, m), 3.87(2H, d, J = 15 Hz), 3.75(1H, d, J = 15 Hz), 3.50(2H, t, J = 6 Hz), 3.43(2H, t, J = 6 Hz), 3.37-3.31(2H, m), 3.24(2H, q, J = 6 Hz), 2.27-2.03 (3H, m), 1.96-1.69(4H, m), 1.67-1.50(2H, m), 1.43-1.35(1H, m) |

[Table 16-2]

| Compound | NMR data (δ: ppm) |
|---|---|
| 8-5 | $CDCl_3$: 6.89(1H, brs), 4.27-4.22(1H, m), 4.07(1H, d, J = 15 Hz), 3.88(1H, d, J = 15 Hz), 3.58-3.47(6H, m), 2.87(2H, t, J = 5 Hz), 2.31-2.10(3H, m), 2.03-1.77(4H, m), 1.73-1.59(2H, m), 1.51-1.43(1H, m) |
| 9-1 | DMSO-$d_6$: 9.39(1H, brs), 7.93(1H, brs), 6.92(1H, t, J = 6 Hz), 3.49(2H, d, J = 6 Hz), 3.25-3.17(4H, m), 1.38(9H, s) |
| 9-2 | DMSO-$d_6$: 9.50(1H, brs), 8.54(1H, brs), 8.01(3H, brs), 3.49(2H, s), 3.28-3.24(4H, m) |
| 9-3 | DMSO-$d_6$: 9.41(1H, brs), 8.03(1H, t, J = 6 Hz), 7.78(1H, t, J = 6 Hz), 4.35-4.29(1H, m), 3.93(1H, d, J = 15 Hz), 3.79(1H, d, J = 15 Hz), 3.68(2H, dd, J = 6, 2 Hz), 3.27-3.16(4H, m), 2.28-2.05(3H, m), 1.99-1.69(4H, m), 1.65-1.54(2H, m), 1.46-1.36(1H, m) |
| 9-4 | DMSO-$d_6$: 7.83(1H, t, J = 6 Hz), 7.78(1H, t, J = 6 Hz), 4.33-4.29(1H, m), 3.92(1H, d, J = 15 Hz), 3.79 (1H, d, J = 15 Hz), 3.69(2H, dd, J = 6, 2 Hz), 3.05(2H, q, J = 6 Hz), 2.55(2H, t, J = 6 Hz), 2.28-2.05 (3H, m), 1.99-1.69(4H, m), 1.63-1.56(2H, m), 1.46-1.36(1H, m) |
| 10-1 | DMSO-$d_6$: 9.39(1H, t, J = 5 Hz), 8.00(1H, t, J = 6 Hz), 6.74(1H, t, J = 6 Hz), 3.21(2H, t, J = 6 Hz), 3.17 (2H, t, J = 6 Hz), 3.14-3.07(2H, m), 2.20(2H, t, J = 7 Hz), 1.37(9H, s) |
| 10-2 | DMSO-$d_6$: 9.47(1H, brs), 8.27(1H, t, J = 6 Hz), 7.74(3H, brs), 3.27-3.17(4H, m), 2.96(2H, t, J = 7 Hz), 2.43(2H, t, J = 7 Hz) |
| 10-3 | DMSO-$d_6$: 9.40(1H, t, J = 5 Hz), 8.04(1H, t, J = 6 Hz), 7.68(1H, t, J = 6 Hz), 4.29-4.23(1H, m), 3.85 (1H, d, J = 15 Hz), 3.73(1H, d, J = 15 Hz), 3.32-3.13(6H, m), 2.25(2H, t, J = 7 Hz), 2.23-2.03(3H, m), 1.95-1.70(4H, m), 1.66-1.50(2H, m), 1.43-1.35(1H, m) |
| 10-4 | DMSO-$d_6$: 7.85(1H, t, J = 6 Hz), 7.68(1H, t, J = 6 Hz), 4.30-4.25(1H, m), 3.86(1H, d, J = 15 Hz), 3.74 (1H, d, J = 15 Hz), 3.32-3.25(2H, m), 3.04(2H, q, J = 6 Hz), 2.55(2H, t, J = 6 Hz), 2.27(2H, t, J = 7 Hz), 2.25-2.04(3H, m), 1.96-1.71(4H, m), 1.67-1.51(2H, m), 1.45-1.35(1H, m) |
| 11-4 | $CDCl_3$: 4.20-4.16(1H, m), 3.61-3.56(1H, m), 3.38-3.33(1H, m), 2.88-2.82(2H, m), 2.30-2.09(3H, m), 2.01-1.41(7H, m) |
| 12-2 | $CDCl_3$: 7.28(1H, brs), 3.80-3.75(2H, m), 3.66-3.56(6H, m), 2.42(1H, brs) |
| 12-3 | $CDCl_3$: 7.04(1H, brs), 6.20(1H, dd, J = 11, 4 Hz), 3.90(1H, dd, J = 10, 5 Hz), 3.71-3.55(7H, m), 3.51-3.44 (1H, m), 2.80-2.69(1H, m), 2.34-2.27(1H, m), 2.09-1.82(4H, m), 1.79-1.67(1H, m), 1.55-1.44(1H, m), 1.35-1.23(1H, m), 0.87-0.76(1H, m) |

[Table 16-3]

| Compound | NMR data (δ: ppm) |
|---|---|
| 12-4 | $CDCl_3$: 7.04(1H, brs), 4.24-4.19(1H, m), 3.75-3.68(1H, m), 3.67-3.61(4H, m), 3.58-3.49(3H, m), 2.30-2.11(3H, m), 2.00-1.66(5H, m), 1.63-1.54(1H, m), 1.47-1.38(1H, m) |
| 12-5 | $CDCl_3$: 4.25-4.21(1H, m), 3.76-3.70(1H, m), 3.64-3.61(2H, m), 3.55-3.49(3H, m), 2.87(2H, t, J = 5 Hz), 2.30-2. 10(3H, m), 2.02-1.55(6H, m), 1.47-1.38(1H, m) |
| 13-2 | DMSO-$d_6$: 12.30(1H, brs), 9.47(1H, brs), 3.37((2H, q, J = 7 Hz), 2.49(2H, t, J = 7 Hz) |
| 13-3 | $CDCl_3$: 7.73(1H, brs), 6.40(1H, brs), 4.86(1H, brs), 3.64(2H, q, J = 6 Hz), 3.56-3.50(4H, m), 3.47-3.43 (2H, m), 3.30(2H, q, J = 5 Hz), 2.53-2.50(2H, m), 1.45(9H, s) |
| 13-4 | DMSO-$d_6$: 9.50(1H, t, J = 5 Hz), 8.15(1H, t, J = 6 Hz), 8.01(3H, brs), 3.58((2H, t, J = 5 Hz), 3.43(2H, t, J = 6 Hz), 3.38(2H, q, J = 7 Hz), 3.24(2H, q, J = 6 Hz), 3.00-2.92(2H, m), 2.39(2H, t, J = 7 Hz) |
| 13-5 | $CDCl_3$: 7.80(1H, brs), 6.80(1H, brs), 6.58(1H, brs), 4.29-4.23(1H, m), 4.06(1H, d, J = 15 Hz), 3.89(1H, d, J = 15 Hz), 3.64(2H, q, J = 6 Hz), 3.60-3.55(4H, m), 3.52-3.46(2H, m), 3.44(2H, q, J = 5 Hz), 2.52 (2H, t, J = 6 Hz), 2.31-2.11(3H, m), 2.02-1.78(4H, m), 1.76-1.61(2H, m), 1.51-1.42(1H, m) |
| 13-6 | $CDCl_3$: 7.33(1H, brs), 6.82(1H, brs), 4.28-4.23(1H, m), 4.06(1H, d, J = 15 Hz), 3.90(1H, d, J = 15 Hz), 3.56(4H, t, J = 5 Hz), 3.51-3.44(4H, m), 3.01(2H, t, J = 6 Hz), 2.35(2H, t, J = 6 Hz), 2.31-2.12(3H, m), 2.02-1.79(4H, m), 1.77-1.60(2H, m), 1.50-1.42(1H, m) |

(continued)

| Compound | NMR data (δ: ppm) |
|---|---|
| 14-2 | $CDCl_3$: 6.66(1H, brs), 3.80(2H, q, J = 6 Hz), 3.53(2H, t, J = 6 Hz) |
| 14-4 | $CDCl_3$: 7.22(2H, d, J = 8 Hz), 6.85(2H, d, J = 8 Hz), 6.74(1H, brs), 4.79(1H, brs), 4.25(2H, d, J = 6 Hz), 4.09(2H, t, J = 5 Hz), 3.78(2H, q, J = 5 Hz), 1.46(9H, s). |
| 14-5 | DMSO-$d_6$: 9.67(1H, brs), 8.14(3H, brs), 7.38(2H, d, J = 9 Hz), 6.98(2H, d, J = 9 Hz), 4. 10(2H, t, J = 6 Hz), 3.94(2H, brs), 3.57(2H, q, J = 6 Hz) |
| 14-6 | $CDCl_3$: 7.23(2H, d, J = 9 Hz), 6.94(1H, brs), 6.85(2H, d, J = 9 Hz), 6.80-6.77(1H, m), 4.42(2H, d, J = 6 Hz), 4.25-4.21(1H, m), 4.11-4.05(3H, m), 3.92(1H, d, J= 15 Hz), 3.78(2H, q, J= 5 Hz), 2.28-2.05(3H, m), 1.99-1.55(6H, m), 1.48-1.39(1H, m) |
| 14-7 | $CDCl_3$: 7.22(2H, d, J = 9 Hz), 6.87(2H, d, J = 9 Hz), 6.75(1H, brs), 4.42(2H, d, J = 6 Hz), 4.25-4.20 (1H, m), 4.10(1H, d, J = 15 Hz), 4.03-3.90(3H, m), 3.08(2H, t, J = 5 Hz), 2.28-2.07(3H, m), 1.99-1.55 (6H, m), 1.48-1.40(1H, m) |

[Table 16-4]

| Compound | NMR data (δ: ppm) |
|---|---|
| 15-2 | DMSO-$d_6$: 7.88(2H, d, J = 8 Hz), 7.73-7.66(3H, m), 7.61-7.57(2H, m), 7.50-7.29(11H, m), 5.02(1H, d, J = 14 Hz), 4.29-4.18(3H, m), 3.62(1H, d, J = 14 Hz), 3.46(2H, d, J = 6 Hz), 3.18-3.08(1H, m), 3.02-2.89 (1H, m), 2.47-2.39(1H, m), 1.85-1.74(1H, m) |
| 15-3 | DMSO-$d_6$: 7.73(1H, brs), 7.64-7.58(2H, m), 7.51-7.29(6H, m), 5.04(1H, d, J = 14 Hz), 3.63(1H, d, J= 14 Hz), 3.18-3.06(1H, m), 3.04-2.95(1H, m), 2.95(2H, s), 2.47-2.39(1H, m), 1.87-1.78(1H, m) |
| 16-2 | $CDCl_3$: 6.80(1H, brs), 4.21(2H, d, J = 5 Hz). |
| 16-3 | $CDCl_3$: 6.03(1H, brs), 4.20-4.16(1H, m), 4.05-4.01(2H, m), 3.64-3.60(1H, m), 3.54-3.44(3H, m), 2.29-2.09(3H, m), 1.98-1.67(6H, m), 1.48-1.40(1H, m) |
| 16-4 | $CDCl_3$: 7.43(1H, brs), 4.21-4.17(1H, m), 3.66-3.62(1H, m), 3.55-3.41(3H, m), 3.36(2H, s), 2.30-2.08 (3H, m), 2.01-1.77(4H, m), 1.72-1.59(2H, m), 1.50-1.42(1H, m) |
| 17-2 | $CDCl_3$: 7.35-7.28(3H, m), 7.14-7.12(2H, m), 6.69(1H, d, J = 7 Hz), 4.96-4.91(1H, m), 4.64(1H, brs), 3.31(1H, dd, J = 14, 6 Hz), 3.22(1H, dd, J = 14, 6 Hz) |
| 17-3 | $CDCl_3$: 7.35-7.26(4H, m), 7.22-7.20(2H, m), 5.71-5.63(1H, m), 4.59-4.53(1H, m), 4.11-3.99(1H, m), 3.54-3.14(5H, m), 3.07-2.96(1H, m), 2.27-2.12(2H, m), 2.10-1.64(6H, m), 1.60-1.55(1H, m), 1.46-1.37 (1H, m) |
| 17-4 | $CDCl_3$: 7.51-7.47(1H, m), 7.33-7.30(2H, m), 7.24-7.21(3H, m), 4.17-4.13(1H, m), 3.63-3.58(2H, m), 3.51-3.38(3H, m), 3.30-3.25(1H, m), 2.71-2.65(1H, m), 2.29-2.05(3H, m), 1.98-1.76(4H, m), 1.70-1.41 (3H, m) |
| 18-2 | $CDCl_3$: 7.98 (2H, d, J = 9 Hz), 6.90 (2H, d, J = 9 Hz), 4.97 (1H, brs), 4.07 (2H, t, J = 5 Hz), 3.88 (3H, s), 3.56 (2H, q, J = 5 Hz), 1.45 (9H, s) |
| 18-4 | $D_2O$: 7.60 (2H, d, J = 9 Hz), 6.93 (2H, d, J = 9 Hz), 4.19 (2H, t, J = 5 Hz), 3.31-3.29 (6H, m), 1.77-1.71 (2H, m) |

[Table 16-5]

| Compound | NMR data (δ: ppm) |
|---|---|
| 20-2 | $CDCl_3$: 7.29 (2H, d, J = 8 Hz), 7.25 (2H, d, J = 8 Hz), 6.51 (1H, br s), 4.86 (1H, brs), 4.51 (2H, d, J = 5 Hz), 4.31 (2H, d, J = 6 Hz), 1.46 (9H, s) |
| 20-3 | $D_2O$: 7.29 (2H, d, J = 8 Hz), 7.25 (2H, d, J = 8 Hz), 4.38 (2H, s), 4.02 (2H, s) |

(continued)

| Compound | NMR data (δ: ppm) |
|---|---|
| 20-4 | $CDCl_3$: 7.31 (2H, d, J = 8 Hz), 7.26 (2H, d, J = 8 Hz), 6.84 (1H, brs), 6.52 (1H, brs), 4.52 (2H, d, J = 6 Hz), 4.49 (2H, d, J = 6 Hz), 4.26-4.23 (1H, m), 4.11 (1H, d, J= 15 Hz), 3.94 (1H, d, J= 15 Hz), 2.26-2.09 (3H, m), 2.00-1.58 (6H, m), 1.48-1.44 (1H, m) |
| 20-5 | $CDCl_3$: 7.31-7.26 (4H, m), 6.80 (1H, brs), 4.48 (2H, d, J = 6 Hz), 4.26-4.21 (1H, m), 4.11 (1H, d, J = 15 Hz), 3.93 (1H, d, J = 15 Hz), 3.86 (2H, s), 2.28-2.07 (3H, m), 1.99-1.40 (7H, m) |

LC-Mass data of intermediate compounds

[Table 17]

| Compound | MS-ESI (m/z) $[M+H]^+$ | Retention time (min.) |
|---|---|---|
| 6-3 | 279 | 1.13 |
| 6-4 | 179 | 0.63 |
| 8-5 | 269 | 0.60 |
| 9-3 | 378 | 0.84 |
| 9-4 | 282 | 0.62 |
| 10-3 | 392, *414 | 0.83 |
| 10-4 | 296 | 0.62 |
| 12-2 | *410, *412 | 1.15 |
| 12-3 | *330 | 1.03 |
| 13-5 | 436, *458 | 0.82 |
| 13-6 | 340 | 0.64 |
| 14-5 | *285 | 0.59 |
| 14-6 | 427 | 1.02 |
| 14-7 | 331 | 0.72 |
| 15-3 | 334, *356 | 0.74 |
| 16-3 | *343 | 0.90 |
| 17-3 | 411 | 1.10 |
| 17-4 | 315 | 0.77 |
| 18-4 | 264 | 0.54 |
| 20-2 | *355 | 0.97 |
| 20-3 | 233 | 0.53 |
| 20-4 | 397 | 0.99 |
| 20-5 | 301 | 0.68 |
| *: [M+Na] | | |

[Measurement of introduction rate of reactive group or complementary reactive group]

The reactive group or complementary reactive group introduction rate means a value expressing the number of the reactive groups or the complementary reactive groups introduced per uronic acid monosaccharide unit, which is the repeating unit of alginic acid.

In the examples, the reactive group or complementary reactive group introduction rate (mol%) was calculated from the integral ratio in $^1$H-NMR. In addition, the amount of alginic acid necessary for the calculation of the introduction rate can be measured by the carbazole sulfate method in which a calibration curve is used, and the amount of the reactive group or the complementary reactive group can also be measured by spectrophotometry in which a calibration curve is used.

[Measurement of molecular weight]

An alginic acid solid into which the reactive group or the complementary reactive group had been introduced, which was obtained in the examples, was dissolved in a 10 mmol/L phosphate buffer solution containing 0.15 mol/L of NaCl (pH: 7.4) to prepare a 0.1% or 0.2% solution, an insoluble matter was removed by passing the solution through a polyether sulfone filtration filter having a pore diameter of 0.22 μm (Minisart High Flow Filter, Sartorius AG), and then the solution was used as a gel filtration sample. The spectrum of each sample was measured with a spectrophotometer DU-800 (Beckman Coulter, Inc.), and the measurement wavelength in the gel filtration of each compound was determined. For compounds having no peculiar absorption wavelength, a differential refractometer was used.

200 μL of the gel filtration sample was fed into a Superose 6 Increase 10/300 GL column (GE Healthcare Corporation). Gel filtration was performed under conditions of room temperature and a flow rate of 0.8 mL/mim using AKTA Explorer 10S as a chromatography device and a 10 mmol/L phosphate buffer solution comprising 0.15 mol/L of NaCl (pH: 7.4) as a developing solvent. The elution profile of the sample was produced by monitoring the absorption of the wavelength determined in each compound. The obtained chromatogram was analyzed with Unicorn 5.31 software (GE Healthcare Corporation), and the peak range was determined.

Regarding the molecular weight of alginic acid into which the reactive group or the complementary reactive group had been introduced, blue dextran (molecular weight: 2,000,000 Da, Sigma-Aldrich), thyroglobulin (molecular weight: 669,000 Da, GE Healthcare Corporation), ferritin (molecular weight: 440,000 Da, GE Healthcare Corporation), aldolase (molecular weight: 158,000 Da, GE Healthcare Corporation), conalbumin (molecular weight: 75,000 Da, GE Healthcare Corporation), ovalbumin (molecular weight: 44,000 Da, GE Healthcare Corporation), ribonuclease A (molecular weight: 13,700 Da, GE Healthcare Corporation) and aprotinin (molecular weight: 6,500 Da, GE Healthcare Corporation) were used as standard products, gel filtration was performed under the same conditions as for alginic acid into which the reactive group or the complementary reactive group had been introduced, and the amount of the eluate of each component was determined with Unicorn software. The amount of the eluate of each component was plotted along the horizontal axis, the absolute value of the molecular weight was plotted along the vertical axis, respectively, and calibration curves were created by linear regression. Two calibration curves were created from blue dextran to ferritin and from ferritin to aprotinin.

The molecular weight (Mi) at an elution time i in the previously-obtained chromatogram was calculated using this calibration curve. Next, the absorbance at the elution time i was read and regarded as Hi. The weight-average molecular weight (Mw) was obtained from this data through the following formula.

[Math. 1]

$$Mw = \frac{\sum_{i=1}^{\infty} (Hi \times Mi)}{\sum_{i=1}^{\infty} Hi}$$

[Measurement of gel stability]

(Measurement of gel stability (1)): Stability in PBS

Each alginic acid derivative of (Ex1-A2), (Ex4-A2), (Ex5-A2) and (Ex19-A2) obtained in Examples 1, 4, 5 and 19 was dissolved in water so that the concentration reached 1.0%, thereby obtaining each of alginic acid aqueous solutions (1-1), (4-1), (5-1) and (19-1). Equal amounts of the alginate aqueous solutions were mixed together in combination of each of (1-1) and (19-1), (4-1) and (19-1) and (5-1) and (19-1) and put into a syringe equipped with an 18-gauge needle, this syringe was installed in a syringe pump set to a flow rate of 1 mL/minute, and the combination was added dropwise to a calcium chloride solution having a concentration of 30 mmol/L for 30 seconds and stirred for five minutes, thereby obtaining alginate gel. This gel was washed with 10 mL of PBS once and placed still in PBS at 37°C

for 10 minutes to perform chemical crosslinking, thereby obtaining gel-like chemically crosslinked alginic acid. Chemically crosslinked alginic acid (beads) produced from (Ex18-A2)/(Ex19-A2) was also prepared in the same manner as described above. 19.5 mL of PBS was added to the obtained chemically crosslinked alginic acid and shaken at 37°C, the aqueous solution was recovered over time, and the same amount of PBS as the recovered amount was supplemented. After the end of a test, 10 μL of alginate lyase (Nippon Gene Co., Ltd., 319-08261) was added to a test solution and shaken at 37°C for three hours or longer to collapse all of the gel, and the aqueous solution was recovered. The alginic acid concentration in the recovered aqueous solution was measured by a carbazole sulfate method, a value expressed in percentage of a value obtained by dividing the amount of alginic acid eluted until each point in time by the total amount of alginic acid calculated from the alginic acid concentrations at all points in time and the alginic acid concentration after the end of the test was regarded as a collapse rate and an index of gel stability.

The results of Fig. 1 were obtained. The chemically crosslinked alginic acid (beads) did not collapse even when 96 hours had elapsed, and the stability of the gel could be confirmed. That is, it was suggested that, due to the formation of a chemical crosslink by a Huisgen reaction, the structure of the produced chemically crosslinked alginic acid was maintained for a long period of time.

(Measurement of gel stability (2)): Stability under EDTA

Equal amounts of the alginate aqueous solutions obtained in (Measurement of gel stability (1)) were mixed together in combination of each of (1-1) and (19-1), (4-1) and (19-1), and (5-1) and (19-1) and put into a syringe equipped with an 18-gauge needle, this syringe was installed in a syringe pump set to a flow rate of 1 mL/minute, and the combination was added dropwise to a calcium chloride solution having a concentration of 30 mmol/L for 30 seconds and stirred for five minutes, thereby obtaining alginate gel. Chemically crosslinked alginic acid (beads) produced from (Ex18-A2)/(Ex19-A2) was also prepared in the same manner as described above. This gel was washed with 10 mL of physiological saline once and placed still in the physiological saline at 37°C for 10 minutes to perform chemical crosslinking, thereby obtaining chemically crosslinked alginic acid. 19.5 mL of 5 mM ethylenediaminetetraacetic acid dipotassium salt dihydrate (EDTA-2K)/physiological saline was added to the obtained chemically crosslinked alginic acid and shaken at 37°C, the aqueous solution was recovered over time, and the same amount of 5 mM EDTA-2K/physiological saline as the recovered amount was supplemented. After the end of a test, 10 μL of alginate lyase (Nippon Gene Co., Ltd., 319-08261) was added to a test solution and shaken at 37°C for three hours or longer to collapse all of the gel, and the aqueous solution was recovered. The alginic acid concentration in the recovered aqueous solution was measured by a carbazole sulfate method, a value expressed in percentage of a value obtained by dividing the amount of alginic acid eluted until each point in time by the total amount of alginic acid calculated from the alginic acid concentrations at all points in time and the alginic acid concentration after the end of the test was regarded as a collapse rate and an index of gel stability.

The results were shown in Fig. 2. The collapse rate of the chemically crosslinked alginic acid (beads) after 24 hours had elapsed was 2% or less. That is, it was suggested that, due to the formation of a crosslink by a Huisgen reaction, the structure of the produced chemically crosslinked alginic acid was maintained even under a condition of a calcium ion-free solution (the physiological concentration or lower for living bodies).

(Measurement of gel stability (3)): Stability in PBS

Each alginic acid derivative of (Ex3-A2), (Ex5-A2), (Ex6-A2), (Ex9a-A2), (Ex10-A2), (Ex12-A2) and (Ex18-A2) obtained in Examples 3, 5, 6, 9, 10, 12 and 18 was dissolved in water so that the concentration reached 1.0%, thereby obtaining each of alginic acid aqueous solutions (3-1), (5-1), (6-1), (9-1), (10-1), (12-1) and (18-1). Equal amounts of the alginate aqueous solutions were mixed together in combination of each of (6-1) and (9-1), (3-1) and (10-1), (5-1) and (10-1), and (18-1) and (12-1) and put into a syringe equipped with an 18-gauge needle, this syringe was installed in a syringe pump set to a flow rate of 1 mL/minute, and the combination was added dropwise to a calcium chloride solution having a concentration of 30 mmol/L for 30 seconds and stirred for five minutes, thereby obtaining alginate gel. This gel was washed with 10 mL of PBS once and placed still in PBS at 37°C for 10 minutes to perform chemical crosslinking, thereby obtaining chemically crosslinked alginic acid. Chemically crosslinked alginic acid gel (beads) produced from (Ex18-A2)/(Ex19-A2) was also prepared in the same manner as described above. 19.5 mL of PBS was added to the obtained chemically crosslinked alginic acid and shaken at 37°C, the aqueous solution was recovered over time, and the same amount of PBS as the recovered amount was supplemented. After the end of a test, 10 μL of alginate lyase (Nippon Gene Co., Ltd., 319-08261) was added to a test solution and shaken at 37°C for three hours or longer to collapse all of the gel, and the aqueous solution was recovered. The alginic acid concentration in the recovered aqueous solution was measured by a carbazole sulfate method, a value expressed in percentage of a value obtained by dividing the amount of alginic acid eluted until each point in time by the total amount of alginic acid calculated from the alginic acid concentrations at all points in time and the alginic acid concentration after the end of the test was regarded as a collapse rate and an index of gel stability.

The results of Fig. 3 were obtained. The chemically crosslinked alginic acid (beads) did not collapse even when 96 hours had elapsed, and the stability of the gel could be confirmed. That is, it was suggested that, due to the formation of a chemical crosslink by a Huisgen reaction, the structure of the produced chemically crosslinked alginic acid was maintained for a long period of time.

(Measurement of gel stability (4)): Stability under EDTA

Equal amounts of the alginate aqueous solutions obtained in (Measurement of gel stability (3)) were mixed together in combination of each of (6-1) and (9-1), (3-1) and (10-1), (5-1) and (10-1), and (18-1) and (12-1) and put into a syringe equipped with an 18-gauge needle, this syringe was installed in a syringe pump set to a flow rate of 1 mL/minute, and the combination was added dropwise to a calcium chloride solution having a concentration of 30 mmol/L for 30 seconds and stirred for five minutes, thereby obtaining alginate gel. This gel was washed with 10 mL of physiological saline once and placed still in the physiological saline at 37°C for 10 minutes to perform chemical crosslinking, thereby obtaining chemically crosslinked alginic acid. Chemically crosslinked alginic acid (beads) produced from (Ex18-A2)/(Ex19-A2) was also prepared in the same manner as described above. 19.5 mL of 5 mM ethylenediaminetetraacetic acid dipotassium salt dihydrate (EDTA 2K)/physiological saline was added to the obtained chemically crosslinked alginic acid and shaken at 37°C, the aqueous solution was recovered over time, and the same amount of 5 mM EDTA-2K/physiological saline as the recovered amount was supplemented. After the end of a test, 10 $\mu$L of alginate lyase (Nippon Gene Co., Ltd., 319-08261) was added to a test solution and shaken at 37°C for three hours or longer to collapse all of the gel, and the aqueous solution was recovered. The alginic acid concentration in the recovered aqueous solution was measured by a carbazole sulfate method, a value expressed in percentage of a value obtained by dividing the amount of alginic acid eluted until each point in time by the total amount of alginic acid calculated from the alginic acid concentrations at all points in time and the alginic acid concentration after the end of the test was regarded as a collapse rate and an index of gel stability.

The results were shown in Fig. 4. The collapse rate of the chemically crosslinked alginic acid (beads) after 24 hours had elapsed was 4% or less. That is, it was suggested that, due to the formation of a crosslink by a Huisgen reaction, the structure of the produced chemically crosslinked alginic acid was maintained even under a condition of a calcium ion-free solution (the physiological concentration or lower for living bodies).

(Measurement of gel stability (5)): Stability in PBS

Each alginic acid derivative of (Ex4-A2), (EX9a-A2), (Ex16-A2), (Ex18-A2) and (Ex20-B2) obtained in Examples 4, 9, 16, 18 and 20 was dissolved in water so that the concentration reached 1.0%, thereby obtaining each of alginic acid aqueous solutions (4-1), (9-1), (16-1), (18-1) and (20-1). Equal amounts of the alginate aqueous solutions were mixed together in combination of each of (4-1) and (20-1), (18-1) and (9-1), and (18-1) and (16-1) and put into a syringe equipped with an 18-gauge needle, this syringe was installed in a syringe pump set to a flow rate of 1 mL/minute, and the combination was added dropwise to a calcium chloride solution having a concentration of 30 mmol/L for 30 seconds and stirred for five minutes, thereby obtaining alginate gel. This gel was washed with 10 mL of PBS once and placed still in PBS at 37°C for 10 minutes to perform chemical crosslinking, thereby obtaining chemically crosslinked alginic acid. Chemically crosslinked alginic acid (beads) produced from (Ex18-A2)/(Ex19-A2) was also prepared in the same manner as described above. 19.5 mL of PBS was added to the obtained chemically crosslinked alginic acid and shaken at 37°C, the aqueous solution was recovered over time, and the same amount of PBS as the recovered amount was supplemented. After the end of a test, 10 $\mu$L of alginate lyase (Nippon Gene Co., Ltd., 319-08261) was added to a test solution and shaken at 37°C for three hours or longer to collapse all of the gel, and the aqueous solution was recovered. The alginic acid concentration in the recovered aqueous solution was measured by a carbazole sulfate method, a value expressed in percentage of a value obtained by dividing the amount of alginic acid eluted until each point in time by the total amount of alginic acid calculated from the alginic acid concentrations at all points in time and the alginic acid concentration after the end of the test was regarded as a collapse rate and an index of gel stability.

The results were shown in Fig. 5. The collapse rate of the chemically crosslinked alginic acid produced by the above-described method was 12% or less even after 96 hours had elapsed. That is, it was suggested that, due to the formation of a chemical crosslink by a Huisgen reaction, the structure of the produced chemically crosslinked alginic acid was maintained.

(Measurement of gel stability (6)): Stability under EDTA

Equal amounts of the alginate aqueous solutions obtained in (Measurement of gel stability (5)) were mixed together in combination of each of (4-1) and (20-1), (18-1) and (9-1), and (18-1) and (16-1) and put into a syringe equipped with an 18-gauge needle, this syringe was installed in a syringe pump set to a flow rate of 1 mL/minute, and the combination

was added dropwise to a calcium chloride solution having a concentration of 30 mmol/L for 30 seconds and stirred for five minutes, thereby obtaining alginate gel. This gel was washed with 10 mL of physiological saline once and placed still in the physiological saline at 37°C for 10 minutes to perform chemical crosslinking, thereby obtaining chemically crosslinked alginic acid. Chemically crosslinked alginic acid (beads) produced from (Ex18-A2)/(Ex19-A2) was also prepared in the same manner as described above. 19.5 mL of 5 mM ethylenediaminetetraacetic acid dipotassium salt dihydrate (EDTA-2K)/physiological saline was added to the obtained chemically crosslinked alginic acid and shaken at 37°C, the aqueous solution was recovered over time, and the same amount of 5 mM EDTA-2K/physiological saline as the recovered amount was supplemented. After the end of a test, 10 μL of alginate lyase (Nippon Gene Co., Ltd., 319-08261) was added to a test solution and shaken at 37°C for three hours or longer to collapse all of the gel, and the aqueous solution was recovered. The alginic acid concentration in the recovered aqueous solution was measured by a carbazole sulfate method, a value expressed in percentage of a value obtained by dividing the amount of alginic acid eluted until each point in time by the total amount of alginic acid calculated from the alginic acid concentrations at all points in time and the alginic acid concentration after the end of the test was regarded as a collapse rate and an index of gel stability.

The results of Fig. 6 were obtained. The collapse rate of the chemically crosslinked alginic acid (beads) after 24 hours had elapsed was 18% or less. That is, it was suggested that, due to the formation of a crosslink by a Huisgen reaction, the structure of the produced chemically crosslinked alginic acid was maintained even under a condition of a calcium ion-free solution (the physiological concentration or lower for living bodies).

[Measurement of gel permeability]

(Measurement of gel permeability (1))

Each alginic acid derivative (Ex1-A2), (Ex3-A2), (Ex4-A2), (Ex5-A2) and (Ex18-A2) obtained in Examples 1, 3, 4, 5 and 18 was dissolved in water so that the concentration reached 2.0% to prepare an alginic acid aqueous solution, and a 2/5 volume of fluorescein isothiocyanate-dextran having a molecular weight of 150,000 (Sigma-Aldrich Co. LLC, FD150S) prepared to 1 mg/mL and a 315 volume of water were added to this alginic acid aqueous solution, thereby each of obtaining 0.2 mg/mL fluorescein isothiocyanate-dextran-containing 1.0% alginic acid aqueous solutions (1-2), (3-2), (4-2), (5-2) and (18-2).

Furthermore, each alginic acid derivative of (ExIO-A2), (Ex12-A2) and (Ex19-A2) obtained in Examples 10, 12 and 19 was dissolved in water so that the concentration reached 1.0%, thereby obtaining each of alginic acid aqueous solutions (10-1), (12-1) and (19-1).

Equal amounts of the alginate aqueous solutions were mixed together in combination of each of (1-2) and (19-1), (4-2) and (19-1), (5-2) and (19-1), (3-2) and (10-1), and (18-2) and (12-1), 40 mL of a calcium chloride solution having a concentration of 30 mmol/L was added thereto and stirred for five minutes, thereby obtaining alginate gel. This gel was washed with 10 mL of physiological saline once and placed still in the physiological saline at 37°C for 10 minutes to perform chemical crosslinking, thereby obtaining fluorescein isothiocyanate-dextran-containing chemically crosslinked alginic acid. Fluorescein isothiocyanate-dextran-containing chemically crosslinked alginic acid produced from (Ex18-A2)/(Ex19-A2) was also prepared in the same manner as described above. 19.5 mL of physiological saline was added to the obtained chemically crosslinked alginic acid and shaken at 37°C, the aqueous solution was recovered over time, and the same amount of physiological saline as the recovered amount was supplemented. After the end of a test, 10 μL of alginate lyase (Nippon Gene Co., Ltd., 319-08261) was added to a test solution and shaken at 37°C for three hours or longer to collapse all of the gel, and the aqueous solution was recovered. The dextran concentration in the recovered aqueous solution was measured by fluorometry (excitation light: 485 nm, fluorescence: 535 nm), and a value expressed in percentage of a value obtained by dividing the amount of dextran at each point in time by the total amount of dextran after the end of a test was regarded as permeability.

The results of Fig. 7 were obtained. The permeability after 24 hours was within a range of 25% to 40%.

(Measurement of gel permeability (2))

Each alginic acid derivative (Ex4-A2), (Ex5-A2), (Ex6-A2) and (Ex18-A2) obtained in Examples 4, 5, 6 and 18 was dissolved in water so that the concentration reached 2.0% to prepare an alginic acid aqueous solution, and a 2/5 volume of fluorescein isothiocyanate-dextran having a molecular weight of 150,000 (Sigma-Aldrich Co. LLC, FD150S) prepared to 1 mg/mL and a 315 volume of water were added to this alginic acid aqueous solution, thereby each of obtaining 0.2 mg/mL fluorescein isothiocyanate-dextran-containing 1.0% alginic acid aqueous solutions (4-2), (5-2), (6-2) and (18-2).

Furthermore, each alginic acid derivative of (EX9a-A2), (Ex10-A2), (Ex16-A2) and (Ex20-B2) obtained in Examples 9, 10, 16 and 20 was dissolved in water so that the concentration reached 1.0%, thereby obtaining each of alginic

acid aqueous solutions (9-1), (10-1), (16-1) and (20-1).

Equal amounts of the alginate aqueous solutions were mixed together in combination of each of (4-2) and (20-1), (5-2) and (10-1), (6-2) and (9-1), (18-2) and (9-1), and (18-2) and (16-1), 40 mL of a calcium chloride solution having a concentration of 30 mmol/L was added thereto and stirred for five minutes, thereby obtaining alginate gel. This gel was washed with 10 mL of physiological saline once and placed still in the physiological saline at 37°C for 10 minutes to perform chemical crosslinking, thereby obtaining fluorescein isothiocyanate-dextran-containing chemically crosslinked alginic acid. Fluorescein isothiocyanate-dextran-containing chemically crosslinked alginic acid produced from (Ex18-A2)/(Ex19-A2) was also prepared in the same manner as described above. 19.5 mL of physiological saline was added to the obtained chemically crosslinked alginic acid and shaken at 37°C, the aqueous solution was recovered over time, and the same amount of physiological saline as the recovered amount was supplemented. After the end of a test, 10 μL of alginate lyase (Nippon Gene Co., Ltd., 319-08261) was added to a test solution and shaken at 37°C for three hours or longer to collapse all of the gel, and the aqueous solution was recovered. The dextran concentration in the recovered aqueous solution was measured by fluorometry (excitation light: 485 nm, fluorescence: 535 nm), and a value expressed in percentage of a value obtained by dividing the amount of dextran at each point in time by the total amount of dextran after the end of a test was regarded as permeability.

The results of Fig. 8 were obtained. The permeability after 24 hours was within a range of 25% to 30%.

[Biocompatibility evaluation of chemically crosslinked alginic acid]

Each chemically modified alginic acid derivative of (EX4-A2), (EX5-A2), (EX12-A2), (EX16-A2), (EX18-A2), (EX19-A2) and (EX20-B2) obtained in Examples 4, 5, 12, 16, 18, 19 and 20 was dissolved in water to produce a reactive group-introduced alginic acid solution (in the present specification, there are cases where alginic acid aqueous solutions are simply expressed as alginic acid solutions, and, unless particularly otherwise described, alginic acid solutions indicate aqueous solutions of corresponding alginic acids, chemically modified derivatives or mixtures thereof). Filter sterilization was performed on this with Minisart High Flow (Sartorius AG, 16532GUK), and then a 1.0% reactive group-introduced alginic acid/physiological saline solution was prepared. The 1.0% reactive group-introduced alginic acid/physiological saline solution was added to HeLa cells that had been seeded in a 96 well plate so that the cell concentration reached $5 \times 10^3$ cells/well and cultured for one day so that the final concentration reached 0.1% in a combination of (EX18-A2) and (EX19-A2), (Ex5-A2) and (Ex19-A2), (Ex4-A2) and (Ex20-B2), and (Ex18-A2) and (Ex12-A2) or (Ex16-A2), and, as an index of cytotoxicity, ATP activity was evaluated with CellTiter-Glo Luminescent Cell Viability Assay (Promega Corporation, G7571) after one day of the culture.

The results of Fig. 9 were obtained. The fact that the ATP activity could be confirmed in all of the chemically crosslinked alginic acids evaluated by the above-described method suggested that there was no cytotoxicity in the chemically crosslinked alginic acids and suggested that chemically crosslinked alginic acids in which a chemical crosslink was formed by a Huisgen reaction are biocompatible.

[Production of alginic acid three-layer structure]

Structures of the present invention were molded into a bead shape, a disc shape and a fiber shape using the chemically modified alginic acid derivatives, and the strengths thereof were compared with the strength of a structure produced using natural alginic acid. In addition, in a case where cells were actually encapsulated as a pharmacological ingredient, it was confirmed that the cells survived in the structures and the functions could be exhibited.

(Preparation of chemically modified alginic acid derivative solution)

An aqueous solution of the chemically modified alginic acid derivative that was used in each of examples to be described below was prepared by the following method. In each example where chemically crosslinked alginic acid was manufactured by a Huisgen reaction, an aqueous solution of each of the chemically modified alginic acid derivatives of Example 18, Example 19 and (EX20-A2) of Example 20 was prepared. In addition, in examples where chemically crosslinked alginic acid was manufactured by a photocrosslinking reaction, chemically modified alginic acid derivatives (C-IA and C-IB) represented by the following formula were prepared according to a method disclosed in PCT/JP2019/007655 (filed on February 27, 2019), and aqueous solutions thereof were prepared. These solutions will be abbreviated as a solution of Example 18, a solution of Example 19, a solution of Example 20, a solution of Example C-IA and a solution of Example C-IB, respectively.

[C177]

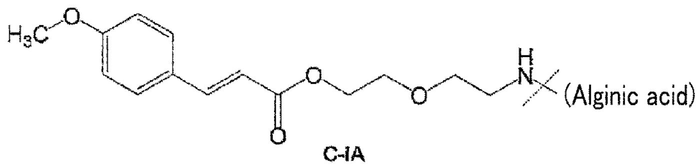

C-IA

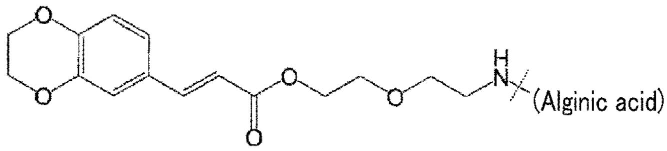

C-IB

[In the formula, a nitrogen atom on the left side of the broken line bonds to a carboxy group of alginic acid on the right side of the broken line (manufactured by MOCHIDA PHARMACEUTICAL CO., LTD.: manufactured using A-2 as a raw material)]

<Aqueous solutions used in Examples A to C>

For each of the chemically modified alginic acid derivatives manufactured by the methods in Examples 18 to 20 and the compounds C-IA and C-IB, a 1 wt% physiological saline solution was prepared.

<Preparation of aqueous solution used in Example D>

For each of the chemically modified alginic acid derivatives manufactured by the methods in Examples 18 to 20, a 3 wt% aqueous solution was prepared using MilliQ water.

<Preparation of aqueous solution used in Example E>

For each of the chemically modified alginic acid derivatives manufactured by the methods in Examples 18 and 20, a 2.6 wt% physiological saline solution was prepared, and filter sterilization was performed on this using Minisart High Flow (Sartorius AG, 0.2 μm, Cat. 16532GUK). The salt concentration of the aqueous solution after the filter sterilization was adjusted, and the aqueous solution was used as a 2.4 wt% physiological saline solution.

In the following examples, unless particularly otherwise described, each solution that was used in each step was prepared using physiological saline.

[Example A] Production of alginic acid three-layer structure (bead)

In the present test, it was verified that, in a case where the structure of the present invention is molded into a bead shape, it is possible to produce a large structure compared with conventional structures for which natural alginic acid is used.

<Step 1a> Production of alginic acid bead (1 mm bead)

An alginic acid bead having a diameter of approximately 1 mm was produced by the following method. In the production of the bead, a commercially available encapsulating device (manufactured by BÜCHI, Encapsulator B-390) was used.

First, a magnetic stirrer was placed below the nozzle of the encapsulating device, a crystal dish having a diameter of 95 mm and a height of 55 mm was placed thereon, 100 mL of a 100 mmol/L calcium chloride solution and a stirrer were put thereinto and stirred at 300 rpm. Next, an aqueous solution of sodium alginate (manufactured by MOCHIDA PHARMACEUTICAL CO., LTD.: AL100 [corresponding to A-2 in Table 13] prepared to 1 wt% (which was a sodium alginate solution and will be referred to as the solution of AL 100 as appropriate, which will be same below) was put into a pressure bottle, the pressure of an oilless air compressor was adjusted to 1.5 to 2 bar, and then the air pressure was set to approximately 700 mbar with a pressure adjustment system of the device main body. While observing a water flow irradiated with a strobe light, the flow amount or vibration frequency at which regularly spaced droplets continued was adjusted until the continuous water flow was divided into regularly spaced droplets from a point 3 to 5 cm below the nozzle and could be confirmed across several centimeters. An electrostatic dispersion unit was operated from 500 V,

an electric current was passed by changing the voltage by 100 V, and a state in which the flow of a capsule sphere dispersed in a conical shape from 3 to 10 cm was formed. Once spherical and stable circular distribution was obtained, the bead was added dropwise into the calcium chloride solution and stirred for 30 minutes.

<Step 1b> Production of alginic acid bead (5 mm bead)

An alginic acid bead having a diameter of approximately 5 mm was produced by the following method. First, a syringe connected to a 12-gauge needle was filled with a variety of alginic acid solutions prepared to 1 wt%. 40 mL of a 100 mmol/L calcium chloride solution was set such that the distance from the tip of the needle to the liquid surface became 8 mm and stirred using the magnetic stirrer at 400 rpm. The alginic acid solutions were added dropwise thereto for 30 seconds at a flow rate of 1 mL/min using a syringe pump and stirred for 30 minutes.

The alginic acid solutions used for the alginic acid bead produced herein are as described below.

· Solution of AL 100
· Solution obtained by mixing the solution of Example 18 and the solution of Example 19 in a volume ratio of 1:1 (hereinafter, also referred to as the solution of Examples 18/19)
· Solution obtained by mixing the solution of Example 18 and the solution of Example 20 in a volume ratio of 1:1 (hereinafter, also referred to as the solution of Examples 18/20)
· Solution of Example C-IA

<Step 1c> PLO coating

5 mL of a poly-L-ornithine (SIGMA-ALDRICH Co. LLC, P2533, MW: 15,000 to 30,000) solution prepared to 0.1 wt% (hereinafter, particularly otherwise described, poly-L-ornithine solutions are prepared using a 100 mmol/L calcium chloride-comprising physiological saline solution) was added to a plastic petri dish including the alginic acid bead obtained in the step 1a or the step 1b and placed still at 37°C for 10 minutes. After that, the solution was once removed, 5 mL of a poly-L-ornithine solution prepared to 0.05 wt% was added thereto and placed still at 37°C for six minutes, and the alginic acid bead was washed twice using 10 mL of physiological saline.

<Step 1d> Alginic acid coating

5 mL of an alginic acid solution prepared to 0.15 wt% was added to a plastic petri dish including the PLO-coated bead obtained in the step 1c and placed still at 37°C for 10 minutes, and the bead was washed twice using 10 mL of physiological saline. The alginic acid solution used is the same as that in the step 1b.

<Step 1e> Light irradiation

On the bead for which the alginic acid solution that was crosslink-reacted by light irradiation in the step 1b and the step 1d (the solution in Example C-I), UV irradiation was performed for 10 minutes with an UV irradiation device (manufactured by Sen Lights Corp., HB100A-1) after the end of the step 1d.

<Step 1f> Chelate treatment and stability evaluation

20 mL of a 55 mM sodium citrate solution was added to a plastic petri dish including the bead obtained through each step. It was confirmed that the alginic acid bead derived from the AL100 solution produced in the step 1a or 1b was dissolved rapidly, the alginic acid bead produced in the step 1d or 1e was shaken (water bath shaker (TAITEC Corporation, personal II), 180 rpm) at 37°C for one day in a tube including 20 mL of a phosphate buffer solution, and the shape was observed. The observation results are shown in Table 18 below. The stability of the alginic acid bead was visually evaluated based on the following indexes. As reference of each index, a photograph of Example A-6 evaluated as "3" is shown in Fig. 10, a photograph of Example A-3 evaluated as "2" is shown in Fig. 11(b), and a photograph of Example A-2 evaluated as "1" is shown in Fig. 12(b), respectively.
Indexes of stability evaluation:

3: The collapse/dissolution/deformation or the like of the bead are all not recognized.
2: Large collapse/dissolution/deformation or the like is recognized in a part of the bead.
1: Clear collapse/dissolution/deformation or the like are recognized in the bead, and the function of the structure is not kept.

[Table 18]

| Example | Steps | | | | | Stability evaluation (scores) | |
|---|---|---|---|---|---|---|---|
| | Step 1a | Step 1b | Step 1c | Step 1d | Step 1e | After chelate treatment | After shaking |
| A-1 | AL100 | - | Performed | AL100 | - | 3 | 3 |
| A-2 | - | AL 100 | Performed | AL100 | - | 3 | 1 |
| A-3 | - | AL 100 | Performed | Examples 18/19 | - | 3 | 2 |
| A-4 | - | AL 100 | Performed | Examples 18/20 | - | 3 | 3 |
| A-5 | - | Examples 18/19 | Performed | AL100 | - | 3 | 3 |
| A-6 | - | Examples 18/20 | Performed | AL100 | - | 3 | 3 |
| A-7 | - | Examples 18/19 | Performed | Examples 18/19 | - | 3 | 3 |
| A-8 | - | Examples 18/20 | Performed | Examples 18/20 | - | 3 | 3 |
| A-9 | - | AL 100 | Performed | AL100 | Performed | 3 | 1 |
| A-10 | - | AL 100 | Performed | Example C-IA | Performed | 3 | 1 |
| A-11 | - | Example C-IA | Performed | AL100 | Performed | 3 | 3 |
| A-12 | - | Example C-IA | Performed | Example C-IA | Performed | 3 | 3 |

For beads in which AL100 was used in a core layer, a bead having a diameter of approximately 1 mm was capable of producing a stable three-layer structure (Example A-1), but a bead having a diameter of approximately 5 mm held the shape at the time of the chelate treatment (Fig. 12(a)), but the bead collapseed after shaking (Fig. 12(b), Example A-2). On the other hand, in the structures (Examples A-5 to A-8) of the present invention manufactured from the Huisgen-type chemically modified alginic acid derivative, the shapes were held in all of the structures even after shaking (a photograph of Example A-6 after shaking is exemplified in Fig. 10(b)). In cases where the chemically modified alginic acid derivative was used in an anionic polymer layer (Examples A-3 and A-4), the beads were stable compared with A-2, but deformation was recognized after shaking (a photograph of Example A-3 after shaking is exemplified in Fig. 11(b)), and sufficient strengths were not exhibited. The same tendency was recognized even in cases where the photocrosslinked alginic acid derivative was used as the chemically modified alginic acid derivative (Examples A-9 to A-12). As described above, the structures of the present invention exhibit favorable stability even in large beads compared with conventional beads.

[Example B] Production of alginic acid three-layer structure (disc (sheet) having diameter of 20 mm (2 mm thick))

The structure of the present invention can be molded into an arbitrary shape using a mold. In the present test, it was verified that it is possible to produce a disc (flat plate)-like structure and the strength is favorable compared with those of conventional structures for which natural alginic acid is used.

<Step 2a> Production of alginic acid disc

An alginic acid disc is produced by the following method. First, a semipermeable membrane (manufactured by Repligen Corporation, catalog No. 132670) cut to 5 cm × 5 cm was immersed in a 100 mmol/L calcium chloride solution and adapted for 30 minutes or longer. After that, a silicon rubber formwork hollowed out in a disc shape having a diameter of 20 mm was placed on the semipermeable membrane from which moisture on the surface had been wiped with

KimWipes, 1.3 mL of an alginic acid solution was caused to flow thereinto, a semipermeable membrane from which moisture on the surface had been wiped was overlaid from above, and the semipermeable membranes and the silicon rubber form were fixed using clips. These were immersed in a 100 mmol/L calcium chloride solution all night, and then an alginic acid disc was removed from the formwork. The alginic acid solution used is the same as that in the step 1b.

<Step 2b> PLO coating

10 mL of a poly-L-ornithine solution prepared to 0.1 wt% was added to a 60 mm plastic petri dish including the alginic acid disc obtained in the step 2a and placed still at 37°C for 10 minutes. After that, the solution was once removed, 10 mL of a poly-L-ornithine solution prepared to 0.05 wt% was added thereto and placed still at 37°C for six minutes, and the alginic acid bead was washed twice using 20 mL of physiological saline.

<Step 2c> Alginic acid coating

10 mL of an alginic acid solution prepared to 0.15 wt% was added to a plastic petri dish including the PLO-coated disc obtained in the step 2b and placed still at 37°C for 10 minutes, and the bead was washed twice using 20 mL of physiological saline. The alginic acid solution used is the same as that in the step 1b except that, in Example B-9 to Example B-11, the solution of Example C-IB was used.

<Step 2d> Light irradiation

On the disc for which the alginic acid solution that was crosslink-reacted by light irradiation in the step 2a and the step 2c (the solution in Example C-IB), UV irradiation was performed for 10 minutes with an UV irradiation device (manufactured by Sen Lights Corp., HB 100A-1) after the end of the step 2c.

<Step 2e> Chelate and stability evaluation

40 mL of a 55 mM sodium citrate solution was added to a plastic petri dish including the disc obtained through each step. At a point in time where it was possible to confirm that the alginic acid disc derived from the AL100 solution produced in the step 2a was dissolved, the shapes of remaining discs were observed. The observation results are shown in Table 19 below. The stability of the alginic acid disc was visually evaluated based on the same indexes as those for the stability of the alginic acid bead.

[Table 19]

| Example | Steps | | | | Stability evaluation (scores) |
|---|---|---|---|---|---|
| | Step 2a | Step 2b | Step 2c | Step 2d | After chelate treatment |
| B-1 | AL100 | Performed | AL100 | - | 1 |
| B-2 | AL100 | Performed | Examples 18/19 | - | 1 |
| B-3 | AL100 | Performed | Examples 18/20 | - | 1 |
| B-4 | Examples 18/19 | Performed | AL100 | - | 3 |
| B-5 | Examples 18/20 | Performed | AL100 | - | 3 |
| B-6 | Examples 18/19 | Performed | Examples 18/19 | - | 3 |
| B-7 | Examples 18/20 | Performed | Examples 18/20 | - | 3 |
| B-8 | AL100 | Performed | AL100 | Performed | 1 |
| B-9 | AL100 | Performed | Example C-IB | Performed | 1 |
| B-10 | Example C-IB | Performed | AL100 | Performed | 3 |
| B-11 | Example C-IB | Performed | Example C-IB | Performed | 3 |

As a result, it was possible to produce stable three-layer structures like the case of the bead even in cases where the Huisgen-type chemically modified alginic acid derivative was used (Examples B-4 to B-7) and in cases where the photocrosslinked chemically modified alginic acid derivative was used (Examples B-10 and B-11) in the core layer.

In contrast, the shapes of the structures were not held at the time of the chelate treatment in all of cases where AL100 was used in the core layer and cases where the chemically crosslinked alginic acid was used only in the anionic polymer layer (Examples B-1 to B-3, Examples B8 and B9). As described above, the structure of the present invention exhibited significant stability even in the case of being molded into a disc shape.

[Example C] Production of alginic acid three-layer structure (disc (sheet) having diameter of 50 mm (2 mm thick))

In the present test, it was verified that, in a case where a disc-like structure is produced by the same method as in Example B, it is possible to produce a structure having a larger diameter.

<Step 3a> Production of alginic acid disc

An alginic acid disc was produced by the following method. First, a semipermeable membrane (manufactured by Repligen Corporation, catalog No. 132670) cut to 5 cm × 5 cm was immersed in a 100 mmol/L calcium chloride solution and adapted for 30 minutes or longer. After that, a silicon rubber formwork hollowed out in a disc shape having a diameter of 50 mm was placed on the semipermeable membrane from which moisture on the surface had been wiped with KimWipes, 5.65 mL of an alginic acid solution was caused to flow thereinto, a semipermeable membrane from which moisture on the surface had been wiped was overlaid from above, and the semipermeable membranes and the silicon rubber form were fixed using clips. These were immersed in a 100 mmol/L calcium chloride solution all night, and then an alginic acid disc was removed from the formwork. The alginic acid solution used is the same as that in the step 1b.

<Step 3b> PLO coating

20 mL of a poly-L-ornithine solution prepared to 0.1 wt% was added to a 100 mm plastic petri dish including the alginic acid disc obtained in the step 3a and placed still at 37°C for 10 minutes. After that, the solution was once removed, 20 mL of a poly-L-ornithine solution prepared to 0.05 wt% was added thereto and placed still at 37°C for six minutes, and the alginic acid bead was washed twice using 40 mL of physiological saline.

<Step 3c> Alginic acid coating

20 mL of an alginic acid solution prepared to 0.15 wt% was added to a plastic petri dish including the PLO-coated disc obtained in the step 3b and placed still at 37°C for 10 minutes, and the bead was washed twice using 40 mL of physiological saline. The alginic acids used are the solution of AL100, the solution of Examples 18/19 and the solution of Examples 18/20.

<Step 3d> Chelate and stability evaluation

200 mL of a 55 mM sodium citrate solution was added to a plastic petri dish including the disc obtained through each step. At a point in time where it was possible to confirm that the alginic acid disc derived from the AL100 solution produced in the step 3a was dissolved, the shapes of remaining discs were observed. The observation results are shown in Table 20 below. The stability of the alginic acid disc was visually evaluated based on the same indexes as those for the stability of the alginic acid bead.

[Table 20]

| Example | Steps | | | Stability evaluation (scores) |
|---|---|---|---|---|
| | Step 3a | Step 3b | Step 3c | After chelate treatment |
| C-1 | AL100 | Performed | AL100 | 1 |
| C-2 | Examples 18/19 | Performed | Examples 18/19 | 3 |
| C-3 | Examples 18/20 | Performed | Examples 18/20 | 3 |

As a result, in cases where the Huisgen-type chemically modified alginic acid derivative was used in the core layer (Examples C-2 and C-3), it was possible to produce stable three-layer structures; however, in a case where AL100 was used in the core layer (Example C-1), the shape of the structure was not held at the time of the chelate treatment. As described above, the structure of the present invention exhibited significant stability even in the case of being molded into a large disc shape having a diameter of 50 mm.

[Example D] Production of alginic acid three-layer structure (fiber)

In the present test, it was verified that it is possible to mold the structure of the present invention into a fiber shape and the strength is favorable compared with those of conventional structures for which natural alginic acid is used.

<Step 4a> Production of alginic acid fiber

Equal amounts of an alginic acid solution prepared to 3.0 wt% and 1.8 wt% saline containing 20 mg/mL of blue dextran (manufactured by Cytiva, Blue Dextran 2000, code No. 17036001) were mixed together, and a Hamilton syringe was filled with the mixture. A metal needle (manufactured by Musashi Engineering, Inc., SNA-19G-B), a silicon tube (manufactured by AS ONE Corporation, $\varphi 1 \times \varphi 2$) and a glass capillary (manufactured by NARISHIGE Group, G-1) were sequentially connected to the syringe and set in a syringe pump. The tip of the glass capillary was immersed in a 100 mmol/L calcium chloride solution (prepared with MilliQ water), and the mixture was sent at a flow rate of 250 μL/minute for one minute. A recovered alginic acid fiber was placed still in the same concentration of a calcium chloride aqueous solution (10 mL) for 30 minutes. The alginic acids used are the solution of AL 100 and the solution of Examples 18/20.

<Step 4b> PLO coating

The alginic acid fiber was separated from the calcium chloride solution containing the alginic acid fiber obtained in step 4a using a cell strainer. The separated alginic acid fiber was added to a poly-L-ornithine solution prepared to 0.1 wt% (prepared using a 100 mmol/L calcium chloride solution prepared using MilliQ water) (5 mL) and shaking-stirred at 37°C and 125 rpm for 20 minutes. After that, the alginic acid fiber was separated from the poly-L-ornithine solution using the cell strainer and washed using 5 mL of physiological saline twice.

<Step 4c> Alginic acid coating

The PLO-coated alginic acid fiber obtained in the step 4b was added to an alginic acid solution prepared to 0.15 wt% (5 mL) and shaking-stirred at 37°C and 125 rpm for 20 minutes. After that, the alginic acid fiber was separated from the alginic acid solution using the cell strainer and washed using 5 mL of physiological saline twice. The alginic acids used are the solution of AL 100 and the solution of Examples 18/20.

<Step 4d> Chelate treatment

The alginic acid fiber obtained in the step 4c was added to 20 mM EDTA-2K/physiological saline (5 mL) and shaking-stirred at 37°C and 125 rpm for 20 minutes. After that, the alginic acid fiber was separated from the EDTA-2K/physiological saline using the cell strainer and washed using 5 mL of physiological saline twice. A recovered alginic acid fiber was immersed in 5 mL of physiological saline until a stability evaluation test was performed.

<Step 4e> Stability evaluation

The alginic acid fiber obtained in the step 4d was moved a 15 mL centrifuge tube to which 5 mL of a PBS solution had been added and shaken at 37°C for one day. After that, the fiber was moved to a 25 mL centrifuge tube to which 10 mL of a PBS solution had been added and further shaken at 37°C for one hour. The observation results are shown in Table 21 below. The stability of the alginic acid fiber was visually evaluated based on the following indexes. Photograph of the alginic acid fibers according to the individual examples after the end of the step 4e are shown in Figs. 13 to 16.
Stability evaluation (score):

3: The collapse/dissolution/deformation/blue dextran elution or the like of the fiber are all not recognized.
2: Collapse/dissolution/deformation/blue dextran elution or the like are recognized in a part of the fiber.
1: Clear collapse/dissolution/deformation/blue dextran elution or the like are recognized in the fiber, and the function of the structure is not kept.

[Table 21]

| Example | Steps | | | Stability evaluation (scores) | |
|---|---|---|---|---|---|
| | Step 4a | Step 4b | Step 4c | After chelate treatment | After shaking |
| D-1 | AL100 | Performed | AL100 | 2 | 1 |
| D-2 | AL100 | Performed | Examples 18/20 | 3 | 2 |
| D-3 | Examples 18/20 | Performed | AL100 | 3 | 3 |
| D-4 | Examples 18/20 | Performed | Examples 18/20 | 3 | 3 |

In Example D-1 where AL100 was used in the core layer, as shown in Fig. 13, the collapse of the fiber began after the chelate treatment, and, after the shaking, blue dextran, which was a dyeing agent, was completely eluted in association with the clear collapse of the structure. In Example D-2 where the same core layer was used, the chemically crosslinked alginic acid of Examples 18/20 was used in the anionic polymer layer; however, as shown in Fig. 14, the collapse of the structure was not recognized. In contrast, in Example D-3 and Example D-4, as shown in Fig. 15 and Fig. 16, as a result of using the chemically crosslinked alginic acid of Examples 18/20 as the core layer, the collapse of the structure was not recognized regardless of the kind of the anionic polymer. As described above, the structure of the present invention exhibited favorable stability even in the case of being molded into a fiber shape.

[Example D(2)] Production of alginic acid three-layer structure (barium ion fiber)

The structure of the present invention can be manufactured in the same manner even when barium chloride is used as a divalent metal ion that is used to form the core layer instead of calcium chloride.

That is, in the <step 4a> of Example D, a barium chloride solution (concentration: 20 mmol/L, prepared with physiological saline) was used instead of the calcium chloride solution, in the <step 4b>, the poly-L-ornithine solution was prepared using a 20 mmol/L barium chloride-containing physiological saline solution, and the same test as the <step 4a> to the <step 4e> was performed. In the stability evaluation of the <step 4e>, shaking was performed for 41 hours.

The observation results are shown in Table 22. The stability of the alginic acid fiber was visually evaluated based on the following indexes. A photograph of the alginic acid fiber according to each example after the end of the step 4e is shown in Fig. 17.

Stability evaluation (score)

3: The collapse/dissolution/deformation/blue dextran elution or the like of the fiber are all not recognized.
2: Collapse/dissolution/deformation/blue dextran elution or the like are recognized in a part of the fiber.
1: Clear collapse/dissolution/deformation/blue dextran elution or the like are recognized in the fiber, and the function of the structure is not kept.

[Table 22]

| Example | Steps | | | Stability evaluation (scores) | |
|---|---|---|---|---|---|
| | Step 4a | Step 4b | Step 4c | After chelate treatment | After shaking |
| D(2)-1 | AL100 | Performed | AL100 | 3 | 2 |
| D(2)-2 | Examples 18/20 | Performed | AL100 | 3 | 3 |

As shown in Table 22, in a fiber of natural alginic acid (Example D(2)-1), it was confirmed that the destruction of the structure was not recognized after the chelate treatment and the stability was enhanced due to the use of a barium ion instead of a calcium ion. However, when the shaking in the PBS solution continued for a long period of time, in Example D(2)-1, the rupture of the fiber occurred. In contrast, in Example D(2)-2 where the chemically crosslinked alginic acid was used in the core layer, the collapse of the structure was not recognized. As described above, the same results as in Example D were obtained even in the present test, and it was possible to confirm that, even in the case of using a barium ion instead of a calcium ion as the divalent metal ion at the time of forming the core layer, the obtained structure

of the present invention had favorable stability.

From the above-described tests, it was possible to confirm that the structure of the present invention has a degree of freedom enabling the structure to be processed in a variety of shapes and sizes and thus has favorable stability compared with structures in which conventional natural alginic acid is used in the core layer.

[Example E] Production of alginic acid three-layer structure containing cell in core layer (Min6 cell-containing bead)

In the present test, it was verified that, in a case where a cell capable of releasing a bioactive substance is encapsulated in the core layer of the structure of the present invention, the cell survives in the structure and the functions are maintained.

<Step 5a> Production of alginic acid bead

An alginic acid bead containing a Min6 cell, which is a cell line of the β cell of the islands of Langerhans, was produced. First, a syringe connected to a 12-gauge needle was filled with the alginic acid solution of Examples 18/20 in which Min6 cells (manufactured by AddexBio Technologies) were suspended so that the number of the cells reached $9.17 \times 10^6$ cells/mL. 40 mL of a 100 mmol/L calcium chloride solution was set such that the distance from the tip of the needle to the liquid surface became 10 mm and stirred using the magnetic stirrer at 200 rpm. The alginic acid solutions were added dropwise thereto for 15 seconds at a flow rate of 1 mL/min using a syringe pump and stirred for 90 seconds.

<Step 5b> PLO coating

5 mL of a poly-L-ornithine solution prepared to 0.1 wt% was added to a mini cup including the alginic acid bead obtained in the step 5a and placed still at 37°C for 10 minutes. After that, the solution was once removed, 5 mL of a poly-L-ornithine solution prepared to 0.05 wt% was added thereto and placed still at 37°C for six minutes, and the alginic acid bead was washed once using 20 mL of physiological saline.

<Step 5c> Alginic acid coating

5 mL of an alginic acid solution of AL 100 prepared to 0.15 wt% was added to the mini cup including the PLO-coated bead obtained in the step 5b and placed still at 37°C for 10 minutes, and the bead was washed once using 20 mL of physiological saline.

<Step 5d> Chelate treatment

40 mL of a 55 mM sodium citrate solution was added to the mini cup including the bead obtained in the step 5c, placed still at room temperature for two minutes and washed twice using 30 mL of physiological saline.

<Step 5e> Culture

AMin6 cell-containing bead obtained in the step 5d was cultured in a 6 well plate containing 5 mL of a culture medium having the composition shown in Table 23 below. In addition, as a control, untreated Min6 cells (the number of the cells was the same as that in the Min6 cell-containing bead) were also cultured in a 6 well plate containing 5 mL of the same culture medium.

[Table 23]

| Culture medium composition: Prepared by adding a variety of additives in the following table to an optimized DMEM culture medium | | | | |
|---|---|---|---|---|
| | Sample | Maker | Amount added (mL) | Final concentration |
| Culture medium | Optimized DMEM | AddexBi o | 420 | |
| Additives | Fetal bovine serum (FB S) | NICHIR EI | 75 | 15% |
| | Penicillin Streptmycin | Gibco | 5 | 1% |
| | 2-mercaptoethanol | Gibco | 0.455 | 0.05 mM |

<Step 5f> Insulin secretory ability evaluation

The insulin secretory abilities of the Min6 cells and the untreated Min6 cells that were contained in the core layers of the alginic acid beads cultured for nine days in the step 5e were evaluated. The Min6 cells and the untreated Min6 cells were cultured for two hours in 10 mL of a low glucose solution (2 mM glucose/KRBH/0.1% BSA), the low glucose solution was exchanged with 10 mL of a high glucose solution (20 mM glucose/KRBH/0.1% BSA), and then the cells were further cultured for two hours. After that, the solution was exchanged with 10 mL of a low glucose solution again, and the cells were cultured for two hours. The insulin concentrations in the solution were measured using an ultra sensitive mouse insulin ELISA kit (manufactured by Morinaga Institute of Biological Science, Inc.) upon the end of each step. The results are shown in Table 24.

[Table 24]

| | Low glucose solution (1) | High glucose solution | Low glucose solution (2) |
|---|---|---|---|
| Insulin concentration (ng/mL) | 3.3 | 27 | 3.7 |

<Step 5g> ATP quantification

ATP's of the Min6 cells and the untreated Min6 cells that were contained in the core layers of the alginic acid beads cultured for 10 days in the step 5e were quantitatively evaluated. The quantitative evaluation was performed using CellTiter-Glo (registered trademark) 3D Cell Viability Assay (manufactured by Promega Corporation). The results are shown in Table 25. As shown in Table 25, the ATP amount of the Min6 cells that were contained in the core layer of the alginic acid bead was substantially the same as that of the untreated Min6 cells.

[Table 25]

| | Min6 cells in bead | Untreated Min6 cells |
|---|---|---|
| Relative light unit (RLU) | 339967 | 297092 |

As a result of encapsulating Min6 cells in the structure produced by combining Example 18 and Example 20 and measuring the insulin secretory ability after nine days of culture, as shown in Table 24, it was confirmed that the cells secreted insulin in a glucose concentration-dependent manner. In addition, as a result of measuring the ATP production amount of the Min6 after 10 days of culture, as shown in Table 25, it was confirmed that the amount was substantially the same as that of untreated Min6 that was not encapsulated in the structure.

From the above-described results, it was confirmed that, in a case where living cells are encapsulated, the structure of the present invention is an environment suitable for survival and it is possible to release a bioactive substance from the structure.

## Claims

**1.** A structure comprising:

a core layer comprising a pharmacological ingredient embedded in a chemically crosslinked alginic acid;
a cationic polymer layer coating the core layer; and
an anionic polymer layer coating the cationic polymer layer.

**2.** The structure according to claim 1,
wherein the chemically crosslinked alginic acid is obtained by forming a crosslink between a chemically modified alginic acid derivative represented by Formula (H-I) and a chemically modified alginic acid derivative represented by Formula (H-II) using the two alginic acid derivatives:

[chemically modified alginic acid derivative represented by Formula (H-I)]
chemically modified alginic acid represented by Formula (H-I):

[C178]

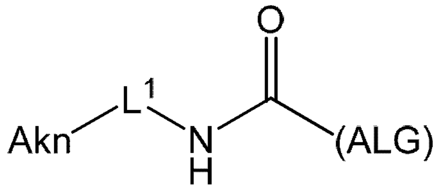

(H-I)

[in Formula (H-I), (ALG) represents alginic acid; -NHCO- represents an amide bond through an arbitrary carboxyl group of the alginic acid; $-L^1-$ is a divalent linker that bonds to a cyclic alkyne group (Akn)]; and [chemically modified alginic acid derivative represented by Formula (H-II)]
chemically modified alginic acid represented by Formula (H-II):

[C179]

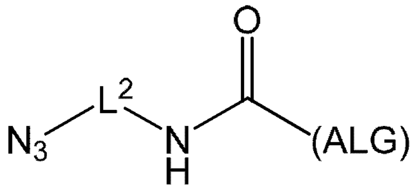

(H-II)

[in Formula (H-II), (ALG) represents alginic acid; -NHCO- represents an amide bond through an arbitrary carboxyl group of the alginic acid; $-L^2-$ is a divalent linker that bonds to an azide group].

**3.** The structure according to claim 2,

wherein, in Formula (H-I), $-L^1-$ is $-(CH_2)_{n1}-$ (here, n1 is 1 to 50, $-CH_2-$ in the group is optionally substituted with one to 10 groups selected from -CO-, -CONH-, -NHCO-, -O-CONH-, -NHCO-O-, -O- and -NH- or a benzene ring, and a hydrogen atom in the $-CH_2-$ is optionally substituted with a $C_{1-3}$ alkyl group or a $C_{1-3}$ alkyl group substituted with a phenyl group),
Akn is a compound that is an eight-membered cyclic alkyne group (here, the cyclic alkyne group is an eight-membered cyclic group in which, furthermore, one or two benzene rings, cyclopropane rings or 1,2,3-triazole rings are optionally condensed and optionally bond to $-L^1-$ in a condensed ring, and $-CH_2-$ in the eight-membered cyclic alkyne group is optionally substituted with one or two groups selected from -C(=O)-, -CONH- and -NH-, and a hydrogen atom in $-CH_2-$ in the alkyne group is optionally substituted with one or two groups selected from a $C_{1-3}$ alkyl group, a fluorine atom, a hydroxyl group or a $C_{1-3}$ alkyloxy group), and
in Formula (H-II), $-L^2-$ is $-(CH_2)_{n2}-$ (here, n2 is 1 to 50, $-CH_2-$ in the group is optionally substituted with one to 10 groups selected from -CONH-, -NHCO-, -O- and -NH-, a benzene ring or a pyridine ring, and a hydrogen atom in the $-CH_2-$ is optionally substituted with a $C_{1-3}$ alkyl group).

**4.** The structure according to claim 3,

wherein, in Formula (H-I), $-L^1-$ is $-(CH_2)_{n1}-$ (here, n1 is 2 to 15, $-CH_2-$ in the group is optionally substituted with one to five groups selected from -CO-, -CONH-, -NHCO-, -O-CONH-, -NHCO-O-, -O- and -NH- or a benzene ring, and a hydrogen atom in the $-CH_2-$ is optionally substituted with a $C_{1-3}$ alkyl group or a $C_{1-3}$ alkyl group substituted with a phenyl group),
Akn is a compound that is an eight-membered cyclic alkyne group (here, the cyclic alkyne group is an eight-membered cyclic group in which, furthermore, one or two benzene rings are optionally condensed or $-CH_2-$ in the eight-membered cyclic alkyne group is optionally substituted with -NH-), and
in Formula (H-11), $-L^2-$ is $-(CH_2)_{n2}-$ (here, n2 is 2 to 15, $-CH_2-$ in the group is optionally substituted with one to five groups selected from -CONH-, -NHCO-, -O- and -NH- or a benzene ring).

**5.** The structure according to claim 4,

wherein the chemically modified alginic acid derivative represented by Formula (H-I) is Formula (A01) or (A02) below, and the chemically modified alginic acid derivative represented by Formula (H-II) is Formula (N01), (N02), (N03) or (N04) below:

[C180]

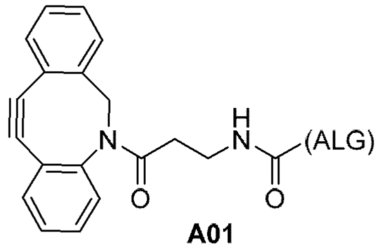

A01

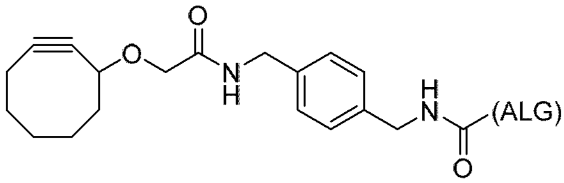

A02

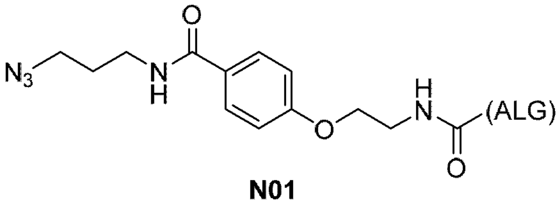

N01

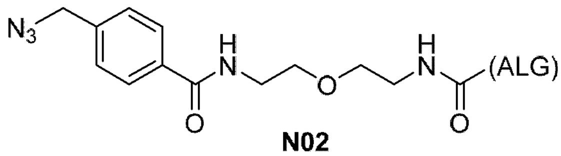

N02

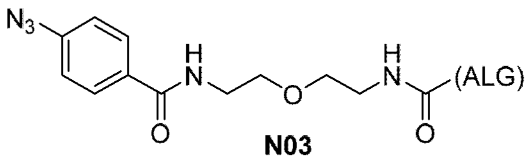

N03

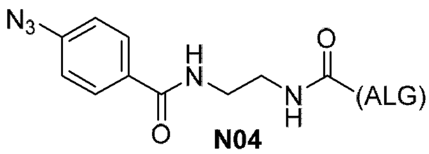

N04

.

6. The structure according to any one of claims 1 to 5,
wherein an anionic polymer is alginic acid or chemically crosslinked alginic acid.

7. The structure according to any one of claims 1 to 6,
wherein a pharmacological ingredient embedded in a chemically crosslinked alginic acid in the core layer is a cell.

8. The structure according to any one of claims 1 to 6,
wherein a pharmacological ingredient embedded in a chemically crosslinked alginic acid in the core layer is a bioactive substance-producing cell.

9. The structure according to any one of claims 1 to 8,
wherein a cationic polymer is poly-L-ornithine or poly-L-lysine.

10. The structure according to any one of claims 1 to 9,
wherein a shape of the structure is a bead, a sheet or a fiber.

11. The structure according to any one of claims 1 to 10, the structure being intended to be used in a living body.

12. A medical material comprising, as an effective ingredient,
a pharmacological ingredient encapsulated in the structure according to any one of claims 1 to 10.

13. The medical material according to claim 12, the medical material being used in a wound dressing, a postoperative adhesion barrier, a sustained drug release substrate, a cell transplantation substrate, a prosthetic material, a formulation coating material or a bioprinter.

**14.** A method for manufacturing the structure according to any one of claims 1 to 11, the method comprising steps (a) to (c) below:

step (a): a step of gelation of a solution of a chemically modified alginic acid derivative comprising a pharmacological ingredient [a mixture of the derivative represented by Formula (H-I) and the derivative represented by Formula (H-II)] by bringing the solution into contact with a solution comprising a divalent metal ion;
step (b): a step of bringing gel obtained in the step (a) into contact with a solution comprising a cationic polymer to coat the gel with the cationic polymer; and
step (c): a step of bringing a manufactured product obtained in the step (b) into contact with a solution comprising an anionic polymer to further coat the manufactured product with the anionic polymer.

**15.** The method for manufacturing the structure according to claim 14, the method further comprising a step (d) below:
step (d): a step of performing a chelate treatment on a structure obtained in the step (c) with a chelating agent.

Fig. 1

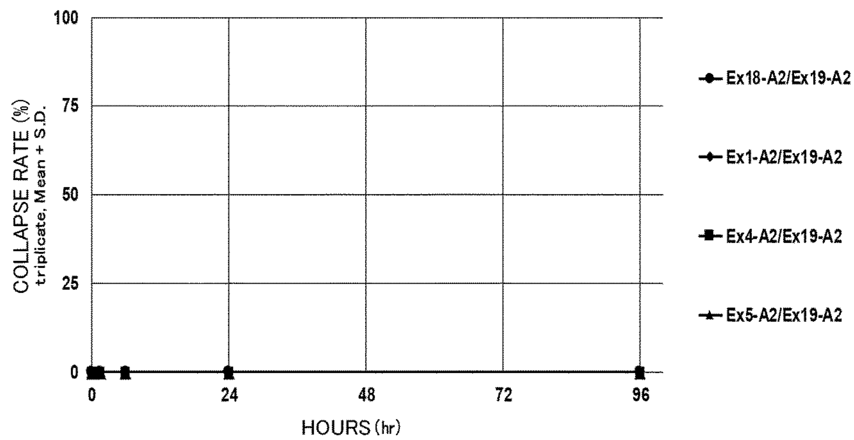
100
75
50
25
0
0
24
48
72
96
COLLAPSE RATE (%)
triplicate, Mean + S.D.
HOURS (hr)
Ex18-A2/Ex19-A2
Ex1-A2/Ex19-A2
Ex4-A2/Ex19-A2
Ex5-A2/Ex19-A2

Fig. 2

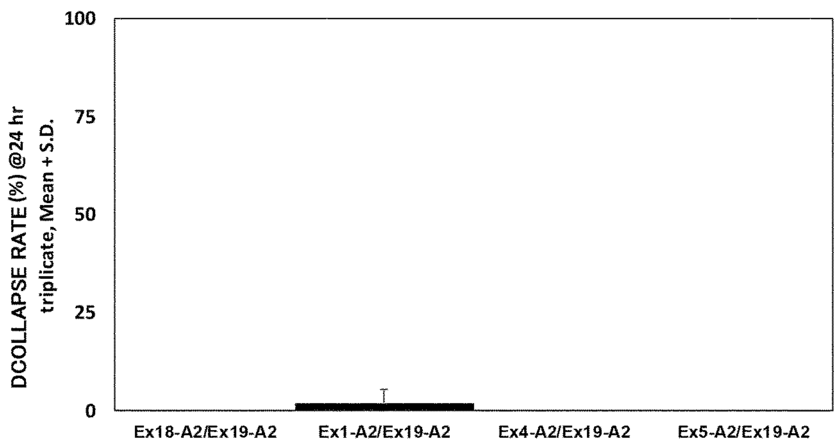
100
75
50
25
0
DCOLLAPSE RATE (%) @24 hr
triplicate, Mean + S.D.
Ex18-A2/Ex19-A2
Ex1-A2/Ex19-A2
Ex4-A2/Ex19-A2
Ex5-A2/Ex19-A2

Fig. 3

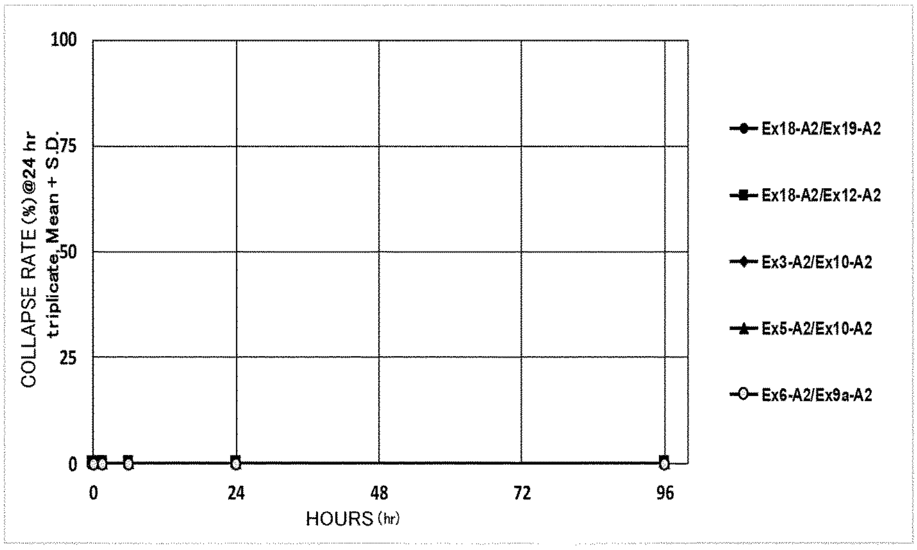
100
75
50
25
0
0
24
48
72
96
HOURS (hr)
COLLAPSE RATE (%) @24 hr triplicate, Mean + S.D.
Ex18-A2/Ex19-A2
Ex18-A2/Ex12-A2
Ex3-A2/Ex10-A2
Ex5-A2/Ex10-A2
Ex6-A2/Ex9a-A2

Fig. 4

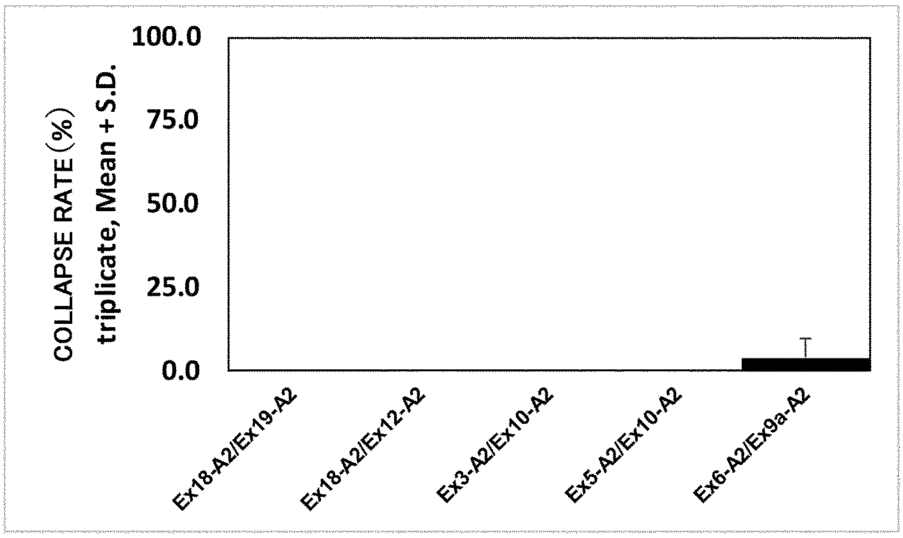
100.0
75.0
50.0
25.0
0.0
COLLAPSE RATE (%) triplicate, Mean + S.D.
Ex18-A2/Ex19-A2
Ex18-A2/Ex12-A2
Ex3-A2/Ex10-A2
Ex5-A2/Ex10-A2
Ex6-A2/Ex9a-A2

Fig. 5

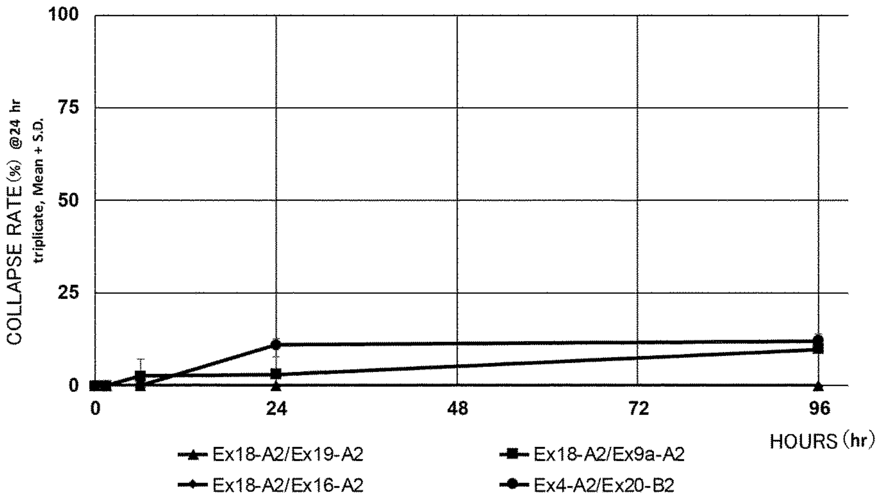
100
75
50
25
0
COLLAPSE RATE (%) @24 hr
triplicate, Mean + S.D.
0
24
48
72
96
HOURS (hr)
Ex18-A2/Ex19-A2
Ex18-A2/Ex9a-A2
Ex18-A2/Ex16-A2
Ex4-A2/Ex20-B2

Fig. 6

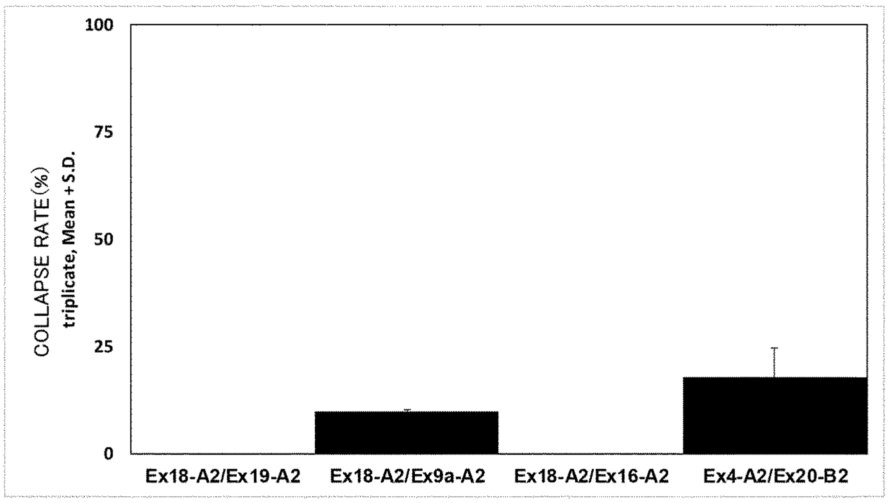
100
75
50
25
0
COLLAPSE RATE (%)
triplicate, Mean + S.D.
Ex18-A2/Ex19-A2
Ex18-A2/Ex9a-A2
Ex18-A2/Ex16-A2
Ex4-A2/Ex20-B2

Fig. 7

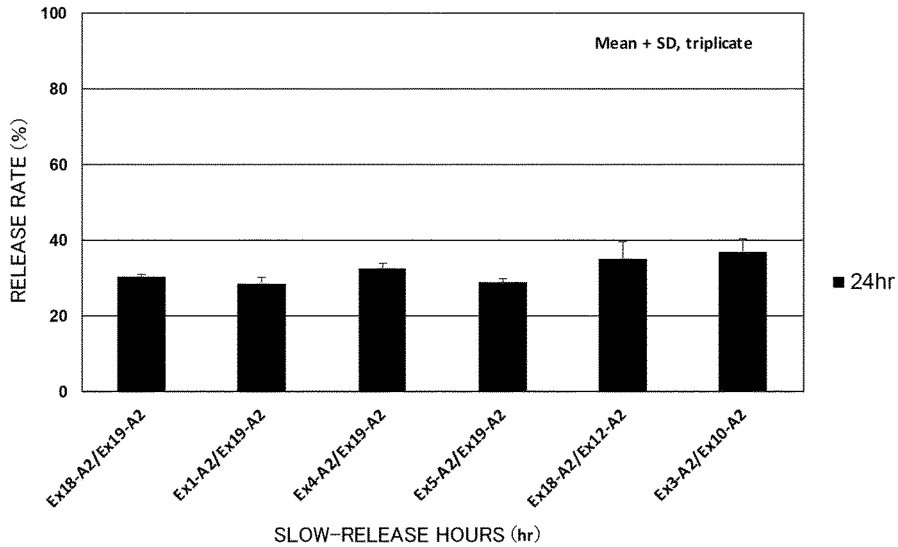
100
80
60
40
20
0
RELEASE RATE (%)
Mean + SD, triplicate
■ 24hr
Ex18-A2/Ex19-A2
Ex1-A2/Ex19-A2
Ex4-A2/Ex19-A2
Ex5-A2/Ex19-A2
Ex18-A2/Ex12-A2
Ex3-A2/Ex10-A2
SLOW-RELEASE HOURS (hr)

Fig. 8

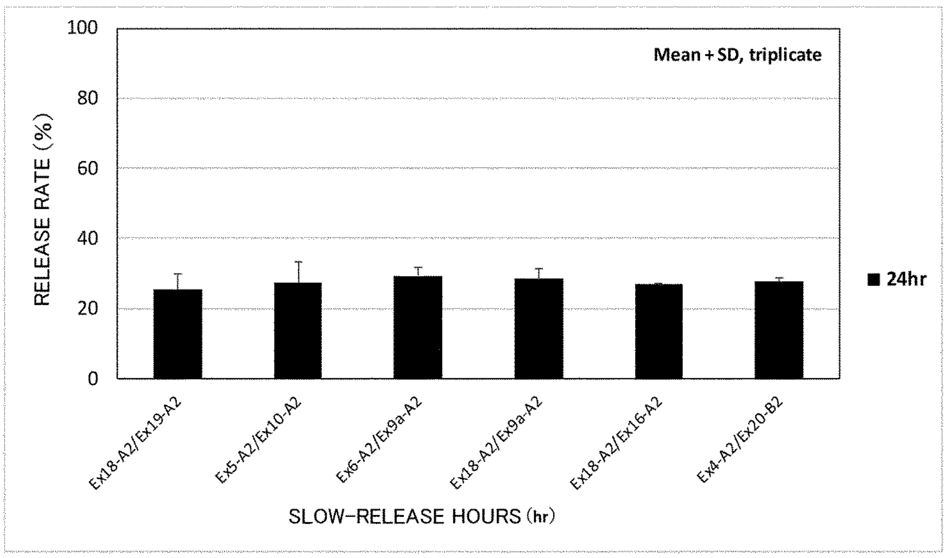
100
80
60
40
20
0
RELEASE RATE (%)
Mean + SD, triplicate
■ 24hr
Ex18-A2/Ex19-A2
Ex5-A2/Ex10-A2
Ex6-A2/Ex9a-A2
Ex18-A2/Ex9a-A2
Ex18-A2/Ex16-A2
Ex4-A2/Ex20-B2
SLOW-RELEASE HOURS (hr)

Fig. 9

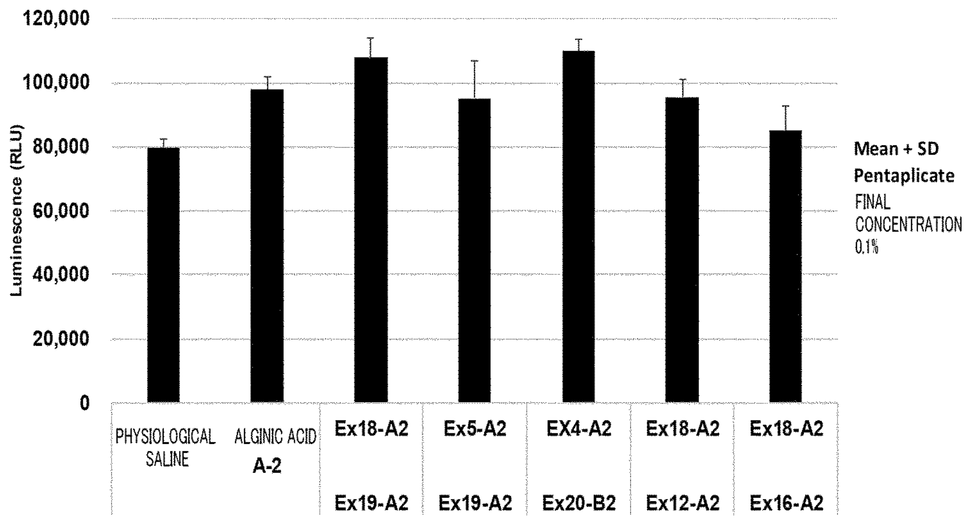
120,000
100,000
80,000
60,000
40,000
20,000
0
Luminescence (RLU)
PHYSIOLOGICAL SALINE
ALGINIC ACID A-2
Ex18-A2 Ex19-A2
Ex5-A2 Ex19-A2
EX4-A2 Ex20-B2
Ex18-A2 Ex12-A2
Ex18-A2 Ex16-A2
Mean + SD
Pentaplicate
FINAL CONCENTRATION 0.1%

Fig. 10

(a)

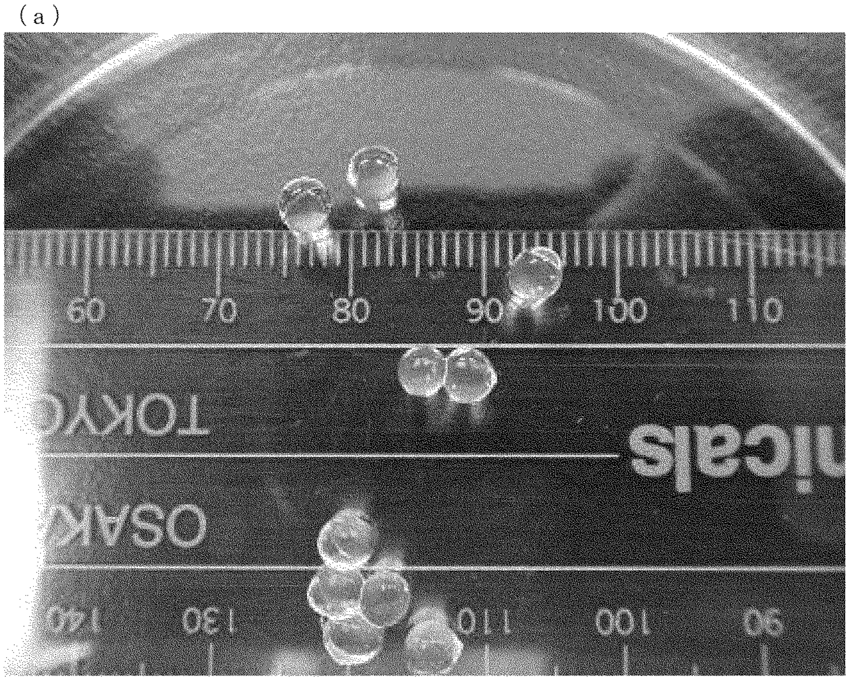


(b)

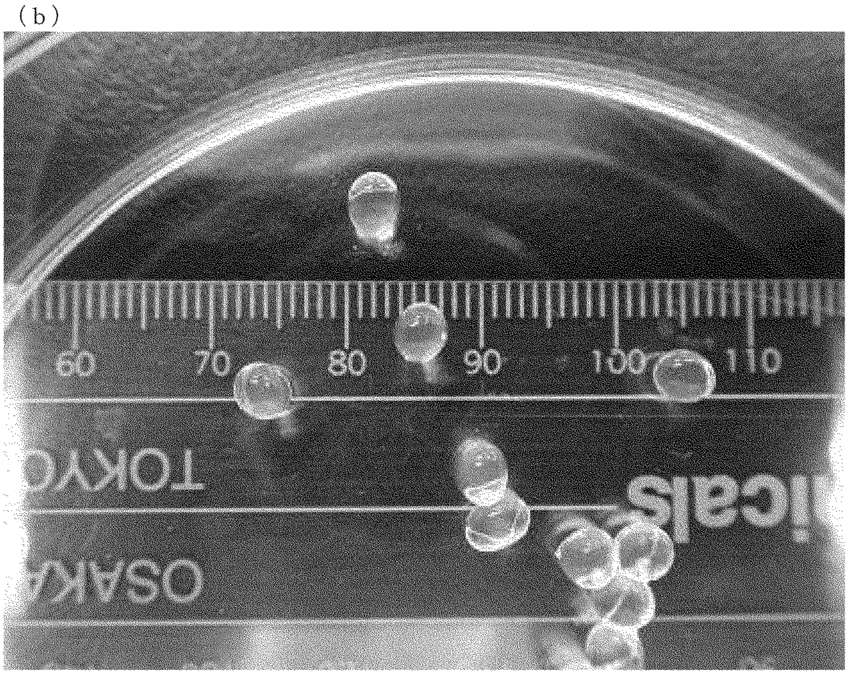

Fig. 11

(a)

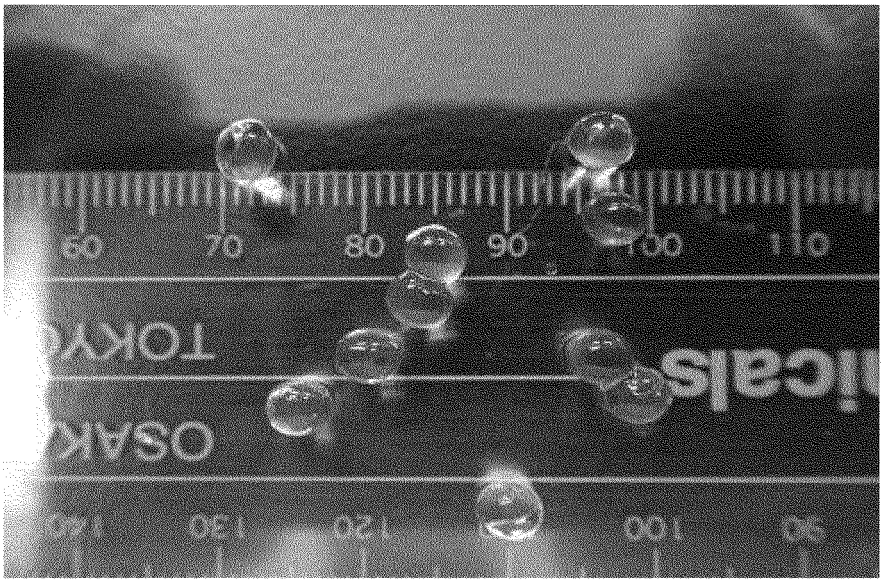


(b)

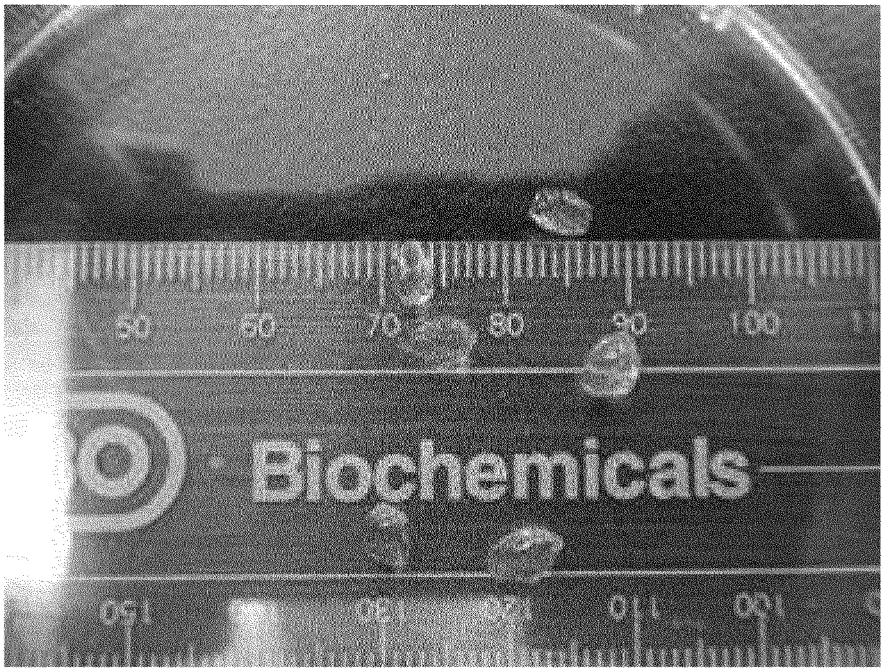

Fig. 12

(a)

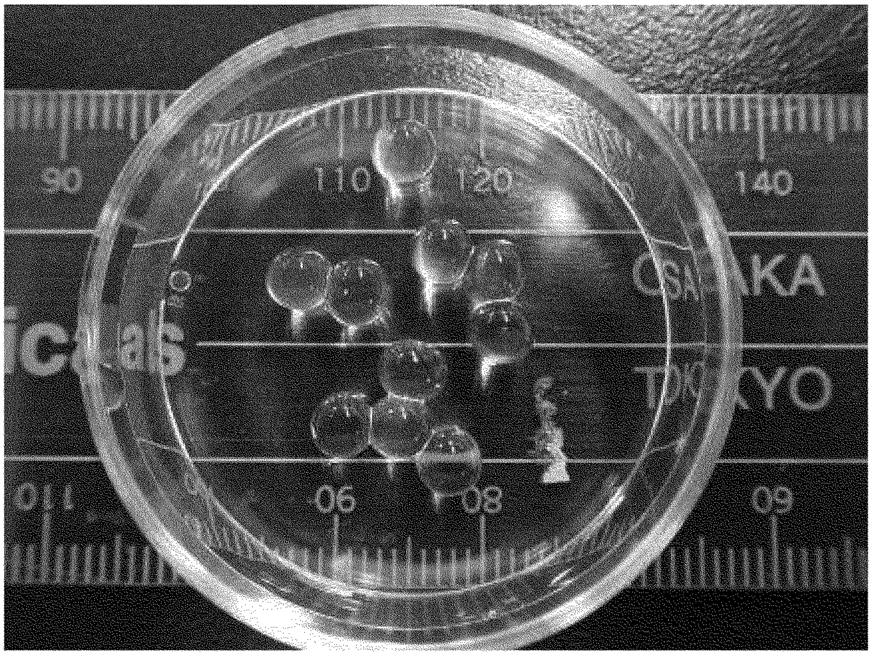


(b)

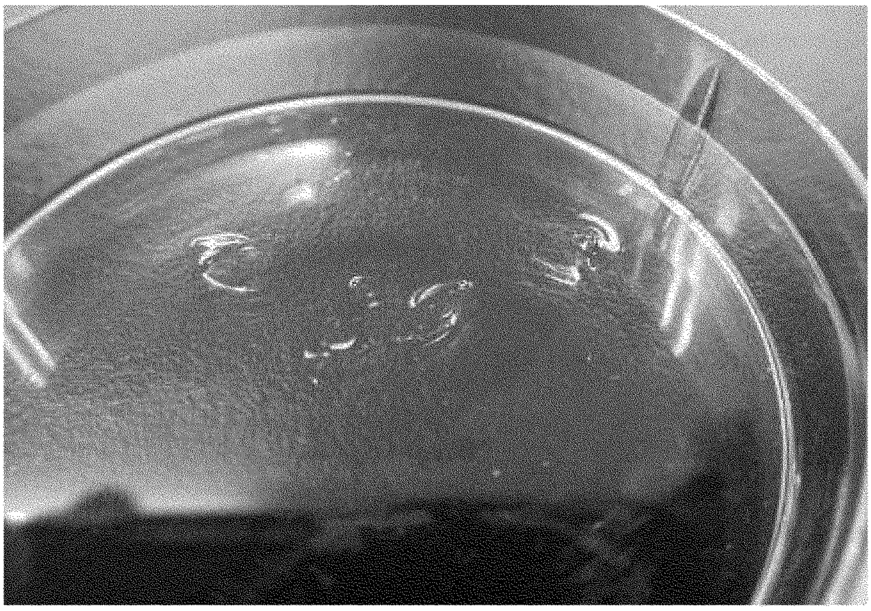

Fig. 13

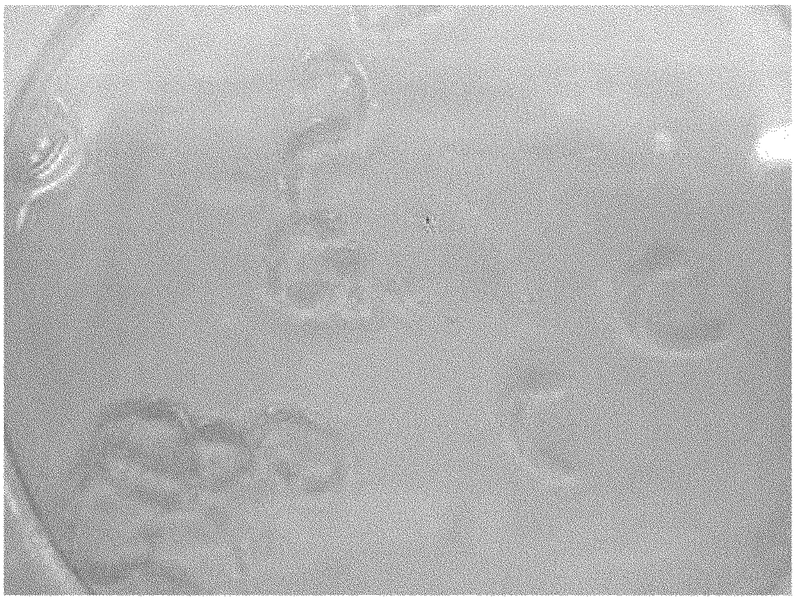

Fig. 14

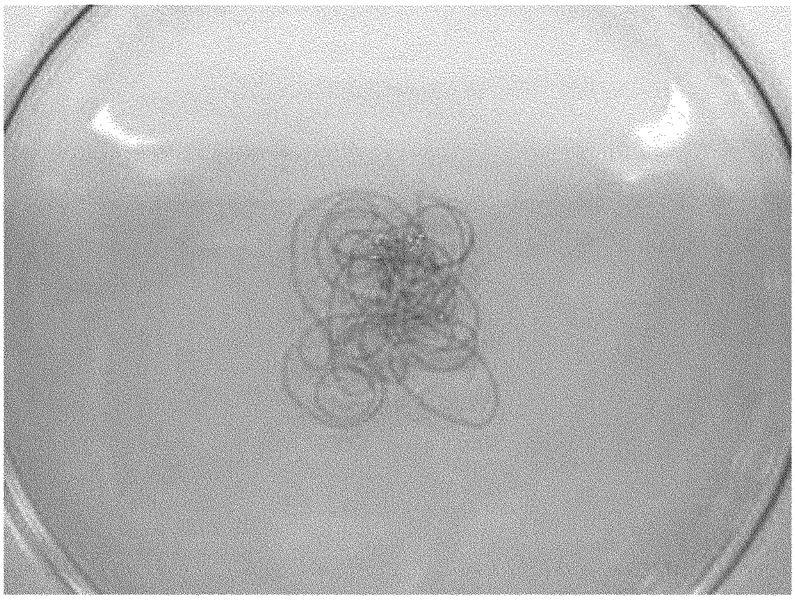

Fig. 15

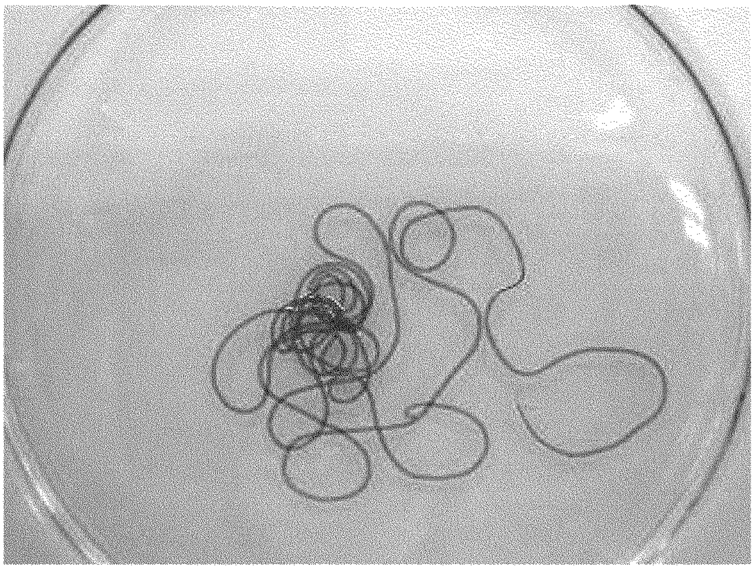

Fig. 16

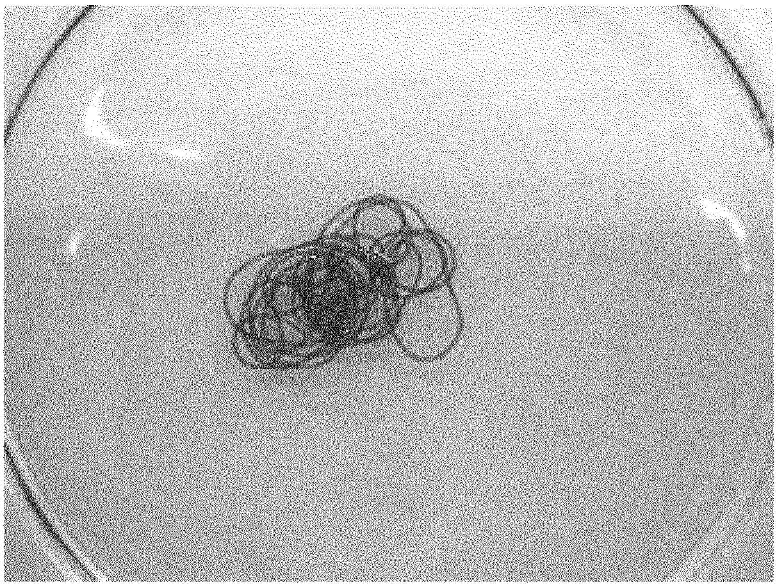

Fig. 17

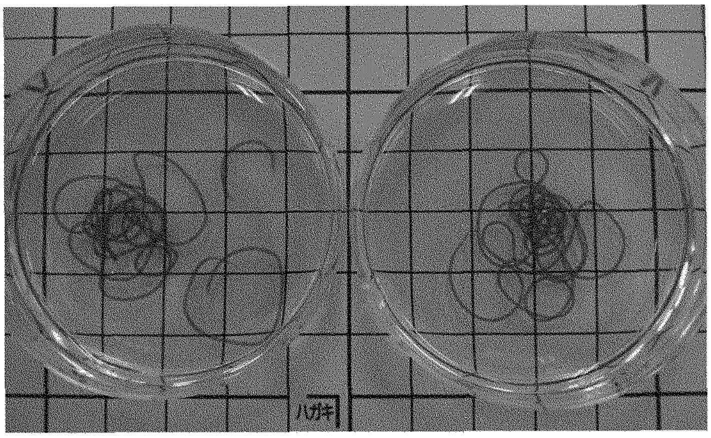

LEFT: EXAMPLE D(2)-1 RIGHT: EXAMPLE D(2)-3

## INTERNATIONAL SEARCH REPORT

International application No. **PCT/JP2021/048566**

**A. CLASSIFICATION OF SUBJECT MATTER**

***A61L 27/44***(2006.01)i; ***A61K 35/12***(2015.01)i; ***A61L 27/20***(2006.01)i; ***A61L 27/34***(2006.01)i; ***A61L 27/38***(2006.01)i; ***A61L 27/50***(2006.01)i; ***A61L 27/58***(2006.01)i
FI: A61L27/44; A61K35/12; A61L27/20; A61L27/34; A61L27/38; A61L27/50; A61L27/58

According to International Patent Classification (IPC) or to both national classification and IPC

**B. FIELDS SEARCHED**

Minimum documentation searched (classification system followed by classification symbols)

A61L27/44; A61K35/12; A61L27/20; A61L27/34; A61L27/38; A61L27/50; A61L27/58

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/240219 A1 (MOCHIDA PHARM CO LTD) 19 December 2019 (2019-12-19) claims 1-9, 11-15, paragraphs [0018], [0257], [0268], [0309], [0361], [0375], [0424], [0466], [0513]-[0516], [0530]-[0531], [0542]-[0555] | 1-15 |
| Y | ULUDAG, H. et al. Technology of mammalian cell encapsulation. Advanced Drug Delivery Reviews. 2000, vol. 42, pp. 29-64 title, abstract, p. 33, right column, line 3 to p. 34, left column, line 20 | 1-15 |
| Y | JP 2019-520837 A (ETH ZURICH) 25 July 2019 (2019-07-25) paragraphs [0102], [0136] | 1-15 |
| Y | JP 2014-506926 A (WAKE FOREST UNIVERSITY HEALTH SCIENCES) 20 March 2014 (2014-03-20) claims, paragraphs [0015]-[0018] | 1-15 |
| Y | JP 2016-508369 A (SHANGHAI INSTITUTES FOR BIOLOGICAL SCIENCES, CHINESE ACADEMY OF SCIENCES) 22 March 2016 (2016-03-22) paragraphs [0078], [0093] | 1-15 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 January 2022** | **15 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/048566**

| Patent document cited in search report | Publication date (day/month/year) | Patent family member(s) | Publication date (day/month/year) |
|---|---|---|---|
| WO 2019/240219 A1 | 19 December 2019 | US 2021/0095053 A1<br>claims, paragraphs [0029], [0410], [0423], [0463], [0515], [0529], [0577], [0619], [0669]-[0673], [0693]-[0696], [0708]-[0725]<br>EP 3808783 A1<br>CN 112236457 A<br>KR 10-2021-0019441 A | |
| JP 2019-520837 A | 25 July 2019 | US 2019/0282710 A1<br>paragraphs [0116], [0162]<br>WO 2018/015330 A1<br>EP 3272877 A1<br>KR 10-2019-0026786 A<br>CN 109563487 A | |
| JP 2014-506926 A | 20 March 2014 | US 2014/0127308 A1<br>claims, paragraphs [0023]-[0026]<br>WO 2012/121874 A1<br>EP 2680862 A1 | |
| JP 2016-508369 A | 22 March 2016 | US 2015/0190427 A1<br>paragraphs [0183], [0224]<br>WO 2014/121758 A1<br>CN 103981147 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2001052871 A
- WO 2014171842 A
- WO 2018151186 A
- WO 98049202 A
- CN 105078923 A
- WO 2010139061 A
- WO 2019240219 A
- WO 2019168058 A
- WO 2021125255 A
- JP H09324001 B, Yoshida
- JP H08269102 B, William
- JP 2002530440 W, James
- WO 9313136 A, Lewis
- US 5589591 A, Herman Frank
- JP 2005036036 A
- JP 2019023478 W
- JP 2019007655 W
- WO 2004035017 A
- WO 2015140807 A


### Non-patent literature cited in the description


- *Yakugaku Zasshi,* 2005, vol. 125 (8), 601-615
- *Biomaterials,* 2005, vol. 26 (34), 6846-52
- *American Diabetes Association 60th Scientific Sessions,* 2000
- **CHIKAKO YOMOTA.** *Bull. Natl. Health Sci.,* 1999, vol. 117, 135-139
- **CHIKAKO YOMOTA.** *Bull. Natl. Inst. Health Sci.,* 2003, vol. 121, 30-33
- *Appl Microbiol Biotechnol,* 1994, vol. 40, 638-643
- Synthesis IV of Organic Compounds, Carboxylic Acids and Derivatives, Esters. Experimental Chemistry. 35-70
- *Acid Amides and Acid Imides,* 118-154
- **MARUZEN.** *Amino Acids/Peptides,* 2007, 258-283
- **GREENE.** Protective Groups in Organic Synthesis. John Wiley & Sons, 2007
- *Journal of the America Chemical Society,* 2004, vol. 126 (46), 15056-15047
- *Organometallics,* 2010, vol. 29 (23), 6619-6622
- *Bioorganic & Medicinal Chemistry,* 2015, vol. 23 (22), 7150-7157
- Experimental Chemistry. Maruzen, 2007
- Comprehensive Organic Transformations. A Guide to Functional Group Preparations, 2018
- Strategic Applications of Named Reactions in Organic Synthesis. Academic Press, 2005
- **STAHL ; WERMUTH.** Handbook of Pharmaceutical Salts: Properties, Selection, and Use. Wiley-VCH, 2002
- *CHEMICAL ABSTRACTS,* 88192-19-2
- *CHEMICAL ABSTRACTS,* 4530-20-5
- *CHEMICAL ABSTRACTS,* 3303-84-2
- *Organic Letters,* 2017, vol. 19 (23), 6400-6403
- *CHEMICAL ABSTRACTS,* 18523-48-3
- *CHEMICAL ABSTRACTS,* 108468-00-4
- *CHEMICAL ABSTRACTS,* 24541-43-3
- *CHEMICAL ABSTRACTS,* 39684-80-5
- *CHEMICAL ABSTRACTS,* 57260-73-8
- *Org. Process Res. Dev.,* 2018, vol. 22, 108-110
- *CHEMICAL ABSTRACTS,* 127828-22-2
- *Org. Process Res. Dev,* 2018, vol. 22, 108-110
- *CHEMICAL ABSTRACTS,* 929-06-6
- *CHEMICAL ABSTRACTS,* 107-95-9
- *CHEMICAL ABSTRACTS,* 2576-47-8
- *CHEMICAL ABSTRACTS,* 149505-94-2
- *CHEMICAL ABSTRACTS,* 1255942-06-3
- *CHEMICAL ABSTRACTS,* 29022-11-5
- *CHEMICAL ABSTRACTS,* 63-91-2
- *Bioorganic & Medicinal Chemistry,* 2003, vol. 11, 4189-4206